# EUROPEAN PATENT APPLICATION

(11) **EP 0 661 266 A1**
(43) Date of publication of application: **05.07.1995**
(21) Application number: 94120698.9
(22) Date of filing: 27.12.1994
(51) Int. Cl.: C07D 211/32, A61K 31/395, C07D 401/06, C07D 405/12, C07D 417/06, C07D 401/12, C07D 295/14, C07D 295/12, C07D 417/12

(54) **Substituted cyclic amine compounds as 5HT2 antagonists**

(30) Priority: 27.12.1993 JP 346805/93
(71) Applicant: TOA EIYO LTD., Chuo-ku Tokyo 104 (JP)
(72) Inventor: Aoki, Tsuyoshi, Fukushima-shi, Fukushima (JP); Takahashi, Atsuo, Fukushima-shi, Fukushima (JP); Sato, Hiroyasu, Fukushima-shi, Fukushima (JP); Shimanuki, Eiji, Fukushima-shi, Fukushima (JP); Gengyou, Kaoru, Fukushima-shi, Fukushima (JP); Nishimata, Toyoki, Fukushima-shi, Fukushima (JP); Ishigami, Sachiko, Fukushima-shi, Fukushima (JP); Yamada, Shin-ichi, Fukushima-shi, Fukushima (JP); Yamaguchi, Takahiro, Fukushima-shi, Fukushima (JP); Manome, Yoichi, Fukushima-shi, Fukushima (JP); Sato, Isamu, Fukushima-shi, Fukushima (JP); Kogi, Kentaro, Shiraishi-shi, Miyagi (JP); Narita, Sen-ichi, Omiya-shi, Saitama (JP)
(74) Representative: Kraus, Walter, Dr.

(57) **Abstract**

A substituted cyclic amine compound represented by the following general formula (1) 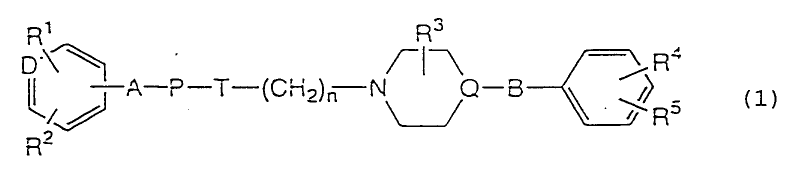
wherein each of R¹ to R⁵ represents a hydrogen atom, a hydroxyl group, an alkyl group or the like, D represents a methine moiety, a nitrogen atom or the like, P represents -CR⁶(R⁷)- or -NR⁸-, Q represents a methine moiety or a nitrogen atom, each of T and B is a single bond or represents a methylene moiety, a carbonyl group or the like, n is an integer of 1 to 6 and each of R⁶, R⁷ and R⁸ represents a hydrogen atom, an alkyl group or the like; and synthetic intermediates thereof.

The inventive compound is useful in preventing and treating circulatory organ-related diseases such as hypertension, ischemic heart disease, cerebrovascular disease, peripherol circulatory disease and the like.

## Description

### FIELD OF THE INVENTION

This invention relates to a novel substituted cyclic amine compound and a pharmaceutical composition which contains the novel compound.

The substituted cyclic amine compound and salts thereof of the present invention show a strong serotonin 2 receptor antagonism with no central nervous action and are useful as pharmaceutical drugs for the prevention and treatment of circulatory organ-related diseases such as hypertension, ischemic heart disease and other diseases caused by the cerebrovascular and peripheral circulatory disturbances.

### BACKGROUND OF THE INVENTION

In recent years, attention has been paid to the participation of serotonin in the generation mechanism of unstable angina and myocardial infarction caused by coronary arteriosclerosis and hypertension

That is, platelet aggregation is apt to occur in blood vessels where arteriosclerosis or endothelial disorder is generated, and platelets in the aggregation-caused vessel region release a high concentration of serotonin which subsequently increases the platelet aggregation to form thrombi and induces strong angiospasm mediated by the serotonin 2 receptor. Since peripheral-selective serotonin 2 receptor antagonists seem to be effective in inhibiting such phenomena, studies have been made on such antagonists. For example, a quinazoline derivative, ketanserin, has been disclosed in U.S. Patent 4,335,127 and JP-A-55-105679 (the term "JP-A" as used herein means an "unexamined published Japanese patent application). However, since most of the prior art serotonin 2 receptor antagonists show not only peripheral serotonin 2 antagonism but also central nervous actions, these compounds are problematic as pharmaceutical drugs for circulatory organ use.

### SUMMARY OF THE INVENTION

As a result of extensive investigation to develop a novel serotonin 2 receptor antagonist which shows strong serotonin 2 receptor antagonism and from which the central nervous action is separated, the present invention has been completed.

According to the present invention, there is provided a substituted cyclic amine compound represented by the following general formula (1) 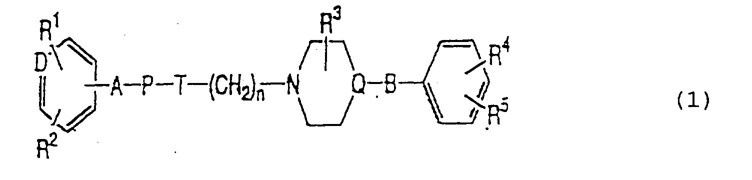
wherein R¹ and R² may be the same or different from each other and each represents a hydrogen atom, a hydroxyl group, a straight or branched-chain alkyl group, an alkenyl group, a substituted or unsubstituted aralkyl group, an alkoxy group, a hydroxyalkyl group, an acyl group, a substituted or unsubstituted amino group, a substituted or unsubstituted aminoalkyl group, a substituted or unsubstituted imino group, a substituted or unsubstituted iminoalkyl group, a substituted or unsubstituted guanidino group, a substituted or unsubstituted guanidinoalkyl group, a substituted or unsubstituted amidino group, a substituted or unsubstituted amidinoalkyl group, a substituted or unsubstituted ureido group, a substituted or unsubstituted ureidoalkyl group, a substituted or unsubstituted thioureido group, a substituted or unsubstituted thioureidoalkyl group, a carboxyl group, an alkoxycarbonyl group, a formyl group, a substituted or unsubstituted carbamoyl group, a substituted or unsubstituted carbamoylalkyl group, a formylalkyl group, an alkoxycarbonylalkyl group, a carboxyalkyl group, a mercapto group, an alkylthio group, a mercaptoalkyl group, a nitro group, a cyano group, a halogenoalkyl group, a trihalogenomethyl group or a halogen atom, R³ represents a hydrogen atom, a hydroxyl group, a straight or branched-chain alkyl group or an alkoxy group, R⁴ and R⁵ may be the same or different from each other and each represents a hydrogen atom, a hydroxyl group, a straight or branched-chain alkyl group, an alkenyl group, a halogen atom, an alkoxy group, a mercapto group, an alkylthio group, a substituted or unsubstituted amino group or a trihalogenomethyl group, D represents a methine moiety, a nitrogen atom or =N(→O)-, A represents a methylene moiety, a carbonyl group or a sulfonyl group, P is a group represented by -CR⁶(R⁷)- or -NR⁸-, each of R⁶, R⁷ and R⁸ represents a hydrogen atom, a straight or branched-chain alkyl group, a cyclic alkyl group, an alkenyl group, a substituted or unsubstituted aralkyl group, a substituted or unsubstituted phenyl group, a substituted or unsubstituted naphthyl group, a substituted or unsubstituted heterocyclic moiety, an acyl group, a hydroxyalkyl group, a substituted or unsubstituted aminoalkyl group, a cyano group, a substituted or unsubstituted carbamoylalkyl group, a carboxyalkyl group or an alkoxycarbonylalkyl group, Q represents a methine group or a substituted or unsubstituted nitrogen atom, each of T and B is a single bond or represents a group selected from the class consisting of methylene, carbonyl, -CH(OH)- and -C(=NH)-, and n is an integer of 1 to 6; or a pharmaceutically acceptable salt thereof.

The present invention also relates to a process for the production of the compound of general formula (1), to its use as a pharmaceutical composition for circulatory organ use, to a synthetic intermediate of the compound (1), represented by the following general formula (2) 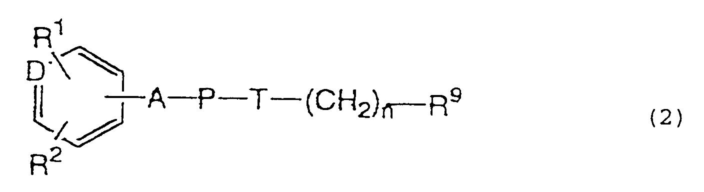
wherein R⁹ represents a hydroxyl group, a p-toluenesulfonyloxy group, a methanesulfonyloxy group, trifluoromethanesulfonyloxy group, a halogen atom, a tetrahydropyranyloxy group, a methoxy group, an ethoxy group, a tert-butoxy group, a benzyloxy group, an acetoxy group, a trimethylsilyloxy group, a tert-butyldimethyloxy group or a pivaloyloxy group, and R¹, R², D, A, P, T and n are as defined above, and to another synthetic intermediate of the compound (1), represented by the following general formula (3) 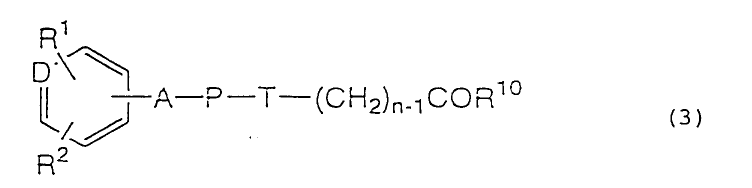
wherein R¹⁰ represents a hydrogen atom, a hydroxyl group, a methoxy group, an ethoxy group or a tert-butoxy group, and R¹, R², D, A, P, T and n are as defined above.

Other objects and advantages of the present invention will be made apparent as the description progresses.

### DETAILED DESCRIPTION OF THE INVENTION

Alkyl groups of the substituents R¹, R², R³, R⁴, R⁵, R⁶, R⁷ and R⁸ may be either in the straight or branched form, and their illustrative examples include those having 1 to 6 carbon atoms, such as methyl, ethyl, n-propyl, isopropyl, n-butyl, isobutyl, tert-butyl and the like. Illustrative examples of alkenyl groups of R¹, R², R³, R⁵, R⁶, R⁷ and R⁸ include those having 1 to 5 carbon atoms, such as vinyl, 1-propenyl, 2-propenyl, 1-butenyl and the like. Illustrative examples of halogen atoms of R¹, R², R⁴, R⁵, R⁹ and R¹¹ include fluorine, chlorine, bromine and iodine. Illustrative examples of alkoxy groups of R¹, R², R³, R⁴, R⁵ and R¹¹ include those either in the straight or branched form and having 1 to 6 carbon atoms, such as methoxy, ethoxy, n-propoxy, isopropoxy, n-butoxy, tert-butoxy and the like.

Illustrative examples of hydroxyalkyl groups of R¹, R², R⁶, R⁷ and R⁸ include those having 1 to 4 carbon atoms, such as hydroxymethyl, 2-hydroxyethyl, 1-hydroxyethyl, 3-hydroxypropyl, 2-hydroxypropyl, 4-hydroxybutyl, 3-hydroxybutyl and the like, which may be protected by protecting groups such as methyl, ethyl, phenyl, acetyl, pivaloyl, benzyl, trimethylsilyl, tert-butyldimethylsilyl, tetrahydropyranyl, methanesulfonyl, p-toluenesulfonyl and the like. Amino groups of R¹, R², R⁴ and R⁵ may be unsubstituted, monosubstituted or disubstituted amino groups, and illustrative examples of the substituents include lower alkyl groups such as methyl, ethyl, propyl, butyl and the like, acyl groups such as acetyl, trifluoroacetyl, propionyl, butylyl, benzoyl and the like, amino acid residues such as glycyl, alanyl, phenylalanyl and the like, sulfonyl groups such as methanesulfonyl, benzenesulfonyl, p-toluenesulfonyl and the like and alkoxycarbonyl groups such as methoxycarbonyl, ethoxycarbonyl, tert-butylcarbonyl and the like. In this instance, two of such substituents may be linked to each other to form a ring such as pyrrole, pyrrolidinyl, piperidino, morpholino, piperazinyl, succinimide, phthalimide or the like.

Illustrative examples of aminoalkyl groups of R¹, R², R⁶, R⁷ and R⁸ include those having 1 to 4 carbon atoms, such as aminomethyl, 2-aminoethyl, 1-aminoethyl, 3-aminopropyl, 2-aminopropyl, 4-aminobutyl, 3-aminobutyl and the like, and the amino group of these aminoalkyl groups may be unsubstituted, monosubstituted or disubstituted amino group, with illustrative examples of the substituents including lower alkyl groups such as methyl, ethyl, propyl, butyl and the like, acyl groups such as acetyl, trifluoroacetyl, propionyl, butylyl, benzoyl and the like, amino acid residues such as glycyl, alanyl, phenylalanyl and the like, sulfonyl groups such as methanesulfonyl, benzenesulfonyl, p-toluenesulfonyl and the like and alkoxycarbonyl groups such as methoxycarbonyl, ethoxycarbonyl, tert-butylcarbonyl and the like. In this instance, two of such substituents may be linked to each other to form a ring such as pyrrolidinyl, piperidino, morpholino, piperazinyl, succinimide, phthalimide or the like. Illustrative examples of alkylthio groups of R¹, R², R⁴, R⁵ and R¹¹ include those either in the straight or branched form having 1 to 6 carbon atoms, such as methylthio, ethylthio, isopropylthio, butylthio and the like.

Illustrative examples of aralkyl groups of R¹, R², R⁶, R⁷ and R⁸ include benzyl, phenethyl, pyridylmethyl, pyridylethyl, imidazomethyl and the like which may be substituted, and illustrative examples of the substituents include lower alkyl groups such as methyl, ethyl, propyl, isopropyl and the like, alkoxy groups such as methoxy, ethoxy, propoxy, isopropoxy and the like, alkenyl groups such as vinyl, 1-propenyl, 2-propenyl, 3-butenyl and the like, formyl group, cyano group, nitro group, amino groups such as amino, methylamino, dimethylamino, morpholino and the like, imino groups such as imino, N-methylimino, N-hydroxyimino and the like, amidino group in which 1 or 2 nitrogen atoms may be substituted with nitro or cyano, carboxyl group, alkoxycarbonyl groups such as methoxycarbonyl, ethoxycarbonyl and the like, trihalogenomethyl groups such as trichloromethyl, trifluoromethyl and the like, hydroxyl group, lower hydroxyalkyl groups such as hydroxymethyl, hydroxyethyl and the like which may be protected, mercapto group, lower alkylthio groups such as methylthio, ethylthio and the like, lower mercaptoalkyl groups such as mercaptomethyl, mercaptoethyl and the like which may be protected, fluorine, chlorine, bromine and iodine. Illustrative examples of halogenoalkyl groups of R¹ and R² are halogenomethyl, 2-halogenoethyl, 1-halogenoethyl, 3-halogenopropyl, 2-halogenopropyl, 4-halogenobutyl, 3-halogenobutyl and the like halogenoalkyl groups having 1 to 4 carbon atoms, such as chloroalkyl group, bromoalkyl group, iodoalkyl group, fluoroalkyl group and the like.

Illustrative examples of trihalogenomethyl groups of R¹, R², R⁴ and R⁵ include trichloromethyl, trifluoromethyl and the like. Illustrative examples of carbamoyl groups of R¹ and R² include unsubstituted, monosubstituted or disubstituted carbamoyl groups in which carboxyl group is condensed with amines such as ammonia, methylamine, ethylamine, propylamine, dimethylamine, diethylamine, aniline, morpholine and the like. Examples of carbamoylalkyl groups of R¹, R², R⁶, R⁷ and R⁸ include carbamoylmethyl, carbamoylethyl, carbamoylpropyl, carbamoylbutyl and the like, and illustrative examples of the carbamoyl group of these carbamoylalkyl groups include unsubstituted, monosubstituted or disubstituted carbamoyl groups in which carboxyl group is condensed with amines such as ammonia, methylamine, ethylamine, propylamine, dimethylamine, diethylamine, aniline, morpholine and the like. Illustrative examples of acyl groups of R¹, R², R⁶, R⁷ and R⁸ include aliphatic groups such as acetyl, propionyl and the like and aromatic groups such as benzoyl, naphthoyl and the like. Illustrative examples of alkoxycarbonylalkyl groups of R¹, R², R⁶, R⁷ and R⁸ include those having 2 to 10 carbon atoms, such as methoxycarbonylmethyl, ethoxycarbonylmethyl, methoxycarbonylethyl, ethoxycarbonylethyl and the like. Illustrative examples of alkoxycarbonyl groups of R¹ and R² include methoxycarbonyl, ethoxycarbonyl, propoxycarbonyl, tert-butoxycarbonyl and the like.

Illustrative examples of carboxyalkyl groups of R¹, R², R⁶, R⁷ and R⁸ include carboxymethyl, 2-carboxyethyl, 3-carboxypropyl and the like. Illustrative examples of formylalkyl groups of R¹ and R² include formylmethyl, 2-formylethyl, 3-formylpropyl and the like which may be protected. Illustrative examples of imino groups of R¹ and R² include those in which carbonyl group is condensed with amines such as ammonia, methylamine, ethylamine, propylamine, aniline, hydroxylamine, methoxyamine and the like. Examples of iminoalkyl groups of R¹ and R² include those having 1 to 4 carbon atoms, such as iminomethyl, 2-iminoethyl, 1-iminoethyl, 3-iminopropyl, 2-iminopropyl, 4-iminobutyl, 3-iminobutyl and the like, and illustrative examples of the imino group of these iminoalkyl groups include those in which carbonyl group is condensed with amines such as ammonia, methylamine, ethylamine, propylamine, aniline, hydroxylamine, methoxyamine and the like. One or two nitrogen atoms of the guanidino group of R¹ and R² may be substituted, and illustrative examples of such substituents include methyl, ethyl, benzyl, acetyl, trifluoroacetyl, methanesulfonyl, alkoxycarbonyl such as methoxy carbonyl, ethoxycarbonyl and the like, cyano and nitro.

One or two nitrogen atoms of the guanidinoalkyl group of R¹ and R² may be substituted with methyl, ethyl, benzyl, acetyl, trifluoroacetyl, methanesulfonyl, alkoxycarbonyl such as methoxy carbonyl, ethoxycarbonyl or the like, cyano or nitro, and its illustrative examples include guanidinomethyl, guanidinoethyl, guanidinopropyl, guanidinobutyl and the like. One or two nitrogen atoms of the amidino group of R¹ and R² may be substituted, and illustrative examples of such substituents include methyl, ethyl, benzyl, acetyl, trifluoroacetyl, propionyl, alkoxycarbonyl such as methoxy carbonyl, ethoxycarbonyl and the like, cyano, nitro,methanesulfonyl, p-toluenesulfonyl and the like. One or two nitrogen atoms of the amidinoalkyl group of R¹ and R² may be substituted with methyl, ethyl, benzyl, acetyl, trifluoroacetyl, propionyl, alkoxycarbonyl such as methoxy carbonyl, ethoxycarbonyl and the like, cyano, nitro,methanesulfonyl, p-toluenesulfonyl and the like, and its illustrative examples include amidinomethyl, amidinoethyl, amidinopropyl, amidinobutyl and the like.

One or two nitrogen atoms of the ureido group of R¹ and R² may be substituted, and illustrative examples of such substituents include methyl, ethyl, propyl, tert-butyl and the like. One or two nitrogen atoms of the ureidoalkyl group of R¹ and R² may be substituted, and illustrative examples of such substituents include methyl, ethyl, propyl, tert-butyl and the like. One or two nitrogen atoms of the thioureido group of R¹ and R² may be substituted, and illustrative examples of such substituents include methyl, ethyl, propyl, tert-butyl and the like. One or two nitrogen atoms of the thioureidoalkyl group of R¹ and R² may be substituted, and illustrative examples of such substituents include methyl, ethyl, propyl, tert-butyl and the like. The phenyl groups of R⁶, R⁷ and R⁸ are mono- to trisubstituted phenyl groups, and illustrative examples of the substituents include lower alkyl groups such as methyl, ethyl, propyl, isopropyl and the like, alkoxy groups such as methoxy, ethoxy, propoxy, isopropoxy and the like, alkenyl groups such as vinyl, 1-propenyl, 2-propenyl, 3-butenyl and the like, formyl group, cyano group, amino groups such as amino, methylamino, dimethylamino, morpholino and the like, nitro group, imino groups such as imino, N-methylimino, N-hydroxyimino and the like, amidino group in which 1 or 2 nitrogen atoms may be substituted with nitro or cyano, carboxyl group, alkoxycarbonyl groups such as methoxycarbonyl, ethoxycarbonyl and the like, trihalogenomethyl groups such as trichloromethyl, trifluoromethyl and the like, hydroxyl group, lower hydroxyalkyl groups such as hydroxymethyl, hydroxyethyl and the like which may be protected, mercapto group, lower alkylthio groups such as methylthio, ethylthio and the like, lower mercaptoalkyl groups such as mercaptomethyl, mercaptoethyl and the like which may be protected, fluorine, chlorine, bromine and iodine.

The naphthyl groups of R⁶, R⁷ and R⁸ are mono- to trisubstituted naphthyl groups, and illustrative examples of the substituents include lower alkyl groups such as methyl, ethyl, propyl, isopropyl and the like, alkoxy groups such as methoxy, ethoxy, propoxy, isopropoxy and the like, alkenyl groups such as vinyl, 1-propenyl, 2-propenyl, 3-butenyl and the like, formyl group, cyano group, amino groups such as amino, methylamino, dimethylamino, morpholino and the like, nitro group, imino groups such as imino, N-methylimino, N-hydroxyimino and the like, amidino group in which 1 or 2 nitrogen atoms may be substituted with nitro or cyano, carboxyl group, alkoxycarbonyl groups such as methoxycarbonyl, ethoxycarbonyl and the like, trihalogenomethyl groups such as trichloromethyl, trifluoromethyl and the like, hydroxyl group, lower hydroxyalkyl groups such as hydroxymethyl, hydroxyethyl and the like which may be protected, mercapto group, lower alkylthio groups such as methylthio, ethylthio and the like, lower mercaptoalkyl groups such as mercaptomethyl, mercaptoethyl and the like which may be protected, fluorine, chlorine, bromine and iodine.

Illustrative examples of heterocyclic rings of R⁶, R⁷ and R⁸ include furan, thiophene, pyrrole, imidazole, pyridine, oxazole, oxazolidine, oxazoline, thiazole, thiazolidine, thiazoline, pyrimidine, pyrazine, indole, benzimidazole, quinoline, isoquinoline and the like which may be substituted, and illustrative examples of the substituents include lower alkyl groups such as methyl, ethyl, propyl, isopropyl and the like, alkoxy groups such as methoxy, ethoxy, propoxy, isopropoxy and the like, alkenyl groups such as vinyl, 1-propenyl, 2-propenyl, 3-butenyl and the like, formyl group, cyano group, amino groups such as amino, methylamino, dimethylamino, morpholino and the like, nitro group, imino groups such as imino, N-methylimino, N-hydroxyimino and the like, amidino group in which 1 or 2 nitrogen atoms may be substituted with nitro or cyano, carboxyl group, alkoxycarbonyl groups such as methoxycarbonyl, ethoxycarbonyl and the like, trihalogenomethyl groups such as trichloromethyl, trifluoromethyl and the like, hydroxyl group, lower hydroxyalkyl groups such as hydroxymethyl, hydroxyethyl and the like which may be protected, mercapto group, lower alkylthio groups such as methylthio, ethylthio and the like, lower mercaptoalkyl groups such as mercaptomethyl, mercaptoethyl and the like which may be protected, fluorine, chlorine, bromine and iodine.

Illustrative examples of cyclic alkyl groups of R⁶, R⁷ and R⁸ include 3- to 7-membered rings such as cyclopentane, cyclohexane, cycloheptane and the like which may be substituted, and illustrative examples of the substituents include lower alkyl groups such as methyl, ethyl, propyl, isopropyl and the like, alkoxy groups such as methoxy, ethoxy, propoxy, isopropoxy and the like, alkenyl groups such as vinyl, 1-propenyl, 2-propenyl, 3-butenyl and the like, formyl group, cyano group, amino groups such as amino, methylamino, dimethylamino, morpholino and the like, nitro group, imino groups such as imino, N-methylimino, N-hydroxyimino and the like, amidino group in which 1 or 2 nitrogen atoms may be substituted with nitro or cyano, carboxyl group, alkoxycarbonyl groups such as methoxycarbonyl, ethoxycarbonyl and the like, trihalogenomethyl groups such as trichloromethyl, trifluoromethyl and the like, hydroxyl group, lower hydroxyalkyl groups such as hydroxymethyl, hydroxyethyl and the like which may be protected, mercapto group, lower alkylthio groups such as methylthio, ethylthio and the like, lower mercaptoalkyl groups such as mercaptomethyl, mercaptoethyl and the like which may be protected, fluorine, chlorine, bromine and iodine.

Illustrative examples of salts of the compound (1) include acid addition salts such as of hydrochloric acid, sulfuric acid, nitric acid, phosphoric acid and the like mineral acids, methanesulfonic acid, benzenesulfonic acid, toluenesulfonic acid and the like organic sulfonic acids and acetic acid, tartaric acid, maleic acid, fumaric acid, oxalic acid, lactic acid, citric acid and the like organic carboxylic acids.

Processes for the production of the intended compound (1) of the present invention and its synthetic intermediates (2) and (3) are described in detail in the following.

The novel substituted cyclic amine compound of the present invention represented by the aforementioned general formula (1) and its salts can be produced in accordance with the following reaction formulae. 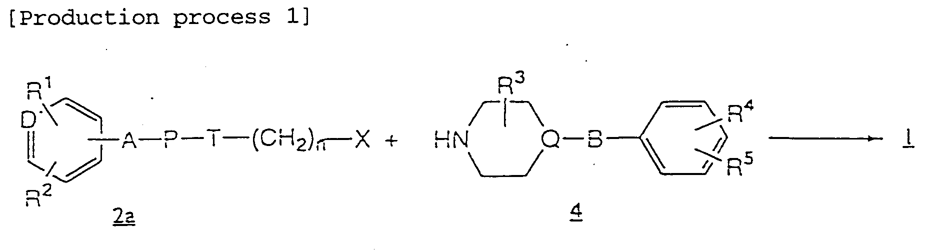
In this formula, X represents a halogen atom, a p-toluenesulfonyloxy group, a methanesulfonyloxy group or a trifluoromethanesulfonyloxy group, and R¹, R², R³, R⁴, R⁵, A, D, P, T, n, Q and B are as defined in the foregoing. 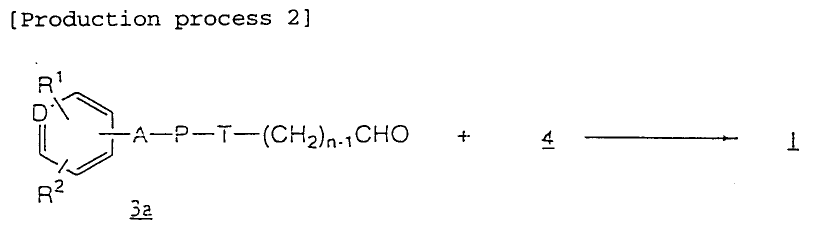
In this formula, R¹, R², R³, R⁴, R⁵, A, D, P, T, n, Q and B are as defined in the foregoing. 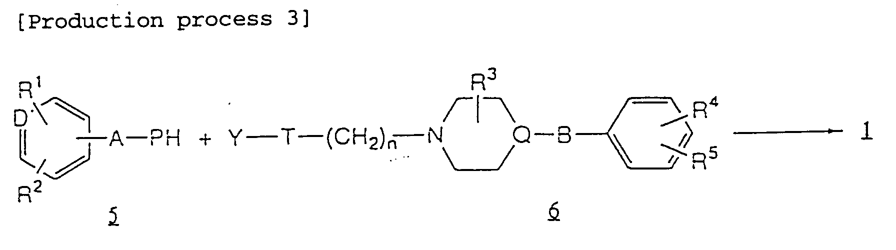
In this formula, Y represents a halogen atom, a p-toluenesulfonyloxy group, a methanesulfonyloxy group or a trifluoromethanesulfonyloxy group, and R¹, R², R³, R⁴, R⁵, A, D, P, T, n, Q and B are as defined in the foregoing. 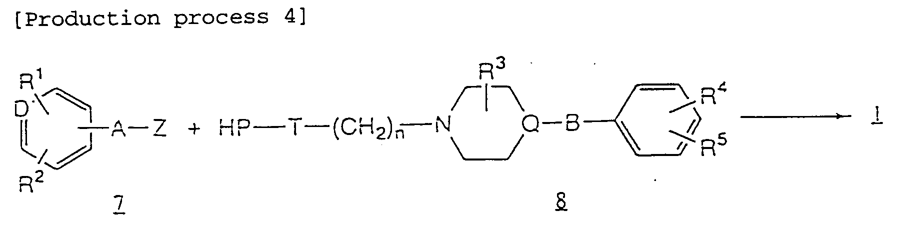
In this formula, Z represents a halogen atom, a p-toluenesulfonyloxy group, a methanesulfonyloxy group or a trifluoromethanesulfonyloxy group, and R¹, R², R³, R⁴, R⁵, A, D, P, T, n, Q and B are as defined in the foregoing. 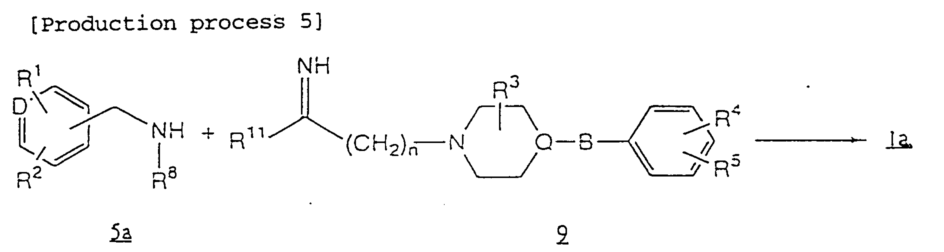
In this formula R¹¹ represents an alkoxy group, an alkylthio group or a halogen atom and R¹, R², R³, R⁴, R⁵, R⁸, D, n, Q and B are as defined in the foregoing.

### [Production process 1]

The compound (1) can be produced by allowing the compound (2a) to react with the compound (4). Examples of the base to be used in the reaction include organic bases such as triethylamine and the like, alkali metal salts such as sodium hydride, n-butyl lithium, sec-butyl lithium, tert-butyl lithium, tert-butoxy potassium and the like and alkali metal carbonates such as potassium carbonate. Examples of the solvent to be used in the reaction include tetrahydrofuran (THF), N,N-dimethylformamide (DMF), dimethyl sulfoxide (DMSO), methylene chloride, benzene, toluene, acetone, methyl ethyl ketone and the like. The reaction temperature can be selected within the range of from 0 to 100°C, and the reaction is completed within 2 to 24 hours. It is desirable to carry out this reaction in an atmosphere of an inert gas such as argon or nitrogen.

### [Production process 2]

The compound (1) can be produced by allowing the compound (3a) to react with the compound (4). The reaction is carried out in an alcohol such as methanol, ethanol or the like or in a solvent such as ethylene chloride, chloroform, THF or the like, in the presence of a boron base reducing agent such as sodium cyanoborohydride or the like alkali metal cyanoborohydride, sodium borohydride or the like alkali metal borohydride or diborane, or in an atmosphere of hydrogen in the presence of a transition metal catalyst such as palladium carbon, palladium black, rhodium carbon or the like. The reaction temperature can be selected within the range of from 0 to room temperature. If necessary, a catalytically effective amount of an organic acid such as acetic acid, p-toluenesulfonic acid or the like, or a dehydrating agent such as molecular sieves, magnesium sulfate or the like, may be added to the reaction system. The reaction is completed within 1 to 4 hours. It is desirable to carry out this reaction in an atmosphere of an inert gas such as argon or nitrogen.

### [Production process 3]

The compound (1) can be produced by allowing the compound (5) to react with the compound (6). Examples of the base to be used in the reaction include organic bases such as triethylamine and the like, alkali metal amide bases such as lithium diisopropylamide (LDA), sodium amide and the like, alkali metal salts such as sodium hydride, n-butyl lithium, sec-butyl lithium, tert-butyl lithium, tert-butoxy potassium and the like and alkali metal carbonates such as potassium carbonate. Examples of the solvent to be used in the reaction include THF, DMF, DMSO, methylene chloride, benzene, toluene, acetone, methyl ethyl ketone and the like. The reaction temperature can be selected within the range of from 0 to 100°C, and the reaction is completed within 2 to 24 hours. It is desirable to carry out this reaction in an atmosphere of an inert gas such as argon or nitrogen. As occasion demands, an alkali metal halide such as sodium iodide, potassium iodide or the like may be added to the reaction system.

### [Production process 4]

The compound (1) can be produced by allowing the compound (7) to react with the compound (8). Examples of the base to be used in the reaction include organic bases such as triethylamine and the like, alkali metal amide bases such as LDA, sodium amide and the like, alkali metal salts such as sodium hydride, n-butyl lithium, sec-butyl lithium, tert-butyl lithium, tert-butoxy potassium and the like and alkali metal carbonates such as potassium carbonate. Examples of the solvent to be used in the reaction include diethyl ether, THF, DMF, DMSO, methylene chloride, benzene, toluene, acetone, methyl ethyl ketone and the like. The reaction temperature can be selected within the range of from 0 to 100°C, and the reaction is completed within 2 to 24 hours. It is desirable to carry out this reaction in an atmosphere of an inert gas such as argon or nitrogen.

### [Production process 5]

The compound (1a) can be produced by allowing the compound (5a) to react with the compound (9). The reaction is carried out without solvent or in an alcohol such as methanol, ethanol or the like or in a solvent such as methylene chloride, chloroform, THF or the like, at a temperature of from 0 to 100°C for a period of from 1 to 24 hours. It is desirable to carry out this reaction in an atmosphere of an inert gas such as argon or nitrogen.

The compound of the present invention represented by the aforementioned general formula (2) as a synthetic intermediate of the compound (1) can be produced in accordance with the following reaction formulae. 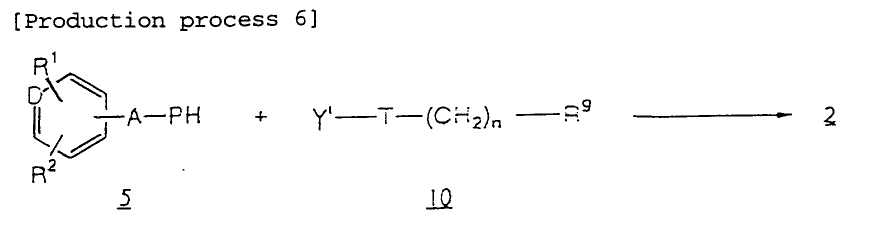
In this formula, Y' represents a halogen atom or a sulfonyloxy group, and R¹, R², R⁹, A, D, P, T and n are as defined in the foregoing. 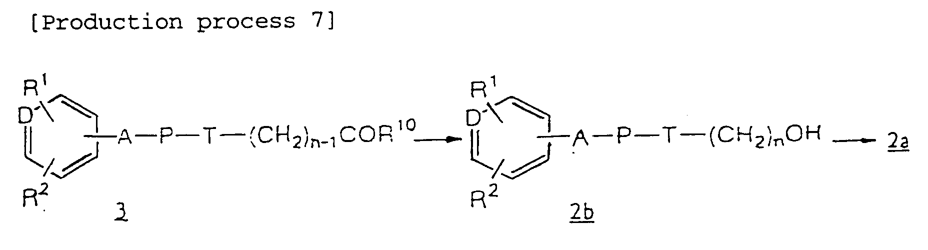
In this formula, R¹, R², R¹⁰, A, D, P, T, X and n are as defined in the foregoing. 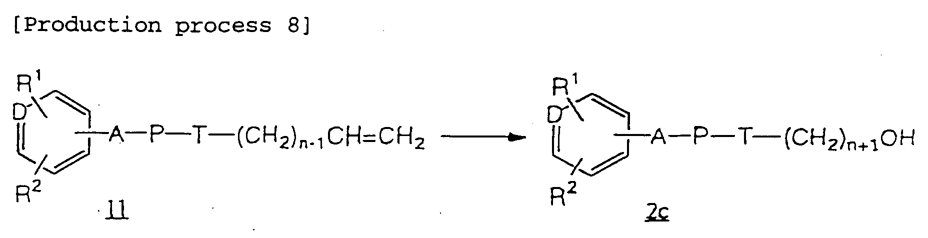
In this formula, R¹, R², A, D, P, T, and n are as defined in the foregoing.

### [Production process 6]

The compound (2) can be produced by allowing the compound (5) to react with the compound (10). In the compound (10), Y' represents a reactive leaving group such as chloro, bromo, iodo or the like halogen atom or methanesulfonyloxy, p-toluenesulfonyloxy or the like sulfonyloxy group. Examples of the base to be used in the reaction include organic bases such as triethylamine, pyridine and the like, alkali metal amide bases such as LDA, sodium amide and the like, alkali metal salts such as sodium hydride, n-butyl lithium, sec-butyl lithium, tert-butyl lithium, tert-butoxy potassium and the like and alkali metal carbonates such as potassium carbonate. Examples of the solvent to be used in the reaction include diethyl ether, THF, DMF, DMSO, methylene chloride, benzene, toluene, acetone, methyl ethyl ketone and the like. The reaction temperature can be selected within the range of from 0 to 100°C, and the reaction is completed within 2 to 24 hours. It is desirable to carry out this reaction in an atmosphere of an inert gas such as argon or nitrogen. As occasion demands, an alkali metal halide such as sodium iodide, potassium iodide or the like may be added to the reaction system.

### [Production process 7]

The compound (2b) can be produced by reducing the compound (3). The reducing reaction is carried out in an alcohol such as methanol, ethanol or the like in the presence of a boron hydride base reducing agent such as sodium borohydride, sodium cyanoborohydride or the like, or in a solvent such as toluene, THF, diethyl ether, ethylene glycol dimethyl ether (DME) or the like in the presence of an aluminum hydride base reducing agent such as lithium aluminum hydride, diisobutylaluminum hydride (DIBAH) or the like. The reaction temperature can be selected within the range of from -78°C to boiling point, and the reaction is completed within 1 to 4 hours. It is desirable to carry out this reaction in an atmosphere of an inert gas such as argon or nitrogen. Also, the compound (2a) can be produced by substituting the hydroxyl group of the compound (2b) with a halogen atom or sulfonylating the hydroxyl group. The substitution reaction with a halogen atom may be carried out using a reaction agent such as hydrogen chloride, hydrogen bromide or the like hydrogen halide or a solution thereof, thionyl chloride, thionyl bromide or the like thionyl halide, oxalyl chloride or the like oxalyl halide, phosphorous oxychloride, phosphorous oxybromide, phosphorous trichloride, phosphorous tribromide or the like phosphorous halide or carbon tetrachloride, carbon tetrabromide or the like carbon tetrahalide. The sulfonylation reaction may be carried out in the presence of a tertiary amine such as triethylamine, pyridine or the like using a reaction agent such as methanesulfonyl chloride, p-toluenesulfonyl chloride or the like sulfonic acid chloride or trifluoromethanesulfonic acid anhydride or the like sulfonic acid anhydride. The reaction may be carried out without solvent or in a solvent such as methylene chloride, chloroform, benzene, toluene, THF, DMF, DME or the like. The reaction temperature is in the range of from -20°C to boiling point, and the reaction is completed within 1 to 24 hours.

### [Production process 8]

The compound (2c) can be produced by subjecting the compound (11) to hydroboration and then to oxidation using hydrogen peroxide under an alkaline condition. Examples of the hydroboration agent include diborane, 9-borabicyclo[3.3.1]nonane (9-BBN), diamyl borane and the like. Examples of the solvent to be used in the reaction include THF, diethyl ether and the like. The reaction temperature can be selected within the range of from -78°C to room temperature. The reaction is completed within 6 to 6 hours, and the subsequent oxidation reaction is completed within 1 to 5. It is desirable to carry out this reaction in an atmosphere of an inert gas such as argon or nitrogen.

The compound of the present invention represented by the aforementioned general formula (3) as a synthetic intermediate of the compound (1) can be produced in accordance with the following reaction formulae. 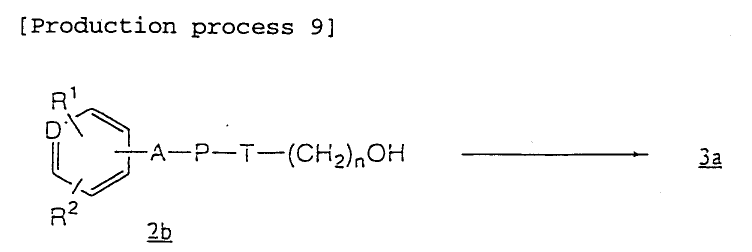
In this formula, R¹, R², A, D, P, T, and n are as defined in the foregoing. 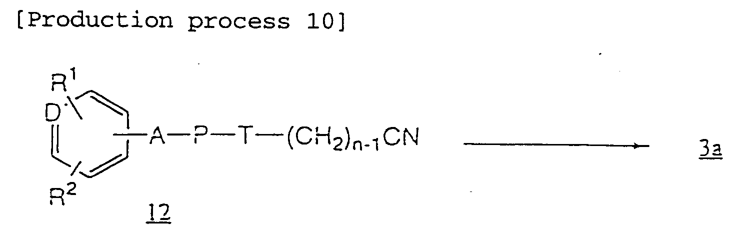
In this formula, R¹, R², A, D, P, T, and n are as defined in the foregoing. 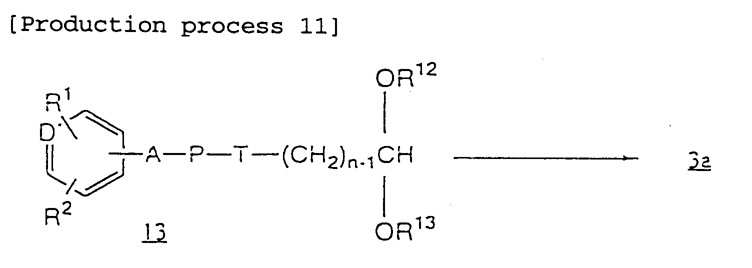
In this formula, R¹² and R¹³ may be the same or different from each other and each represents a straight or branched-chain lower alkyl group such as methyl, ethyl, isobutyl or the like, R¹² and R¹³ may be linked to each other to form a lower alkyl chain such as -C₂H₅- or the like, and R¹, R², A, D, P, T, and n are as defined in the foregoing. 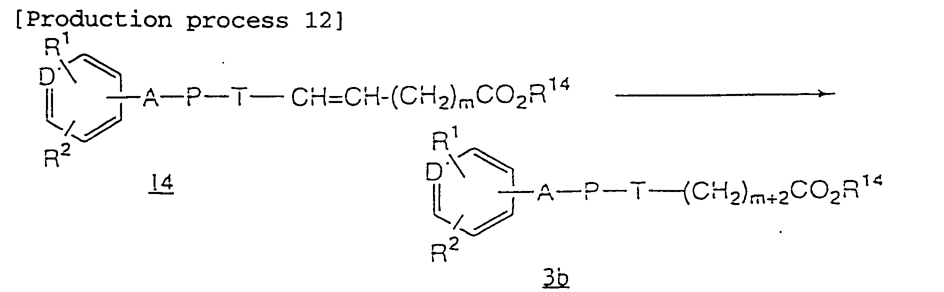
In this formula, m is an integer of 0 to 3, R¹⁴ represents a lower alkyl group such as methyl, ethyl or the like, and R¹, R², A, D, P and T are as defined in the foregoing.

### [Production process 9]

The compound (3a) can be produced by subjecting the compound (2b) to oxidation reaction. The oxidation reaction may be carried out in a hydrocarbon halide solvent such as methylene chloride or the like in the presence of Collins reagent or a chromic acid base oxidizing agent such as pyridinium chlorochromate or the like, or Swern oxidation or the like oxidation agent or a sulfur trioxide-pyridine complex in DMSO solvent. The reaction temperature can be selected within the range of from -78°C to room temperature, and the reaction is completed within 1 to 12 hours. It is desirable to carry out this reaction in an atmosphere of an inert gas such as argon or nitrogen.

### [Production process 10]

The compound (3a) can be produced by reducing the compound (12) into iminium salt which is then subjected to hydrolysis. The reduction reaction may be carried out in a hydrocarbon solvent such as hexane, toluene or the like in the presence of an aluminum hydride base reducing agent such as lithium aluminum hydride, DIBAH or the like, and the subsequent hydrolysis may be carried out using a mineral acid such as sulfuric acid, hydrochloric acid or the like. The reaction temperature can be selected within the range of from -78°C to room temperature, and the reaction is completed within 1 to 12 hours. It is desirable to carry out this reaction in an atmosphere of an inert gas such as argon or nitrogen.

### [Production process 11]

The compound (3a) can be produced by deprotecting the acetal moiety of the compound (13). The reaction may be carried out in a polar solvent such as THF, acetone or the like using a mineral acid such as hydrochloric acid, sulfuric acid or the like, an organic acid such as acetic acid, trifluoroacetic acid or the like or a Lewis acid such as aluminium chloride, silica gel, trimethylsilane iodide. The reaction temperature can be selected within the range of from -20°C to boiling point, and the reaction is completed within 30 minutes to to 24 hours.

### [Production process 12]

The compound (3b) can be produced by subjecting the double bond of compound (14) to catalytic hydrogenation. The reaction may be carried out in a solvent such as methanol, ethanol, ethyl acetate or the like in the presence of a transition metal catalyst such as palladium carbon, palladium black, rhodium carbon or the like, in an atmosphere of hydrogen under normal pressure or pressurized condition. The reaction temperature can be selected within the range of from room temperature to 100°C, and the reaction is completed within 1 to 12 hours.

The compound of the present invention represented by the aforementioned general formula (11) as a synthetic intermediate of the compound (2b) can be produced in accordance with the following reaction formulae. 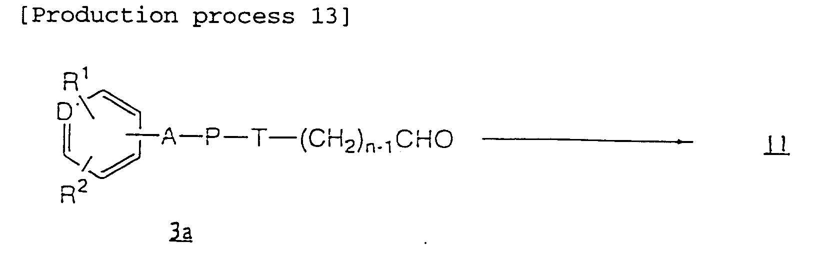
In this formula, R¹, R², A, D, P, T and n are as defined in the foregoing.

### [Production process 13]

The compound (11) can be produced by subjecting the compound (3a) to methylenization reaction. The reaction may be effected in an ether base solvent such as THF, diethyl ether or the like using a ylide obtained from methyltriphenylphosphonium bromide and a base such as tert-butoxy potassium. The reaction temperature can be selected within the range of from -78°C to room temperature, and the reaction is completed within 30 minutes to 3 hours. It is desirable to carry out this reaction in an atmosphere of an inert gas such as argon or nitrogen.

The compound of the present invention represented by the aforementioned general formula (12) as a synthetic intermediate of the compound (3a) can be produced in accordance with the following reaction formulae. 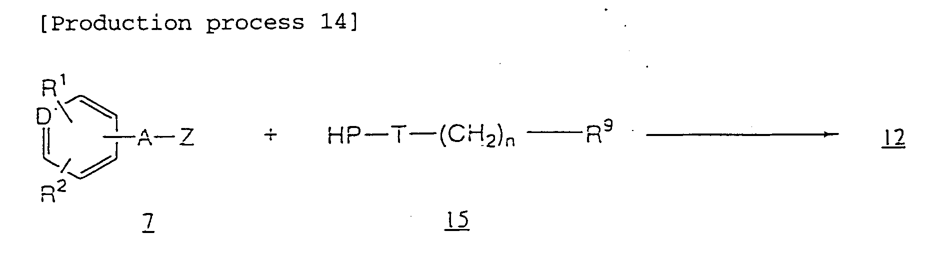
In this formula, R¹, R², R⁹, A, D, Z, P, T and n are as defined in the foregoing.

### [Production process 14]

The compound (12) can be produced by allowing the compound (7) to react with the compound (15). Examples of the base to be used in the reaction include organic bases such as triethylamine and the like, alkali metal amide bases such as LDA, sodium amide and the like, alkali metal salts such as sodium hydride, n-butyl lithium, sec-butyl lithium, tert-butyl lithium, tert-butoxy potassium and the like and alkali metal carbonates such as potassium carbonate. Examples of the solvent to be used in the reaction include diethyl ether, THF, DMF, DMSO, methylene chloride, benzene, toluene, acetone, methyl ethyl ketone and the like. The reaction temperature can be selected within the range of from 0 to 100°C, and the reaction is completed within 2 to 24 hours. It is desirable to carry out this reaction in an atmosphere of an inert gas such as argon or nitrogen.

The compound of the present invention represented by the aforementioned general formula (14) as a synthetic intermediate of the compound (3b) can be produced in accordance with the following reaction formulae. 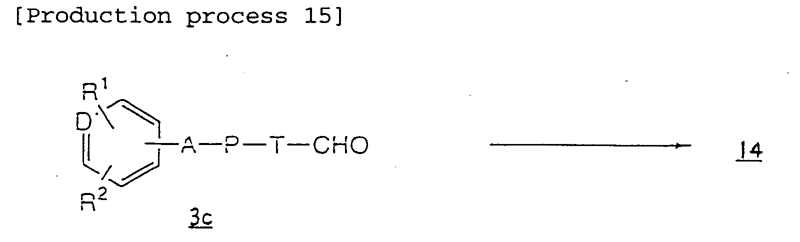
In this formula, R¹, R², R¹⁴, A, D, P, T and m are as defined in the foregoing.

### [Production process 15]

The compound (14) can be produced by subjecting the compound (3c) to Witting reaction or Horner-Emmons reaction. Witting reaction may be carried out using methyl (triphenylphosphoranidene)acetate in an ether base solvent such as THF, diethyl ether or the like,or using a ylide obtained from a phosphonium salt of (4-carboxybutyl)triphenylphosphonium bromide, (3-ethoxycarboxypropyl)triphenylphosphonium bromide or the like and a base such as tert-butoxy potassium or the like, in a solvent such as benzene, toluene or the like. In the case of Horner-Emmons reaction, it may be carried out in an ether base solvent such as THF, diethyl ether or the like using an phosphonium anion obtained from trimethyl phosphonoacetate and a base such as sodium hydride or the like. The reaction temperature can be selected within the range of from -78°C to boiling point, and the reaction is completed within 30 minutes to 6 hours. It is desirable to carry out this reaction in an atmosphere of an inert gas such as argon or nitrogen.

Compounds to be used as starting materials are known compounds disclosed in literatures or can be prepared in accordance with the procedures disclosed in literatures.

The serotonin 2 receptor antagonism, platelet aggregation inhibition activity and serotonin (5-hydroxy-L-tryptophan: 5-HTP)-induced head-twitch inhibition activity of representative compounds of the general formula (1) in the present invention are described in the following in detail.
4-(4-Fluorobenzoyl)-1-[3-(2-methoxyphenyl)-4-(3-methoxyphenyl)butyl]piperidine hydrochloride (compound A: Inventive Example 200)
N-{2-[4-(4-Fluorobenzoyl)piperidino]ethyl}-3-methoxy-N-(2-methoxyphenyl)benzamide oxalate (compound B: Inventive Example 212)
N-{2-[4-(4-Fluorobenzoyl)piperidino]ethyl}-4-methoxy-N-(2-methoxyphenyl)benzenesulfonamide oxalate (compound C: Inventive Example 213)
4-Fluoro-N-{2-[4-(4-fluorobenzoyl)piperidino]ethyl}-N-(2-methoxyphenyl)benzenesulfonamide oxalate (compound D: Inventive Example 214)
N-Cyclohexyl-N-{2-[4-(4-fluorobenzoyl)piperidino]ethyl}-4-methoxybenzenesulfonamide oxalate (compound E: Inventive Example 228)
N-{2-[4-(4-Fluorobenzoyl)piperidino]ethyl}-4-hydroxymethyl-N-(2-methoxyphenyl)benzenesulfonamide oxalate (compound F: Inventive Example 237)
N-{2-[4-(4-Fluorobenzoyl)piperidino]ethyl}-4-hydroxymethyl-N-(2-methoxyphenyl)benzamide oxalate (compound G: Inventive Example 238)
4-Aminomethyl-N-{2-[4-(4-fluorobenzoyl)piperidino]ethyl}-N-(2-methoxyphenyl)benzamide oxalate (compound H: Inventive Example 246)
3-[4-(4-Fluorobenzoyl)piperidino]-N-(4-methoxybenzenesulfonyl)propionamidine oxalate (compound I: Inventive Example 262)
4-Acetylamino-N-{2-[4-(4-fluorobenzoyl)piperidino]ethyl}-N-(3-methoxyphenyl)benzamide fumarate (compound J: Inventive Example 269)
4-Acetylamino-N-{2-[4-(4-fluorobenzoyl)piperidino]ethyl}-N-(phenyl)benzamide fumarate (compound K: Inventive Example 289) Ketanserin (comparative compound)

### [Measurement of serotonin 2 receptor antagonistic activity]

Male rats of Wistar-KY line (body weight, approximately 220 to 370 g) were sacrificed and subjected to bloodletting to excise abdominal side tail arteries. A piece of wire was inserted into each of the thus excised blood vessels to prepare a spiral specimen (approximately 1.5 x 30 mm). The specimen was suspended in a Magnus tube (10 ml; Krebs-Hensereit solution) with a load of 500 mg at 37°C, and a gas mixture of 95% O₂ + CO₂ was passed through the tube. Tensile force was measured using a tension transducer (TB-621T, manufactured by Nippon Koden) and writing the data on an ink recorder (FBA-253A, manufactured by Toa Denpa) via a pressure amplifier (AP-621G, manufactured by Nippon Koden). After 1 hour of equilibration time, the specimen was contracted with 10⁻⁵ M of serotonin, washed and then subjected to cumulative administration of 10⁻⁸ to 3 x 10⁻⁵ M serotonin at intervals of 45 minutes to record the resulting contraction twice. The second recording was used as a control. Thereafter, each of the drugs to be tested was administered 10 minutes before the commencement of the cumulative administration to evaluate its serotonin antagonism. The serotonin antagonism of these test drugs on the serotonin contraction was expressed by IC₅₀ values against the contraction of 3 x 10⁻⁶ serotonin, with the results shown in Table 1. In the table, each result was indicated by "+" when the IC₅₀ value was 1.0 x 10⁻⁷ M or more, "++" when the value was 9.9 x 10⁻⁸ to 1.0 x 10⁻⁸ M and "+++" when the value was 9.9 x 10⁻⁹ M or smaller.

**Table 1**

| Serotonin 2 receptor antagonism | |
|---|---|
| Compound | IC₅₀ value |
| A | ++ |
| B | ++ |
| D | ++ |
| E | ++ |
| F | +++ |
| H | ++ |
| I | ++ |
| J | ++ |
| K | ++ |
| ketanserin | ++ |

### [Measurement of platelet aggregation inhibition activity]

Measurement of platelet aggregation was carried out using a platelet aggregation measuring apparatus NSB Hematracer 601.

Blood samples were collected from auricular arteries of male Japanese white rabbits (2 to 3 kg) using a syringe containing 1 volume of 3.8% sodium citrate per 9 volumes of blood and centrifuged at 900 rpm for 10 minutes. After collecting the resulting supernatant fluid as platelet rich plasma (PRP), the remaining lower layer was centrifuged at 3,000 rpm for 10 minutes to obtain platelet poor plasma (PPP). Platelets in the thus obtained PRP were counted using a microcell counter (Sysmex F-800, manufactured by Toa Iyo Denshi) and diluted with the PPP to a density of 30 x 10⁴/µl. Firstly, aggregation of the diluted PRP by collagen alone was examined to find a collagen concentration which does not cause aggregation.

A 220 µl portion of PRP was mixed with 5 µl of 104 mM CaCl₂, incubated at 37°C for 1 minute and then mixed with 5 µl of a drug to be tested or physiological saline as a control. To this were added 5 µl of 5-HTP (final concentration, 3 µM) 2 minutes thereafter and collagen 1 minute further thereafter in a concentration which alone does not cause aggregation, thereby inducing platelet aggregation reaction. Activities to inhibit the aggregation reaction were measured to calculate 50% inhibition concentration (IC₅₀) by probit analysis, with the results shown in Table 2. In the table, each result was indicated by "+" when the IC₅₀ value was 1.0 x 10⁻⁶ M or more, "++" when the value was 9.9 x 10⁻⁷ to 1.0 x 10⁻⁷ M and "+++" when the value was 9.9 x 10⁻⁸ M or smaller.

**Table 2**

| Platelet aggregation inhibition activity | |
|---|---|
| Compound | IC₅₀ value |
| C | + |
| G | ++ |
| H | ++ |
| J | +++ |
| ketanserin | ++ |

### [Measurement of activity to inhibit 5-HTP-induced head-twitch]

To each ICR male mouse of 4 weeks of age were administered 300 mg/kg of 5-HTP by intraperitoneal injection (i.p.) and then 0.03 to 3 mg/kg of each test drug 25 minutes thereafter by intravenous injection, thereby counting the number of head-twitch induced after 5 minutes of the administration (for 20 minutes) and calculating the IC₅₀ value. The results shown in Table 3. In the table, each result was indicated by "+" when the IC₅₀ value was 1,000 mg/kg or more, "++" when the value was 100 to 999 mg/kg and "+++" when the value was 99 mg/kg or less.

**Table 3**

| Activity to inhibit 5-HTP-induced head-twitch | |
|---|---|
| Compound | IC₅₀ value |
| G | +++ |
| H | + |
| J | ++ |
| ketanserin | +++ |

As is evident from the results of the above pharmacological tests, the substituted cyclic amine compound represented by the general formula (1) and salts thereof have a strong serotonin 2 receptor antagonism, because they showed activities similar to or higher than those of the control drug ketanserin in the serotonin 2 antagonism test in which excised rat blood vessels were used and in the inhibition test of rabbit platelet aggregation induced by serotonin. On the other hand, since the activity to inhibit serotonin-induced head-twitch in mice is weaker than that of ketanserin, it is evident that the central action of the inventive compound is separated.

Since the substituted cyclic amine compound represented by the general formula (1) and salts thereof show strong serotonin 2 receptor antagonism and excellent reaction selectivity, they are useful as pharmaceutical drugs for the prevention and treatment of circulatory organ-related diseases such as hypertension, ischemic heart disease and other diseases caused by the cerebrovascular and peripheral circulatory disturbances. The compound (1) or a salt thereof can be used in oral or parenteral administration as it is or in various dosage forms such as powders, granules, tablets, capsules, injections and the like by mixing it with optionally selected pharmaceutically acceptable carriers, fillers, diluents and the like. Its dose varies depending on the diseases to be treated, conditions of the symptoms and patients, administration methods and the like. In general, when administered to adults, it may be used in an amount of from 1 to 200 mg per day in the case of oral administration or from 0.5 to 50 mg per day in the case of intravenous injection, and the daily dose recited above may preferably be divided into 1 to 3 doses per day.

The following examples are provided to further illustrate the present invention. It is to be understood, however, that the examples are for purpose of illustration only and are not intended as a definition of the limits of the present invention. All the persents and ratios are by weight unless otherwise indicated.

### Reference Example 1

### 2-(2-Methoxyphenyl)-3-(3-methoxyphenyl)propionitrile

In an atmosphere of argon, diisopropylamine (1.5 ml, 10.8 mmol) was dissolved in THF (5 ml) to which was added dropwise n-butyl lithium (7.4 ml, 1.46 M, 10.8 mmol) at -40°C. After 20 minutes of stirring at the same temperature, to this were added dropwise a solution (10 ml) of (2-methoxyphenyl)acetonitrile (1.62 g, 10.8 mmol) dissolved in THF and, after 20 minutes of stirring, a solution (5 ml) of 3-methoxybenzyl chloride (1.8 ml, 12.0 mmol) dissolved in THF. After 1 hour of stirring at the same temperature, the resulting reaction mixture was warmed to room temperature, mixed with saturated ammonium chloride aqueous solution (50 ml), extracted with ether and then washed with water and saturated brine. After drying the organic layer on anhydrous magnesium sulfate, the solvent was removed by evaporation to obtain 3.32 g (115%) of the title compound as a light yellow oily material which was used in the following reaction without further purification.
IR (neat): 2930, 2240, 1602, 1586, 1494, 1464, 1438, 1250, 1154, 1050, 1028, 754 cm⁻¹
NMR (CDCl₃) δ: 3.09 (2H, d, J = 7 Hz), 3.74, 3.83 (each 3H, s), 4.39 (1H, t, J = 7 Hz), 6.55 - 7.50 (8H, m)

### Reference Example 2

### 3-Methoxy-1-[2-(2-methoxyphenyl)-3-butenyl]benzene

In an atmosphere of argon and at room temperature, methyltriphenylphosphonium bromide (956 mg, 2.68 mmol) was suspended in THF (5 ml) to which was added dropwise tert-butoxy potassium (300 mg, 2.68 mmol) which has been dissolved in THF (5 ml). After 20 minutes of stirring at the same temperature, to this was added dropwise a solution (5 ml) of 2-(2-methoxyphenyl)-3-(3-methoxyphenyl)propanal (362 mg, 1.34 mmol) dissolved in THF. After 10 minutes of stirring at room temperature, the resulting reaction mixture was mixed with saturated ammonium chloride aqueous solution (10 ml) and extracted with ether. After drying the organic layer on anhydrous magnesium sulfate, the solvent was removed by evaporation to obtain a yellow oily material which was subsequently purified by a silica gel column chromatography (ether) to obtain 360 mg (100%) of the title compound as a light yellow oily material.
IR (neat): 2940, 1600, 1584, 1492, 1464, 1242, 1152, 1050, 752 cm⁻¹
NMR (CDCl₃) δ: 2.96 (2H, d, J = 7.5 Hz), 3.71 (6H, s), 4.07 (1H, d, J = 7.5 Hz, 7.5 Hz), 4.96 (1H, d, J = 18 Hz), 4.99 (1H, d, J = 10.5 Hz), 5.83 - 6.30 (1H, m), 6.55 - 7.30 (8H, m)

### Reference Example 3

### Methyl 4-(2-methoxyphenyl)-5-(3-methoxyphenyl)-2-pentenate

In an atmosphere of argon, 2-(2-methoxyphenyl)-3-(3-methoxyphenyl)propanal (90 mg, 0.333 mmol) was dissolved in toluene (3 ml). Methyl (triphenylphosphoranilidene)acetate (180 mg, 0.538 mmol) was added to the resulting solution and stirred at 60°C for 21 hours. After removing the solvent by evaporation, the resulting residue was purified by a silica gel column chromatography (ether:hexane = 1:8) to obtain 109 mg (100%) of the title compound as a colorless oily material.
IR (neat): 2948, 1722, 1650, 1600, 1586, 1492, 1464, 1436, 1314, 1262, 1242, 1154, 1048, 754 cm⁻¹
NMR (CDCl₃) δ: 3.04 (2H, d, J = 7.5 Hz), 3.66, 3.70, 3.73 (each 3H, s), 4.19 (1H, dt, J = 7.5 Hz, 7.5 Hz), 5.71 (1H, d, J = 16.5 Hz), 6.55 - 7.34 (9H, m)

### Reference Example 4

### N-(2-Methoxyphenyl)-3-pyridinesulfonamide

o-Anisidine (377 mg, 3.0 mmol) was dissolved in toluene (10 ml) to which were subsequently added pyridine (0.48 ml, 6.0 mmol) and 3-pyridinesulfonylchloride hydrochloride (642 mg, 3.0 mmol) at room temperature. After 1.5 hours of stirring at 100°C, the resulting reaction solution was mixed with water (20 ml), adjusted to pH 7 to 8 with anhydrous sodium carbonate, extracted with ethyl acetate and then washed with water and saturated brine. After drying on anhydrous sodium carbonate and removing the solvent by evaporation, the resulting orange solid was purified by a silica gel column chromatography (ether:hexane = 3:1) to obtain a light orange solid which was subsequently washed with an ether-hexane (1:3) mixture solution to obtain 662 mg (83.5%) of the title compound in the form of colorless prism crystals.
Melting point: 101.5 - 103°C
IR (KBr): 3008, 2712, 1586, 1498, 1420, 1336, 1320, 1280, 1256, 1194, 1110, 1020, 762, 744, 600, 578, 544 cm⁻¹
NMR (CDCl₃) δ: 3.58 (3H, s), 6.71 (1H, dd, J = 7.5 Hz, 2 Hz), 6.80 - 7.14 (3H, m), 7.31 (1H, dd, J = 8 Hz, 5 Hz), 7.54 (1H, dd, J = 7.5 Hz, 2 Hz), 7.96 (1H, dd, J = 8 Hz, 2 Hz), 8.70 (1H, dd, J = 5 Hz, 2 Hz), 8.93 (1H, d, J = 2 Hz)

### Reference Example 5

### N-(2-Methoxyphenyl)-p-toluenesulfonamide

o-Anisidine (2.34 ml, 20 mmol) was dissolved in toluene (60 ml) to which were subsequently added, with cooling in an ice bath, pyridine (4.58 ml, 60 mmol), p-toluenesulfonyl chloride (3.89 g, 20 mmol) and a catalytically effective amount of 4-dimethylaminopyridine. After 2.5 hours of stirring at room temperature, the resulting reaction solution was mixed with water (50 ml), extracted with ethyl acetate and then washed with water, 10% sodium hydroxide aqueous solution, 1 N hydrochloric acid, water and saturated brine in that order. After drying on anhydrous magnesium sulfate and removing the solvent by evaporation, the resulting orange solid was dissolved in ethyl acetate (50 ml), adsorbed to anhydrous magnesium sulfate (20 g), eluted with ether and then subjected to evaporation to remove the solvent. The resulting colorless solid which was then washed with an ether-hexane (1:1) mixture solution to obtain 3.44 g (62.0%) of the title compound in the form of colorless prism crystals.
Melting point: 126.5 - 128.5°C
IR (KBr): 3336, 1594, 1498, 1440, 1286, 1256, 1158, 1112, 1088, 1024, 822, 752, 660, 554, 534 cm⁻¹
NMR (CDCl₃) δ: 2.35 (3H, s), 3.63 (3H, s), 6.58 - 7.34 (7H, m), 7.35 - 7.75 (2H, m)

### Reference Example 6

### 4-Methoxy-N-(2-methoxyphenyl)benzenesulfonamide

Using o-anisidine (1.0 ml, 8.55 mmol) and 4-methoxybenzenesulfonyl chloride (1.78 g, 8.55 mmol), the procedure of Reference Example 5 was repeated to obtain 2.26 g (90.1%) of the title compound in the form of colorless powder.
Melting point: 85.5 - 87°C
IR (KBr): 3620, 3540, 3248, 1594, 1500, 1448, 1342, 1286, 1246, 1156, 1114, 1094, 1024, 910, 836, 754, 678, 582, 568, 546 cm⁻¹
NMR (CDCl₃) δ: 3.63, 3.77 (each, 3H, s), 6.55 - 7.15 (6H, m), 7.35 - 7.90 (3H, m)

### Reference Example 7

### 4-Fluoro-N-(2-methoxyphenyl)benzenesulfonamide

Using o-anisidine (1.0 ml, 8.55 mmol) and 4-fluorobenzenesulfonyl chloride (1.7 g, 8.55 mmol), the procedure of Reference Example 5 was repeated to obtain 2.24 g (93.1%) of the title compound in the form of colorless prism crystals.
Melting point: 101 - 102.5°C
IR (KBr): 3272, 1598, 1494, 1396, 1342, 1254, 1222, 1178, 1154, 1112, 1088, 840, 754, 690, 552, 538 cm⁻¹
NMR (CDCl₃) δ: 3.60 (3H, s), 6.60 - 7.25 (6H, m), 7.37 - 7.94 (3H, m)

### Reference Example 8

### N-(2-Methoxyphenyl)-3-nitrobenzenesulfonamide

Using o-anisidine (1.0 ml, 8.55 mmol) and 3-nitrobenzenesulfonyl chloride (1.95 g, 8.55 mmol), the procedure of Reference Example 5 was repeated to obtain 2.36 g (89.5%) of the title compound in the form of light yellow needle crystals.
Melting point: 130 - 131.5°C
IR (KBr): 3252, 1608, 1532, 1496, 1406, 1354, 1258, 1154, 1112, 746, 684, 668 cm⁻¹
NMR (CDCl₃) δ: 3.63 (3H, s), 6.65 - 7.15 (4H, m), 7.56 (1H, d, J = 7.5 Hz), 7.63 (1H, d, J = 7.5 Hz), 8.05 (1H, d, J = 7.5 Hz), 8.33 (1H, d, J = 7.5 Hz), 8.60 (1H, br-s)

### Reference Example 9

### 4-Methoxy-N-(2-thiazolyl)benzenesulfonamide

Using 2-aminothiazole (883 mg, 8.55 mmol) and 4-methoxybenzenesulfonyl chloride (1.78 g, 8.55 mmol), the procedure of Reference Example 5 was repeated to obtain 1.47 g (63.6%) of the title compound in the form of light yellow needle crystals.
Melting point: 162 - 164°C
IR (KBr): 1594, 1580, 1518, 1498, 1372, 1290, 1270, 1170, 1144, 1134, 1084, 834, 688, 656, 556 cm⁻¹
NMR (CDCl₃) δ: 3.84 (3H, s), 6.37 (1H, d, J = 5 Hz), 6.86 (1H, d, J = 9 Hz), 6.89 (1H, d, J = 9 Hz), 7.44 (1H, d, J = 5 Hz), 7.84 (1H, d, J = 9 Hz), 7.91 (1H, d, J = 9 Hz)

### Reference Example 10

### 4-Methoxy-N-(3-methoxyphenyl)benzenesulfonamide

Using m-anisidine (0.99 ml, 8.55 mmol) and 4-methoxybenzenesulfonyl chloride (1.78 g, 8.55 mmol), the procedure of Reference Example 5 was repeated to obtain 2.50 g (99.7%) of the title compound in the form of light yellow oil.
IR (neat): 3268, 1596, 1500, 1328, 1262, 1152, 1094, 834, 692, 570, 550 cm⁻¹
NMR (CDCl₃) δ: 3.72, 3.81 (each 3H, s), 6.45 - 7.40 (7H, m), 7.74 (2H, d, J = 9 Hz)

### Reference Example 11

### N-(2-Cyanophenyl)-4-methoxybenzenesulfonamide

Using 2-aminobenzonitrile (1.03 g, 8.55 mmol) and 4-methoxybenzenesulfonyl chloride (1.78 g, 8.55 mmol), the procedure of Reference Example 5 was repeated to obtain 1.57 g (63.7%) of the title compound in the form of colorless solid.
Melting point: 102 - 103°C
IR (KBr): 3252, 2228, 1596, 1578, 1496, 1456, 1416, 1340, 1310, 1180, 1162, 1092, 1024, 908, 834, 762, 670, 592 cm⁻¹
NMR (CDCl₃) δ: 3.83 (3H, s), 6.92 (2H, d, J = 9 Hz), 7.50 - 7.29 (2H, m), 7.35 - 7.90 (5H, m)

### Reference Example 12

### N-(2-Trifluoromethylphenyl)-4-methoxybenzenesulfonamide

Using 2-trifluoroaniline (1.39 g, 8.55 mmol) and 4-methoxybenzenesulfonyl chloride (1.78 g, 8.55 mmol), the procedure of Reference Example 5 was repeated to obtain 2.14 g (75.5%) of the title compound in the form of colorless solid.
Melting point: 92 - 93°C
IR (KBr): 3292, 1596, 1494, 1416, 1346, 1318, 1270, 1254, 1164, 1112, 1094, 1026, 834, 758, 670, 556 cm⁻¹
NMR (CDCl₃) δ: 3.82 (3H, s), 6.65 - 7.00 (3H, m), 7.03 - 7.30 (1H, m), 7.35 - 7.90 (5H, m)

### Reference Example 13

### 4-Methoxy-N-cyclohexylbenzenesulfonamide

Using cyclohexylamine (1.98 ml, 17.1 mmol) and 4-methoxybenzenesulfonyl chloride (1.78 g, 8.55 mmol), the procedure of Reference Example 5 was repeated to obtain 2.30 g (100%) of the title compound in the form of light yellow oil.
IR (neat): 3248, 2936, 2856, 1598, 1580, 1452, 1324, 1258,
1156, 1096, 1080, 1026, 834, 672, 576, 556 cm⁻¹
NMR (CDCl₃) δ: 0.76 - 1.95 (10H, m), 3.05 (1H, br-s), 3.85 (3H, s), 4.95 (1H, br-s), 6.95 (2H, d, J = 9 Hz), 7.83 (2H, d, J = 9 Hz)

### Reference Example 14

### 4-[(2-Methoxyanilino)sulfonyl]benzoic acid

Using o-anisidine (0.585 ml, 5.0 mmol) and 4-(chlorosulfonyl)benzoic acid (1.15 g, 5.0 mmol), the procedure of Reference Example 5 was repeated to obtain 1.47 g (95.5%) of the title compound in the form of pink powder.
Melting point: 202 - 205°C
IR (KBr): 3268, 1688, 1502, 1406, 1346, 1314, 1286, 1258, 1166, 744, 724 cm⁻¹
NMR (CDCl₃) δ: 3.57 (3H, s), 6.55 - 7.20 (3H, m), 7.25 - 7.60 (2H, m), 7.76 (2H, m, J = 8.5 Hz), 8.07 (2H, m, J = 8.5 Hz), 8.35 (1H, br-s)

### Reference Example 15

### 3-Methoxy-N-(2-methoxyphenyl)benzamide

Using o-anisidine (1.0 ml, 8.55 mmol) and m-anisoyl chloride (1.2 ml, 8.55 mmol), the procedure of Reference Example 5 was repeated to obtain 2.2 g (100%) of the title compound in the form of brown oil.
IR (neat): 3436, 2940, 1674, 1600, 1526, 1482, 1460, 1434, 1336, 1288, 1274, 1250, 1220, 1120, 1046, 1028, 748 cm⁻¹
NMR (CDCl₃) δ: 3.88, 4.02 (each 3H, s), 6.80 - 7.20 (5H, m), 7.30 - 7.55 (3H, m), 8.50 (1H, br-s)

### Reference Example 16

### 4-Cyano-N-(2-methoxyphenyl)benzamide

In an atmosphere of dry air, 4-cyanobenzoic acid (1.5 g, 10 mmol) was dissolved in benzene (5 ml), and DMF (0.1 ml) and thionyl chloride (2.2 ml, 30 mmol) were added dropwise to the resulting solution at room temperature, followed by 30 minutes of heating under reflux. After removing the solvent by evaporation, the resulting residue was subjected to azeotropy using benzene (10 ml x 2) to obtain a yellow solid which was subsequently dissolved in methylene chloride (10 ml) and mixed with o-anisidine (1.16 ml, 10 mmol) and 20% sodium hydroxide aqueous solution (4 ml) with cooling in an ice bath. After 20 minutes of stirring at the same temperature, the resulting mixture was extracted with methylene chloride and then washed with 1 N hydrochloric acid, water and saturated brine in that order. After drying on anhydrous magnesium sulfate, the solvent was removed by evaporation, and the resulting light yellow solid was purified by recrystallization (methylene chloride-ether) to obtain 2.36 g (93.6%) of the title compound in the form of cream crystals.
Melting point: 157.5 - 160°C
IR (KBr): 3312, 2228, 1666, 1644, 1600, 1534, 1486, 1460, 1434, 1334, 1288, 1256, 1218, 1022, 742 cm⁻¹
NMR (CDCl₃) δ: 3.93 (3H, s), 6.81 - 7.20 (3H, m), 7.77 (2H, d, J = 8.5 Hz), 7.98 (2H, d, J = 8.5 Hz), 8.32 - 8.58 (2H, m)

### Reference Example 17

### 4-[(2-Methoxyanilino)carbonyl]pyridine

Using o-anisidine (2.34 g, 19.0 mmol) and 4-carboxypyridine (2.29 g, 19.0 mmol), the procedure of Reference Example 16 was repeated to obtain 4.22 g (97.2%) of the title compound in the form of colorless powder.
Melting point: 79 - 80°C
IR (KBr): 3316, 1665, 1596, 1533, 1485, 1464, 1440, 747 cm⁻¹
NMR (CDCl₃) δ: 3.92 (3H, s), 6.85 - 7.16 (3H, m), 7.72 (2H, d, J = 6.0 Hz), 8.36 - 8.60 (2H, m), 8.78 (2H, d, J = 6.0 Hz)

### Reference Example 18

### 4-Chloromethyl-N-(2-methoxyphenyl)benzamide

o-Anisidine (0.69 ml, 6.0 mmol) was dissolved in methylene chloride (10 ml) and, with cooling in an ice bath, mixed with 20% sodium hydroxide (5 ml) and 4-chloromethylbenzoyl chloride (1.17 g, 6.0 mmol). After 30 minutes of stirring at the same temperature, the reaction mixture was extracted with methylene chloride and then washed with 1 N hydrochloric acid, water and saturated brine in that order. After drying on anhydrous magnesium sulfate, the solvent was removed by evaporation, and the resulting light beige solid was purified by recrystallization (ether=hexane) to obtain 1.56 g (94.2%) of the title compound in the form of colorless needle crystals.
Melting point: 101 - 103°C
IR (KBr): 3448, 1668, 1600, 1536, 1510, 1484, 1460, 1438, 1344, 1290, 1248, 1220, 1022, 750, 702, 592, 556 cm⁻¹
NMR (CDCl₃) δ: 3.93 (3H, s), 4.64 (2H, s), 6.80 - 7.16 (3H, m), 7.51 (2H, d, J = 8.5 Hz), 7.98 (2H, d, J = 8.5 Hz), 8.33 - 8.66 (2H, m)

### Reference Example 19

### 3-Methoxy-N-(2-pyridyl)benzamide

Using 2-aminopyridine (670 mg, 7.12 mmol) and m-anisoyl chloride (1.2 ml, 8.55 mmol), the procedure of Reference Example 18 was repeated to obtain 341 mg (21.0%) of the title compound in the form of light yellow oil.
IR (neat): 3250, 1676, 1580, 1526, 1488, 1464, 1432, 1306, 1272, 1226, 1042, 778 cm⁻¹
NMR (CDCl₃) δ: 3.80 (3H, s), 6.85 - 7.17 (2H, m), 7.20 - 7.57 (3H, m), 7.72 (1H, dd, J = 7.5 Hz, 7.5 Hz), 8.11 (1H, br-s), 8.40 (1H, d, J = 8 Hz), 9.37 (1H, br-s)

### Reference Example 20

### 4-Chloromethyl-N-(3-methoxybenzyl)benzamide

Using 3-methoxtbenzylamine (754 mg, 5.39 mmol) and 4-chloromethylbenzoyl chloride (1.17 g, 6.0 mmol), the procedure of Reference Example 18 was repeated to obtain 1.31 g (83.9%) of the title compound in the form of colorless crystals.
Melting point: 108 - 109.5°C
IR (KBr): 3292, 1634, 1612, 1584, 1572, 1552, 1438, 1304, 1266, 1234, 1052, 784, 740, 700, 678 cm⁻¹
NMR (CDCl₃) δ: 3.78 (3H, s), 4.57 (2H, s), 4.62 (2H, d, J = 5 Hz), 6.16 - 6.55 (1H, m), 6.66 - 7.03 (3H, m), 7.10 - 7.31 (1H, m), 7.43 (2H, d, J = 8 Hz), 7.78 (2H, d, J = 8 Hz)

### Reference Example 21

### 4-Chloromethyl-N-(2-methoxyethyl)benzamide

Using 2-methoxyethylamine (0.53 ml, 6.0 mmol) and 4-chloromethylbenzoyl chloride (780 mg, 4.0 mmol), the procedure of Reference Example 18 was repeated to obtain 878 mg (96.4%) of the title compound in the form of colorless crystals.
Melting point: 125 - 126°C
IR (KBr): 3348, 2900, 1638, 1560, 1510, 1446, 1340, 1296, 1266, 1156, 1122, 1114, 956, 672, 640 cm⁻¹
NMR (CDCl₃) δ: 3.39 (3H, s), 3.46 - 3.79 (4H, m), 4.60 (2H, s), 6.50 (1H, br-s), 7.43 (2H, d, J = 8 Hz), 7.76 (2H, d, J = 8 Hz)

### Reference Example 22

### 4-Nitro-N-(2-methoxyphenyl)benzamide

With cooling in an ice bath, 1-(3-dimethylaminopropyl)-3-ethylcarbodiimide hydrochloride (EDC·HCl) (630 mg, 3.29 mmol) was added to 15 ml of methylene chloride solution containing o-anisidine (368 mg, 2.99 mmol) and 4-nitrobenzoic acid (500 mg, 2.99 mmol). After 1 hour of stirring at the same temperature, the reaction mixture was roughly adjusted to pH 8 with saturated sodium bicarbonate aqueous solution, extracted with methylene chloride and then washed with water and saturated brine in that order. After drying on anhydrous magnesium sulfate, the solvent was removed by evaporation, and the resulting solid material was purified by silica gel column chromatography (methylene chloride) to obtain 712 mg (87.4%) of the title compound in the form of yellow powder.
Melting point: 146 - 147°C
IR (KBr): 3322, 1647, 1599, 1545, 1521, 1461, 1338, 1266, 741 cm⁻¹
NMR (CDCl₃) δ: 3.93 (3H, s), 6.81 - 7.18 (3H, m), 8.03 (2H, d, J = 10.0 Hz), 8.34 (2H, d, J = 10.0 Hz), 8.40 (1H, m)

### Reference Example 23

### 4-Nitro-N-(3-methoxyphenyl)benzamide

Using m-anisidine (2.50 g, 15.0 mmol) and 4-nitrobenzoic acid (2.03 g, 16.5 mmol), the procedure of Reference Example 22 was repeated to obtain 3.80 g (93.2%) of the title compound in the form of light yellow needle crystals.
Melting point: 189 - 191°C
IR (KBr): 3316, 1642, 1598, 1536, 1522, 1434, 1320, 1296, 1162, 1046, 970 cm⁻¹
NMR (CDCl₃) δ: 3.83 (3H, s), 6.60 - 6.80 (1H, m), 7.10 - 7.43 (4H, m), 8.05 (2H, d, J = 9.0 Hz), 8.33 (2H, d, J = 9.0 Hz)

### Reference Example 24

### 4-Nitro-N-(4-methoxyphenyl)benzamide

Using p-anisidine (492.6 mg, 4.0 mmol) and 4-nitrobenzoic acid (668.5 mg, 4.0 mmol), the procedure of Reference Example 22 was repeated to obtain 904.9 mg (83.2%) of the title compound in the form of light yellow powder.
Melting point: 199 - 200°C
IR (KBr): 3296, 1644, 1600, 1530, 1516, 1462, 1348, 1322, 1302, 1248, 1104, 1028, 872, 828, 704, 690 cm⁻¹
NMR (CDCl₃) δ: 3.82 (3H, s), 6.62 (2H d, J = 9.0 Hz), 7.53 (2H, d, J = 9.0 Hz), 8.01 (2H, d, J = 8.7 Hz), 8.33 (2H, d, J = 8.7 Hz)

### Reference Example 25

### 4-Nitro-N-(2,5-di-methoxyphenyl)benzamide

Using 2,5-dimethoxyaniline (2.53 g, 16.5 mmol) and 4-nitrobenzoic acid (2.51 g, 15.0 mmol), the procedure of Reference Example 22 was repeated to obtain 3.50 g (77.2%) of the title compound in the form of orange needle crystals.
Melting point: 184 - 186°C
IR (KBr): 3424, 1686, 1604, 1536, 1346, 1220, 1042, 850, 614 cm⁻¹
NMR (CDCl₃) δ: 3.83 (3H, s), 3.91 (3H, s), 6.65 (1H, dd, J = 2.9 Hz, 9.0 Hz), 6.85 (1H, d, J = 9.0 Hz), 8.06 (2H, dd, J = 7.0 Hz, 2.0 Hz), 8.21 - 8.65 (3H, m)

### Reference Example 26

### 3-Nitro-N-(3-methoxyphenyl)benzamide

Using m-anisidine (2.03 mg, 16.5 mmol) and 3-nitrobenzoic acid (2.51 g, 15.0 mmol), the procedure of Reference Example 22 was repeated to obtain 3.72 g (91.3%) of the title compound in the form of colorless powder.
Melting point: 120 - 121.5°C
IR (KBr): 3300, 1648, 1608, 1600, 1534, 1452, 1432, 1358, 1272, 1156 cm⁻¹
NMR (CDCl₃) δ: 3.83 (3H, s), 6.66 - 6.83 (1H, m), 7.06 - 7.33 (3H, m), 7.33 - 7.45 (1H, m), 7.68 (1H, dd, each J = 9.0 Hz), 8.20 - 8.46 (2H, m), 8.70 - 8.88 (1H, m)

### Reference Example 27

### 4-Nitro-N-(2,4-dimethoxyphenyl)benzamide

Using 2,4-dimethoxyaniline (2.53 g, 16.0 mmol) and 4-nitrobenzoic acid (2.50 g, 14.8 mmol), the procedure of Reference Example 22 was repeated to obtain 3.72 g (91.3%) of the title compound in the form of yellow needle crystals.
Melting point: 172 - 174°C
IR (KBr): 1680, 1522, 1502, 1422, 1342, 1286, 1252, 1212, 1156, 1136, 1032, 852, 836, 708, 550 cm⁻¹
NMR (CDCl₃) δ: 3.82 (3H, s), 3.91 (3H, s), 6.51- 6.85 (2H, m), 7.76 - 8.29 (1H, m), 8.03 (2H, dd, each J = 8.8 Hz), 8.34 (2H, d, J = 8.8 Hz), 8.39 (1H, d, J = 4.2 Hz)

### Reference Example 28

### 4-Nitro-N-(3-methylphenyl)benzamide

Using m-toluidine (1.61 g, 15.0 mmol) and 4-nitrobenzoic acid (2.76 g, 16.5 mmol), the procedure of Reference Example 22 was repeated to obtain 3.34 g (84.3%) of the title compound in the form of colorless needle crystals.
Melting point: 140.5 - 146.5°C
IR (KBr): 3298, 1641, 1599, 1533, 1515, 1449, 1347, 1320, 1302, 1260, 867, 708 cm⁻¹
NMR (CDCl₃) δ: 2.37 (3H, s) 6.87 - 7.53 (4H, m), 7.64 - 8.10 (1H, m), 7.99 (2H, dd, each J = 9.0 Hz), 8.32 (2H, d, J = 9.0 Hz)

### Reference Example 29

### 4-Nitro-N-(phenyl)benzamide

Using aniline (1.50 ml, 16.5 mmol) and 4-nitrobenzoic acid (1.77 g, 10.5 mmol), the procedure of Reference Example 22 was repeated to obtain 2.48 g (97.5%) of the title compound in the form of colorless powder.
Melting point: 90 - 93°C
IR (KBr): 3324, 1652, 1598, 1530, 1494, 1440, 1348, 1324, 1300, 1264, 852, 758, 722, 694 cm⁻¹
NMR (CDCl₃) δ: 7.25 (1H, t, J = 5.7 Hz), 7.41 (2H, dd, J = 5.7 Hz), 7.64 (2H, d, J = 7.9 Hz), 7.68 - 7.99 (1H, s), 8.03 (2H, d, J = 8.8 Hz), 8.35 (2H, d, J = 8.8 Hz)

### Reference Example 30

### 4-Nitro-N-(3-methylthiophenyl)benzamide

Using 3-methylthioaniline (1.40 ml, 11.0 mmol) and 4-nitrobenzoic acid (1.67 g, 10.0 mmol), the procedure of Reference Example 22 was repeated to obtain 2.46 g (85.2%) of the title compound in the form of yellow powder.
Melting point: 158 - 161°C
IR (KBr): 3280, 1648, 1598, 1540, 1518, 1476, 1344, 1326, 1310, 1300, 1264, 868, 848, 716, 684 cm⁻¹
NMR (CDCl₃) δ: 2.51 (3H, s), 7.08 - 7.35 (3H, m), 7.63 (1H, s), 7.81 (1H, s), 8.02 (2H, d, J = 8.8 Hz), 8.35 (2H, d, J = 8.8 Hz)

### Reference Example 31

### 4-Nitro-N-(2-trifluoromethylphenyl)benzamide

Using 2-trifluoromethylaniline (1.77 g, 11.0 mmol) and 4-nitrobenzoic acid (1.67 g, 10.0 mmol), the procedure of Reference Example 22 was repeated to obtain 474 mg (15.3%) of the title compound in the form of colorless powder.
Melting point: 125.6 - 126.1°C
IR (KBr): 3308, 1660, 1524, 1348, 1318, 1294, 1164, 1114, 762 cm⁻¹
NMR (CDCl₃) δ: 7.53 (1H, d, J = 7.7 Hz), 7.66 (2H, t, J = 7.7 Hz), 7.98 - 8.43 (5H, m)

### Reference Example 32

### 4-Nitro-N-(3-trifluoromethylphenyl)benzamide

Using 3-trifluoromethylaniline (1.77 g, 11.0 mmol) and 4-nitrobenzoic acid (1.67 g, 10.0 mmol), the procedure of Reference Example 22 was repeated to obtain 2.48 g (80.0%) of the title compound in the form of colorless powder.
Melting point: 209.6 - 210.3°C
IR (KBr): 3304, 1658, 1652, 1350, 1332, 714, 698 cm⁻¹
NMR (CDCl₃) δ: 7.50 (1H, dd, J = 2.0 Hz, 6.8 Hz), 7.83 - 8.09 (4H, m), 8.36 (2H, m, J = 2.0 Hz, 6.8 Hz)

### Reference Example 33

### 4-Nitro-N-(3-pyridyl)benzamide

Using 3-aminopyridine (1.55 g, 16.5 mmol) and 4-nitrobenzoic acid (2.5 g, 15.0 mmol), the procedure of Reference Example 22 was repeated to obtain 1.81 g (49.6%) of the title compound in the form of colorless powder.
Melting point: 191 - 194.5°C
IR (KBr): 3305, 3025, 1665, 1602, 1539, 1521, 1422, 1353, 1305, 1281, 1236, 711 cm⁻¹
NMR (CDCl₃-CD₃OD) δ: 7.15 - 7.47 (2H, m), 8.08 (2H, d, J = 9.0 Hz), 8.36 (2H, d, J = 9.0 Hz), 8.19 - 8.54 (2H, m), 8.58 - 8.82 (1H, m)

### Reference Example 34

### 2-Nitro-N-(3-methoxyphenyl)benzamide

Using m-anisidine (2.03 g, 16.5 mmol) and 2-nitrobenzoic acid (2.51 g, 15.0 mmol), the procedure of Reference Example 22 was repeated to obtain 3.47 g (84.9%) of the title compound in the form of light yellow needle crystals.
Melting point: 156 - 158°C
IR (KBr): 3252, 1656, 1612, 1598, 1488, 1470, 1350, 1266, 1202, 1030 cm⁻¹
NMR (CDCl₃-CD₃OD) δ: 3.80 (3H, s), 6.58 - 6.80 (1H, m), 7.00 - 7.40 (3H, m), 7.43 - 7.75 (4H, m), 7.96 - 8.18 (1H, m)

### Reference Example 35

### 4-Nitro-N-(3-nitrophenyl)benzamide

Using 3-nitroaniline (2.76 g, 16.5 mmol) and 4-nitrobenzoic acid (2.51 g, 15.0 mmol), the procedure of Reference Example 22 was repeated to obtain 3.58 g (83.2%) of the title compound in the form of light yellow crystals.
Melting point: 229.2 - 232°C
IR (KBr): 3394, 3106, 3076, 1680, 1602, 1548, 1518, 1428, 1344, 1281, 1086, 870 cm⁻¹
NMR (CDCl₃-CD₃OD) δ: 7.40 - 7.83 (3H, m), 8.11 (2H, d, J = 9.0 Hz), 8.36 (2H, d, J = 9.0 Hz), 7.92 - 8.60 (2H, m)

### Reference Example 36

### 4-Nitro-N-(2-methoxycarbonylphenyl)benzamide

Using methyl anthranilate (3.02 g, 20.0 mmol) and 4-nitrobenzoic acid (3.67 g, 22.0 mmol), the procedure of Reference Example 22 was repeated to obtain 4.20 g (72.9%) of the title compound in the form of light yellow prism crystals.
Melting point: 196 - 199°C
IR (KBr): 3368, 1676, 1606, 1520, 1346, 1276, 758, 697 cm⁻¹
NMR (CDCl₃) δ: 3.99 (3H, s), 7.18 (1H, t, J = 7.3 Hz), 7.64 (1H, t, J = 7.3 Hz), 8.06 - 8.44 (5H, m), 8.89 (1H, d, J = 8.4 Hz)

### Reference Example 37

### 4-Cyano-N-(4-methoxyphenyl)benzamide

Using p-anisidine (1.35 g, 11.0 mmol) and 4-cyanobenzoic acid (1.47 g, 10.0 mmol), the procedure of Reference Example 22 was repeated to obtain 2.31 g (91.7%) of the title compound in the form of colorless needle crystals.
Melting point: 154.6 - 157.3°C
IR (KBr): 3440, 2945, 1646, 1348, 1258, 973, 850, 715, 601 cm⁻¹
NMR (CDCl₃) δ: 3.82 (3H, s), 6.91 (1H, t, J = 9.0 Hz), 7.51 (2H, d, J = 9.0 Hz), 7.77 (2H, d, J = 8.6 Hz), 7.94 (2H, d, J = 8.6 Hz)

### Reference Example 38

### 3-Methyl-4-nitro-N-(4-methoxyphenyl)benzamide

Using p-anisidine (738 mg, 6.0 mmol) and 3-methyl-4-nitrobenzoic acid (906 mg, 5.0 mmol), the procedure of Reference Example 22 was repeated to obtain 1.33 g (93.0%) of the title compound in the form of yellow powder.
Melting point: 167.8 - 169.1°C
IR (KBr): 3276, 1642, 1534, 1514, 1356, 1248, 1031, 826, 713, 522 cm⁻¹
NMR (CDCl₃) δ: 2.63 (3H, s), 3.81 (3H, s), 6.90 (2H, dd, J = 2.2 Hz, 6.8 Hz), 7.42 (2H, dd, J = 2.2 Hz, 6.8 Hz), 7.71 - 8.04 (3H, m)

### Reference Example 39

### 3-Methoxy-4-nitro-N-(4-methoxyphenyl)benzamide

Using p-anisidine (738 mg, 6.0 mmol) and 3-methoxy-4-nitrobenzoic acid (986 mg, 5.0 mmol), the procedure of Reference Example 22 was repeated to obtain 1.40 g (92.7%) of the title compound in the form of colorless powder.
Melting point: 168 - 169.3°C
IR (KBr): 3312, 1650, 1526, 1514, 1238, 1028, 803, 680 cm⁻¹
NMR (CDCl₃) δ: 3.82 (3H, s), 4.01 (3H, s), 6.92 (2H, d, J = 9.0 Hz), 7.31 - 7.65 (3H, m), 7.84 (2H, d, J = 8.1 Hz)

### Reference Example 40

### 4-Nitro-N-(3-methylphenyl)benzamide

Using p-toluidine (1.19 g, 11.0 mmol) and 4-nitrobenzoic acid (1.77 g, 10.5 mmol), the procedure of Reference Example 22 was repeated to obtain 2.68 g (99.6%) of the title compound in the form of light yellow needle crystals.
Melting point: 140.5 - 146.5°C
IR (KBr): 3298, 1641, 1599, 1533, 1515, 1449, 1347, 1320, 1302, 1260, 867, 708 cm⁻¹
NMR (CDCl₃) δ: 2.37 (3H, s), 6.87 - 7.53 (4H, m), 7.64 - 8.10 (1H, m), 7.99 (2H, d, J = 9.0 Hz), 8.32 (2H, d, J = 9.0 Hz)

### Reference Example 41

### 4-Nitro-N-(4-fluorophenyl)benzamide

Using 4-fluoroaniline (1.0 ml, 9.93 mmol) and 4-nitrobenzoic acid (1.58 g, 9.36 mmol), the procedure of Reference Example 22 was repeated to obtain 1.60 g (65.8%) of the title compound in the form of light yellow needle crystals.
Melting point: 143 - 146°C
IR (KBr): 3280, 1648, 1598, 1554, 1524, 1504, 1348, 1322, 1244, 1212, 838, 824 cm⁻¹
NMR (CDCl₃) δ: 7.09 (2H, t, J = 8.8 Hz), 7.63 (2H, dd, J = 4.6, 8.8 Hz), 8.05 (2H, d, J = 8.6 Hz), 8.10 (1H, s), 8.34 (2H, d, J = 8.6 Hz)

### Reference Example 42

### 3-Nitro-N-(phenyl)benzamide

Using aniline (1.50 ml, 16.5 mmol) and 3-nitrobenzoic acid (2.64 g, 15.0 mmol), the procedure of Reference Example 22 was repeated to obtain 2.84 g (78.2%) of the title compound in the form of light yellow needle crystals.
Melting point: 153 - 154°C
IR (KBr): 1654, 1600, 1528, 1494, 1444, 1348, 1326, 1304, 1260, 756, 714 cm⁻¹
NMR (CDCl₃) δ: 7.19 - 7.48 (2H, m), 7.60 - 7.79 (4H, m), 7.86 (1H, brs), 8.25 (1H, d, J = 7.7 Hz), 8.40 (1H, d, J = 7.7 Hz), 8.69 (1H, s)

### Reference Example 43

### 3-Nitro-N-(4-methylphenyl)benzamide

Using p-toluidine (1.19 g, 11.0 mmol) and 3-nitrobenzoic acid (1.76 g, 10.0 mmol), the procedure of Reference Example 22 was repeated to obtain 2.27 g (88.3%) of the title compound in the form of colorless needle crystals.
Melting point: 160 - 162°C
IR (KBr): 3304, 1648, 1522, 1350, 1322, 814 cm⁻¹
NMR (CDCl₃) δ: 2.37 (3H, s), 7.18 - 7.28 (3H, m), 7.51 - 7.81 (4H, m), 8.28 (1H, d, J = 8.6 Hz), 8.43 (1H, d, J = 8.6 Hz), 8.72 (1H, s)

### Reference Example 44

### 3-Nitro-N-(4-fluorophenyl)benzamide

Using 4-fluoroaniline (1.1 ml, 10.9 mmol) and 3-nitrobenzoic acid (1.76 g, 10.0 mmol), the procedure of Reference Example 22 was repeated to obtain 1.98 g (76.0%) of the title compound in the form of light yellow needle crystals.
Melting point: 167 - 168°C
IR (KBr): 3312, 1650, 1614, 1528, 1504, 1408, 1350, 1322, 1264, 1240, 1210, 1098, 832, 712, 518 cm⁻¹
NMR (CDCl₃) δ: 6.98 - 7.18 (3H, m), 7.53 - 7.79 (3H, m), 7.79 - 7.98 (1H, m), 8.24 (1H, d, J = 7.8 Hz), 8.40 (1H, d, J = 7.8 Hz), 8.68 (1H, s)

### Reference Example 45

### 3-Nitro-N-(3-methylthiophenyl)benzamide

Using 3-methylthioaniline (2.1 ml, 16.5 mmol) and 3-nitrobenzoic acid (2.64 g, 15.0 mmol), the procedure of Reference Example 22 was repeated to obtain 4.09 g (94.6%) of the title compound in the form of light yellow needle crystals.
Melting point: 145°C
IR (KBr): 3300, 1658, 1596, 1528, 1476, 1432, 1404, 1350, 1316, 1304, 832 cm⁻¹
NMR (CDCl₃) δ: 2.51 (3H, s), 7.04 - 7.35 (3H, m), 7.62 (1H, s), 7.73 (1H, d, J = 8.7 Hz), 7.70 - 7.98 (1H, m), 8.24 (1H, d, J = 7.8 Hz), 8.40 (1H, d, J = 7.8 Hz), 8.68 (1H, s)

### Reference Example 46

### 4-Nitro-N-(3,4-dimethylphenyl)benzamide

Using 3,4-xylidine (1.35 g, 11.0 mmol) and 4-nitrobenzoic acid (1.69 g, 10.0 mmol), the procedure of Reference Example 22 was repeated to obtain 2.70 g (quantitative) of the title compound in the form of light yellow crystals.
Melting point: 221 - 223°C
IR (KBr): 3292, 1650, 1600, 1540, 1514, 1500, 1414, 1342, 1326, 1312, 1300, 1288, 1262, 848, 812, 706 cm⁻¹
NMR (CDCl₃) δ: 2.27 (6H, s), 7.09 - 7.41 (3H, m), 7.80 - 7.81 (1H, m), 8.02 (2H, d, J = 8.6 Hz), 8.33 (2H, d, J = 8.6 Hz)

### Reference Example 47

### 4-Nitro-N-(3,5-dimethylphenyl)benzamide

Using 3,5-xylidine (1.42 ml, 11.0 mmol) and 4-nitrobenzoic acid (1.76 g, 10.4 mmol), the procedure of Reference Example 22 was repeated to obtain 2.72 g (quantitative) of the title compound in the form of light yellow crystals.
Melting point: 206 - 210°C
IR (KBr): 3296, 1648, 1618, 1600, 1562, 1522, 1462, 1344, 1326, 1290, 1254, 864, 850, 838, 704, 682 cm⁻¹
NMR (CDCl₃) δ: 2.33 (6H, s), 6.84 (1H, s), 7.26 (2H, s), 8.00 (2H, d, J = 8.8 Hz), 8.33 (2H, d, J = 8.8 Hz)

### Reference Example 48

### 4-Nitro-N-(4-chlorophenyl)benzamide

Using 4-chloroaniline (1.42 g, 11.0 mmol) and 4-nitrobenzoic acid (1.76 g, 10.4 mmol), the procedure of Reference Example 22 was repeated to obtain 2.84 g (98.5%) of the title compound in the form of yellow crystals.
Melting point: 233 - 235°C
IR (KBr): 3424, 1680, 1604, 1532, 1512, 1492, 1394, 1346, 1328, 1310, 1300, 1248 cm⁻¹
NMR (CDCl₃) δ: 7.36 (2H, d, J = 9.0 Hz), 7.67 (2H, d, J = 9.0 Hz), 7.84 - 7.88 (1H, m), 8.04 (2H, d, J = 8.8 Hz), 8.35 (2H, d, J = 8.8 Hz)

### Reference Example 49

### 4-Nitro-N-(3,4-methylenedioxyphenyl)benzamide

Using 3,4-methylenedioxyaniline (1.44 g, 10.5 mmol) and 4-nitrobenzoyl chloride (1.86 g, 10.0 mmol), the procedure of Reference Example 16 was repeated to obtain 2.49 g (87.2%) of the title compound in the form of yellow crystals.
Melting point: 231 - 234°C
IR (KBr): 3316, 1650, 1599, 1533, 1512, 1494, 1452, 1344, 1320, 1278, 1245, 1200, 1035, 924, 864, 852 cm⁻¹
NMR (CDCl₃) δ: 5.98 (2H, s), 6.80 (2H, d, J = 8.1 Hz), 7.06 (1H, dd, J = 8.1 Hz, 1.9 Hz), 8.07 (2H, d, J = 8.7 Hz), 8.32 (2H, d, J = 8.7 Hz)

### Reference Example 50

### 4-Nitro-N-(1-naphtyl)benzamide

Using 1-aminonaphthalene (751.7 mg, 5.25 mmol) and 4-nitrobenzoyl chloride (927.9 mg, 5.00 mmol), the procedure of Reference Example 16 was repeated to obtain 1.57 g (quantitative) of the title compound in the form of light yellow crystals.
Melting point: 197 - 203°C
IR (KBr): 3232, 3064, 1644, 1602, 1545, 1518, 1485, 1443, 1404, 1344, 1320, 1287, 1104, 864, 849, 789, 762 cm⁻¹
NMR (CDCl₃) δ: 7.40 - 7.63 (3H, m), 7.70 - 8.00 (4H, m), 8.14 (2H, d, J = 8.0 Hz), 8.36 (2H, d, J = 8.0 Hz)

### Reference Example 51

### 3-[(2-methoxyanilino)sulfonyl]pyridine-N-oxide

N-(2-Methoxyphenyl)-3-pyridine sulfonamide (132 mg, 0.5 mmol) was dissolved in methylene chloride (5 ml), and m-chloroperbenzoic acid (185 mg, 70%, 0.75 mmol) was added to the solution at room temperature. After 7 hours of stirring at the same temperature, the resulting reaction solution was mixed with saturated sodium thiosulfate aqueous solution (5 ml) and saturated sodium bicarbonate aqueous solution (5 ml), saturated with sodium chloride and then extracted with ethyl acetate. After drying on anhydrous sodium carbonate, the solvent was removed by evaporation, and the resulting yellow solid material was washed with ethyl acetate to obtain 124 mg (88.5%) of the title compound in the form of cream powder.
IR (KBr): 3000, 2710, 1605, 1496, 1446, 1426, 1342, 1290, 1260, 1236, 1168, 1158, 1110, 606, 592 cm⁻¹
NMR (CDCl₃-CD₃OD) δ: 3.66 (3H, s), 6.70 - 7.65 (6H, m), 8.26 (1H, d, J = 6 Hz), 8.60 (1H, br-s)

### Reference Example 52

### 4-[(2-methoxyanilino)carbonyl]pyridine-N-oxide

Using 4-{(2-methoxyanilino)carbonyl}pyridine (500 mg, 2.19 mmol) and 89% m-chloroperbenzoic acid (637 mg, 3.28 mmol), the procedure of Reference Example 23 was repeated to obtain 501 mg (93.6%) of the title compound in the form of colorless powder.
Melting point: 182 - 184°C
IR (KBr): 3610, 2998, 1665, 1614, 1596, 1536, 1503, 1479, 1464, 1260, 1176 cm⁻¹
NMR (CDCl₃) δ: 3.94 (3H, s), 6.83 - 7.19 (3H, m), 7.76 (2H, d, J = 6.0 Hz), 8.27 (2H, d, J = 6.0 Hz), 8.35 - 8.55 (2H, m)

### Reference Example 53

### 4-Hydroxymethyl-N-(2-methoxyphenyl)benzenesulfonamide

In an atmosphere of argon and with cooling in an ice bath, 4-[(2-methoxyanilino)sulfonyl]benzoic acid (150 mg, 0.488 mmol) was dissolved in THF (5 ml), to which were subsequently added dropwise triethylamine (0.14 ml, 1.0 mmol) and ethyl chloroformate (50 µl, 0.509 mmol). After 30 minutes of stirring at the same temperature, the thus formed precipitate was removed by filtration, and the resulting filtrate was concentrated. The thus obtained residue was dissolved in 5 ml of THF, and, with cooling in an ice bath, sodium borohydride (46.5 mg, 1.22 mmol) and water (1 ml) were added to the resulting solution. After 40 minutes of stirring at the same temperature, the reaction mixture was adjusted to pH 4 to 5 with 2 N hydrochloric acid and extracted with ether. The organic layer was washed with saturated brine and dried on anhydrous magnesium sulfate. After removing the solvent by evaporation, the resulting colorless oily material was purified by silica gel column chromatography (ether) to obtain 99.6 mg (69.6%) of the title compound in the form of colorless solid.
Melting point: 101 - 104°C
IR (KBr): 3528, 3180, 1598, 1502, 1448, 1412, 1332, 1254, 1182, 1154, 1114, 1088, 1052, 922, 754, 590, 544 cm⁻¹
NMR (CDCl₃) δ: 1.79 (1H, t, J = 6 Hz), 3.63 (3H, s), 4.72 (2H, d, J = 6 Hz), 6.72 (1H, dd, J = 8 Hz, 2 Hz), 6.83 - 7.16 (3H, m), 7.38 (2H, d, J = 9 Hz), 7.51 (1H, dd, J = 8 Hz, 2 Hz), 7.74 (2H, d, J = 9 Hz)

### Reference Example 54

### 4-Methoxymethyl-N-(2-methoxyphenyl)benzamide

4-Chloromethyl-N-(2-methoxyphenyl)benzamide (138 mg, 0.50 mmol) was dissolved in a methanol-THF (1:1) mixture solution (6 ml) to which was subsequently added 10% sodium hydroxide aqueous solution (3 ml) at room temperature. After 14 hours of stirring at the same temperature, the reaction solution was subjected to 3 hours of ultrasonic treatment. After removing the organic solvent by evaporation, the resulting residue was extracted with methylene chloride and dried on anhydrous magnesium sulfate. After removing the solvent by evaporation, the resulting colorless oily material was purified by silica gel column chromatography (ether:hexane = 1:2) to obtain 126 mg (92.9%) of the title compound in the form of colorless oil.
IR (neat): 3440, 2930, 1674, 1602, 1530, 1510, 1482, 1460, 1434, 1252, 1102, 748 cm⁻¹
NMR (CDCl₃) δ: 3.41 (3H, s), 3.92 (3H, s), 4.52 (2H, s), 6.80 - 7.14 (3H, m), 7.45 (2H, d, J = 8.5 Hz), 7.88 (2H, d, J = 8.5 Hz), 8.35 - 8.68 (2H, m)

### Reference Example 55

### 3-Hydroxymethyl-N-(2-methoxyphenyl)benzamide

N-Bromosuccinimide (1.29 g, 7.2 mmol) and AIBN (50 mg, 0.30 mmol) were suspended in carbon tetrachloride (80 ml), mixed with m-toluyl chloride (0.8 ml, 6.0 mmol) and then heated under reflux for 4 hours while exposing to light. After concentrating the reaction solution to 1/3 volume, insoluble materials were removed by filtration. To the resulting filtrate cooled in an ice bath were added dropwise o-anisidine (0.69 ml, 6 mmol) and 20% sodium hydroxide aqueous solution (5 ml) in that order. The resulting reaction mixture was stirred for 20 minutes at room temperature, extracted with methylene chloride and then washed with 10% citric acid aqueous solution, water and saturated brine in that order. After drying on anhydrous magnesium sulfate and removing the solvent by evaporation, the resulting light beige solid material was subjected to silica gel column chromatography (ether:hexane = 1:1) to obtain creamy powder. To this were added precipitated calcium carbonate (2.34 g, 23.4 mmol) and a dioxane-water (1:1) mixture solution (20 ml), followed by 6 hours of heating under reflux. After adding THF (50 ml) and filtering off the formed insoluble materials, the organic solvent was removed by evaporation. The resulting residue was extracted with ethyl acetate, and the organic layer was washed with saturated brine and dried on anhydrous magnesium sulfate, followed by the removal of the solvent by evaporation to obtain an orange oily material. This was dissolved in methanol (15 ml), and sodium borohydride (90 mg, 2.34 mmol) was added to the solution in one portion at -15°C. After 1 hour of stirring at the same temperature, the reaction mixture was mixed with acetone (1 ml), warmed to room temperature and then mixed with saturated ammonium chloride aqueous solution (10 ml). After removing methanol by evaporation, ethyl acetate extraction was carried out, and the resulting organic layer was washed with saturated brine, dried on anhydrous magnesium sulfate and then subjected to evaporation to remove the solvent. The thus obtained residue was subjected to silica gel column chromatography (ether:hexane = 3:1), and the resulting colorless solid material was washed with an ether-hexane (2:1) mixture solution to obtain 1.02 g (66.1%) of the title compound in the form of colorless powder.
Melting point: 101.5 - 102.5°C
IR (KBr): 3312, 1650, 1594, 1534, 1494, 1462, 1434, 1332, 1288, 1256, 1224, 1030 cm⁻¹
NMR (CDCl₃) δ: 1.95 (1H, t, J = 5 Hz), 3.93 (3H, s), 4.79 (2H, d, J = 5 Hz), 6.76 - 7.18 (3H, m), 7.35 - 7.65 (2H, m), 7.67 - 8.00 (2H, m), 8.35 - 8.70 (2H, m)

### Reference Example 56

### 4-Hydroxymethyl-N-(2-methoxyphenyl)benzamide

4-Chloromethyl-N-(2-methoxyphenyl)benzamide (500 mg, 1.81 mmol) and precipitated calcium carbonate (970 mg, 9.7 mmol) were suspended in a dioxane-water (1:1) mixture solution (9 ml) and subjected to 25 hours of heating under reflux. After adding THF (25 ml) and filtering off the formed insoluble materials, the organic solvent was removed by evaporation. The thus obtained residue was extracted with ethyl acetate, and the resulting organic layer was washed with saturated brine and dried on anhydrous magnesium sulfate. After removing the solvent by evaporation, the resulting colorless solid material was purified by subjecting it to a silica gel column chromatography (ether:hexane = 3:1), thereby obtaining 411 mg (88.3%) of the title compound in the form of colorless crystals. Melting point: 104 - 105.5°C

IR (KBr): 3428, 1648, 1604, 1536, 1510, 1488, 1456, 1438, 1346, 1292, 1254, 1038, 744, 614 cm⁻¹
NMR (CDCl₃) δ: 1.88 (1H, t, J = 5 Hz), 3.93 (3H, s), 4.80 (2H, d, J = 5 Hz), 6.76 - 7.16 (3H, m), 7.49 (2H, d, J = 8 Hz), 7.89 (2H, d, J = 8 Hz), 8.30 - 8.70 (2H, m)

### Reference Example 57

### 4-Hydroxymethyl-N-(3-methoxybenzyl)benzamide

Using 4-chloromethyl-N-(3-methoxybenzyl)benzamide (500 mg, 1.73 mmol), the procedure of Reference Example 28 was repeated to obtain 389 mg (82.9%) of the title compound in the form of colorless powder.
Melting point: 63 - 67.5°C
IR (KBr): 3320, 1640, 1614, 1546, 1492, 1454, 1436, 1308, 1264, 1048, 734, 694 cm⁻¹
NMR (CDCl₃) δ: 2.04 (1H, t, J = 6 Hz), 3.79 (3H, s), 4.60 (2H, d, J = 6 Hz), 4.73 (2H, d, J = 4.5 Hz), 6.20 - 6.58 (1H, m), 6.68 - 7.04 (3H, m), 7.10 - 7.32 (1H, m), 7.39 (2H, d, J = 8 Hz), 7.76 (2H, d, J = 8 Hz)

### Reference Example 58

### 4-Hydroxymethyl-N-(2-methoxyethyl)benzamide

Using 4-chloromethyl-N-(2-methoxyethyl)benzamide (228 mg, 1.0 mmol), the procedure of Reference Example 28 was repeated to obtain 209 mg (100%) of the title compound in the form of colorless solid.
Melting point: 84 - 86°C
IR (KBr): 3300, 2888, 1634, 1554, 1510, 1334, 1308, 1120, 1034, 748, 658 cm⁻¹
NMR (CDCl₃) δ: 2.07 (1H, t, J = 5.2 Hz), 3.39 (3H, s), 3.47 - 3.88 (4H, m), 4.72 (2H, d, J = 5.2 Hz), 6.52 (1H, br-s), 7.40 (2H, d, J = 8 Hz), 7.76 (2H, d, J = 8 Hz)

### Reference Example 59

### 4-Tetrahydropyranyloxymethyl-N-(2-methoxyphenyl)benzenesulfonamide

4-Hydroxymethyl-N-(2-methoxyphenyl)benzesulfonamide (99.6 mg, 0.34 mmol) was dissolved in methylene chloride (3 ml) to which, with cooling in an ice bath, were subsequently added 3,4-dihydro-2H-pyrane (48 µl, 0.51 mmol) and a catalytically effective amount of p-toluenesulfonic acid monohydrate. After 90 minutes of stirring at the same temperature, the reaction solution was mixed with saturated sodium bicarbonate (5 ml) and extracted with ether. The resulting organic layer was washed with saturated brine and dried on anhydrous magnesium sulfate, followed by evaporation of the solvent. Thereafter, the thus obtained light yellow oily material was purified by a silica gel column chromatography (ether:hexane = 1:1) to obtain 117 mg (91.2%) of the title compound in the form of colorless solid.
Melting point: 67 - 70°C
IR (KBr): 3272, 2932, 1598, 1502, 1398, 1342, 1256, 1168, 1114, 1090, 1028, 972, 908, 752, 696, 548 cm⁻¹
NMR (CDCl₃) δ: 1.34 - 2.10 (6H, m), 3.30 - 4.10 (2H, m), 3.63 (3H, s), 4.46 (1H, d, J = 13.2 Hz), 4.65 (1H, br-s), 4.78 (1H, d, J = 13.2 Hz), 6.56 - 7.14 (4H, m), 7.19 - 7.60 (1H, m), 7.36 (2H, d, J = 8 Hz), 7.74 (2H, d, J = 8 Hz)

### Reference Example 60

### 4-Tetrahydropyranyloxymethyl-N-(2-methoxyphenyl)benzamide

Using 4-hydroxymethyl-N-(2-methoxyphenyl)benzamide (129 mg, 0.5 mmol), the procedure of Reference Example 31 was repeated to obtain 166 mg (97.2%) of the title compound in the form of colorless solid.
Melting point: 67 - 70°C
IR (KBr): 3445, 2944, 1676, 1602, 1528, 1510, 1482, 1460, 1434, 1340, 1288, 1124, 1032, 748 cm⁻¹
NMR (CDCl₃) δ: 1.60 - 2.10 (6H, m), 3.40 - 3.72 (1H, m), 3.73 - 4.15 (1H, m), 3.92 (3H, s), 4.57 (1H, d, J = 12.5 Hz), 4.73 (1H, br-s), 4.85 (1H, d, J = 12.5 Hz), 6.80 - 7.15 (3H, m), 7.49 (2H, d, J = 8 Hz), 7.87 (2H, d, J = 8 Hz), 8.35 - 8.67 (2H, m)

### Reference Example 61

### 4-Tetrahydropyranyloxymethyl-N-(2-methoxyphenyl)benzamide

Using 3-hydroxymethyl-N-(2-methoxyphenyl)benzamide (129 mg, 0.5 mmol), the procedure of Reference Example 31 was repeated to obtain 159 mg (93.1%) of the title compound in the form of colorless oil.
IR (neat): 3435, 2944, 1676, 1602, 1526, 1460, 1432, 1338, 1288, 1250, 1120, 1030, 746 cm⁻¹
NMR (CDCl₃) δ: 1.40 - 2.00 (6H, m), 3.40 - 3.70 (1H, m), 3.78 - 4.11 (1H, m), 3.92 (3H, s), 4.57 (1H, d, J = 12.5 Hz), 4.75 (1H, br-s), 4.89 (1H, d, J = 12.5 Hz), 6.80 - 7.15 (3H, m), 7.40 - 7.64 (2H, m), 7.67 - 7.98 (2H, m), 8.35 - 8.66 (2H, m)

### Reference Example 62

### 4-Tetrahydropyranyloxymethyl-N-(3-methoxybenzyl)benzamide

Using 4-hydroxymethyl-N-(3-methoxybenzyl)benzamide (136 mg, 0.5 mmol), the procedure of Reference Example 31 was repeated to obtain 178 mg (100%) of the title compound in the form of colorless solid.
Melting point: 66 - 68.5°C
IR (KBr): 3308, 2944, 1640, 1552, 1492, 1352, 1320, 1260, 1140, 1116, 1032, 980, 770, 674 cm⁻¹
NMR (CDCl₃) δ: 1.35 - 2.23 (6H, m), 3.37 - 4.13 (2H, m), 3.79 (3H, s), 4.35 - 4.97 (5H, m), 6.15 - 6.55 (1H, m), 6.67 - 7.06 (3H, m), 7.12 - 7.33 (1H, m), 7.42 (2H, d, J = 8 Hz), 7.77 (2H, d, J = 8 Hz)

### Reference Example 63

### 4-Tetrahydropyranyloxymethyl-N-(2-methoxyethyl)benzamide

Using 4-hydroxymethyl-N-(2-methoxyethyl)benzamide (209 mg, 1.0 mmol), the procedure of Reference Example 31 was repeated to obtain 268 mg (91.4%) of the title compound in the form of colorless oil.
IR (neat): 3336, 2940, 2872, 1640, 1544, 1504, 1304, 1198, 1120, 1078, 1064, 1034 cm⁻¹
NMR (CDCl₃) δ: 1.35 - 2.10 (6H, m), 3.38 (3H, s), 3.42 - 4.06 (6H, m), 4.54 (1H, d, J = 13 Hz), 4.70 (1H, br-s), 4.83 (1H, d, J = 13 Hz), 6.49 (1H, br-s), 7.40 (2H, d, J = 8 Hz), 7.75 (2H, d, J = 8 Hz)

### Reference Example 64

### N-(2-Methoxyphenyl)-4-phthalimidomethylbenzamide

In an atmosphere of argon, phthalimide (150 mg, 1.0 mmol) was dissolved in THF (10 ml) to which was subsequently added sodium hydride (44 mg, 60%, 1.1 mmol) at room temperature. After 40 minutes of stirring at the same temperature, to this was added a DMF solution (5 ml) containing 4-chloromethyl-N-(2-methoxyphenyl)benzamide (276 mg, 1.0 mmol) and a catalytically effective amount of sodium iodide. After 2 hours of stirring at 80°C and subsequent removal of the solvent by evaporation, the resulting residue was mixed with water (10 ml) and extracted with an ethyl acetate-methylene chloride (1:2) mixture solution. The resulting organic layer was washed with water and saturated brine and dried on anhydrous magnesium sulfate. Thereafter, the solvent was removed by evaporation, and the resulting residue was subjected to a silica gel column chromatography (methylene chloride-ether:hexane = 2:1) and then washed with ether to obtain 300 mg (77.6%) of the title compound in the form of colorless solid.
Melting point: 176 - 179.5°C
IR (KBr): 3350, 1710, 1658, 1522, 1462, 1432, 1394, 1288, 938, 748, 724 cm⁻¹
NMR (CDCl₃) δ: 3.90 (3H, s), 4.91 (2H, s), 6.80 - 7.14 (3H, m), 7.54 (2H, d, J = 8.5 Hz), 7.65 - 8.10 (6H, m), 8.30 - 8.67 (2H, m)

### Reference Example 65

### N-(2-Methoxyphenyl)-4-morpholinomethylbenzamide

In an atmosphere of argon, morpholine (192 mg, 2.2 mmol) was dissolved in ether (5 ml) to which, with cooling in an ice bath, was subsequently added n-butyl lithium (1.45 ml, 1.52 M, 2.2 mmol). After 5 minutes of stirring at room temperature, to this was added 4-chloromethyl-N-(2-methoxyphenyl)benzamide (276 mg, 1.0 mmol) at the same temperature. After adding THF (5 ml) and stirring for 2.5 hours at 60°C, the reaction solution was mixed with water (20 ml) and extracted with ethyl acetate. After removing insoluble materials by filtration, the organic layer was washed with water and saturated brine and then dried on anhydrous sodium carbonate. Thereafter, the solvent was removed by evaporation, and the resulting residue was purified by subjecting it to a silica gel column chromatography (ether) to obtain 128 mg (39.2%) of the title compound in the form of light yellow solid.
Melting point: 90 - 93.5°C
IR (KBr): 3440, 1666, 1600, 1530, 1510, 1486, 1460, 1440, 1340, 1292, 1252, 1112, 1020, 866, 754 cm⁻¹
NMR (CDCl₃) δ: 2.46 (4H, t, J = 5 Hz), 3.56 (2H, s), 3.72 (4H, t, J = 5 Hz), 3.92 (3H, s), 6.70 - 7.14 (3H, m), 7.45 (2H, d, J = 8.5 Hz), 7.85 (2H, d, J = 8.5 Hz), 8.33 - 8.70 (2H, m)

### Reference Example 66

### N-(2-Methoxyphenyl)-4-dimethylaminomethylbenzamide

In an atmosphere of argon, 4-chloromethyl-N-(2-methoxyphenyl)benzamide (276 mg, 1.0 mmol) was put into a 25 ml capacity eggplant type flask equipped with a cold finger and dissolved in dioxane (3 ml), followed by the addition of 50% dimethylamine aqueous solution (3 ml) at room temperature. After 2.5 hours of stirring at 80°C and subsequent removal of dioxane by evaporation, the resulting residue was diluted with ethyl acetate (30 ml). The thus diluted solution was dried on anhydrous sodium carbonate, and the resulting residue was purified by subjecting it to a silica gel column chromatography (ether-ethyl acetate-ethyl acetate:methanol = 10:1) to obtain 284 mg (100%) of the title compound in the form of colorless oil.
IR (neat): 3440, 2944, 2816, 2772, 1676, 1602, 1530, 1482, 1460, 1434, 1338, 1290, 1250, 1028, 748 cm⁻¹
NMR (CDCl₃) δ: 2.25 (6H, s), 3.48 (2H, s), 3.93 (3H, s), 6.80 - 7.13 (3H, m), 7.42 (2H, d, J = 8.5 Hz), 7.84 (2H, d, J = 8.5 Hz), 8.35 - 8.66 (2H, m)

### Reference Example 67

### N-(2-Methoxyphenyl)-4-(methylamino)methylbenzamide

4-Chloromethyl-N-(2-methoxyphenyl)benzamide (400 mg, 1.45 mmol) was dissolved in dioxane (4 ml) and then mixed with 50% methylamine aqueous solution (4 ml) at room temperature. After 1 hour of stirring at the same temperature, dioxane was removed by evaporation, and the resulting residue was diluted with methylene chloride (10 ml). After drying on anhydrous sodium carbonate, the solvent was removed by evaporation to obtain 500 mg (100%) of the title compound in the form of colorless oil.
IR (neat): 3436, 2956, 2848, 1674, 1602, 1527, 1485, 1461, 1251, 1122, 747 cm⁻¹
NMR (CDCl₃) δ: 1.51 (1H, s), 2.47 (3H, s), 3.84 (2H, s), 3.92 (3H, s), 6.81 - 7.10 (3H, m), 7.42 (2H, d, J = 8.5 Hz), 7.83 (2H, d, J = 8.5 Hz), 8.42 - 8.58 (2H, m)

### Reference Example 68

### 4-(N-tert-Butoxycarbonyl-N-methylamino)methyl-N-(2-methoxyphenyl)benzamide

N-(2-Methoxyphenyl)-4-(methylamino)methylbenzamide (500 mg, 1.45 mmol) was dissolved in a dioxane-water (2:1) mixture solution (5 ml) to which, with cooling in an ice bath, were subsequently added 1 N sodium hydroxide aqueous solution (2 ml) and di-tert-butyl dicarbonate (380 mg, 1.74 mmol). After 14 hours of stirring at room temperature, the resulting solution was extracted with methylene chloride, washed with saturated brine and then dried on anhydrous sodium carbonate. Thereafter, the solvent was removed by evaporation, and the resulting residue was purified by subjecting it to a silica gel column chromatography (ethyl acetate:hexane = 1:2) to obtain 537 mg (100%) of the title compound in the form of colorless oil.
IR (neat): 2974, 2932, 1689, 1605, 1527, 1485, 1461, 1395, 1248, 1146 cm⁻¹
NMR (CDCl₃) δ: 1.57 (9H, s), 2.85 (3H, s), 3.93 (3H, s), 4.49 (2H, s), 6.89 - 7.12 (3H, m), 7.33 (2H, d, J = 8.5 Hz), 7.85 (2H, d, J = 8.5 Hz), 8.42 - 8.55 (2H, m)

### Reference Example 69

### 4-Chlorometyl-N-(2-methoxyphenyl)-N-(1,3-dioxolan-2-yl)methylbenzamide

In an atmosphere of argon, a mixture consisting of o-anisidine (500 mg, 4.06 mmol), triethylamine (1.6 ml, 11.5 mmol) and 2-bromomethyl-1,3-dioxolan (0.96 ml, 8.18 mmol) was stirred at 80°C for 4 days, dissolved in methylene chloride (8 ml) and then, with cooling in an ice bath, mixed with 20% sodium hydroxide aqueous solution (4 ml) and 4-chloromethylbenzoyl chloride (768 mg, 4.06 mmol). After 1 hour of stirring at the same temperature, the resulting mixture was extracted with methylene chloride, washed with 10% citric acid aqueous solution and saturated brine and then dried on anhydrous sodium sulfate. Thereafter, the solvent was removed by evaporation, and the resulting residue was purified by subjecting it to a silica gel column chromatography (ethyl acetate:hexane = 2:5) to obtain 704 mg (47.9%) of the title compound in the form of colorless solid.
Melting point: 126 - 129°C
IR (KBr): 1644, 1500, 1418, 1384, 1306, 1276, 1162, 1134, 1074, 1030, 746 cm⁻¹
NMR (CDCl₃) δ: 3.68 (3H, s), 3.80 - 4.02 (1H, m), 3.91 (4H, d, J = 3 Hz), 4.20 - 4.65 (1H, m), 4.45 (2H, s), 5.25 (1H, t, J = 5 Hz), 6.74 (1H, d, J = 8.5 Hz), 6.85 (1H, d, J = 7.5 Hz), 6.96 - 7.43 (6H, m)

### Reference Example 70

### N-(2-Methoxyphenyl)-N-(1,3-dioxolan-2-yl)methyl-4-phthalimidomethylbenzamide

Using 4-chloromethyl-N-(2-methoxyphenyl)-N-(1,3-dioxolan-2-yl)methylbenzamide (556 mg, 1.54 mmol), the procedure of Reference Example 36 was repeated to obtain 546 mg (75.1%) of the title compound in the form of colorless solid.
Melting point: 150 - 152°C
IR (KBr): 1716, 1641, 1503, 1422, 1392, 1347, 1308, 1278, 1251, 1134, 1122, 1086, 1035, 1014, 942, 744, 720 cm⁻¹
NMR (CDCl₃) δ: 3.47 - 4.02 (1H, m), 3.66 (3H, s), 3.88 (4H, d, J = 3 Hz), 4.20 - 4.55 (1H, m), 4.72 (2H, s), 5.23 (1H, t, J = 5 Hz), 6.71 (1H, d, J = 8 Hz), 6.81 (1H, d, J = 7.5 Hz), 6.96 - 7.38 (6H, m), 7.55 - 7.93 (4H, m)

### Reference Example 71

### 4-(4-Fluorobenzoyl)-1-(2-hydroxyethyl)piperidine

In an atmosphere of argon, 4-(4-fluorobenzoyl)piperidine (2.07 g, 10 mmol) was dissolved in triethylamine (30 ml) to which was subsequently added dropwise 2-bromoethanol (1.1 ml, 14.7 mmol) at room temperature. After 1 hour of heating under reflux, triethylamine was removed by evaporation, and the resulting residue was mixed with saturated sodium carbonate aqueous solution (40 ml) and extracted with ethyl acetate. The resulting organic layer was dried on anhydrous sodium carbonate, the solvent was removed by evaporation and then the resulting residue was purified by subjecting it to a silica gel column chromatography (ethyl acetate-methylene chloride:methanol = 10:1) to obtain 1.96 g (78.0%) of the title compound in the form of light yellow solid.
Melting point: 45 - 47°C
IR (KBr): 3428, 2944, 1680, 1596, 1278, 1238, 1204 cm⁻¹
NMR (CDCl₃) δ: 1.71 - 1.95 (4H, m), 2.01 - 2.43 (3H, m), 2.54 (2H, t, J = 6 Hz), 2.83 - 3.38 (3H, m), 3.60 (2H, t, J = 6 Hz), 7.12 (2H, dd, J = 9 Hz, 9 Hz), 7.93 (2H, dd, J = 9 Hz, 6 Hz)

### Reference Example 72

### 4-(4-Fluorophenyl)-1-(3-hydroxypropyl)piperazine

Using 4-(4-fluorophenyl)piperazine (3.68 g, 20 mmol) and 3-bromopropanol (3.42 ml, 36.0 mmol), the procedure of Reference Example 43 was repeated to obtain 3.87 g (81.2%) of the title compound in the form of light yellow solid.
Melting point: 77 - 79°C
IR (KBr): 3406, 3262, 2938, 2836, 1512, 1452, 1269, 1242, 1152, 1122, 1053 cm⁻¹
NMR (CDCl₃) δ: 1.69 - 1.88 (2H, m), 2.51 - 2.76 (6H, m), 2.98 - 3.20 (4H, m), 3.80 (2H, t, J = 6.6 Hz), 4.60 (1H, br-s), 6.70 - 7.00 (4H, m)

### Reference Example 73

### 1-(2-Chloroethyl)-4-(4-fluorobenzoyl)piperidine hydrochloride

In an atmosphere of dry air, 4-(4-fluorobenzoyl)-1-(2-hydroxyethyl)piperidine (1.96 g, 7.8 mmol) was dissolved in methylene chloride (10 ml) to which, with cooling in an ice bath, were subsequently added dropwise DMF (0.1 ml) and thionyl chloride (2.5 ml, 34.2 mmol). After 7 hours of stirring at room temperature, the solvent was removed by evaporation, and the resulting residue was subjected to azeotropic distillation using benzene (15 ml x 2). Thereafter, the thus obtained residue was washed with an ether-methylene chloride (4:1) mixture solution (30 ml) to obtain 2.3 g (96.3%) of the title compound in the form of light beige powder.
IR (KBr): 2620, 2520, 1678, 1598, 1506, 1446, 1408, 1278, 1224, 1158, 950, 840, 606 cm⁻¹
NMR (CDCl₃) δ: 1.75 - 2.90 (4H, m), 2.90 - 4.50 (9H, m), 6.90 - 7.40 (2H, m), 7.65 - 8.30 (2H, m)

### Reference Example 74

### 1-(3-Bromopropyl)-4-(4-fluorophenyl)piperazine dihydrobromate

4-(4-Fluorophenyl)-1-(3-hydroxypropyl)piperazine (1.0 g, 4.20 mmol) was mixed with 47% hydrobromic acid (10 ml) and subjected to 4 hours of heating under reflux. After removing the solvent by evaporation, the resulting residue was subjected to azeotropic distillation using a benzene-methanol (1:1) mixture solution (40 ml x 2). Thereafter, the thus obtained residue was washed with an ether-methylene chloride (4:1) mixture solution (30 ml) to obtain 1.76 g (93.2%) of the title compound in the form of beige powder which is insoluble in ether, methylene chloride, methanol, DMSO and acetone.
IR (KBr): 2980, 2620, 2508, 2428, 1508, 1476, 1454, 1232, 846, 544 cm⁻¹

### Reference Example 75

### 1-(2-Chloroethyl)-4-(4-fluorobenzoyl)piperidine

1-(2-Chloroethyl)-4-(4-fluorobenzoyl)piperidine hydrochloride (800 mg, 2.61 mmol) was suspended in ether (20 ml). With cooling in an ice bath, saturated sodium carbonate aqueous solution (5 ml) was added dropwise to the suspension. The reaction solution was extracted with ether and washed with saturated brine, and the resulting organic layer was dried on anhydrous sodium carbonate. Thereafter, the organic layer was filtered through silica gel (5 g), the solvent was removed by evaporation and then the resulting residue was purified by subjecting it to a silica gel column chromatography (ether) to obtain 650 mg (92.3%) of the title compound in the form of light yellow solid.
Melting point: 33 - 37°C
IR (KBr): 2944, 2812, 1666, 1598, 1446, 1412, 1376, 1298, 1264, 1230, 1208, 1164, 1132, 1104, 976, 852 cm⁻¹
NMR (CDCl₃) δ: 1.67 - 1.96 (4H, m), 2.07 - 2.43 (2H, m), 2.75 (2H, t, J = 7.5 Hz), 2.86 - 3.30 (3H,m), 3.60 (2H, d, J = 7.5 Hz), 7.12 (2H, dd, J = 9 Hz, 9 Hz), 7.93 (2H, dd, J = 9 Hz, 6 Hz)

### Reference Example 76

### N-{2-[4-(4-Fluorobenzoyl)piperidino]ethyl}phthalimide

In an atmosphere of argon, 4-(4-fluorobenzoyl)piperidine (3.11 g, 15 mmol) was dissolved in triethylamine (15 ml) to which was subsequently added N-(2-bromoethyl)phthalimide (6.08 g, 22.5 mmol) at room temperature. After 2 hours of heating under reflux, triethylamine was removed by evaporation, and the resulting residue was mixed with water (30 ml) and extracted with methylene chloride. The resulting organic layer was washed with water and saturated brine and dried on anhydrous sodium carbonate, the solvent was removed by evaporation and then the resulting residue was purified by subjecting it to a silica gel column chromatography (ethyl acetate:hexane = 1:2 to 2:3) to obtain 2.72 g (47.7%) of the title compound in the form of light yellow solid.
Melting point: 143 - 147°C
IR (KBr): 2940, 2812, 1776, 1712, 1666, 1596, 1396, 1376, 1286, 1228, 1210, 1166, 1098, 710 cm⁻¹
NMR (CDCl₃) δ: 1.50 - 1.95 (4H, m), 1.95 - 2.40 (2H, m), 2.67 (2H, t, J = 6.5 Hz), 2.86 - 3.38 (3H, m), 3.83 (2H, t, J = 6.5 Hz), 7.11 (2H, dd, J = 9 Hz, 9 Hz), 7.55 - 8.08 (6H, m)

### Reference Example 77

### N-{3-[4-(4-Fluorophenyl)piperazinyl)propyl}phthalimide

Using 4-(4-fluorophenyl)piperazine (1.0 g, 5.55 mmol) and N-(3-bromopropyl)phthalimide (1.59 g, 5.55 mmol), the procedure of Reference Example 48 was repeated to obtain 1.85 g (90.5%) of the title compound in the form of light yellow solid.
Melting point: 94 - 96°C
IR (KBr): 2820, 1698, 1508, 1398, 1232, 1148, 1022, 818, 716 cm⁻¹
NMR (CDCl₃) δ: 1.73 - 2.15 (2H, m), 2.35 - 2.70 (6H, m), 2.84 - 3.10 (4H, m), 3.77 (2H, t, J = 7 Hz), 6.55 - 7.05 (4H, m), 7.53 - 7.89 (4H, m)

### Reference Example 78

### 1-(2-Aminoethyl)-4-(4-fluorobenzoyl)piperidine

N-{2-[4-(4-Fluorobenzoyl)piperidino]ethyl}phthalimide (190 mg, 0.50 mmol) was dissolved in a methanol-THF (3:2) mixture solution (2.5 ml) to which, with cooling in an ice bath, was subsequently added dropwise hydrazine hydrate (1.5 ml). After 20 minutes of stirring at room temperature, the solvent was removed by evaporation, and the resulting residue was dissolved in methylene chloride and then washed with saturated sodium carbonate aqueous solution. The resulting organic layer was dried on anhydrous sodium carbonate, and the solvent was removed by evaporation to obtain 125 mg (100%) of the title compound in the form of light yellow solid.
IR (KBr): 3365, 2944, 2804, 1680, 1598, 1506, 1300, 1262, 1228, 1158, 976, 854 cm⁻¹
NMR (CDCl₃) δ: 1.49 (2H, br-s), 1.63 - 2.30 (6H, m), 2.43 (2H, t, J = 6 Hz), 2.61 - 3.40 (5H, m), 7.13 (2H, dd, J = 9 Hz, 9 Hz), 7.97 (2H, dd, J = 9 Hz, 6 Hz)

### Reference Example 79

### 1-(3-aminopropyl)-4-(4-fluorophenyl)piperazine

The procedure of Reference Example 50 was repeated using N-{3-[4-(4-fluorophenyl)piperazinyl]propyl}phthalimide (1.10 g, 2.99 mmol), thereby obtaining 702 mg (98.9%) of the title compound in the form of yellow oil.
IR (neat): 2938, 2818, 1512, 1455, 1380, 1302, 1287, 1233, 1161, 1143, 819 cm⁻¹
NMR (CDCl₃) δ: 1.38 (2H, s), 1.50 - 2.00 (2H, m), 2.30 - 2.90 (8H, m), 2.90 - 3.55 (4H, m), 6.74 - 7.12 (4H, m)

### Reference Example 80

### 3-[4-(4-Fluorobenzoyl)piperidino]propionitrile

In an atmosphere of argon, 4-(4-fluorobenzoyl)piperidine (1.24 g, 6.0 mmol) was dissolved in triethylamine (10 ml) to which was subsequently added 3-bromopropionitrile (0.6 ml, 7.14 mmol) at room temperature. After 1 hour of heating under reflux, triethylamine was removed by evaporation, and the resulting residue was purified by subjecting it to a silica gel column chromatography (methylene chloride-ether:hexane = 2:1-ether) to obtain 1.11 g (71.1%) of the title compound in the form of light yellow solid.
Melting point: 67 - 69°C
IR (KBr): 2952, 2836, 2800, 2240, 1682, 1598, 1412, 1372, 1276, 1224, 1200, 1164, 1136, 970, 854, 824, 604 cm⁻¹
NMR (CDCl₃) δ: 1.60 - 2.03 (4H, m), 2.03 - 3.50 (9H, m), 7.13 (2H, dd, J = 9 Hz, 9 Hz), 7.96 (2H, dd, J = 9 Hz, 6 Hz)

### Reference Example 81

### 3-[4-(4-Fluorophenyl)piperazinyl]propionitrile

Using 4-(4-fluorophenyl)piperazine (1.84 g, 10.0 mmol), the procedure of Reference Example 80 was repeated to obtain 1.46 g (62.6%) of the title compound in the form of light yellow solid.
Melting point: 87 - 89°C
IR (KBr): 2820, 2240, 1514, 1448, 1296, 1278, 1262, 1236, 1162, 1134, 824, 524 cm⁻¹
NMR (CDCl₃) δ: 2.40 - 2.93 (8H, m), 2.97 - 3.26 (4H, m), 6.70 - 7.12 (4H, m)

### Reference Example 82

### Ethyl 3-[4-(4-fluorobenzoyl)piperidinopropionimidate dihydrochloride

In an atmosphere of dry air, 3-[4-(4-fluorobenzoyl)piperidino]propionitrile (300 mg, 1.15 mmol) was dissolved in ethanol (6 ml), and hydrogen chloride gas was gradually introduced into the solution at -10°C spending 1 hour. After warming from -10°C to room temperature spending 2.5 hours, ethanol was removed by evaporation, and the resulting residue was washed with ether to obtain 436 mg (100%) of the title compound in the form of orange oil to be used in the subsequent reaction without further purification.

### Reference Example 83

### Ethyl 3-[4-(4-fluorophenyl)piperazinyl]propionimidate trihydrochloride

Using 3-[4-(4-fluorophenyl)piperazinyl]propionitrile (1.06 g, 4.56 mmol), the procedure of Reference Example 82 was repeated to obtain 1.73 g (100%) of the title compound in the form of colorless powder to be used in the subsequent reaction without further purification.

### Reference Example 84

### 3-[4-(4-Fluorobenzoyl)piperidino]propionamidine

In an atmosphere of dry air, 10% ammonia/methanol solution (2 ml) was added at room temperature to crude 3-[4-(4-fluorobenzoyl)piperidino]propione imidate dihydrochloride (436 mg, 1.15 mmol), followed by 1 hour of stirring at the same temperature. After removing methanol by evaporation, the resulting residue was mixed with a methylene chloride-methanol (3:1) mixture solution (5 ml), insoluble materials were removed by filtration, the solvent was removed by evaporation, and the thus obtained residue was purified by subjecting it to a silica gel column chromatography (methylene chloride:5% ammonia/methanol solution = 8:1) to obtain a light beige solid material. This was mixed with a methylene chloride-ethanol (1:1) mixture solution (5 ml), and insoluble materials were removed by filtration to obtain 296 mg (92.8%) of the title compound in the form of colorless powder.
Melting point: 167.5 - 169°C (decomposition)
IR (KBr): 3048, 1680, 1596, 1272, 1226, 1202, 1160, 856, 670 cm⁻¹
NMR (CDCl₃-CD₃OD) δ: 1.66 - 2.13 (4H, m), 2.44 (2H, dt, J = 11 Hz, 4 Hz), 2.83 (4H, dd, J = 9 Hz, 5 Hz), 3.00 - 3.60 (3H, m), 7.20 (2H, dd, J = 9 Hz, 9 Hz), 8.02 (2H, dd, J = 9 Hz, 6 Hz) MS (FAB, m/z): 220, 278 (M⁺ + H)

### Reference Example 85

### 3-[4-(4-Fluorobenzoyl)piperazinylpropionamidine

Using crude 3-[4-(4-fluorophenyl)piperazinyl]propione imidate trihydrochloride (603 mg, 1.6 mmol), the procedure of Reference Example 84 was repeated to obtain 376 mg (93.7%) of the title compound in the form of colorless powder.
Melting point: 135 - 140°C
IR (KBr): 3048, 2824, 1672, 1510, 1450, 1342, 1266, 1236, 1140, 815, 746, 714 cm⁻¹
NMR (CDCl₃-CD₃OD) δ: 2.50 - 2.98 (8H, m), 3.00 - 3.40 (4H, m), 6.70 - 7.15 (4H, m)

### Reference Example 86

### 1-(2,2-Diethoxyethyl)-4-(4-fluorobenzoyl)piperidine

4-(4-Fluorobenzoyl)piperidine (207 mg, 1.0 mmol) was dissolved in methylene chloride (5 ml) to which were subsequently added bromoacetoaldehyde diethylacetal (305 mg, 1.5 mmol) and triethylamine (0.5 ml). After 12 hours of heating under reflux, the solvent was removed by evaporation, and the resulting residue purified by subjecting it to a silica gel column chromatography (ether) to obtain 81 mg (25.0%) of the title compound in the form of orange oil.
IR (neat): 2976, 2944, 1682, 1598, 1278, 1230, 1206, 1156, 1120, 1062, 976, 852 cm⁻¹
NMR (CDCl₃) δ: 1.21 (6H, t, J = 7 Hz), 1.50 - 1.98 (4H, m), 2.02 - 2.44 (2H, m), 2.56 (2H, d, J = 5.6 Hz), 2.85 - 3.30 (3H, m), 3.58, 3.66 (each 2H, q, J = 7 Hz), 4.65 (1H, t, J = 5.6 Hz), 7.12 (2H, dd, J = 9 Hz, 9 Hz), 7.95 (2H, dd, J = 9 Hz, 6 Hz)

### Reference Example 87

### 4-(4-Fluorobenzoyl)-1-[2-(2-methoxyanilino)ethyl]piperidine

1-(2,2-Diethoxyethyl)-4-(4-fluorobenzoyl)piperidine (81 mg, 0.5 mmol) was dissolved in THF (3 ml) to which was subsequently added 10% hydrochloric acid (2 ml) at room temperature. After 1 hour of stirring at the same temperature, the solvent was removed by evaporation, and the resulting residue was subjected to azeotropic distillation using benzene (5 ml x 4) to obtain an orange glutinous material. The thus obtained residue was dissolved in methanol (2 ml) to which were subsequently added o-anisidine (29 µl, 0.25 mmol) and sodium cyanoborohydride (11 mg, 0.166 mmol) at room temperature. After 2 hours of stirring at the same temperature, the reaction solution was mixed with saturated sodium carbonate aqueous solution (10 ml), extracted with ethyl acetate and then washed with water and saturated brine. The resulting organic layer was dried on anhydrous sodium carbonate, the solvent was removed by evaporation and then the resulting residue was purified by subjecting it to a silica gel column chromatography (ether:hexane = 2:1-ether) to obtain 59 mg (66.2%) of the title compound in the form of colorless solid.
Melting point: 116 - 120°C
IR (KBr): 3410, 2956, 2810, 1678, 1600, 1510, 1450, 1288, 1268, 1234, 1224, 1204, 1154, 1022, 728 cm⁻¹
NMR (CDCl₃) δ: 1.60 - 2.38 (7H, m), 2.70 (2H, t, J = 6 Hz), 2.83 - 3.45 (5H, m), 3.85 (3H, s), 6.47 - 6.92 (4H, m), 7.13 (2H, dd, J = 9 Hz, 9 Hz), 7.96 (2H, dd, J = 9 Hz, 6 Hz)

### Inventive Example 1

### 2-(2-Methoxyphenyl)-3-(3-methoxyphenyl)propanal

In an atmosphere of argon, crude 2-(2-methoxyphenyl)-3-(3-methoxyphenyl)propionitrile (1.0 g, 3.74 mmol) was dissolved in toluene (20 ml) to which was subsequently added dropwise diisobutylaluminum hydride (2.8 ml, 1.5 M, 4.20 mmol) while cooling in an ice bath. After 1 hour of stirring, the reaction mixture was poured in 5% sulfuric acid aqueous solution (50 ml), extracted with ether and then washed with water and saturated brine. The resulting organic layer was dried on anhydrous magnesium sulfate, the solvent was removed by evaporation and then the resulting yellow oily residue was purified by subjecting it to a silica gel column chromatography (ether:hexane = 1:20 - 1:15) to obtain 681 mg (67.4%, 2 steps 77.5%) of the title compound in the form of colorless oil.
IR (neat): 2950, 2850, 1724, 1600, 1581, 1492, 1246, 1152, 1048, 754, 696 cm⁻¹
NMR (CDCl₃) δ: 2.93 (1H, dd, J = 14.5 Hz, 9 Hz), 3.46 (1H, dd, J = 14.5 Hz, 6 Hz), 3.71, 3.76 (each 3H, s), 3.91 - 4.20 (1H, m), 6.50 - 7.40 (8H, m), 9.74 (1H, s)

### Inventive Example 2

### 3-(2-Methoxyphenyl)-4-(3-methoxyphenyl)butyl alcohol

In an atmosphere of argon, 1-[2-(2-methoxyphenyl)-3-butenyl]-3-methoxybenzene (360 mg, 1.34 mmol) was dissolved in THF (5 ml) to which was subsequently added dropwise 9-BBN dimer (490 mg, 2.0 mmol) which has been dissolved in THF (7 ml), while cooling in an ice bath. After 2.5 hours of stirring at the same temperature, to the resulting reaction mixture cooled in an ice bath were added dropwise 6 N sodium hydroxide aqueous solution (1.9 ml) and hydrogen peroxide (1.33 ml). After 20 minutes of stirring at room temperature, THF was removed by evaporation, and the resulting residue was extracted with an ether-hexane (1:1) mixture solution. The resulting organic layer was dried on anhydrous magnesium sulfate, the solvent was removed by evaporation and then the resulting residue was purified by subjecting it to a silica gel column chromatography (ether:hexane = 3:4) to obtain 359 mg (93.6%) of the title compound in the form of colorless oil.
IR (neat): 3400, 2936, 1600, 1584, 1490, 1464, 1438, 1260, 1240, 1152, 1044, 752 cm⁻¹
NMR (CDCl₃) δ: 1.40 - 2.25 (3H, m), 2.88 (1H, d, J = 9 Hz), 2.92 (1H, d, J = 6 Hz), 3.20 - 3.90 (3H, m), 3.70, 3.75 (each 3H, s), 6.50 - 7.30 (8H, m)

### Inventive Example 3

### Methyl 4-(2-methoxyphenyl)-5-(3-methoxyphenyl)valerate

In an atmosphere of argon, 5% palladium carbon (11 mg) was added to a methanol solution (6 ml) containing methyl 4-(2-methoxyphenyl)-5-(3-methoxyphenyl)-2-pentenate (109 mg, 0.333 mmol), and the mixture was stirred for 4 hours at room temperature in an atmosphere of hydrogen. After replacing the atmosphere by argon, the reaction mixture was diluted with ether (10 ml). Thereafter, palladium carbon was removed by filtration, and the solvent was removed by evaporation to obtain 110 mg (100%) of the title compound in the form of light yellow oil.
IR (neat): 2948, 1736, 1600, 1584, 1492, 1456, 1438, 1242, 1152, 1030, 754 cm⁻¹
NMR (CDCl₃) δ: 1.75 - 2.29 (4H, m), 2.85 (1H, d, J = 8 Hz), 2.87 (1H, d, J = 6 Hz), 3.29 - 3.90 (1H, m), 3.56 (3H, s), 3.71 (6H, s), 6.55 - 7.30 (8H, m)

### Inventive Example 4

### 4-(2-Methoxyphenyl)-5-(3-methoxyphenyl)pentyl alcohol

In an atmosphere of argon, methyl 4-(2-methoxyphenyl)-5-(3-methoxyphenyl)valerate (110 mg, 0.333 mmol) was dissolved in ether (5 ml) to which was subsequently added litium aluminum hydride (26.6 mg, 0.666 mmol), followed by 15 minutes of stirring in an ice bath. At the same temperature, to this were added sodium sulfate decahydrate (300 mg) and water (5 drops), followed by stirring and subsequent separation of ether layer by decantation. Thereafter, the ether layer was filtered (ether) using silica gel (2 g) to obtain 100 mg (100%) of the title compound in the form of colorless oil.
IR (neat): 3384, 2940, 1600, 1584, 1492, 1454, 1438, 1258, 1240, 1152, 1050, 752 cm⁻¹
NMR (CDCl₃) δ: 1.10 - 1.89 (5H, m), 2.82 (1H, d, J = 9 Hz), 2.84 (1H, d, J = 6 Hz), 3.20 - 3.80 (3H, m), 3.70 (6H, s), 6.45 - 7.28 (8H, m)

### Inventive Example 5

### 4-Tetrahydropyranyloxy-2-(3-methoxybenzyl)-2-(2-methoxyphenyl)butyronitrile

In an atmosphere of argon, diisopropylamine (0.28 ml, 2.0 mmol) was dissolved in THF (5 ml), n-butyl lithium (1.37 ml, 1.46 M, 2.0 mmol) was added dropwise to the resulting solution at -78°C, and the mixture was stirred for 30 minutes. To this was added dropwise a THF solution (5 ml) containing crude 2-(2-methoxyphenyl)-3-(3-methoxyphenyl)propionitrile (500 mg, 1.87 mmol) at the same temperature, followed by 30 minutes of stirring. At the same temperature, to this was further added dropwise 2-tetrahydropyranyloxyethyl bromide (500 mg, 2.39 mmol) which has been dissolved in THF (2 ml). The resulting reaction mixture was warmed to -5°C spending 2 hours and then stirred at the same temperature for 15 hours. This was mixed with saturated ammonium chloride aqueous solution (10 ml), extracted with ether and then washed with water and saturated brine. The resulting organic layer was dried on anhydrous magnesium sulfate, the solvent was removed by evaporation and then the resulting light yellow oily residue was purified by subjecting it to a silica gel column chromatography (ether:hexane = 1:5 to 1:3) to obtain 228 mg (32.1%, 2 steps 36.9%) of the title compound in the form of colorless oil.
IR (neat): 2944, 1600, 1584, 1492, 1466, 1438, 1248, 1122, 1034, 750 cm⁻¹
NMR (CDCl₃) δ: 1.10 - 1.90 (6H, m), 2.31, 2.83 (each 1H, dt, J = 7 Hz, 14 Hz), 3.10 - 4.05 (6H, m), 3.63, 3.92 (each 3H, s), 4.40 (1H, br-s), 4.44 - 7.41 (8H, m)

### Inventive Example 6

### N-(2-Tetrahydropyranyloxyethyl)-3-methoxy-N-(2-methoxyphenyl)benzamide

In an atmosphere of argon, 3-methoxy-N-(2-methoxyphenyl)benzamide (500 mg, 1.94 mmol) was dissolved in DMF (10 ml) to which was subsequently added sodium hydride (85 mg, 60%, 2.13 mmol) at room temperature, followed by 20 minutes of stirring. After additional 5 minutes of stirring under an ultrasonic irradiation condition at the same temperature, to this was added 2-tetrahydropyranyloxyethyl bromide (446 mg, 2.13 mmol) at room temperature. After 1 hour of stirring at room temperature and subsequent stirring at 70°C for 6 hours, DMF was removed by evaporation, and the resulting residue was mixed with water (20 ml), extracted with ether and then washed with water and saturated brine. The resulting organic layer was dried on anhydrous magnesium sulfate, the solvent was removed by evaporation and then the resulting orange oily residue was purified by subjecting it to a silica gel column chromatography (ether:hexane = 2:1) to obtain 501 mg (67.0%) of the title compound in the form of yellow oil.
IR (neat): 2944, 1650, 1586, 1502, 1456, 1434, 1384, 1318, 1286, 1252, 1122, 1074, 1034, 750 cm⁻¹
NMR (CDCl₃) δ: 1.20 - 2.00 (6H, m), 3.25 - 4.40 (6H, m), 3.63, 3.66 (each 3H, s), 4.57 (1H, br-s), 6.58 - 7.30 (8H, m)

### Inventive Example 7

### 4-Methoxy-N-(hydroxyethyl)-N-(2-methoxyphenyl)benzene sulfonamine

In an atmosphere of argon, 4-methoxy-N-(2-methoxyphenyl)benzene sulfonamide (300 mg, 1.02 mmol) was dissolved in DMF (6 ml) to which was subsequently added sodium hydride (49 mg, 60%, 1.23 mmol) at room temperature. After 1 hour of stirring, to this was added 2-tetrahydropyranyloxyethyl bromide (258 mg, 1.23 mmol) at room temperature. After 4 hours of stirring at 70°C, DMF was removed by evaporation, and the resulting residue was mixed with saturated ammonium chloride aqueous solution (10 ml), extracted with ethyl acetate and then washed with water and saturated brine, followed by drying on anhydrous magnesium sulfate and distillation removal of the solvent to obtain a light yellow oily material. The thus obtained residue was dissolved in methanol (5 ml) and stirred for 3 hours at room temperature in the presence of a catalytically effective amount of p-toluenesulfonic acid. After removing methanol by evaporation, the reaction solution was mixed with saturated sodium bicarbonate (10 ml), extracted with ethyl acetate and then washed with saturated brine. The resulting organic layer was dried on anhydrous magnesium sulfate, the solvent was removed by evaporation and then the resulting light yellow oily residue was purified by subjecting it to a silica gel column chromatography (ether:hexane = 4:1) to obtain 235 mg (68.3%) of the title compound in the form of colorless oil.
IR (neat): 3532, 2944, 1596, 1498, 1462, 1342, 1302, 1260, 1156, 1116, 1072, 1042, 1024, 586, 562 cm⁻¹
NMR (CDCl₃) δ: 2.65 - 2.97 (1H, m), 3.38 - 3.85 (4H, m), 3.63, 3.87 (each 3H, s), 6.70 - 7.45 (6H, m), 7.67 (2H, d, J = 9 Hz)

### Inventive Example 8

### 4-Fluoro-N-(2-hydroxyethyl)-N-(2-methoxyphenyl)benzene sulfonamine

Using 4-fluoro-N-(2-methoxyphenyl)benzene sulfonamide (282 mg, 1.0 mmol), the procedure of Inventive Example 7 was repeated to obtain 302 mg (92.8%) of the title compound in the form of colorless oil.
IR (neat): 3536, 2940, 1592, 1494, 1344, 1290, 1234, 1166, 1118, 1088, 1072, 1042, 1024, 838, 756, 586, 554 cm⁻¹
NMR (CDCl₃) δ: 2.44 - 2.73 (1H, m), 3.30 - 3.83 (4H, m), 3.56 (3H, s), 6.65 - 7.43 (6H, m), 7.73 (2H, dd, J = 9 Hz, 6 Hz)

### Inventive Example 9

### 4-Hydroxy-2-(3-methoxybenzyl)-2-(2-methoxyphenyl)butyronitrile

4-Tetrahydropyranyloxy-2-(3-methoxybenzyl)-2-(2-methoxyphenyl)butyronitrile (227 mg, 0.598 mmol) was dissolved in methanol (2 ml), and the solution was stirred for 3 hours at room temperature in the presence of a catalytically effective amount of p-toluenesulfonic acid monohydrate. The reaction solution was mixed with saturated sodium bicarbonate (10 ml), extracted with ether and then washed with water and saturated brine. The resulting organic layer was dried on anhydrous magnesium sulfate, the solvent was removed by evaporation and then the resulting colorless oily residue was purified by subjecting it to a silica gel column chromatography (ether:hexane = 3:1) to obtain 163 mg (87.5%) of the title compound in the form of colorless oil.
IR (neat): 3436, 2936, 1600, 1584, 1492, 1466, 1438, 1250, 1046, 752 cm⁻¹
NMR (CDCl₃) δ: 1.54 (1H, br-s), 2.21, 2.78 (each 1H, dt, J = 7 Hz, 14 Hz), 3.28 (2H, dd, J = 7 Hz, 7 Hz), 3.39 - 3.80 (3H, m), 3.61, 3.90 (each 3H, s), 6.41 - 7.40 (8H, m)

### Inventive Example 10

### N-(2-Hydroxyethyl)-3-methoxy-N-(2-methoxyphenyl)benzamide

Using 3-methoxy-N-(2-methoxyphenyl)-N-(2-tetrahydropyranyloxyethyl)benzamide (501 mg, 1.30 mmol), the procedure of Inventive Example 9 was repeated to obtain 340 mg (86.8%) of the title compound in the form of light yellow oil.
IR (neat): 3432, 1638, 1580, 1502, 1458, 1434, 1396, 1320, 1288, 1250, 1046, 1026, 750 cm⁻¹
NMR (CDCl₃) δ: 3.30 - 4.13 (5H, m), 3.63, 3.73 (each 3H, s), 6.60 - 7.30 (8H, m)

### Inventive Example 11

### 3-Formyl-2-(3-methoxybenzyl)-2-(2-methoxyphenyl)propionitrile

In an atmosphere of argon at room temperature, 4-hydroxy-2-(3-methoxybenzyl)-2-(2-methoxyphenyl)butyronitrile (162 mg, 0.520 mmol) was dissolved in DMSO (2 ml) to which were subsequently added dropwise triethylamine (0.362 ml, 2.60 mmol) and a sulfur trioxide-pyridine complex (422 mg, 260 mmol) which has been dissolved in DMSO (2 ml). After 20 minutes of stirring at the same temperature, this was mixed with ice water (10 ml), extracted with ethyl acetate and then washed with water and saturated brine. The resulting organic layer was dried on anhydrous magnesium sulfate, and the solvent was removed by evaporation to obtain 164 mg (101.9%) of the title compound in the form of light yellow oil which was used in the subsequent reaction without further purification.
IR (neat): 1728, 1600, 1584, 1492, 1466, 1438, 1250, 1042, 1022, 754, 704 cm⁻¹
NMR (CDCl₃) δ: 3.26 - 3.75 (4H, m), 3.68, 3.94 (each 3H, s), 6.45 - 7.46 (8H, m), 9.55 - 9.70 (1H, d, J = 7.5 Hz, 2 Hz), (1H, d, J = 7.5 Hz, 2 Hz), (1H, m)

### Inventive Example 12

### N-Formylmethyl-3-methoxy-N-(2-methoxyphenyl)benzamide

Using N-(2-hydroxyethyl)-3-methoxy-N-(2-methoxyphenyl)benzamide (339 mg, 1.12 mmol), the procedure of Inventive Example 11 was repeated to obtain 220 mg (65.6%) of the title compound in the form of light yellow solid.
Melting point: 77 - 81°C
IR (KBr): 1732, 1648, 1588, 1502, 1462, 1432, 1372, 1322, 1280, 1252, 1044, 1026 cm⁻¹
NMR (CDCl₃) δ: 3.64, 3.77 (each 3H, s), 4.40 (2H, s), 6.55 - 7.30 (8H, m), 9.76 (1H, s)

### Inventive Example 13

### N-Formylmethyl-N-(2-methoxyphenyl)-4-phthalimidomethylbenzamide

N-(2-Methoxyphenyl)-N-(1,3-dioxolan-2-yl)methyl-4-phthalimidomethylbenzamide (450 mg, 0.953 mmol) was dissolved in THF (6 ml) to which was subsequently added 10% hydrochloric acid (4 ml) at room temperature. After 2 hours of stirring at 60°C, the resulting reaction solution was extracted with chloroform and washed with saturated brine. The resulting organic layer was dried on anhydrous magnesium sulfate, the solvent was removed by evaporation and then the resulting residue was mixed with 5 ml of chloroform to remove insoluble materials by filtration, thereby obtaining 300 mg (73.5%) of the title compound in the form of yellow oil which was used in the subsequent reaction without further purification.
IR (neat): 1716, 1644, 1500, 1428, 1392, 1248, 747, 717 cm⁻¹
NMR (CDCl₃) δ: 3.73 (3H, s), 4.35 (2H, s), 4.72 (2H, s), 6.63 - 7.35 (8H, m), 7.60 - 7.88 (4H, m), 9.61 (1H, s)

### Inventive Example 14

### 4-(4-Fluorobenzoyl)-1-[3-(3-methoxyphenyl)-2-(methoxyphenyl)propyl]piperidine

2-(2-Methoxyphenyl)-3-(3-methoxyphenyl)propanal (90 mg, 0.333 mmol) and 4-(4-fluorobenzoyl)piperidine (83 mg, 0.40 mmol) were dissolved in methanol (3 ml) to which was subsequently added molecular sieve 4A (200 mg) at room temperature. After 1 hour of stirring at the same temperature, sodium cyanoborohydride (8 mg, 0.127 mmol) was added to the above mixture, followed by 45 minutes of stirring and subsequent addition of acetone (1 ml). The reaction solution was diluted with ether (10 ml) and filtered through cerite, and the solvent was removed by evaporation. The thus obtained colorless oily residue was dissolved in ether (5 ml), mixed with saturated hydrogen chloride/ether solution (5 ml) and stirred for 5 minutes. After removing the ether layer, the reaction solution was mixed with water (5 ml) and potassium carbonate (1 g), extracted with ether and dried on potassium carbonate, and then the solvent was removed by evaporation. Thereafter, the resulting colorless oily residue was purified by subjecting it to a silica gel column chromatography (ether:hexane = 3:2) to obtain 98 mg (63.8%) of the title compound in the form of colorless oil.
IR (neat): 2944, 2250, 1680, 1596, 1490, 1464, 1258, 1238, 1154, 974, 908, 750, 730 cm⁻¹
NMR (CDCl₃) δ: 1.60 - 2.30 (6H, m), 2.57 (1H, d, J = 6.5 Hz), 2.60 (1H, d, J = 7.5 Hz), 2.70 - 3.27 (5H, m), 3.47 - 3.93 (1H, m), 3.68, 3.73 (each 3H, s), 6.47 - 7.30 (10H, m), 7.95 (2H, dd, J = 9 Hz, 6 Hz)

### Inventive Example 15

### 4-[4-(4-Fluorobenzoyl)piperidino]-2-(3-methoxybenzyl)-2-(2-methoxyphenyl)butyronitrile

Using crude 3-formyl-2-(3-methoxybenzyl)-2-(2-methoxyphenyl)propionitrile (81.5 mg, 0.263 mmol), the procedure of Inventive Example 14 was repeated to obtain 66.5 mg (50.5%) of the title compound in the form of colorless oil.
IR (neat): 2944, 1680, 1598, 1492, 1262, 1248, 1156, 752, 732 cm⁻¹
NMR (CDCl₃) δ: 1.52 - 2.36 (8H, m), 2.36 - 3.20 (5H, m), 3.27, 3.45 (each 1H, s), 3.61, 3.90 (each 3H, s), 6.35 - 7.40 (10H, m), 7.90 (2H, dd, J = 9 Hz, 6 Hz)

### Inventive Example 16

### 4-[4-(4-Fluorophenyl)piperazinyl]-2-(3-methoxybenzyl)-2-(2-methoxyphenyl)butyronitrile

Using crude 3-formyl-2-(3-methoxybenzyl)-2-(2-methoxyphenyl)propionitrile (82.5 mg, 0.263 mmol), the procedure of Inventive Example 14 was repeated to obtain 51 mg (40.3%) of the title compound in the form of colorless oil.
IR (neat): 2944, 2828, 1600, 1584, 1510, 1492, 1456, 1236, 1154, 1024, 824, 752 cm⁻¹
NMR (CDCl₃) δ: 2.00 - 2.83 (4H, m), 2.53, 3.05, (each 4H, t, J = 5.5 Hz), 3.31, 3.48 (each 1H, s), 3.63, 3.93 (each 3H, s), 6.40 - 7.11 (10H, m), 7.13 - 7.40 (2H, m)

### Inventive Example 17

### N-{2-[4-(4-Fluorobenzoyl)piperidino]ethyl}-3-methoxy-N-(2-methoxyphenyl)benzamide

Using N-formylmethyl-3-methoxy-N-(2-methoxyphenyl)benzamide (141 mg, 0.473 mmol), the procedure of Inventive Example 14 was repeated to obtain 107 mg (46.1%) of the title compound in the form of light yellow oil.
IR (neat): 2944, 1678, 1638, 1596, 1500, 1458, 1390, 1278, 1248, 1156, 1046, 748 cm⁻¹
NMR (CDCl₃) δ: 1.58 - 1.93 (4H, m), 2.00 - 2.36 (2H, m), 2.63 (2H, t, J = 7.5 Hz), 2.80 - 3.30 (3H, m), 3.64, 3.70 (each 3H, s), 3.90 - 4.40 (2H, m), 6.55 - 7.30 (10H, m), 7.95 (2H, dd, J = 9 Hz, 6 Hz)

### Inventive Example 18

### N-{2-[4-(4-Fluorobenzoyl)piperidino]ethyl}-4-methoxy-N-(2-methoxyphenyl)benzene sulfonamide

In an atmosphere of argon at room temperature, N-hydroxyethyl-4-methoxy-N-(2-methoxyphenyl)benzene sulfonamide (235 mg, 0.697 mmol) was dissolved in DMSO (3 ml) to which were subsequently added dropwise triethylamine (0.48 ml, 3.44 mmol) and sulfur trioxide-pyridine complex (558 mg, 3.44 mmol) which has been dissolved in DMSO (3 ml). After 20 minutes of stirring at the same temperature, ice water (10 ml) was added to the reaction solution, and the mixture was extracted with ethyl acetate and then washed with water and saturated brine. The resulting organic layer was dried on anhydrous magnesium sulfate, and the solvent was removed by evaporation to obtain a light yellow oily material. The thus obtained residue was dissolved in methanol (5 ml) to which were subsequently added 4-(4-fluorobenzoyl)piperidine (108 mg, 0.52 mmol) and molecular sieve 4A (200 mg) at room temperature. After 45 minutes of stirring at the same temperature, sodium cyanoborohydride (11 mg, 0.166 mmol) was added to the reaction mixture which was stirred for additional 30 minutes, followed by the addition of acetone (1 ml). The resulting reaction solution was diluted with ether (10 ml) and filtered through cerite, followed by the removal of the solvent by evaporation. The thus obtained colorless oily residue was dissolved in ether (10 ml) and extracted with 2 N hydrochloric acid (10 ml). After removing the ether layer, the resulting reaction solution was adjusted to pH 9 to 10 with 10% sodium hydroxide aqueous solution and extracted with methylene chloride, the resulting organic layer was dried on potassium carbonate and then the solvent was removed by evaporation. Thereafter, the resulting yellow oily residue was purified by subjecting it to a silica gel column chromatography (ether:hexane = 7:1) to obtain 56.7 mg (15.4%) of the title compound in the form of colorless oil.
IR (neat): 2944, 1680, 1598, 1498, 1342, 1258, 1158, 732, 588, 562 cm⁻¹
NMR (CDCl₃) δ: 1.53 - 2.30 (6H, m), 2.52 (2H, t, J = 7.5 Hz), 2.73 - 3.30 (3H, m), 3.43 (3H, s), 3.72 (2H, t, J = 7.5 Hz), 3.84 (3H, s), 6.67 - 7.40 (8H, m), 7.62 (2H, d, J = 9 Hz), 7.93 (2H, dd, J = 9 Hz, 6 Hz)

### Inventive Example 19

### 4-Fluoro-N-{2-[4-(4-fluorobenzoyl)piperidino]ethyl}-N-(2-methoxyphenyl)benzene sulfonamide

Using 4-fluoro-N-hydroxymethyl-N-(2-methoxyphenyl)benzene sulfonamide (302 mg, 0.928 mmol), the procedure of Inventive Example 18 was repeated to obtain 81 mg (16.9%) of the title compound in the form of yellow oil.
IR (neat): 2948, 1680, 1596, 1494, 1344, 1234, 1156, 1094, 838, 732, 586, 554 cm⁻¹
NMR (CDCl₃) δ: 1.50 - 2.31 (6H, m), 2.51 (2H, t, J = 7.5 Hz), 2.72 - 3.30 (3H, m), 3.39 (3H, s), 3.66 - 3.90 (2H, m), 6.60 - 7.50 (8H, m), 7.69, 7.93 (each 2H, dd, J = 9 Hz, 6 Hz)

### Inventive Example 20

### 4-(4-Fluorobenzoyl)-1-[3-(2-methoxyphenyl)-4-(3-methoxyphenyl)butyl]piperidine

In an atmosphere of argon, 3-(2-methoxyphenyl)-4-(3-methoxyphenyl)butyl alcohol (143 mg, 0.50 mmol) was dissolved in methylene chloride (5 ml) to which were subsequently added triethylamine (0.28 ml, 2.0 mmol), p-toluenesulfonyl chloride (105 mg, 0.55 mmol) and a catalytically effective amount of 4-dimethylaminopyridine at room temperature. After 15 hours of stirring at the same temperature, the reaction solution was mixed with 4-(4-fluorobenzyl)piperidine hydrochloride (182 mg, 0.75 mmol) and heated under reflux for 2.5 days. The reaction solution was diluted with water (10 ml), mixed with 2 N hydrochloric acid (5 ml), extracted with methylene chloride and then washed with 5% sodium hydroxide aqueous solution and water. After drying the resulting organic layer on anhydrous magnesium sulfate, the solvent was removed by evaporation to obtain an orange oily residue which was subsequently purified by a silica gel column chromatography (methylene chloride:ether = 1:1) to obtain 54 mg (22.7%) of the title compound in the form of colorless oil.
IR (neat): 2944, 1680, 1596, 1490, 1464, 1260, 1238, 1154, 752, 732 cm⁻¹
NMR (CDCl₃) δ: 1.36 - 2.35 (12H, m), 2.70 - 3.20 (4H, m), 3.70, 3.73 (each 3H, s), 6.50 - 7.30 (10H, m), 7.91 (2H, dd, J = 9 Hz, 6 Hz)

### Inventive Example 21

### 4-(4-Fluorobenzoyl)-1-[4-(2-methoxyphenyl)-5-(3-methoxyphenyl)pentyl]piperidine

Using 4-(2-methoxyphenyl)-5-(3-methoxyphenyl)pentyl alcohol (100 mg, 0.333 mmol), the procedure of Inventive Example 20 was repeated in an atmosphere of argon to obtain 21 mg (12.9%) of the title compound in the form of colorless oil.
IR (neat): 2940, 1680, 1598, 1490, 1262, 1238, 1156, 732 cm⁻¹
NMR (CDCl₃) δ: 1.15 - 2.40 (14H, m), 2.70 - 3.20 (4H, m), 3.71 (6H, s), 5.50 - 7.30 (10H, m), 7.92 (2H, dd, J = 9 Hz, 6 Hz)

### Inventive Example 22

### 4-Chloromethyl-N-{2-[4-(4-fluorobenzoyl)piperidino]ethyl-N-(2-methoxyphenyl)benzamide

Using 4-(4-fluorobenzoyl)-1-[2-(2-methoxyanilino)ethyl]piperidine (264 mg, 0.741 mmol) and 4-chloromethylbenzoyl chloride (145 mg, 0.741 mmol), the procedure of Reference Example 15 was repeated to obtain 314 mg (83.2%) of the title compound in a light yellow amorphous form.
IR (KBr): 2944, 1680, 1644, 1596, 1502, 1440, 1392, 1278, 1238, 1156, 750 cm⁻¹
NMR (CDCl₃) δ: 1.57 - 2.00 (4H, m), 2.00 - 2.45 (2H, m), 2.48 - 2.80 (2H, m), 2.80 - 3.38 (3H, m), 3.48 - 3.94 (1H, m), 3.66 (3H, s), 3.96 - 4.32 (1H, m), 4.44 (2H, s), 6.73 (1H, d, J = 7.5 Hz), 6.84 (1H, d, J = 8 Hz), 6.92 - 7.43 (8H, m), 7.94 (2H, dd, J = 9 Hz, 6 Hz)

### Inventive Example 23

### N-{2-[4-(4-Fluorobenzoyl)piperidino]ethyl}-4-methoxybenzene sulfonamide

In an atmosphere of argon, 1-(2-aminoethyl)-4-(4-fluorobenzoyl)piperidine (125 mg, 0.50 mmol) was dissolved in methylene chloride (3 ml) to which were subsequently added 4-methoxybenzenesulfonyl chloride (104 mg, 0.50 mmol) and triethylamine (0.07 ml, 0.50 mmol) at room temperature. After 1 hour of stirring at the same temperature, the reaction solution was mixed with saturated sodium carbonate aqueous solution (0.5 ml) under stirring. The resulting reaction solution was diluted with methylene chloride (15 ml) and dried on anhydrous sodium carbonate. After removing the solvent by evaporation, the resulting residue was purified by subjecting it to a silica gel column chromatography (ethyl acetate:methylene chloride = 3:1) to obtain 177 mg (84.2%) of the title compound in the form of colorless solid.
Melting point: 140 - 142°C
IR (KBr): 3288, 2948, 2816, 1680, 1598, 1500, 1322, 1260, 1158, 1096, 834, 560 cm⁻¹
NMR (CDCl₃) δ: 1.55 - 2.23 (6H, m), 2.42 (2H, t, J = 5 Hz), 2.55 - 2.82 (2H, m), 2.85 - 3.40 (1H, m), 2.99 (2H, t, J = 5 Hz), 3.85 (3H, s), 5.10 (1H, br-s), 6.83 - 7.30 (4H, m), 7.60 - 8.10 (4H, m)

### Inventive Example 24

### N-{3-[4-(4-Fluorophenyl)piperazinyl]propyl}-4-methoxybenzene sulfonamide

Using 1-(3-aminopropyl)-4-(4-fluorophenyl)piperazine (122 mg, 0.514 mmol) and 4-methoxybenzenesulfonyl chloride (107 mg, 0.514 mmol), the procedure of Inventive Example 23 was repeated to obtain 207 mg (98.8%) of the title compound in the form of colorless solid.
Melting point: 127 - 128.5°C
IR (KBr): 3060, 2960, 2840, 1600, 1508, 1326, 1262, 1218, 1156, 1084, 838, 576, 558 cm⁻¹
NMR (CDCl₃) δ: 1.46 - 1.87 (2H, m), 2.25 - 2.76 (6H, m), 2.90 - 3.30 (7H, m), 3.86 (3H, s), 6.66 - 7.13 (6H, m), 7.78 (2H, d, J = 9 Hz)

### Inventive Example 25

### N-Benzoyl-N-{2-[4-(4-fluorobenzoyl)piperidino]ethyl}-4-methoxybenzene sulfonamide

In an atmosphere of argon at room temperature, N-{2-[4-(4-fluorobenzoyl)piperidino]ethyl}-4-methoxybenzene sulfonamide (42 mg, 0.10 mmol) was dissolved in DMF (2 ml) to which was subsequently added sodium hydride (10 mg, 60%, 0.25 mmol), followed by 45 minutes of stirring under an ultrasonic irradiation condition and subsequent dropwise addition of benzoyl chloride (21 µl, 0.18 mmol). After 1 hour of stirring at room temperature, the reaction solution was mixed with saturated sodium bicarbonate (10 ml), extracted with ethyl acetate, washed with water and saturated brine, and then dried on anhydrous sodium carbonate. Thereafter, the solvent was removed by evaporation, and the resulting yellow oily residue was purified by subjecting it to PTLC (ether:hexane = 5:1) to obtain 23.7 mg (45.2%) of the title compound in the form of colorless oil.
IR (neat): 2948, 1680, 1596, 1580, 1500, 1448, 1352, 1314, 1262, 1160, 1090, 1014, 976, 834, 730, 578, 556 cm⁻¹
NMR (CDCl₃) δ: 1.47 - 1.82 (4H, m), 1.93 - 2.28 (2H, m), 2.43 - 2.90 (4H, m), 2.90 - 3.25 (1H, m), 3.87 (3H, s), 3.94 (2H, t, J = 7.5 Hz), 6.80 - 7.65 (9H, m), 7.70 - 8.10 (4H, m)

### Inventive Example 26

### N-Acetyl-N-{2-[4-(4-fluorobenzoyl)piperidino]ethyl}-4-methoxybenzene sulfonamide

Using N-{2-[4-(4-fluorobenzoyl)piperidino]ethyl}-4-methoxybenzene sulfonamide (42 mg, 0.10 mmol) and acetic anhydride (25 µl, 0.18 mmol), the procedure of Inventive Example 25 was repeated to obtain 27.6 mg (59.7%) of the title compound in the form of colorless oil.
IR (neat): 2940, 1682, 1596, 1500, 1352, 1310, 1262, 1160, 1090, 974, 834, 730, 578, 558 cm⁻¹
NMR (CDCl₃) δ: 1.63 - 2.00 (4H, m), 2.00 - 2.45 (2H, m), 2.31 (3H, s), 2.67 (2H, t, J = 7.5 Hz), 2.88 - 3.40 (3H, m), 3.89 (3H, s), 3.92 (2H, t, J = 6 Hz), 6.83 - 7.30 (4H, m), 7.73 - 8.10 (4H, m)

### Inventive Example 27

### N-Benzyl-N-{2-[4-(4-fluorobenzoyl)piperidino]ethyl}-4-methoxybenzene sulfonamide

Using N-{2-[4-(4-fluorobenzoyl)piperidino]ethyl}-4-methoxybenzene sulfonamide (42 mg, 0.10 mmol) and benzyl chloride (21 µl, 0.18 mmol), the procedure of Inventive Example 25 was repeated to obtain 22.8 mg (44.7%) of the title compound in the form of colorless oil.
IR (neat): 2946, 1680, 1598, 1498, 1338, 1302, 1260, 1094, 730, 560 cm⁻¹
NMR (CDCl₃) δ: 1.52 - 2.16 (6H, m), 2.34 (2H, t, J = 6.5 Hz), 2.59 - 2.90 (2H, m), 2.90 - 3.40 (1H, m), 3.23 (2H, t, J = 6.5 Hz), 3.88 (3H, s), 4.39 (2H, s), 6.87 - 7.35 (4H, m), 7.29 (5H, s), 7.65 - 8.05 (4H, m)

### Inventive Example 28

### N-{2-[4-(4-Fluorobenzoyl)piperidino]ethyl}-4-methoxy-N-(2-propenyl)benzene sulfonamide

Using N-{2-[4-(4-fluorobenzoyl)piperidino]ethyl}-4-methoxybenzene sulfonamide (68 mg, 0.162 mmol) and allyl chloride (20 µl, 0.224 mmol), the procedure of Inventive Example 25 was repeated to obtain 63 mg (84.4%) of the title compound in the form of colorless oil.
IR (neat): 2944, 1680, 1596, 1500, 1302, 1230, 1206, 1156, 1094, 976, 834, 586, 560 cm⁻¹
NMR (CDCl₃) δ: 1.56 - 2.31 (6H, m), 2.54 (2H, t, J = 7 Hz), 2.75 - 3.39 (5H, m), 3.86 (5H, s), 5.13 (1H, d, J = 12 Hz), 5.15 (1H, d, J = 16.5 Hz), 5.44 - 5.94 (1H, m), 6.70 - 7.30 (4H, m), 7.50 - 8.10 (4H, m)

### Inventive Example 29

### N-Ethoxycarbonylmethyl-N-{2-[4-(4-fluorobenzoyl)piperidino]ethyl}-4-methoxybenzene sulfonamide

Using N-{2-[4-(4-fluorobenzoyl)piperidino]ethyl}-4-methoxybenzene sulfonamide (68 mg, 0.162 mmol) and ethyl bromoacetate (25 µl, 0.224 mmol), the procedure of Inventive Example 25 was repeated to obtain 61 mg (74.3%) of the title compound in the form of light yellow oil.
IR (neat): 2948, 1750, 1680, 1596, 1500, 1338, 1302, 1260, 1228, 1206, 1156, 1094 cm⁻¹
NMR (CDCl₃) δ: 1.22 (3H, t, J = 7.5 Hz), 1.44 - 2.27 (6H, m), 2.57 (2H, t, J = 6 Hz), 2.70 - 3.50 (3H, m), 3.37 (2H, t, J = 6 Hz), 3.85 (3H, s), 4.10 (2H, q, J = 7.5 Hz), 4.21 (2H, s), 6.80 - 7.30 (4H, m), 7.65 - 8.25 (4H, m)

### Inventive Example 30

### N-Benzoyl-N-{3-[4-(4-fluorophenyl)piperazinyl]propyl}-4-methoxybenzene sulfonamide

Using N-{3-[4-(4-fluorophenyl)piperazinyl]propyl}-4-methoxybenzene sulfonamide (47 mg, 0.115 mmol) and benzoyl chloride (21 µl, 0.18 mmol), the procedure of Inventive Example 25 was repeated to obtain 42.2 mg (71.7%) of the title compound in the form of colorless oil.
IR (neat): 2940, 2820, 1684, 1596, 1512, 1500, 1356, 1262, 1232, 1162, 1090, 1018, 830, 730, 700, 578, 558 cm⁻¹
NMR (CDCl₃) δ: 2.70 - 3.22 (4H, m), 3.55 - 4.03 (2H, m), 3.85 (3H, s), 6.59 - 7.10 (6H, m), 7.17 - 7.60 (5H, m), 7.81 (2H, d, J = 9 Hz)

### Inventive Example 31

### N-{2-[4-(4-fluorobenzoyl)piperidino]ethyl}-4-methoxymethyl-N-(2-methoxyphenyl)benzamide

In an atmosphere of argon, 4-methoxymethyl-N-(2-methoxyphenyl)benzamide (126 mg, 0.464 mmol), 1-(2-chloroethyl)-4-(4-fluorobenzoyl)piperidine hydrochloride (157 mg, 0.511 mmol) and sodium iodide (154 mg, 1.02 mmol) were dissolved in DMF (3 ml) to which was subsequently added sodium hydride (40 mg, 60%, 1.0 mmol) at room temperature. After stirring at room temperature for 10 minutes and then at 60°C for 3.5 hours, DMF was removed by evaporation. The resulting residue was diluted with water (10 ml) and extracted with ethyl acetate, and the organic layer was washed with water and saturated brine and then dried on anhydrous sodium carbonate. Thereafter, the solvent was removed by evaporation, and the resulting residue was purified by subjecting it to a silica gel column chromatography (methylene chloride:methanol = 80:1) to obtain 128 mg (54.7%) of the title compound in the form of colorless oil.
IR (neat): 2940, 1680, 1644, 1598, 1410, 1378, 1308, 1278, 1262, 1240, 1158, 1112, 752, 604 cm⁻¹
NMR (CDCl₃) δ: 1.55 - 1.95 (4H, m), 1.98 - 2.36 (2H, m), 2.63 (2H, t, J = 7.5 Hz), 2.80 - 3.46 (3H, m), 3.29 (3H, s), 3.48 - 3.92 (1H, m), 3.67(3H, s), 3.94 - 4.50 (1H, m), 4.33 (2H, s), 6.73 (1H, d, J = 8 Hz), 6.83 (1H, d, J = 8 Hz), 6.93 - 7.43 (8H, m), 7.95 (2H, dd, J = 9 Hz, 6 Hz)

### Inventive Example 32

### N-{2-[4-(4-fluorobenzoyl)piperidino]ethyl}-3-methoxy-N-(2-pyridyl)benzamide

Using 3-methoxy-N-(2-pyridyl)benzamide (41 mg, 0.18 mmol), the procedure of Inventive Example 31 was repeated to obtain 19 mg (25.9%) of the title compound in the form of yellow oil.
IR (neat): 2928, 1680, 1634, 1598, 1572, 1544, 1502, 1452, 1388, 1350, 1286, 1228, 1156, 766, 730 cm⁻¹
NMR (CDCl₃) δ: 1.40 - 2.06 (4H, m), 2.06 - 2.47 (2H, m), 2.57 - 3.40 (5H, m), 3.85 (3H, s), 4.43 (2H, t, J = 6 Hz), 6.35 - 6.62 (1H, m), 6.70 - 7.70 (7H, m), 7.70 - 8.07 (3H, m), 8.35 (1H, d, J = 9 Hz)

### Inventive Example 33

### N-{2-[4-(4-fluorobenzoyl)piperidino]ethyl}-4-tetrahydropyranyloxymethyl-N-(2-methoxyphenyl)benzamide

Using 4-tetrahydropyranyloxymethyl-N-(methoxyphenyl)benzamide (166 mg, 0.486 mmol), the procedure of Inventive Example 31 was repeated to obtain 163 mg (58.4%) of the title compound in the form of light yellow oil.
IR (neat): 2944, 1680, 1644, 1598, 1502, 1390, 1278, 1240, 1202, 1156, 1118, 1032, 974, 752, 604 cm⁻¹
NMR (CDCl₃) δ: 1.36 - 2.00 (10H, m), 2.00 - 2.40 (2H, m), 2.63 (2H, t, J = 7.5 Hz), 2.80 - 3.30 (3H, m), 3.30 - 4.30 (4H, m), 3.78 (3H, s), 4.36 (1H, d, J = 12.5 Hz), 4.60 (1H, br-s), 4.66 (1H, d, J = 12.5 Hz), 6.72 (1H, d, J = 8 Hz), 6.82 (1H, d, J = 7.5 Hz), 6.93 - 7.40 (8H, m), 7.93 (2H, dd, J = 9 Hz, 6 Hz)

### Inventive Example 34

### N-{2-[4-(4-fluorobenzoyl)piperidino]ethyl}-3-tetrahydropyranyloxymethyl-N-(2-methoxyphenyl)benzamide

Using 3-tetrahydropyranyloxymethyl-N-(2 methoxyphenyl)benzamide (159 mg, 0.466 mmol), the procedure of Inventive Example 31 was repeated to obtain 191 mg (71.3%) of the title compound in the form of light yellow oil.
IR (neat): 2944, 1680, 1646, 1598, 1502, 1386, 1278, 1118, 1026 cm⁻¹
NMR (CDCl₃) δ: 1.38 - 2.00 (10H, m), 2.00 - 2.36 (2H, m), 2.63 (2H, t, J = 6.5 Hz), 2.80 - 3.30 (3H, m), 3.35 - 4.30 (4H, m), 3.68 (3H, s), 4.33 (1H, d, J = 12.5 Hz), 4.54 (1H, br-s), 4.61 (1H, d, J = 12.5 Hz), 6.72 (1H, d, J = 8 Hz), 6.82 (1H, d, J = 8 Hz), 6.91 - 7.40 (8H, m), 7.95 (2H, dd, J = 9 Hz, 6 Hz)

### Inventive Example 35

### N-{2-[4-(4-fluorobenzoyl)piperidino]ethyl}-4-tetrahydropyranyloxymethyl-N-(3-methoxybenzyl)benzamide

Using 4-tetrahydropyranyloxymethyl-N-(3-methoxybenzyl)benzamide (178 mg, 0.50 mmol), the procedure of Inventive Example 31 was repeated to obtain 130 mg (44.2%) of the title compound in the form of colorless oil.
IR (neat): 2944, 1680, 1636, 1598, 1422, 1262, 1234, 1156, 1128, 1034, 974 cm⁻¹
NMR (CDCl₃) δ: 1.38 - 2.32 (14H, m), 2.36 - 3.68 (6H, m), 3.70 - 4.10 (1H, m), 3.80 (3H, s), 4.37 - 4.95 (5H, m), 6.60 - 6.95 (2H, m), 7.07 - 7.57 (6H, m), 7.14 (2H, dd, J = 9 Hz, 9 Hz), 7.95 (2H, dd, J = 9 Hz, 6 Hz)

### Inventive Example 36

### 4-Nitro-N-{2-[4-(4-fluorobenzoyl)piperidino]ethyl}-N-(2-methoxyphenyl)benzamide

Using 4-nitro-N-(2-methoxyphenyl)benzamide (272 mg, 1 mmol), the procedure of Inventive Example 31 was repeated to obtain 348 mg (68.8%) of the title compound in the form of light yellow powder.
Melting point: 166 - 168°C
IR (KBr): 2938, 2818, 1680, 1644, 1596, 1518, 1503, 1380, 1344, 1302, 1275 cm⁻¹
NMR (CDCl₃) δ: 1.60 - 1.95 (4H, m), 2.00 - 2.34 (2H, m), 2.45 - 2.76 (2H, m), 2.80 - 3.30 (3H, m), 3.68 (3H, s), 3.60 - 3.85 (1H, m), 4.02 - 4.34 (1H, m), 6.62 - 6 90 (2H, m), 6.98 - 7.26 (4H, m), 7.40 (2H, d, J = 8.0 Hz), 7.80 - 8.03 (4H, m)

### Inventive Example 37

### 4-{[N-[2-[4-(4-Fluorobenzoyl)piperidino]ethyl]-2-methoxyanilino]carbonyl}pyridine

Using 4-{(2-methoxyanilino)carbonyl}pyridine (200 mg, 0.87 mmol), the procedure of Inventive Example 31 was repeated to obtain 215 mg (53.2%) of the title compound in a colorless amorphous form.
IR (KBr): 2944, 2818, 1674, 1647, 1598, 1503, 1413, 1230, 1206, 747 cm⁻¹
NMR (CDCl₃) δ: 1.65 - 2.35 (6H, m), 2.50 - 2.76 (2H, m), 2.80 - 3.28 (3H, m), 3.70 (3H, s), 3.55 - 3.82 (1H, m), 4.05 - 4.33 (1H, m), 6.64 - 6.92 (2H, m), 7.00 - 7.24 (6H, m), 7.80 - 8.07 (2H, m), 8.40 (2H, d, J = 6.0 Hz)

### Inventive Example 38

### 4-{N-[2-[4-(4-Fluorobenzoyl)piperidino]ethyl]-2-methoxyanilino]carbpnyl}pyridine N-oxide

Using 4-[(2-methoxyanilino)carbonyl)pyridine N-oxide (200 mg, 0.82 mmol), the procedure of Inventive Example 31 was repeated to obtain 302 mg (77.2%) of the title compound in the form of light yellow powder amorphous.
IR (KBr): 2944, 1680, 1647, 1596, 1503, 1443, 1398, 1260, 1167 cm⁻¹
NMR (CDCl₃) δ: 1.60 - 2.40 (6H, m), 2.49 - 2.72 (2H, m), 2.78 - 3.29 (3H, m), 3.69 (3H, s), 3.66 - 3.87 (1H, m), 3.97 - 4.25 (1H, m), 6.70 - 7.34 (8H, m), 7.80 - 8.06 (4H, m)

### Inventive Example 39

### N-{2-[4-(4-Fluorobenzoyl)piperidino]ethyl}-N-(2-methoxyphenyl)-3-nitrobenzene sulfonamide

Using N-(2-methoxyphenyl)-3-nitrobenzene sulfonamide (190 mg, 0.616 mmol), the procedure of Inventive Example 31 was repeated to obtain 70 mg (21.0%) of the title compound in the form of yellow oil.
IR (neat): 2950, 1678, 1596, 1530, 1496, 1350, 1280, 1260, 1160, 1122, 974, 910, 754, 732, 592, 576 cm⁻¹
NMR (CDCl₃) δ: 1.54 - 2.32 (6H, m), 2.53 (2H, t, J = 7.5 Hz), 2.70 - 3.30 (3H, m), 3.38 (3H, s), 3.57 - 4.00 (2H, m), 6.67 - 7.77 (7H, m), 7.77 - 8.14 (3H, m), 8.37 (1H, d, J = 7.5 Hz), 8.57 (1H, br-s)

### Inventive Example 40

### N-{2-[4-(4-Fluorobenzoyl)piperidino]ethyl}-4-methoxy-N-(2-thiazolyl)benzene sulfonamide

Using 4-methoxy-N-(2-thiazolyl)benzene sulfonamide (135 mg, 0.50 mmol), the procedure of Inventive Example 31 was repeated to obtain 53 mg (21.0%) of the title compound in the form of beige powder.
IR (KBr): 1680, 1596, 1510, 1290, 1256, 1232, 1140, 1086, 930, 564 cm⁻¹
NMR (CDCl₃) δ: 1.50 - 1.95 (4H, m), 1.96 - 2.33 (2H, m), 2.44 - 3.37 (5H, m), 3.81 (3H, s), 4.02 (2H, t, J = 6 Hz), 6.70 - 7.33 (5H, m), 7.73 - 8.20 (4H, m)

### Inventive Example 41

### N-{2-[4-(4-Fluorobenzoyl)piperidino]ethyl}-4-methoxy-N-(3-methoxyphenyl)benzene sulfonamide

Using 4-methoxy-N-(3-methoxyphenyl)benzene sulfonamide (174 mg, 0.593 mmol), the procedure of Inventive Example 31 was repeated to obtain 87.4 mg (28.0%) of the title compound in the form of colorless flocculent crystals.
Melting point: 129 - 132°C
IR (KBr): 1674, 1598, 1496, 1344, 1258, 1238, 1210, 1160, 1098, 1030, 690, 586, 562 cm⁻¹
NMR (CDCl₃) δ: 1.44 - 1.90 (4H, m), 1.97 - 2.32 (2H, m), 2.49 (2H, t, J = 7.5 Hz), 2.68 - 3.33 (3H, m), 3.67 (2H, t, J = 7.5 Hz), 3.74, 3.85 (each 3H, s), 6.47 - 7.33 (8H, m), 7.54 (2H, d, J = 9 Hz), 7.93 (2H, dd, J = 9 Hz, 6 Hz)

### Inventive Example 42

### N-(2-Cyanophenyl)-N-{2-[4-(4-fluorobenzoyl)piperidino]ethyl}-4-methoxybenzene sulfonamide

Using N-(2-cyanophenyl)-4-methoxybenzene sulfonamide (234 mg, 0.812 mmol), the procedure of Inventive Example 31 was repeated to obtain 107 mg (25.3%) of the title compound in a light yellow amorphous form.
IR (KBr): 2950, 2228, 1680, 1596, 1496, 1350, 1262, 1158, 1092, 834, 688, 576, 552 cm⁻¹
NMR (CDCl₃) δ: 1.35 - 2.22 (6H, m), 2.54 (2H, t, J = 6.5 Hz), 2.65 - 3.28 (3H, m), 3.73 (2H, t, J = 6.5 Hz), 3.87 (3H, s), 6.77 - 7.35 (5H, m), 7.35 - 7.77 (5H, m), 7.91 (2H, dd, J = 9 Hz, 6 Hz)

### Inventive Example 43

### N-{2-[4-(4-Fluorobenzoyl)piperidino]ethyl}-N-(2-trifluoromethylphenyl)-4-methoxybenzene sulfonamide

Using N-(2-trifluoromethylphenyl)-4-methoxybenzene sulfonamide (225 mg, 0.679 mmol), the procedure of Inventive Example 31 was repeated to obtain 46.7 mg (12.2%) of the title compound in the form of colorless oil.
IR (neat): 2948, 1680, 1598, 1498, 1450, 1352, 1316, 1262, 1228, 1206, 1158, 1112, 1092, 1036, 836, 730, 664, 604, 580, 556 cm⁻¹
NMR (CDCl₃) δ: 1.40 - 2.20 (6H, m), 2.30 - 3.30 (5H, m), 3.40 - 3.78 (2H, m), 3.88 (3H, s), 6.83 - 7.27 (5H, m), 7.35 - 7.55 (2H, m), 7.56 - 7.80 (3H, m), 7.90 (2H, dd, J = 9 Hz, 6 Hz)

### Inventive Example 44

### N-Cyclohexyl-N-{2-[4-(4-fluorobenzoyl)piperidino]ethyl}-4-methoxybenzene sulfonamide

Using N-cyclohexyl-4-methoxybenzene sulfonamide (290 mg, 1.08 mmol), the procedure of Inventive Example 31 was repeated to obtain 300 mg (55.3%) of the title compound in a light yellow glutinous form.
IR (neat): 2936, 2856, 1680, 1596, 1498, 1448, 1334, 1300, 1258, 1236, 1150, 1092, 972, 664, 582, 556 cm⁻¹
NMR (CDCl₃) δ: 1.00 - 2.40 (16H, m), 2.48 - 2.80 (2H, m), 2.85 - 3.74 (6H, m), 3.85 (3H, s), 6.94 (2H, d, J = 9 Hz), 7.13 (2H, dd, J = 9 Hz, 9 Hz), 7.76 (2H, d, J = 9 Hz), 7.96 (2H, dd, J = 9 Hz, 6 Hz)

### Inventive Example 45

### N-{2-[4-(4-Fluorobenzoyl)piperidino]ethyl}-N-(2-methoxyphenyl)-3-pyridine sulfonamide

Using N-(2-methoxyphenyl)-3-pyridine sulfonamide (132 mg, 0.50 mmol), the procedure of Inventive Example 31 was repeated to obtain 55 mg (22.1%) of the title compound in the form of colorless solid.
Melting point: 126.5 - 128.5°C
IR (KBr): 2924, 1676, 1594, 1500, 1364, 1284, 1204, 1164, 1116, 982, 744, 608, 600 cm⁻¹
NMR (CDCl₃) δ: 1.40 - 1.90 (4H, m), 1.94 - 2.31 (2H, m), 2.52 (2H, t, J = 7.5 Hz), 2.68 - 3.45 (3H, m), 3.33 (3H, s), 3.50 - 3 90 (2H, m), 6.76 (1H, d, J = 8 Hz), 6.85 - 7.60 (6H, m), 7.73 - 8.20 (2H, m), 8.60 - 9.00 (2H, m)

### Inventive Example 46

### 3-{[N-[2-[4-(4-Fluorobenzoyl)piperidino]ethyl]-2-methoxyanilino]sulfonyl}pyridine N-oxide

Using 3-[(2-methoxyanilino)sulfonyl]pyridine N-oxide (120 mg, 0.428 mmol), the procedure of Inventive Example 31 was repeated to obtain 101 mg (45.9%) of the title compound in the form of yellow oil.
IR (neat): 3430, 3110, 2944, 2805, 1678, 1596, 1498, 1466, 1430, 1358, 1258, 1160, 1010, 976, 912, 854, 786, 720, 670, 588 cm⁻¹
NMR (CDCl₃) δ: 1.55 - 1.95 (4H, m), 1.96 - 2.31 (2H, m), 2.50 (2H, t, J = 7.5 Hz), 2.70 - 3.24 (3H, m), 3.51 (3H, s), 3.60 - 3.91 (2H, m), 6.83 (1H, d, J = 8 Hz), 7.00 (1H, d, J = 7.5 Hz), 7.13 (2H, dd, J = 9 Hz, 9 Hz), 7.20 - 7.58 (4H, m), 7.95 (2H, dd, J = 9 Hz, 6 Hz), 8.27 (1H, d, J = 6 Hz), 8.50 (1H, br-s)

### Inventive Example 47

### 4-{[N-2-[4-(4-Fluorobenzoyl)piperidino]ethyl}-2-methoxyanilino]sulfonyl}benzoic acid

In an atmosphere of argon, 4-[(2-methoxyanilino)sulfonyl]benzoic acid (261 mg, 0.85 mmol), 1-(2-chloroethyl)-4-(4-fluorobenzoyl)piperidine hydrochloride (286 mg, 0.935 mmol) and sodium iodide (281 mg, 1.87 mmol) were dissolved in DMF (8 ml) to which was subsequently added sodium hydride (136 mg, 60%, 3.4 mmol) at room temperature. After 8 hours of stirring at 60°C, DMF was removed by evaporation. The thus obtained residue was diluted with water (10 ml), adjusted to pH 6 to 7 with 2 N hydrochloric acid and extracted with ethyl acetate (salting out). The resulting organic layer was washed with water and saturated brine and dried on anhydrous sodium sulfate. Thereafter, the solvent was removed by evaporation, and the resulting residue was purified by a silica gel column chromatography (methylene chloride:methanol = 50:1 - 40:1 - 30:1) to obtain 135 mg (29.4%) of the title compound in the form of beige amorphous powder.
IR (KBr): 1680, 1596, 1504, 1384, 1344, 1226, 1160 cm⁻¹
NMR (CDCl₃) δ: 1.75 - 2.50 (4H, m), 2.65 - 4.30 (12H, m), 6.50 - 8.20 (13H, m)
MS (FAB, m/z): 321, 357, 467, 499, 541 (M⁺ + 1)

### Inventive Example 48

### N-{2-[4-(4-Fluorobenzoyl)piperidino]ethyl}-N-(2-methoxyphenyl)-p-toluene sulfonamide

In an atmosphere of argon, N-(2-methoxyphenyl)-p-toluene sulfonamide (277 mg, 1.0 mmol) and a catalytically effective amount of sodium iodide were dissolved in DMF (3 ml) to which was subsequently added sodium hydride (44 mg, 60%, 1.1 mmol) at room temperature. After stirring at the same temperature for 30 minutes and then at 60°C for 10 minutes, 1-(2-chloroethyl)-4-(4-fluorobenzoyl)piperidine (324 mg, 1.2 mmol) which has been divided into 3 portions, each being dissolved in DMF (1 ml), was added to the reaction solution at 1 hour intervals at 60°C. After 1.5 hours of additional stirring at the same temperature, DMF was removed by evaporation. The thus obtained residue was diluted with water (10 ml) and extracted with ether. The resulting organic layer was washed with water and saturated brine, and dried on anhydrous sodium carbonate. Thereafter, the solvent was removed by evaporation, and the resulting residue was purified by a silica gel column chromatography (ether:hexane = 2:1) to obtain 467 mg (91.5%) of the title compound in the form of colorless oil.
IR (neat): 2944, 1680, 1598, 1496, 1342, 1280, 1262, 1158, 1094, 656 cm⁻¹
NMR (CDCl₃) δ: 1.54 - 1.94 (4H, m), 1.95 - 2.30 (2H, m), 2.40 (3H, s), 2.51 (2H, t, J = 7.5 Hz), 2.72 - 3.25 (3H, m), 3.37 (3H, s), 3.71 (2H, t, J = 7.5 Hz), 6.76 (1H, d, J = 8.5 Hz), 6.95 (1H, d, J = 8.5 Hz), 7.06 - 7.42 (4H, m), 7.12 (2H, dd, J = 9 Hz, 9 Hz), 7.58 (2H, d, J = 8.5 Hz), 7.93 (2H, dd, J = 9 Hz, 6 Hz)

### Inventive Example 49

### N-{2-[4-(4-Fluorobenzoyl)piperidino]ethyl}-4-tetrahydropyranyloxymethyl-N-(2-methoxyphenyl)benzene sulfonamide

Using 4-tetrahydropyranyloxymethyl-N-(2-methoxyphenyl)benzene sulfonamide (377 mg, 1.0 mmol), the procedure of Inventive Example 48 was repeated to obtain 524 mg (85.8%) of the title compound in the form of light yellow oil.
IR (neat): 2924, 1680, 1598, 1498, 1344, 1262, 1160, 1118, 1034, 976, 908, 732, 592 cm⁻¹
NMR (CDCl₃) δ: 1.40 - 2.33 (12H, m), 2.52 (2H, t, J = 7.5 Hz), 2.70 - 3.20 (3H, m), 3.36 (3H, s), 3.50 - 4.06 (4H, m), 4.53 (1H, d, J = 12.5 Hz), 4.70 (1H, br-s), 4.83 (1H, d, J = 12.5 Hz), 6.60 - 7.35 (6H, m), 7.40 (2H, d, J = 9 Hz), 7.65 (2H, d, J = 9 Hz), 7.93 (2H, dd, J = 9 Hz, 6 Hz)

### Inventive Example 50

### N-{2-[4-(4-Fluorobenzoyl)piperidino]ethyl}-4-tetrahydropyranyloxymethyl-N-(2-methoxyethyl)benzamide

Using 4-tetrahydropyranyloxymethyl-N-(2-methoxyethyl)benzamide (268 mg, 0.914 mmol), the procedure of Inventive Example 48 was repeated to obtain 127 mg (26.4%) of the title compound in the form of light yellow solid.
Melting point: 91 - 95°C
IR (KBr): 2944, 1664, 1628, 1596, 1430, 1224, 1114, 1016, 978, 906, 840, 596 cm⁻¹
NMR (CDCl₃) δ: 1.36 - 2.30 (12H, m), 2.36 - 3.37 (5H, m), 3.32 (3H, s), 3.39 - 4.17 (8H, m), 4.51 (1H, d, J = 12.5 Hz), 4.68 (1H, br-s), 4.78 (1H, d, J = 12.5 Hz), 7.12 (2H, dd, J = 9 Hz, 9 Hz), 7.38 (4H, s), 7.93 (2H, dd, J = 9 Hz, 6 Hz)

### Inventive Example 51

### N-{2-[4-(4-Fluorobenzoyl)piperidino]ethyl}-N-(2-methoxyphenyl)-4-phthalimidomethylbenzamide

Method A: Using N-(2-methoxyphenyl)-4-phthalimidomethylbenzamide (167 mg, 0.432 mmol), the procedure of Inventive Example 48 was repeated to obtain 56 mg (20.9%) of the title compound in a light yellow amorphous form.
Method B: Using crude N-formylmethyl-N-(2-methoxyphenyl)-4-phthalimidomethylbenzamide (300 mg, 0.70 mmol), the procedure of Inventive Example 14 was repeated to obtain 186 mg (28.8%) of the title compound.
Method C: Using 4-chloromethyl-N-{2-[4-(4-fluorobenzoyl)piperidino]ethyl}-N-(2-methoxyphenyl)benzamide (310 mg, 0.609 mmol), the procedure of Reference Example 36 was repeated to obtain 330 mg (87.4%) of the title compound.
   IR (KBr): 2944, 1716, 1680, 1642, 1596, 1502, 1392, 1306, 1278, 1238, 974, 938, 752, 716 cm⁻¹
   NMR (CDCl₃) δ: 1.53 - 1.95 (4H, m), 1.97 - 2.33 (2H, m), 2.60 (2H, t, J = 7.5 Hz), 2.77 - 3.37 (3H, m), 3.48 - 3.87 (1H, m), 3.65 (3H, s), 3.96 - 4.36 (1H, m), 4.72 (2H, s), 6.72 (1H, d, J = 8 Hz), 6.80 (1H, d, J = 7.5 Hz), 6.93 - 7.35 (8H, m), 7.55 - 8.13 (6H, m)

### Inventive Example 52

### N-{2-[4-(4-Fluorobenzoyl)piperidino]ethyl}-N-(2-methoxyphenyl)-4-morpholinomethylbenzamide

Using N-(2-methoxyphenyl)-4-morpholinomethylbenzamide (128 mg, 0.392 mmol), the procedure of Inventive Example 48 was repeated to obtain 48.3 mg (22.0%) of the title compound in the form of colorless oil.
IR (neat): 2948, 1680, 1640, 1598, 1502, 1454, 1412, 1390, 1304, 1280, 1262, 1240, 1116, 866, 752, 732, 604 cm⁻¹
NMR (CDCl₃) δ: 1.54 - 1.93 (4H, m), 2.00 - 2.50 (2H, m), 2.63 (2H, t, J = 7.5 Hz), 2.76 - 3.26 (3H, m), 3.36 (2H, s), 3.46 - 3.96 (5H, m), 3.67 (3H, s), 3.98 - 4.40 (1H, m), 6.72 (1H, d, J = 7.5 Hz), 6.81 (1H, d, J = 7.5 Hz), 6.91 - 7.37 (8H, m), 7.94 (2H, dd, J = 9 Hz, 6 Hz)

### Inventive Example 53

### N-{2-[4-(4-Fluorobenzoyl)piperidino]ethyl}-N-(2-methoxyphenyl)-4-dimethylaminomethylbenzamide

Using N-(2-methoxyphenyl)-4-dimethylaminomethylbenzamide (131 mg, 0.461 mmol), the procedure of Inventive Example 48 was repeated to obtain 106 mg (44.4%) of the title compound in a light yellow amorphous form.
IR (KBr): 1680, 1640, 1598, 1412, 1316, 1280, 1222 cm⁻¹
NMR (CDCl₃-CD₃OD) δ: 1.55 - 2.06 (4H, m), 2.06 - 2.56 (2H, m), 2.21 (6H, s), 2.73 (2H, t, J = 7.5 Hz), 2.86 - 3.30 (3H, m), 3.43 (2H, s), 3.56 - 4.40 (2H, m), 3.70 (3H, s), 6.55 - 6.92 (2H, m), 6.94 - 7.43 (8H, m), 7.96 (2H, dd, J = 9 Hz, 6 Hz)

### Inventive Example 54

### 4-(N-tert-Butoxycarbonyl-N-methylamino)methyl-N-{2-[4-(4-fluorobenzoyl)piperidino]ethyl}-N-(2-methoxyphenyl)benzamide

Using 4-(N-tert-butoxycarbonyl-N-methylamino)methyl-N-(2-methoxyphenyl)benzamide (493 mg, 1.33 mmol), the procedure of Inventive Example 48 was repeated to obtain 580 mg (74.0%) of the title compound in the form of colorless oil.
IR (neat): 2938, 1737, 1692, 1644, 1596, 1503, 1392, 1239, 1143 cm⁻¹
NMR (CDCl₃) δ: 1.41 (9H, s), 1.62 - 2.30 (6H, m), 2.52 - 3.26 (5H, m), 2.72 (3H, s), 3.60 - 3.84 (1H, m), 3.69 (3H, s), 4.00 - 4.20 (1H, m), 4.29 (2H, s), 6.65 - 7.30 (10H, m), 7.95 (2H, dd, J = 9 Hz, 6 Hz)

### Inventive Example 55

### 4-Cyano-N-{2-[4-(4-fluorobenzoyl)piperidino]ethyl}-N-(2-methoxyphenyl)benzamide

Using 4-cyano-N-(2-methoxyphenyl)benzamide (252 mg, 1.0 mmol), the procedure of Inventive Example 48 was repeated to obtain 250 mg (51.5%) of the title compound in the form of colorless flocculent crystals.
Melting point: 155 - 158°C
IR (KBr): 2932, 2228, 1678, 1642, 1594, 1502, 1406, 1392, 1306, 1274, 1226, 854 cm⁻¹
NMR (CDCl₃) δ: 1.65 - 1.97 (4H, m), 1.97 - 2.34 (2H, m), 2.42 - 2.74 (2H, m), 2.75 - 3.37 (3H, m), 3.40 - 3.90 (1H, m), 3.69 (3H, s), 4.00 - 4.43 (1H, m), 6.56 - 6.95 (2H, m), 6.96 - 7.70 (8H, m), 7.95 (2H, dd, J = 9 Hz, 6 Hz)

### Inventive Example 56

### 4-Nitro-N-{2-[4-(4-fluorobenzoyl)piperidino]ethyl}-N-(3-methoxyphenyl)benzamide

Using 4-nitro-N-(3-methoxyphenyl)benzamide (1.09 g, 4.00 mmol), the procedure of Inventive Example 48 was repeated to obtain 1.19 g (53.7%) of the title compound in the form of light yellow flocculent crystals.
Melting point: 133 - 135°C
IR (KBr): 1678, 1634, 1600, 1518, 1408, 1380, 1276, 1230, 1136, 1048, 978 cm⁻¹
NMR (CDCl₃) δ: 1.60 - 2.33 (5H, m), 2.60 (2H, t, J = 6.6 Hz), 1.97 - 2.34 (2H, m), 2.80 - 3.35 (4H, m), 3.66 (3H, s), 4.03 (2H, t, J = 6.6 Hz), 6.53 - 6.80 (3H, m), 7.00 - 7.15 (1H, m), 7.10 (2H, dd, each J = 9.0 Hz), 7.42 (2H, d, J = 9.0 Hz), 7.98 (2H, dd, J = 9.0 Hz, 6.0 Hz)

### Inventive Example 57

### 4-Nitro-N-{2-[4-(4-fluorobenzoyl)piperidino]ethyl}-N-(4-methoxyphenyl)benzamide

Using 4-nitro-N-(4-methoxyphenyl)benzamide (857 mg, 3.14 mmol), the procedure of Inventive Example 48 was repeated to obtain 973.5 mg (61.4%) of the title compound in the form of yellow powder.
Melting point: 154 - 156°C
IR (KBr): 1677, 1641, 1599, 1512, 1443, 1377, 1347, 1296, 1275, 1248, 1221, 1200, 1170, 1155, 1131, 1110, 1035, 972, 867 cm⁻¹
NMR (CDCl₃) δ: 1.67 - 2.00 (4H, m), 2.00 - 2.35 (2H, m), 2.60 (2H, t, J = 6.5 Hz), 2.80 - 3.40 (3H, m), 3.74 (3H, s), 4.03 (2H, t, J = 6.5 Hz), 6.72 (2H, d, J = 9.0 Hz), 6.83 - 7.50 (6H, m), 7.80 - 8.10 (4H, m)

### Inventive Example 58

### 4-Nitro-N-{2-[4-(4-fluorobenzoyl)piperidino]ethyl}-N-(2,5-dimethoxyphenyl)benzamide

Using 4-nitro-N-(2,5-dimethoxyphenyl)benzamide (1.21 g, 4.00 mmol), the procedure of Inventive Example 48 was repeated to obtain 1.48 g (69.1%) of the title compound in a yellow amorphous form.
Melting point: 154 - 156°C IR (KBr): 2950, 1682, 1644, 1600, 1504, 1346, 1224, 1048, 976,
714 cm⁻¹
NMR (CDCl₃) δ: 1.77 - 2.31 (6H, m), 2.61 - 3.19 (5H, m), 3.64 (3H, s), 3.69 (3H, s), 4.16 (2H, t, J = 7.0 Hz), 6.71 (2H, dd, J = 2.0 Hz, 8.1 Hz), 7.05 - 7.52 (5H, m), 7.88 - 8.04 (4H, m)

### Inventive Example 59

### 3-Nitro-N-{2-[4-(4-fluorobenzoyl)piperidino]ethyl}-N-(3-methoxyphenyl)benzamide

Using 3-nitro-N-(3-methoxyphenyl)benzamide (816.0 mg, 3.00 mmol), the procedure of Inventive Example 48 was repeated to obtain 823.9 mg (54.7%) of the title compound in the form of colorless powder.
Melting point: 123 - 125°C
IR (KBr): 2950, 1676, 1640, 1598, 1532, 1400, 1352, 1304, 1232, 1208, 1118, 1026 cm⁻¹
NMR (CDCl₃) δ: 1.55 - 2.31 (5H, m), 2.64 (2H, t, J = 6.6 Hz), 2.76 - 3.30 (4H, m), 3.71 (3H, s), 4.07 (2H, t, J = 6.6 Hz), 6.45 - 6.80 (3H, m), 6.90 - 7.38 (1H, m), 7.13 (2H, dd, each J = 8.8 Hz), 7.40 (1H, d, J = 7.7 Hz), 7.50 - 7.72 (1H, m), 7.72 - 8.20 (4H, m)

### Inventive Example 60

### 4-Nitro-N-{2-[4-(4-fluorobenzoyl)piperidino]ethyl}-N-(2,4-dimethoxyphenyl)benzamide

Using 4-nitro-N-(2,4-dimethoxyphenyl)benzamide (908 mg, 3.00 mmol), the procedure of Inventive Example 48 was repeated to obtain 1.46 g (91.0%) of the title compound in the form of orange powder.
Melting point: 51 - 54°C
IR (KBr): 1680, 1646, 1598, 1510, 1410, 1346, 1310, 1280, 1208, 1158, 1142, 1030, 852, 836 cm⁻¹
NMR (CDCl₃) δ: 1.25 - 3.73 (13H, m), 3.70 (3H, s), 3.73 (3H, s), 4.16 (2H, t, J = 7.0 Hz), 6.27 - 6.34 (2H, m), 6.98 - 7.23 (3H, m), 7.41 (2H, d, J = 8.6 Hz), 7.88 - 7.94 (2H, m), 7.99 (2H, d, J = 8.6 Hz)

### Inventive Example 61

### 4-Nitro-N-{2-[4-(4-fluorobenzoyl)piperidino]ethyl}-N-(3-methylphenyl)benzamide

Using 4-nitro-N-(3-methylphenyl)benzamide (640 mg, 2.50 mmol), the procedure of Inventive Example 48 was repeated to obtain 517 mg (42.1%) of the title compound in the form of colorless powder.
Melting point: 150 - 152°C
IR (KBr): 2935, 2830, 1677, 1641, 1599, 1515, 1410, 1344, 1299, 1281, 1227, 1134 cm⁻¹
NMR (CDCl₃) δ: 1.61 - 2.32 (6H, m), 2.26 (3H, s), 2.62 (2H, t, J = 7.0 Hz), 2.81 - 3.41 (3H, m), 4.06 (2H, t, J = 7.0 Hz), 6.71 - 7.71 (8H, m), 7.81 - 8.14 (4H, m)

### Inventive Example 62

### 4-Nitro-N-{2-[4-(4-fluorobenzoyl)piperidino]ethyl}-N-(phenyl)benzamide

Using 4-nitro-N-(phenyl)benzamide (970.0 mg, 4.01 mmol), the procedure of Inventive Example 48 was repeated to obtain 1.13 g (59.5%) of the title compound in the form of colorless powder.
Melting point: 177 - 181°C
IR (KBr): 1678, 1640, 1594, 1516, 1494, 1408, 1380, 1342, 1318, 1300, 1274, 1224, 1136, 976, 870, 852, 718, 698 cm⁻¹
NMR (CDCl₃) δ: 1.66 - 2.32 (6H, m), 2.60 (2H, dd, J = 6.6 Hz, 6.4 Hz), 2.93 - 3.30 (3H, m), 4.07 (2H, dd, J = 6.6 Hz, 6.4 Hz), 7.03 - 7.18 (7H, m), 7.41 (2H, d, J = 8.7 Hz), 7.88 - 8.04 (4H, m)

### Inventive Example 63

### 4-Nitro-N-{2-[4-(4-fluorobenzoyl)piperidino]ethyl}-N-(3-methylthiophenyl)benzamide

Using 4-nitro-N-(3-methylthiophenyl)benzamide (1.15 g, 4.00 mmol), the procedure of Inventive Example 48 was repeated to obtain 1.73 g (81.3%) of the title compound in the form of light yellow powder.
Melting point: 153 - 154°C
IR (KBr): 1680, 1644, 1602, 1580, 1522, 1434, 1372, 1344, 1308, 1298, 1226, 1138 cm⁻¹
NMR (CDCl₃) δ: 1.16 - 2.27 (6H, m), 2.36 (3H, s), 2.62 (2H, t, J = 6.4 Hz), 2.95 - 3.22 (3H, m), 4.07 (2H, t, J = 6.4 Hz), 6.79 - 6.86 (1H, m), 7.00 - 7.23 (5H, m), 7.43 (2H, d, J = 8.6 Hz), 7.93 (2H, d, J = 8.8 Hz), 8.03 (2H, d, J = 8.8 Hz)

### Inventive Example 64

### 4-Nitro-N-{2-[4-(4-fluorobenzoyl)piperidino]ethyl}-N-(2-trifluoromethylphenyl)benzamide

Using 4-nitro-N-(2-trifluoromethylphenyl)benzamide (450.0 mg, 1.45 mmol), the procedure of Inventive Example 48 was repeated to obtain 299 mg (40.6%) of the title compound in the form of yellow needle crystals.
Melting point: 156.6 - 157.8°C
IR (KBr): 2960, 2841, 1678, 1656, 1600, 1524, 1316, 1126, 862, 577, 614 cm⁻¹
NMR (CDCl₃) δ: 1.68 - 2.25 (6H, m), 2.61 - 2.86 (3H, m), 3.05 - 3.43 (2H, m), 4.57 (2H, brs), 7.09 (2H, d, J = 8.6 Hz), 7.33 - 7.61 (6H, m), 7.89 - 8.08 (4H, m)

### Inventive Example 65

### 4-Nitro-N-{2-[4-(4-fluorobenzoyl)piperidino]ethyl}-N-(3-trifluoromethylphenyl)benzamide

Using 4-nitro-N-(3-trifluoromethylphenyl)benzamide (1.55 g, 5.00 mmol), the procedure of Inventive Example 48 was repeated to obtain 1.14 g (42.0%) of the title compound in the form of colorless powder.
Melting point: 123 - 137.1°C
IR (KBr): 2948, 1678, 1648, 1602, 1344, 1136, 859, 722, 601 cm⁻¹
NMR (CDCl₃) δ: 1.79 - 1.95 (4H, m), 2.05 - 2.20 (2H, m), 2.60 (2H, t, J = 6.2 Hz), 2.90 - 3.29 (3H, m), 4.09 (2H, t, J = 9.0 Hz), 7.04 - 7.50 (8H, m), 7.88 - 8.10 (4H, m)

### Inventive Example 66

### 4-Nitro-N-{2-[4-(4-fluorobenzoyl)piperidino]ethyl}-N-(3-pyridyl)benzamide

Using 4-nitro-N-(3-pyridyl)benzamide (973 mg, 4.00 mmol), the procedure of Inventive Example 48 was repeated to obtain 178 mg (9.3%) of the title compound in the form of colorless powder.
IR (KBr): 2935, 1677, 1644, 1599, 1518, 1344, 1299, 1278, 1224, 1134, 849 cm⁻¹
NMR (CDCl₃) δ: 1.41 - 2.36 (6H, m), 2.62 (2H, t, J = 6.0 Hz), 2.76 - 3.41 (3H, m), 4.08 (2H, t, J = 6.0 Hz), 7.21 (2H, t, J = 9.0 Hz), 6.67 - 7.70 (4H, m), 7.70 - 8.17 (4H, m), 8.17 - 8.53 (2H, m)

### Inventive Example 67

### 2-Nitro-N-{2-[4-(4-fluorobenzoyl)piperidino]ethyl}-N-(3-methoxyphenyl)benzamide

Using 2-nitro-N-(3-methoxyphenyl)benzamide (816 mg, 3.00 mmol), the procedure of Inventive Example 48 was repeated to obtain 1.497 g (98.9%) of the title compound in a light yellow amorphous form.
IR (KBr): 2930, 1678, 1648, 1600, 1530, 1488, 1408, 1346, 1208, 1156, 910 cm⁻¹
NMR (CDCl₃) δ: 1.60 - 2.35 (6H, m), 2.67 (2H, t, J = 6.0 Hz), 2.90 - 3.30 (4H, m), 3.63 (3H, s), 4.05 (2H, t, J = 6.0 Hz), 7.10 (2H, dd, each J = 9.0 Hz), 6.53 - 6.80 (3H, m), 6.90 - 7.55 (4H, m), 7.75 - 8.05 (1H, m), 7.95 (2H, dd, J = 9.0 Hz, 6.0 Hz)

### Inventive Example 68

### 4-Nitro-N-{2-[4-(4-fluorobenzoyl)piperidino]ethyl}-N-(3-nitrophenyl)benzamide

Using 4-nitro-N-(3-nitrophenyl)benzamide (718 mg, 2.50 mmol), the procedure of Inventive Example 48 was repeated to obtain 1.11 g (53.4%) of the title compound in the form of light yellow oil.
IR (neat): 2950, 1680, 1656, 1600, 1530, 1408, 1350, 1274, 1226, 1156, 854 cm⁻¹
NMR (CDCl₃) δ: 1.40 - 2.40 (5H, m), 2.43 (2H, t, J = 6.0 Hz), 2.77 - 3.40 (4H, m), 4.09 (2H, t, J = 6.0 Hz), 7.13 (2H, dd, each J = 9.0 Hz), 7.76 - 8.27 (6H, m)

### Inventive Example 69

### 4-Nitro-N-{2-[4-(4-fluorobenzoyl)piperidino]ethyl}-N-(2-ethoxycarbonylphenyl)benzamide

Using 4-nitro-N-(2-ethoxycarbonylphenyl)benzamide (600.0 mg, 2.00 mmol), the procedure of Inventive Example 48 was repeated to obtain 523 mg (49.1%) of the title compound in the form of colorless powder.
Melting point: 187 - 190°C
IR (KBr): 1678, 1656, 1600, 1314, 846, 763, 645, 610 cm⁻¹
NMR (CDCl₃) δ: 1.71 - 1.89 (4H, m), 2.01 - 2.23 (2H, m), 2.26 - 2.83 (2H, m), 2.91 - 3.17 (3H, m), 3.48 - 3.63 (1H, m), 3.88 (3H, s), 4.30 - 4.53 (1H, m), 7.03 - 7.46 (7H, m), 7.76 - 8.02 (5H, m)

### Inventive Example 70

### 4-Cyano-N-{2-[4-(4-fluorobenzoyl)piperidino]ethyl}-N-(4-methoxyphenyl)benzamide

Using 4-cyano-N-(2-methoxyphenyl)benzamide (2.02 g, 8.00 mmol), the procedure of Inventive Example 48 was repeated to obtain 2.32 g (59.8%) of the title compound in the form of colorless powder.
Melting point: 136 - 149°C
IR (KBr): 2940, 2227, 1678, 1640, 1510, 1250, 1035, 972, 832, 601 cm⁻¹
NMR (CDCl₃) δ: 1.78 - 2.31 (6H, m), 2.58 (2H, t, J = 6.6 Hz), 2.94 - 3.30 (3H, m), 3.75 (3H, s), 4.01 (2H, t, J = 6.6 Hz), 6.40 (2H, d, J = 9.0 Hz), 6.94 - 7.42 (8H, m), 7.88 - 8.03 (2H, m)

### Inventive Example 71

### 3-Methyl-4-nitro-N-{2-[4-(4-fluorobenzoyl)piperidino]ethyl}-N-(4-methoxyphenyl)benzamide

Using 3-methyl-4-nitro-N-(4-methoxyphenyl)benzamide (1.14 g, 4.00 mmol), the procedure of Inventive Example 48 was repeated to obtain 1.06 g (51.1%) of the title compound in the form of colorless powder.
Melting point: 125.7 - 126.4°C
IR (KBr): 2930, 1678, 1640, 1510, 1346, 1250, 972, 835, 731, 602 cm⁻¹
NMR (CDCl₃) δ: 1.79 - 2.30 (6H, m), 2.49 (3H, s), 2.59 (2H, t, J = 6.4 Hz), 2.95 - 3.28 (3H, m), 3.75 (3H, s), 4.01 (2H, t, J = 6.4 Hz), 6.75 (2H, d, J = 9.0 Hz), 6.97 - 7.32 (6H, m), 7.11 (1H, d, J = 8.4 Hz), 7.95 (2H, dd, J = 8.8 Hz, 6.5 Hz)

### Inventive Example 72

### 3-Methoxy-4-nitro-N-{2-[4-(4-fluorobenzoyl)piperidino]ethyl}-N-(4-methoxyphenyl)benzamide

Using 3-methoxy-4-nitro-N-(4-methoxyphenyl)benzamide (1.36 g, 4.50 mmol), the procedure of Inventive Example 48 was repeated to obtain 1.60 g (66.5%) of the title compound in the form of light yellow powder.
Melting point: 152 - 153°C
IR (KBr): 2950, 1680, 1640, 1606, 1512, 1250, 975, 837, 720 cm⁻¹
NMR (CDCl₃) δ: 1.79 - 2.30 (6H, m), 2.59 (2H, t, J = 6.6 Hz), 2.95 - 3.36 (3H, m), 3.76 (3H, s), 3.81 (3H, s), 4.02 (2H, t, J = 6.6 Hz), 6.77 (2H, d, J = 9.0 Hz), 6.90 - 7.26 (6H, m), 7.60 (1H, d, J = 8.4 Hz), 7.96 (2H, dd, J = 8.6 Hz, 5.5 Hz)

### Inventive Example 73

### 4-Nitro-N-{2-[4-(4-fluorobenzoyl)piperidino]ethyl}-N-(4-fluorophenyl)benzamide

Using 4-nitro-N-(4-fluorophenyl)benzamide (1.04 g, 4.00 mmol), the procedure of Inventive Example 48 was repeated to obtain 1.41 g (71.6%) of the title compound in the form of light yellow powder.
Melting point: 194 - 196°C
IR (KBr): 1680, 1642, 1600, 1516, 1504, 1376, 1342, 1306, 1276, 1240, 1222, 1136 cm⁻¹
NMR (CDCl₃) δ: 1.78 - 2.18 (6H, m), 2.58 (2H, dd, J = 6.4, 6.2 Hz), 2.93 - 3.05 (3H, m), 4.03 (2H, dd, J = 6.4 Hz, 6.2 Hz), 6.92 - 7.23 (6H, m), 7.40 (2H, d, J = 8.6 Hz), 7.88 - 8.08 (4H, m)

### Inventive Example 74

### 4-Nitro-N-{2-[4-(4-fluorobenzoyl)piperidino]ethyl}-N-(4-methylphenyl)benzamide

Using 4-nitro-N-(4-methylphenyl)benzamide (1.03 g, 4.01 mmol), the procedure of Inventive Example 48 was repeated to obtain 1.61 g (59.1%) of the title compound in the form of light yellow powder.
Melting point: 175 - 178°C
IR (KBr): 1678, 1640, 1600, 1516, 1376, 1342, 1296, 1376, 1222 cm⁻¹
NMR (CDCl₃) δ: 1.66 - 2.16 (6H, m), 2.27 (3H, s), 2.59 (2H, dd, J = 6.6 Hz, 6.4 Hz), 2.95 - 3.10 (3H, m), 4.04 (2H, dd, J = 6.6 Hz, 6.4 Hz), 6.89 - 7.25 (6H, m), 7.41 (2H, d, J = 8.6 Hz), 7.88 - 8.05 (4H, m)

### Inventive Example 75

### 4-Nitro-N-{2-[4-(4-fluorobenzoyl)piperidino]ethyl}-N-(phenyl)benzamide

Using 4-nitro-N-(phenyl)benzamide (969.0 mg, 4.00 mmol), the procedure of Inventive Example 48 was repeated to obtain 1.02 g (53.6%) of the title compound in the form of light yellow powder.
Melting point: 178 - 180°C
IR (KBr): 1680, 1640, 1596, 1530, 1492, 1394, 1352, 1308, 1278, 1226, 1208, 1160, 1126, 970, 724, 706 cm⁻¹
NMR (CDCl₃) δ: 1.66 - 2.32 (6H, m), 2.63 (2H, t, J = 6.6 Hz), 2.94 - 3.27 (3H, m), 4.08 (2H, t, J = 6.6 Hz), 7.03 - 7.64 (9H, m), 7.87 - 8.02 (3H, m), 8.09 (1H, s)

### Inventive Example 76

### 3-Nitro-N-{2-[4-(4-fluorobenzoyl)piperidino]ethyl}-N-(4-methylphenyl)benzamide

Using 3-nitro-N-(4-methylphenyl)benzamide (1.03 g, 4.00 mmol), the procedure of Inventive Example 48 was repeated to obtain 1.07 g (57.0%) of the title compound in the form of light yellow powder.
Melting point: 131 - 134°C
IR (KBr): 1680, 1640, 1598, 1530, 1512, 1346 cm⁻¹
NMR (CDCl₃) δ: 1.66 - 2.16 (6H, m), 2.26 (3H, s), 2.60 (2H, t, J = 6.4 Hz), 2.95 - 3.20 (3H, m), 4.04 (2H, t, J = 6.4 Hz), 6.97 - 7.63 (8H, m), 7.86 - 8.02 (3H, m), 8.10 (1H, s)

### Inventive Example 77

### 3-Nitro-N-{2-[4-(4-fluorobenzoyl)piperidino]ethyl}-N-(4-fluorophenyl)benzamide

Using 3-nitro-N-(4-fluorophenyl)benzamide (1.04 g, 4.00 mmol), the procedure of Inventive Example 48 was repeated to obtain 507.0 mg (25.7%) of the title compound in the form of light yellow powder.
Melting point: 97 - 113°C
IR (KBr): 1656, 1594, 1530, 1508, 1350, 1310, 1216, 724 cm⁻¹
NMR (CDCl₃) δ: 1.66 - 2.31 (6H, m), 2.60 (2H, t, J = 6.6 Hz), 2.92 - 3.19 (3H, m), 4.03 (2H, t, J = 6.6 Hz), 6.82 - 7.22 (6H, m), 7.34 - 7.62 (2H, m), 7.87 - 8.03 (3H, m), 8.10 (1H, s)

### Inventive Example 78

### 3-Nitro-N-{2-[4-(4-fluorobenzoyl)piperidino]ethyl}-N-(3-methylthiophenyl)benzamide

Using 3-nitro-N-(3-methylthiophenyl)benzamide (1.54 g, 4.00 mmol), the procedure of Inventive Example 48 was repeated to obtain 1.04 g (49.6%) of the title compound in the form of light yellow powder.
Melting point: 148 - 149°C
IR (KBr): 1674, 1640, 1594, 1528, 1388, 1376, 1348, 1302, 1222, 1204, 1170, 1142, 726, 692 cm⁻¹
NMR (CDCl₃) δ: 1.69 - 2.26 (6H, m), 2.36 (3H, s), 2.63 (2H, t, J = 6.5 Hz), 2.95 - 3.20 (3H, m), 4.07 (2H, t, J = 6.5 Hz), 6.83 - 7.23 (7H, m), 7.30 (1H, d, J = 7.8 Hz), 7.63 (1H, d, J = 7.8 Hz), 7.92 (1H, d, J = 8.6 Hz), 7.98 (1H, d, J = 8.6 Hz), 7.34 - 7.62 (2H, m), 8.13 (1H, s)

### Inventive Example 79

### 4-Nitro-N-{2-[4-(4-fluorobenzoyl)piperidino]ethyl}-N-(3,4-methylenedioxyphenyl)benzamide

Using 4-nitro-N-(3,4-methylenedioxyphenyl)benzamide (1.14 g, 4.00 mmol), the procedure of Inventive Example 48 was repeated to obtain 1.49 g (72.0%) of the title compound in the form of light yellow powder.
Melting point: 197 - 198°C
IR (KBr): 1680, 1644, 1599, 1518, 1503, 1485, 1446, 1410, 1380, 1341, 1302, 1281, 1269, 1236, 1215, 1173, 1155, 1128, 1110, 1035, 972, 870, 843, 717 cm⁻¹
NMR (CDCl₃) δ: 1.65 - 2.00 (4H, m), 2.00 - 2.43 (2H, m), 2.60 (2H, t, J = 6.5 Hz), 2.83 - 3.33 (3H, m), 4.00 (2H, t, J = 6.5 Hz), 5.96 (2H, s), 6.48 - 6.70 (3H, m), 7.00 - 7.29 (3H, m), 7.45 (2H, d, J = 8.8 Hz), 7.80 - 8.20 (4H, m)

### Inventive Example 80

### 4-Nitro-N-{2-[4-(4-fluorobenzoyl)piperidino]ethyl}-N-(1-naphthyl)benzamide

Using 4-nitro-N-(1-naphthyl)benzamide (584.0 mg, 2.00 mmol), the procedure of Inventive Example 48 was repeated to obtain 375.7 mg (35.8%) of the title compound in the form of light yellow powder.
Melting point: 174 - 175°C
IR (KBr): 1680, 1644, 1599, 1521, 1410, 1380, 1341, 1305,
1278, 1230, 1206, 1155, 1134, 1110, 975, 870, 852, 832 cm⁻¹ NMR (CDCl₃) δ: 1.67 - 1.97 (4H, m), 1.97 - 2.32 (2H, m), 2.63 (2H, t, J = 6.6 Hz), 2.80 - 3.30 (3H, m), 3.74 (1H, quint., J = 6.6 Hz), 4.61 (1H, quint., J = 6.6 Hz), 6.93 - 8.10 (15H, m)

### Inventive Example 81

### 4-Nitro-N-{2-[4-(4-fluorobenzoyl)piperidino]ethyl}-N-(4-chlorophenyl)benzamide

Using 4-nitro-N-(4-chlorophenyl)benzamide (1.11 g, 4.00 mmol), the procedure of Inventive Example 48 was repeated to obtain 1.21 g (59.2%) of the title compound in the form of light yellow powder.
Melting point: 175 - 177°C
IR (KBr): 1680, 1644, 1600, 1490, 1374, 1348, 1306, 1280, 1270, 1222, 1134, 1094 cm⁻¹
NMR (CDCl₃) δ: 1.79 - 2.20 (6H, m), 2.59 (2H, t, J = 6.4 Hz), 2.93 - 3.05 (3H, m), 4.04 (2H, t, J = 6.4 Hz), 7.00 - 7.26 (6H, m), 7.42 (2H, d, J = 8.6 Hz), 7.89 - 8.10 (4H, m)

### Inventive Example 82

### 4-Nitro-N-{2-[4-(4-fluorobenzoyl)piperidino]ethyl}-N-(3,4-dimethylphenyl)benzamide

Using 4-nitro-N-(3,4-dimethylphenyl)benzamide (1.08 g, 4.00 mmol), the procedure of Inventive Example 48 was repeated to obtain 1.26 g (62.3%) of the title compound in the form of light yellow powder.
Melting point: 197 - 199°C
IR (KBr): 1678, 1640, 1600, 1516, 1504, 1410, 1378, 1344, 1328, 1302, 1276, 1228, 1136, 870, 856, 842, 720 cm⁻¹
NMR (CDCl₃) δ: 2.16 (6H, s), 1.59 - 2.33 (6H, m), 2.60 (2H, t, J = 6.6 Hz), 2.95 - 3.28 (3H, m), 4.03 (2H, t, J = 6.6 Hz), 6.23 - 6.71 (5H, m), 7,43 (2H, d, J = 8.6 Hz), 7.89 - 8.05 (4H, m)

### Inventive Example 83

### 4-Nitro-N-{2-[4-(4-fluorobenzoyl)piperidino]ethyl}-N-(3,5-dimethylphenyl)benzamide

Using 4-nitro-N-(3,5-dimethylphenyl)benzamide (1.08 g, 4.00 mmol), the procedure of Inventive Example 48 was repeated to obtain 686.1 mg (34.1%) of the title compound in the form of light yellow powder.
IR (KBr): 1680, 1638, 1598, 1408, 1382, 1332, 1310, 1296, 1234, 1126, 854, 716 cm⁻¹
NMR (CDCl₃) δ: 2.19 (6H, s), 1.69 - 2.32 (6H, m), 2.60 (2H, t, J = 6.6 Hz), 2.95 - 3.20 (3H, m), 4.03 (2H, t, J = 6.6 Hz), 6.70 (2H, s), 6.79 (1H, s), 7.04 - 7.23 (2H, m), 7.43 (2H, d, J = 8.6 Hz), 7.89 - 8.07 (4H, m)

### Inventive Example 84

### 4-Nitro-N-{3-[4-(4-fluorobenzoyl)piperidino]propyl}-N-(3-methoxyphenyl)benzamide

Using 4-nitro-N-(3-methoxyphenyl)benzamide (816.0 mg, 3.00 mmol), the procedure of Inventive Example 48 was repeated to obtain 872.0 mg (56.0%) of the title compound in a light yellow amorphous form.
IR (KBr): 1680, 1644, 1600, 1524, 1490, 1408, 1392, 1348, 1314, 1282, 1234, 1200, 1156, 910, 862 cm⁻¹
NMR (CDCl₃) δ: 1.55 - 2.23 (7H, m), 2.43 (2H, t, J = 7.2 Hz), 2.75 - 3.28 (3H, m), 3.70 (3H, s), 3.98 (2H, t, J = 7.2 Hz), 6.41 - 6.78 (3H, m), 6.90 - 7.28 (1H, m), 7.13 (2H, dd, J = 9.0 Hz), 7.45 (2H, d, J = 8.8 Hz), 7.89 (2H, d, J = 8.8 Hz), 7.95 (2H, dd, J = 9.0 Hz, 6.0 Hz)

### Inventive Example 85

### 4-Nitro-N-{2-[4-(4-fluorobenzoyl)piperidino]ethyl}-N-(3-methoxyphenyl)benzamide

4-Nitro-N-(3-methoxyphenyl)benzamide (50.0 g, 183.6 mmol) was dissolved in dioxane (900 ml) to which was subsequently added potassium hydroxide (30.3 g, 85%, 459.0 mmol) at room temperature. After 1 hour of stirring at 60°C, N-1-(2-chloroethyl)-4-(4-fluorobenzoyl)piperidine hydrochloride (67.5 g, 220.0 mmol) was added to the reaction solution at room temperature. After 24 hours of additional stirring at 50°C, the reaction solution was diluted with ice water and extracted with ethyl acetate. The resulting organic layer was washed with water and saturated brine and dried on anhydrous sodium sulfate. Thereafter, the solvent was removed by evaporation, and the resulting residue was recrystallized from ethyl acetate to obtain 47.2 g (50.8%) of the title compound in the form of light yellow flocculent crystals.
Melting point: 133 - 135°C
IR (KBr): 1678, 1634, 1600, 1518, 1408, 1380, 1276, 1230, 1136, 1048, 978 cm⁻¹
NMR (CDCl₃) δ: 1.60 - 2.33 (5H, m), 2.60 (2H, t, J = 6.6 Hz), 1.97 - 2.34 (2H, m), 2.80 - 3.35 (4H, m), 3.66 (3H, s), 4.03 (2H, t, J = 6.6 Hz), 6.53 - 6.80 (3H, m), 7.00 - 7.15 (1H, m), 7.10 (2H, dd, each J = 9.0 Hz), 7.42 (2H, d, J = 9.0 Hz), 7.98 (2H, dd, J = 9.0 Hz, 6.0 Hz)

### Inventive Example 86

### N-{3-[4-(4-Fluorophenyl)piperazinyl]propyl}-4-methoxy-N-(2-methoxyphenyl)benzene sulfonamide

In an atmosphere of argon, 4-methoxy-N-(2-methoxyphenyl)benzene sulfonamide (200 mg, 0.682 mmol) was dissolved in DMF (3 ml) to which was subsequently added sodium hydride (150 mg, 60%, 3.75 mmol) at room temperature. After 1 hour of stirring at the same temperature, to this was added dropwise 1-(3-bromopropyl)-4-(4-fluorophenyl)piperazine dihydrobromide (347 mg, 0.75 mmol) which has been dissolved in DMF (3 ml). After 5 hours of stirring at 50°C, the reaction solution was diluted with ice water (10 ml) and extracted with ethyl acetate. The resulting organic layer was washed with water and saturated brine, and dried on anhydrous sodium carbonate. Thereafter, the solvent was removed by evaporation, and the resulting residue was purified by a silica gel column chromatography (ether) to obtain 130 mg (37.1%) of the title compound in the form of colorless prism crystals.
Melting point: 121 - 122.5°C
IR (KBr): 2948, 2820, 1596, 1512, 1496, 1460, 1342, 1300, 1256, 1178, 1158, 1114, 1096, 1026, 830, 804, 754, 588, 562 cm⁻¹
NMR (CDCl₃) δ: 1.45 - 1.90 (2H, m), 2.13 - 2.70 (6H, m), 2.87 - 3.26 (4H, m), 3.43 (3H, s), 3.63 (2H, t, J = 7 Hz), 3.83 (3H, s), 6.60 - 7.10 (8H, m), 7.13 - 7.40 (2H, m), 7.60 (2H, d, J = 9 Hz)

### Inventive Example 87

### 4-Fluoro-N-{3-[4-(4-fluorophenyl)piperazinyl]propyl}-N-(2-methoxyphenyl)benzene sulfonamide

Using 4-fluoro-N-(2-methoxyphenyl)benzene sulfonamide (141 mg, 0.50 mmol), the procedure of Inventive Example 56 was repeated to obtain 103 mg (41.1%) of the title compound in the form of colorless powder.
Melting point: 115 - 118°C
IR (KBr): 1508, 1492, 1340, 1288, 1250, 1222, 1162, 816, 782, 554 cm⁻¹
NMR (CDCl₃) δ: 1.40 - 1.88 (2H, m), 2.25 - 2.73 (6H, m), 2.85 - 3.23 (4H, m), 3.40 (3H, s), 3.66 (2H, t, J = 7 Hz), 6.63 - 7.46 (10H, m), 7.66 (2H, dd, J = 9 Hz, 6 Hz)

### Inventive Example 88

### N-{3-[4-(4-Fluorophenyl)piperazinyl]propyl}-N-(2-methoxyphenyl)-3-nitrobenzene sulfonamide

Using N-(2-methoxyphenyl)-3-nitrobenzene sulfonamide (190 mg, 0.616 mmol), the procedure of Inventive Example 56 was repeated to obtain 196 mg (60.2%) of the title compound in the form of yellow powder.
Melting point: 95 - 98°C
IR (KBr): 1528, 1506, 1496, 1348, 1280, 1268, 1246, 1228, 1174, 1162, 828, 786, 592, 576 cm⁻¹
NMR (CDCl₃) δ: 1.40 - 1.92 (2H, m), 2.22 - 2.70 (6H, m), 2.88 - 3.28 (4H, m), 3.37 (3H, s), 3.68 (2H, t, J = 7 Hz), 6.68 - 7.10 (6H, m), 7.18 - 7.45 (2H, m), 7.62 (1H, dd, J = 7.5 Hz, 7.5 Hz), 7.98 (1H, d, J = 7.5 Hz), 8.35 (1H, d, J = 7.5 Hz), 8.55 (1H, br-s)

### Inventive Example 89

### N-{2-[4-(4-fluorobenzoyl)piperidino]ethyl}-4-hydroxymethyl-N-(2-methoxyphenyl)benzene sulfonamide

N-{2-[4-(4-Fluorobenzoyl)piperidino]ethyl}-4-tetrahydropyranyloxymethyl-N-(2-methoxyphenyl)benzene sulfonamide (101 mg, 0.165 mmol) was dissolved in methanol (5 ml) to which was subsequently added p-toluenesulfonic acid monohydrate (35 mg, 0.18 mmol) at room temperature. After 2 hour of stirring at the same temperature, methanol was removed by evaporation, and the resulting reaction solution was mixed with saturated sodium bicarbonate aqueous solution (5 ml), extracted with ethyl acetate, and washed with water and saturated brine. The water layer was extracted with ethyl acetate, and washed with water and saturated brine, and the organic layers were combined and dried on anhydrous sodium sulfate. Thereafter, the solvent was removed by evaporation, and the resulting residue was purified by a silica gel column chromatography (ethyl acetate) to obtain 87 mg (100%) of the title compound in the form of colorless oil.
IR (neat): 3520, 2944, 1680, 1598, 1496, 1342, 1280, 1262, 1238, 1158, 732 cm⁻¹
NMR (CDCl₃) δ: 1.46 - 1.90 (4H, m), 1.90 - 2.15 (1H, m), 2.15 - 2.32 (2H, m), 2.46 (2H, t, J = 7.5 Hz), 2.69 - 2.99 (2H, m), 3.00 - 3.26 (1H, m), 3.41 (3H, s), 3.70 (2H, t, J = 7.5 Hz), 4.75 (2H, s), 6.77 (1H, d, J = 9 Hz), 6.95 (1H, d, J = 9 Hz), 7.11 (2H, dd, J = 9 Hz, 9 Hz), 7.27 (2H, d, J = 9 Hz), 7.40 (2H, d, J = 8.5 Hz), 7.67 (2H, d, J = 8.5 Hz), 7.92 (2H, dd, J = 9 Hz, 6Hz)

### Inventive Example 90

### N-{2-[4-(4-Fluorobenzoyl)piperidino]ethyl}-4-hydroxymethyl-N-(2-methoxyphenyl)benzamide

Using N-{2-[4-(4-fluorobenzoyl)piperidino]ethyl}-4-tetrahydropyranyloxymethyl-N-(2-methoxyphenyl)benzamide (163 mg, 0.284 mmol), the procedure of Inventive Example 59 was repeated to obtain 128 mg (91.9%) of the title compound in the form of colorless solid.
Melting point: 87 - 91°C
IR (KBr): 3408, 2948, 1680, 1638, 1598, 1502, 1410, 1298, 1278, 1264, 1240, 752, 730, 602 cm⁻¹
NMR (CDCl₃) δ: 1.50 - 1.96 (5H, m), 2.00 - 2.40 (2H, m), 2.63 (2H, t, J = 7.5 Hz), 2.76 - 3.40 (3H, m), 3.50 - 3.93 (1H, m), 3.68 (3H, s), 3.96 - 4.33 (1H, m), 4.57 (2H, s), 6.73 (1H, d, J = 8 Hz), 6.84 (1H, d, J = 7.5 Hz), 6.95 - 7.43 (8H, m), 7.95 (2H, dd, J = 9 Hz, 6 Hz)

### Inventive Example 91

### N-{2-[4-(4-Fluorobenzoyl)piperidino]ethyl}-3-hydroxymethyl-N-(2-methoxyphenyl)benzamide

Using N-{2-[4-(4-fluorobenzoyl)piperidino]ethyl}-3-tetrahydropyranyloxymethyl-N-(2-methoxyphenyl)benzamide (191 mg, 0.332 mmol), the procedure of Inventive Example 59 was repeated to obtain 148 mg (90.9%) of the title compound in the form of colorless oil.
IR (neat): 3416, 2944, 1680, 1642, 1598, 1504, 1394, 1278, 1240, 734 cm⁻¹
NMR (CDCl₃) δ: 1.45 - 1.90 (5H, m), 1.98 - 2.36 (2H, m), 2.63 (2H, t, J = 7.5 Hz), 2.79 - 3.22 (3H, m), 3.40 - 3.92 (1H, m), 3.70 (3H, s), 4.04 - 4.35 (1H, m), 4.52 (2H, s), 6.73 (1H, d, J = 8 Hz), 6.83 (1H, d, J = 8 Hz), 6.94 - 7.39 (8H, m), 7.96 (2H, dd, J = 9 Hz, 6 Hz)

### Inventive Example 92

### N-{2-[4-(4-Fluorobenzoyl)piperidino]ethyl}-4-hydroxymethyl-N-(3-methoxybenzyl)benzamide

Using N-{2-[4-(4-fluorobenzoyl)piperidino]ethyl}-4-tetrahydropyranyloxymethyl-N-(3-methoxybenzyl)benzamide (130 mg, 0.221 mmol), the procedure of Inventive Example 59 was repeated to obtain 100 mg (89.7%) of the title compound in the form of colorless oil.
IR (neat): 3416, 2944, 1678, 1600, 1424, 1262, 1234, 1208, 1156, 1048, 974, 730 cm⁻¹
NMR (CDCl₃) δ: 1.45 - 2.30 (8H, m), 2.37 - 3.65 (6H, m), 3.80 (3H, s), 4.70 (4H, br-s), 6.64 - 6.95 (2H, m), 7.10 - 7.60 (6H, m), 7.13 (2H, dd, J = 9 Hz, 9 Hz), 7.95 (2H, dd, J = 9 Hz, 6 Hz)

### Inventive Example 93

### N-{2-[4-(4-Fluorobenzoyl)piperidino]ethyl}-4-hydroxymethyl-N-(2-methoxyethyl)benzamide

Using N-{2-[4-(4-fluorobenzoyl)piperidino]ethyl}-4-tetrahydropyranyloxymethyl-N-(2-methoxyethyl)benzamide (424 mg, 0.805 mmol), the procedure of Inventive Example 59 was repeated to obtain 307 mg (86.2%) of the title compound in the form of colorless solid.
Melting point: 95 - 99°C
IR (KBr): 3450, 2940, 2820, 1664, 1628, 1596, 1464, 1428, 1290, 1280, 1212, 1170, 1112, 1048, 1014, 978 cm⁻¹
NMR (CDCl₃) δ: 1.43 - 2.30 (7H, m), 2.36 - 3.39 (5H, m), 3.32 (3H, s), 3.39 - 3.90 (6H, m), 4.71 (2H, s), 7.12 (2H, dd, J = 9 Hz, 9 Hz), 7.38 (4H, s), 7.94 (2H, dd, J = 9 Hz, 6 Hz)

### Inventive Example 94

### N-{2-[4-(4-Fluorobenzoyl)piperidino]ethyl}-N-(2-methoxyphenyl)-4-dimetylaminomethylbenzene sulfonamide and N-(2-methoxyphenyl)-N-{2-[4-(4-dimethylaminobenzoyl)piperidino]ethyl}-4-dimetylaminomethylbenzene sulfonamide

In an atmosphere of dry air, N-{2-[4-(4-fluorobenzoyl)piperidino]ethyl}-4-hydroxymethyl-N-(2-methoxyphenyl)benzene sulfonamide (80 mg, 0.152 mmol) was dissolved in methylene chloride (2 ml) to which were subsequently added dropwise DMF (1 drop) and thionyl chloride (0.1 ml, 1.37 mmol) while cooling in an ice bath. After 2 hours of stirring at the same temperature, the solvent was removed by evaporation. The thus obtained residue was put into a 25 ml capacity eggplant type flask equipped with a cold finger, dissolved in dioxane (1 ml) and mixed with 50% dimethylamine aqueous solution (1 ml). After 2.5 hours of stirring at 80°C, dioxane was removed by evaporation, and the resulting residue was diluted with ethyl acetate (15 ml) and dried on anhydrous sodium carbonate. Thereafter, the solvent was removed by evaporation, and the resulting residue was purified by a PTLC (methylene chloride:methanol = 35:1) to obtain 33.5 mg (39.8%) of N-{2-[4-(4-fluorobenzoyl)-piperidino]ethyl}-N-(2-methoxyphenyl)-4-dimetylaminomethyl-benzene sulfonamide as a low polarity component in the form of colorless oil and 47.5 mg (54.0%) of N-(2-methoxyphenyl)-N-{2-[4-(4-dimethylaminobenzoyl)piperidino]ethyl}-4-dimetylamino-methylbenzene sulfonamide as a high polarity component in the form of colorless solid.
Low polarity component, N-{2-[4-(4-fluorobenzoyl)piperidino]ethyl}-N-(2-methoxyphenyl)-4-dimetylaminomethylbenzene sulfonamide:
IR (neat): 2944, 2816, 2776, 1680, 1598, 1496, 1458, 1342, 1280, 1262, 1160, 592 cm⁻¹
NMR (CDCl₃) δ: 1,46 - 1.93 (4H, m), 1.95 - 2.35 (2H, m), 2.23 (6H, s), 2.51 (2H, t, J = 7.5 Hz), 2.70 - 3.20 (3H, m), 3.39 (3H, s), 3.44 (2H, s), 3.72 (2H, t, J = 7.5 Hz), 6.76 (1H, d, J = 8.5 Hz), 6.95 (1H, d, J = 8.5 Hz), 7.11 (2H, dd, J = 9 Hz, 9 Hz), 7.15 - 7.49 (4H, m), 7.65 (2H, d, J = 8.5 Hz), 7.93 (2H, dd, J = 9 Hz, 6 Hz)
High polarity component, N-(2-methoxyphenyl)-N-{2-[4-(4-dimethylaminobenzoyl)piperidino]ethyl}-4-dimetylaminomethylbenzene sulfonamide:
Melting point: 152 - 155°C
IR (KBr): 3440, 2816, 1656, 1598, 1496, 1342, 1280, 1264, 1160, 1116, 730, 592 cm⁻¹
NMR (CDCl₃) δ: 1.50 - 1.92 (4H, m), 1.95 - 2.36 (2H, m), 2.23 (6H, s), 2.52 (2H, t, J = 7.5 Hz), 2.72 - 3.24 (3H, m), 3.02 (6H, s), 3.40 (3H, s), 3.46 (2H, s), 3.72 (2H, t, J = 7.5 Hz), 6.45 - 7.05 (4H, m), 7.06 - 7.50 (4H, m), 7.65 (2H, d, J = 8 Hz), 7.83 (2H, d, J = 8.5 Hz)

### Inventive Example 95

### N-{2-[4-(4-Fluorobenzoyl)piperidino]ethyl}-4-formyl-N-(2-methoxyphenyl)benzene sulfonamide

Activated manganese dioxide (870 mg, 10.0 mmol) was suspended in methylene chloride (10 ml), and N-{2-[4-(4-fluorobenzoyl)piperidino]ethyl}-4-hydroxymethyl-N-(2- methoxyphenyl)benzene sulfonamide (259 mg, 0.492 mmol) dissolved in methylene chloride (3 ml) was added to the suspension. After 30 minutes of stirring at room temperature, the reaction mixture was filtered through silica gel (1 g) which was further subjected to elution with ethyl acetate. Thereafter, the filtrate and eluate were combined, and the solvent was removed by evaporation to obtain 211 mg (81.7%) of the title compound in a colorless amorphous form.
IR (KBr): 2940, 1706, 1678, 1598, 1496, 1348, 1294, 1260, 1200, 1158, 1116, 974, 754, 716, 604, 580 cm⁻¹
NMR (CDCl₃) δ: 1.50 - 1.95 (4H, m), 1.95 - 2.32 (2H, m), 2.52 (2H, t, J = 7.5 Hz), 2.70 - 3.47 (3H, m), 3.30 (3H, s), 3.55 - 4.00 (2H, m), 6.76 (1H, d, J = 8 Hz), 6.90 (1H, d, J = 8 Hz), 7.12 (2H, dd, J = 9 Hz, 9 Hz), 7.16 - 7.50 (2H, m), 7.70 - 8.14 (6H, m), 10.1 (1H, s)

### Inventive Example 96

### N-{2-[4-(4-Fluorobenzoyl)piperidino]ethyl}-4-(N-hydroxyimino)-N-(2-methoxyphenyl)benzene sulfonamide

N-{2-[4-(4-Fluorobenzoyl)piperidino]ethyl}-4-formyl-N-(2-methoxyphenyl)benzene sulfonamide (63 mg, 0.12 mmol) and hydroxyamine hydrochloride (9.5 mg, 0.132 mmol) were dissolved in ethanol (2 ml) to which were subsequently added anhydrous sodium carbonate (14 mg, 0.132 mmol) and water (1 ml). After 1.5 hours of stirring at room temperature, ethanol was removed by evaporation, and the resulting residue was mixed with water (10 ml) to collect crystals by filtration. The thus collected crystals were washed with water and hexane, dissolved in methylene chloride (50 ml) and then dried on anhydrous sodium carbonate. Thereafter, the solvent was removed by evaporation to obtain 65 mg (100%) of the title compound in the form of colorless powder.
Melting point: 162 - 168°C
IR (KBr): 3450, 2950, 2840, 1676, 1594, 1500, 1344, 1264, 1204, 1156, 1106, 990, 726, 604, 588 cm⁻¹
NMR (CDCl₃) δ: 1.62 - 1.96 (4H, m), 1.96 - 2.40 (2H, m), 2.60 (2H, t, J = 7.5 Hz), 2.80 - 3.55 (3H, m), 3.31 (3H, s), 3.83 (2H, t, J = 7.5 Hz), 6.75 (1H, d, J = 8 Hz), 6.96 (1H, d, J = 8.5 Hz), 7.12 (2H, dd, J = 9 Hz, 9 Hz), 7.16 - 7.43 (3H, m), 7.46 - 7.80 (4H, m), 7.93 (2H, dd, J = 9 Hz, 6 Hz), 8.11 (1H, s)

### Inventive Example 97

### 4-Carbamoyl-N-{2-[4-(4-fluorobenzoyl)piperidino]ethyl}-N-(2-methoxyphenyl)benzene sulfonamide

In an atmosphere of argon and with cooling in an ice bath, 4-{(N-[2-[4-(4-fluorobenzoyl)piperidino]ethyl}-2-methoxyanilino]sulfonyl}benzoic acid (63 mg, 0.117 mmol) was dissolved in THF (1 ml) to which were subsequently added dropwise N-methylmorpholine (14 µl, 0.13 mmol) and isobutyl chloroformate (16.8 µl, 0.13 mmol). After 15 minutes of stirring at room temperature, 28% liquid ammonia (0.5 ml) was added to the reaction solution which was cooled in an ice bath, followed by additional 10 minutes of stirring at room temperature. The reaction mixture was extracted with methylene chloride, and the resulting organic layer was dried on anhydrous sodium carbonate. Thereafter, the solvent was removed by evaporation, and the resulting light yellow oily residue was purified by a silica gel column chromatography (ether-ethyl acetate-methylene chloride:methanol = 10:1) to obtain 32 mg (50.9%) of the title compound in the form of light yellow oil.
IR (neat): 3368, 2944, 1672, 1596, 1496, 1406, 1342, 1280, 1262, 1240, 1116, 912, 732, 662, 600 cm⁻¹
NMR (CDCl₃) δ: 1.50 - 1.93 (4H, m), 1.95 - 2.30 (2H, m), 2.46 (2H, t, J = 7 Hz), 2.80 - 3.28 (3H, m), 3.38 (3H, s), 3.56 - 3.93 (2H, m), 6.10 (1H, br-s), 6.78 (1H, d, J = 8.5 Hz), 6.97 (1H, d, J = 9 Hz), 7.13 (2H, dd, J = 9 Hz, 9 Hz), 7.17 - 7.50 (2H, m), 7.65 - 8.40 (7H, m)

### Inventive Example 98

### N-{2-[4-(4-Fluorobenzoyl)piperidino]ethyl}-N-(2-methoxyethyl)-4-phthalimidomethylbenzamide

In an atmosphere of argon, N-{2-[4-(4-fluorobenzoyl)piperidino]ethyl}-4-hydroxymethyl-N-(2-methoxyethyl)benzamide (216 mg, 0.488 mmol), phthalimide (86 mg, 0.585 mmol) and triphenylphosphine (155 mg, 0.585 mmol) were dissolved in THF (3 ml) to which was subsequently added dropwise diethyl azodicarboxylate (93 µl, 0.585 mmol) while cooling in an ice bath. After 1 hour of stirring at the same temperature, the reaction solution was diluted with water (10 ml) and extracted with ethyl acetate. The resulting organic layer was washed with water and saturated brine, and dried on anhydrous sodium carbonate. Thereafter, the solvent was removed by evaporation, and the resulting light yellow oily residue was purified by a silica gel column chromatography (ethyl acetate-ethyl acetate:methanol = 20:1) to obtain 259 mg (92.8%) of the title compound in a colorless amorphous form.
IR (KBr): 2940, 1716, 1678, 1630, 1596, 1426, 1392, 1348, 1228, 1112, 974, 718 cm⁻¹
NMR (CDCl₃) δ: 1.47 - 2.30 (6H, m), 2.33 - 3.85 (11H, m), 3.30 (3H, s), 4.85 (2H, s), 7.13 (2H, dd, J = 9 Hz, 9 Hz), 7.34 (2H, d, J = 8.5 Hz), 7.46 (2H, d, J = 8.5 Hz), 7.58 - 8.10 (6H, m)

### Inventive Example 99

### N-{2-[4-(4-Fluorobenzoyl)piperidino]ethyl}-N-(2-methoxyphenyl)-4-phthalimidomethylbenzene sulfonamide

Using N-{2-[4-(4-fluorobenzoyl)piperidino]ethyl}-4-hydroxymethyl-N-(2-methoxyphenyl)benzene sulfonamide (80 mg, 0.152 mmol), the procedure of Inventive Example 68 was repeated in an atmosphere of argon to obtain 78 mg (78.3%) of the title compound in a colorless amorphous form.
IR (KBr): 1716, 1678, 1598, 1496, 1392, 1346, 1280, 1260, 1158, 1092, 716 cm⁻¹
NMR (CDCl₃) δ: 1.52 - 1.92 (4H, m), 1.93 - 2.30 (2H, m), 2.50 (2H, t, J = 7.5 Hz), 2.70 - 3.00 (2H, m), 3.00 - 3.40 (1H, m), 3.24 (3H, s), 3.50 - 3.85 (2H, m), 4.89 (2H, s), 6.60 - 8.10 (16H, m)

### Inventive Example 100

### N-{2-[4-(4-Fluorobenzoyl)piperidino]ethyl}-N-(2-methoxyphenyl)-4-succinimidomethylbenzamide

In an atmosphere of argon, N-{2-[4-(4-fluorobenzoyl)piperidino]ethyl}-4-hydroxymethyl-N-(2-methoxyphenyl)benzamide (54 mg, 0.11 mmol), succinimide (13 mg, 0.132 mmol) and triphenylphosphine (34.6 mg, 0.132 mmol) were dissolved in THF (1 ml) to which was subsequently added dropwise diethyl azodicarboxylate (21 µl, 0.132 mmol) while cooling in an ice bath. After 30 minutes of stirring at room temperature, the reaction solution was diluted with water (10 ml) and extracted with ethyl acetate. The resulting organic layer was washed with water and saturated brine, and dried on anhydrous sodium carbonate. Thereafter, the solvent was removed by evaporation, and the resulting residue was purified by a PTLC (chloroform:methanol = 20:1) to obtain 42 mg (66.8%) of the title compound in a light yellow amorphous form.
IR (KBr): 3472, 2944, 2800, 1704, 1683, 1644, 1401, 1305, 1164 cm⁻¹
NMR (CDCl₃) δ: 1.70 - 1.95 (4H, m), 2.03 - 2.40 (2H, m), 2.66 (4H, s), 2.60 - 2.80 (2H, m), 2.80 - 3.25 (3H, m), 3.79 (3H, s), 3.40 - 3.80 (1H, m), 4.00 - 4.28 (1H, m), 4.53 (2H, s), 6.74 (1H, d, J = 8 Hz), 6.82 (1H, d, J = 8 Hz), 7.00 - 7.30 (8H, m), 7.93 (2H, dd, J = 9 Hz, 6 Hz)

### Inventive Example 101

### 4-Aminomethyl-N-{2-[4-(4-fluorobenzoyl)piperidino]ethyl}-N-(2-methoxyphenyl)benzene sulfonamide

N-{2-[4-(4-Fluorobenzoyl)piperidino]ethyl}-N-(2-methoxyphenyl)-4-phthalimidomethylbenzene sulfonamide (77 mg, 0.117 mmol) was dissolved in a methanol-THF (3:2) mixture solution (2.5 ml) to which was subsequently added hydrazine hydrate (1 ml) while cooling in an ice bath. After 15 minutes of stirring at the same temperature, the organic solvent was removed by evaporation, and the resulting residue was extracted with methylene chloride. The resulting organic layer was washed with saturated sodium bicarbonate aqueous solution and saturated brine, and dried on anhydrous sodium carbonate, followed by the removal of the solvent by evaporation. To the resulting residue was added an acetic acid-THF-water (3:1:1) mixture solution (3 ml). After 2 hours of stirring at room temperature, the solvent was removed by evaporation, and the resulting reaction solution was diluted with ethyl acetate (15 ml), mixed with saturated sodium bicarbonate aqueous solution (5 ml) and then extracted with ethyl acetate. After drying the extract on anhydrous sodium carbonate, the solvent was removed by evaporation, and the resulting light yellow oily residue was purified by a silica gel column chromatography (methylene chloride:methanol = 20:1 to 9:1) to obtain 47 mg (76.4%) of the title compound in a colorless oily form.
IR (neat): 3380, 2944, 1680, 1598, 1496, 1340, 1280, 1262, 1158, 1094, 730 cm⁻¹
NMR (CDCl₃) δ: 1.40 - 1.93 (6H, m), 1.93 - 2.30 (2H, m), 2.50 (2H, t, J = 7 Hz), 2.70 - 3.28 (3H, m), 3.40 (3H, s), 3.72 (2H, t, J = 7.5 Hz), 3.96 (2H, s), 6.77 (1H, d, J = 8.5 Hz), 6.95 (1H, d, J = 8.5 Hz), 7.12 (2H, dd, J = 9 Hz, 9 Hz), 7.15 - 7.50 (2H, m), 7.35 (2H, d, J = 8.5 Hz), 7.66 (2H, d, J = 8.5 Hz), 7.93 (2H, dd, J = 9 Hz, 6 Hz)

### Inventive Example 102

### 4-Aminomethyl-N-{2-[4-(4-fluorobenzoyl)piperidino]ethyl}-N-(2-methoxyethyl)benzamide

Using N-{2-[4-(4-fluorobenzoyl)piperidino]ethyl}-N-(2-methoxyethyl)-4-phthalimidomethylbenzamide (258 mg, 0.451 mmol), the procedure of Inventive Example 71 was repeated to obtain 131 mg (65.8%) of the title compound in the form of colorless oil.
Melting point: 85 - 90°C
IR (KBr): 2944, 2820, 1664, 1628, 1598, 1462, 1428, 1290, 1280, 1222, 1170, 1112, 1012, 978, 854, 840 cm⁻¹
NMR (CDCl₃) δ: 1.50 - 2.35 (8H, m), 2.42 - 3.40 (5H, m), 3.32 (3H, s), 3.40 - 3.76 (6H, m), 3.89 (2H, s), 7.12 (2H, dd, J = 9 Hz, 9 Hz), 7.26 (4H, s), 7.93 (2H, dd, J = 9 Hz, 6 Hz)

### Inventive Example 103

### 4-Aminomethyl-N-{2-[4-(4-fluorobenzoyl)piperidino]ethyl}-N-(2-methoxyphenyl)benzamide

Using N-{2-[4-(4-fluorobenzoyl)piperidino]ethyl}-N-(2-methoxyphenyl)-4-phthalimidomethylbenzamide (56 mg, 0.0904 mmol), the procedure of Inventive Example 71 was repeated to obtain 32 mg (72.3%) of the title compound in the form of colorless oil.
IR (neat): 2944, 1678, 1638, 1598, 1500, 1440, 1410, 1390, 1310, 1278, 1240, 1158, 1140, 1024, 976, 754 cm⁻¹
NMR (CDCl₃) δ: 1.35 - 2.00 (6H, m), 2.00 - 2.36 (2H, m), 2.63 (2H, t, J = 7.5 Hz), 2.80 - 3.36 (3H, m), 3.46 - 3.93 (1H, m), 3.68 (3H, s), 3.75 (2H, s), 3.93 - 4.35 (1H, m), 6.74 (1H, d, J = 8 Hz), 6.83 (1H, d, J = 7.5 Hz), 6.93 - 7.40 (8H, m), 7.95 (2H, dd, J = 9 Hz, 6 Hz)

### Inventive Example 104

### 4-Amino-N-{2-[4-(4-fluorobenzoyl)piperidino]ethyl}-N-(2-methoxyphenyl)benzamide

4-Nitro-N-{2-[4-(4-fluorobenzoyl)piperidino]ethyl}-N-(2-methoxyphenyl)benzamide (264.0 mg, 0.52 mmol) was dissolved in methanol (5 ml) to which were subsequently added concentrated hydrochloric acid (2.0 ml) and tin (powder, 186.0 mg, 1.57 mmol) while cooling in an ice bath. After 1 hour of stirring at room temperature, the reaction solution was poured in ice water, adjusted to pH 9 to 10 with 10% sodium hydroxide aqueous solution, mixed with chloroform and then passed through cerite to remove insoluble materials. The resulting residue was washed with chloroform, the filtrate was extracted with chloroform, and the resulting organic layer was washed with water and saturated brine, and dried on anhydrous sodium sulfate. Thereafter, the solvent was removed by evaporation, and the resulting residue was purified by a silica gel column chromatography (chloroform:methanol = 10:1) to obtain 243.0 mg (97.9%) of the title compound in a colorless amorphous form.
IR (KBr): 3336, 2936, 1678, 1628, 1600, 1438, 1384, 1278 cm⁻¹
NMR (CDCl₃) δ: 1.60 - 1.95 (4H, m), 2.00 - 2.34 (2H, m), 2.45 - 2.76 (2H, m), 2.80 - 3.30 (3H, m), 3.70 (3H, s), 3.60 - 3.85 (1H, m), 4.02 - 4.34 (1H, m), 6.35 (2H, d, J = 9.0 Hz), 6.83 (2H, t, J = 6.0 Hz), 6.95 - 7.30 (6H, m), 7.93 (2H, dd, J = 9.0 Hz, 6.0 Hz)

### Inventive Example 105

### 4-Amino-N-{2-[4-(4-fluorobenzoyl)piperidino]ethyl}-N-(3-methoxyphenyl)benzamide

Using 4-nitro-N-{2-[4-(4-fluorobenzoyl)piperidino]ethyl}-N-(3-methoxyphenyl)benzamide (505.0 mg, 1.00 mmol), the procedure of Inventive Example 104 was repeated to obtain 490.7 mg (quantitative) of the title compound in the form of light yellow powder.
Melting point: 126 - 127.5°C
IR (KBr): 3360, 2944, 1680. 1634, 1600, 1438, 1380, 1310 cm⁻¹
NMR (CDCl₃) δ: 1.60 - 2.35 (5H, m), 2.63 (2H, t, J = 7.0 Hz), 2.85 - 3.20 (4H, m), 3.69 (3H, s), 3.60 - 3.86 (2H, m), 4.02 (2H, t, J = 7.0 Hz), 6.40 (2H, d, J = 8.6 Hz), 6.56 - 6.83 (1H, m), 6.69 (2H, d, J = 8.6 Hz), 6.95 - 7.30 (3H, m), 7.11 (2H, dd, each J = 9.0 Hz), 7.95 (2H, dd, J = 9.0 Hz, 6.0 Hz)

### Inventive Example 106

### 4-Amino-N-{2-[4-(4-fluorobenzoyl)piperidino]ethyl}-N-(4-methoxyphenyl)benzamide

Using 4-nitro-N-{2-[4-(4-fluorobenzoyl)piperidino]ethyl}-N-(4-methoxyphenyl)benzamide (968.6 mg, 1.92 mmol), the procedure of Inventive Example 104 was repeated to obtain 905.7 mg (99.4%) of the title compound in a light yellow amorphous form.
IR (KBr): 2944, 1677, 1626, 1602, 1509, 1440, 1380, 1287, 1245, 1221, 1170, 1155, 1137, 1107, 1029, 972, 834, 759, 603, 591 cm⁻¹
NMR (CDCl₃) δ: 1.61 - 1.98 (4H, m), 1.98 - 2.32 (2H, m), 2.62 (2H, t, J = 7.1 Hz), 2.80 - 3.35 (3H, m), 3.76 (3H, s), 3.99 (2H, t, J = 7.1 Hz), 6.40 (2H, d, J = 8.6 Hz), 6.74 (2H, d, J = 9.0 Hz), 6.85 - 7.25 (6H, m), 7.80 - 8.03 (2H, m)

### Inventive Example 107

### 4-Amino-N-{2-[4-(4-fluorobenzoyl)piperidino]ethyl}-N-(2,5-dimethoxyphenyl)benzamide

Using 4-nitro-N-{2-[4-(4-fluorobenzoyl)piperidino]ethyl}-N-(2,5-dimethoxyphenyl)benzamide (535.0 mg, 1.00 mmol), the procedure of Inventive Example 104 was repeated to obtain 479.0 mg (94.9%) of the title compound in a colorless amorphous form.
IR (KBr): 3370, 2950, 1678, 1630, 1602, 1504, 1222, 1041, 840, 763 cm⁻¹
NMR (CDCl₃) δ: 1.76 - 2.35 (6H, m), 2.63 (2H, t, J = 7.0 Hz), 2.95 - 3.16 (3H, m), 3.61 (3H, s), 3.69 (3H, s), 3.72 - 3.85 (2H, m), 6.38 (2H, brd, J = 8.6 Hz), 6.69 (3H, brs), 7.02 - 7.26 (4H, m), 7.87 - 8.03 (2H, m)

### Inventive Example 108

### 3-Amino-N-{2-[4-(4-fluorobenzoyl)piperidino]ethyl}-N-(3-methoxyphenyl)benzamide

Using 3-nitro-N-{2-[4-(4-fluorobenzoyl)piperidino]ethyl}-N-(3-methoxyphenyl)benzamide (730.0 mg, 1.45 mmol), the procedure of Inventive Example 104 was repeated to obtain 690.0 mg (quantitative) of the title compound in the form of light yellow powder.
Melting point: 141 - 142°C
IR (KBr): 3372, 2944, 1672, 1628, 1600, 1488, 1318, 1210, 1164, 1074, 974 cm⁻¹
NMR (CDCl₃) δ: 1.50 - 2.33 (5H, m), 2.69 (2H, t, J = 6.8 Hz), 2.84 - 3.30 (4H, m), 3.69 (3H, s), 4.02 (2H, t, J = 6.8 Hz), 6.40 - 6.76 (6H, m), 6.86 (1H, d, J = 7.7 Hz), 7.05 (1H, d, J = 7.7 Hz), 7.12 (2H, dd, each J = 8.8 Hz), 7.93 (2H, dd, J = 8.8 Hz, 5.5 Hz)

### Inventive Example 109

### 4-Amino-N-{2-[4-(4-fluorobenzoyl)piperidino]ethyl}-N-(2,4-dimethoxyphenyl)benzamide

Using 4-nitro-N-{2-[4-(4-fluorobenzoyl)piperidino]ethyl}-N-(2,4-dimethoxyphenyl)benzamide (527.0 mg, 0.98 mmol), the procedure of Inventive Example 104 was repeated to obtain 439.3 mg (88.3%) of the title compound in the form of light yellow powder.
Melting point: 135 - 138°C
IR (KBr): 1680, 1602, 1512, 1440, 1410, 1389, 1308, 1224, 1206, 1179, 1158, 1026, 954, 837, 765, 603 cm⁻¹
NMR (CDCl₃) δ: 1.73 - 3.91 (13H, m), 3.69 (3H, s), 3.76 (3H, s), 4.19 (2H, dd, J = 6.6 Hz, 5.3 Hz), 6.34 - 6.43 (4H, m), 6.92 - 7.17 (5H, m), 7.87 - 7.95 (2H, m)

### Inventive Example 110

### 4-Amino-N-{2-[4-(4-fluorobenzoyl)piperidino]ethyl}-N-(3-methylphenyl)benzamide

Using 4-nitro-N-{2-[4-(4-fluorobenzoyl)piperidino]ethyl}-N-(3-methylphenyl)benzamide (517.0 mg, 1.00 mmol), the procedure of Inventive Example 104 was repeated to obtain 485.0 mg (quantitative) of the title compound in a colorless amorphous form.
IR (KBr): 3300, 2935, 2780, 1677, 1626, 1599, 1440, 1377, 1299, 1227, 1155, 834 cm⁻¹
NMR (CDCl₃) δ: 1.40 - 2.38 (6H, m), 2.26 (3H, s), 2.64 (2H, t, J = 7.0 Hz), 2.84 - 3.38 (3H, m), 3.51 - 3.86 (2H, m), 4.20 (2H, t, J = 7.0 Hz), 6.39 (2H, d, J = 9.0 Hz), 6.71 - 7.34 (8H, m), 7.94 (2H, dd, J = 9.0 Hz, 6.0 Hz)

### Inventive Example 111

### 4-Amino-N-{2-[4-(4-fluorobenzoyl)piperidino]ethyl}-N-(phenyl)benzamide

Using 4-nitro-N-{2-[4-(4-fluorobenzoyl)piperidino]ethyl}-N-(phenyl)benzamide (713.0 mg, 1.50 mmol), the procedure of Inventive Example 104 was repeated to obtain 671.9 mg (quantitative) of the title compound in the form of light yellow needle crystals.
Melting point: 156 - 157°C
IR (KBr): 1680, 1626, 1596, 1494, 1376, 1296, 1278, 1222, 1204, 1172, 1156, 1138, 974, 836, 758, 698, 598 cm⁻¹
NMR (CDCl₃) δ: 1.71 - 2.31 (6H, m), 2.64 (2H, t, J = 6.8 Hz), 2.94 - 3.25 (3H, m), 3.72 (2H, brs), 4.03 (2H, t, J = 6.8 Hz), 6.39 (2H, d, J = 8.1 Hz), 7.09 - 7.17 (9H, m), 7.92 (1H, d, J = 8.1 Hz)

### Inventive Example 112

### 4-Amino-N-{2-[4-(4-fluorobenzoyl)piperidino]ethyl}-N-(3-methylthiophenyl)benzamide

Using 4-nitro-N-{2-[4-(4-fluorobenzoyl)piperidino]ethyl}-N-(3-methylthiophenyl)benzamide (793.7 mg, 1.52 mmol), the procedure of Inventive Example 104 was repeated to obtain 274.4 mg (36.7%) of the title compound in a colorless amorphous form.
IR (KBr): 1676, 1628, 1600, 1438, 1374, 1296, 1262, 1224, 1204, 1172, 1156, 1140, 974, 838, 760, 698 cm⁻¹
NMR (CDCl₃) δ: 1.77 - 1.86 (4H, m), 2.04 - 2.22 (2H, m), 2.34 (3H, s), 2.62 (2H, t, J = 6.8 Hz), 2.94 - 3.17 (3H, m), 3.74 (2H, brs), 4.01 (2H, t, J = 6.8 Hz), 6.41 (2H, d, J = 8.4 Hz), 6.82 - 7.22 (8H, m), 7.92 (1H, d, J = 8.6 Hz), 7.98 (1H, d, J = 8.4 Hz)

### Inventive Example 113

### 4-Amino-N-{2-[4-(4-fluorobenzoyl)piperidino]ethyl}-N-(2-trifluoromethylphenyl)benzamide

Using 4-nitro-N-{2-[4-(4-fluorobenzoyl)piperidino]ethyl}-N-(2-trifluoromethylphenyl)benzamide (275.0 mg, 0.51 mmol), the procedure of Inventive Example 104 was repeated to obtain 264.0 mg (quantitative) of the title compound in a colorless amorphous form.
IR (KBr): 3370, 2948, 1632, 1600, 1314, 1172, 1126, 977, 769, 608 cm⁻¹
NMR (CDCl₃) δ: 1.63 - 2.22 (6H, m), 2.45 - 3.54 (6H, m), 3.74 (2H, brs), 4.32 - 4.63 (1H, m), 6.43 (2H, brd, J = 8.4 Hz), 7.03 - 7.46 (8H, m), 7.94 (2H, dd, J = 8.8 Hz, 5.5 Hz)

### Inventive Example 114

### 4-Amino-N-{2-[4-(4-fluorobenzoyl)piperidino]ethyl}-N-(3-trifluoromethylphenyl)benzamide

Using 4-nitro-N-{2-[4-(4-fluorobenzoyl)piperidino]ethyl}-N-(3-trifluoromethylphenyl)benzamide (543.0 mg, 1.00 mmol), the procedure of Inventive Example 104 was repeated to obtain 504.0 mg (98.2%) of the title compound in a colorless amorphous form.
IR (KBr): 3371, 2950, 1680, 1632, 1600, 1332, 1274, 1172, 1126, 975, 841, 702 cm⁻¹
NMR (CDCl₃) δ: 1.60 - 1.89 (4H, m), 2.04 - 2.30 (2H, m), 2.61 (2H, t, J = 6.5 Hz), 2.91 - 3.31 (3H, m), 3.78 (2H, brs), 4.03 (2H, t, J = 6.5 Hz), 6.42 (2H, d, J = 8.6 Hz), 7.03 - 7.46 (8H, m), 7.94 (2H, dd, J = 9.0 Hz, 5.5 Hz)

### Inventive Example 115

### 4-Amino-N-{2-[4-(4-fluorobenzoyl)piperidino]ethyl}-N-(3-pyridyl)benzamide

Using 4-nitro-N-{2-[4-(4-fluorobenzoyl)piperidino]ethyl}-N-(3-pyridyl)benzamide (160.0 mg, 0.34 mmol), the procedure of Inventive Example 104 was repeated to obtain 150.0 mg (quantitative) of the title compound in a light yellow amorphous form.
IR (KBr): 3450, 2950, 1680, 1632, 1602, 1479, 1425, 1374, 1224, 1155, 837 cm⁻¹
NMR (CDCl₃) δ: 1.38 - 2.34 (6H, m), 2.64 (2H, t, J = 6.0 Hz), 2.82 - 3.39 (3H, m), 3.78 (2H, brs), 4.07 (2H, t, J = 6.0 Hz), 6.40 (2H, d, J = 9.0 Hz), 6.92 - 7.60 (6H, m), 7.97 (2H, dd, J = 9.0 Hz, 6.0 Hz), 8.24 - 8.44 (2H, m)

### Inventive Example 116

### 4-Amino-N-{2-[4-(4-fluorobenzoyl)piperidino]ethyl}-N-(3-methoxyphenyl)benzamide

Using 2-nitro-N-{2-[4-(4-fluorobenzoyl)piperidino]ethyl}-N-(3-methoxyphenyl)benzamide (936.4 mg, 1.85 mmol), the procedure of Inventive Example 104 was repeated to obtain 853.3 mg (97.1%) of the title compound in the form of colorless oil.
IR (KBr): 3450, 2950, 1680, 1620, 1598, 1492, 1316, 1246, 1158, 910 cm⁻¹
NMR (CDCl₃) δ: 1.60 - 2.33 (5H, m), 2.60 (2H, t, J = 6.0 Hz), 2.76 - 3.30 (4H, m), 3.63 (3H, s), 4.03 (2H, t, J = 6.0 Hz), 4.60 (2H, brs), 6.13 - 7.00 (8H, m), 7.10 (2H, dd, each J = 9.0 Hz), 7.92 (2H, dd, J = 9.0 Hz, 6.0 Hz)

### Inventive Example 117

### 4-Amino-N-{2-[4-(4-fluorobenzoyl)piperidino]ethyl}-N-(3-aminophenyl)benzamide

Using 4-nitro-N-{2-[4-(4-fluorobenzoyl)piperidino]ethyl}-N-(3-aminophenyl)benzamide (1.1 g, 2.11 mmol), the procedure of Inventive Example 104 was repeated to obtain 557.0 mg (57.0%) of the title compound in the form of light brown powder.
Melting point: 107.5 - 110.5°C
IR (KBr): 3400, 1680, 1626, 1596, 1494, 1392, 1377, 1305, 1218, 1158, 852 cm⁻¹
NMR (CDCl₃-CD₃OD) δ: 1.35 - 2.42 (7H, m), 2.63 (2H, t, J = 7.0 Hz), 2.83 - 3.40 (3H, m), 3.40 - 3.85 (3H, m), 3.99 (2H, t, J = 7.0 Hz), 6.23 - 6.60 (4H, m), 6.76 - 7.33 (6H, m), 7.95 (2H, d, J = 9.0 Hz, 6.0 Hz)

### Inventive Example 118

### 4-Amino-N-{2-[4-(4-fluorobenzoyl)piperidino]ethyl}-N-(2-methoxycarbonylphenyl)benzamide

Using 4-nitro-N-{2-[4-(4-fluorobenzoyl)piperidino]ethyl}-N-(2-methoxycarbonylphenyl)benzamide (506.0 mg, 0.95 mmol), the procedure of Inventive Example 104 was repeated to obtain 307.0 mg (64.2%) of the title compound in a colorless amorphous form.
IR (KBr): 3368, 1722, 1630, 1598, 1296, 976, 821, 761, 600 cm⁻¹
NMR (CDCl₃) δ: 1.66 - 2.15 (6H, m), 2.66 - 3.15 (5H, m), 3.70 (2H, brs), 3.81 (3H, s), 4.06 - 4.45 (2H, m), 6.36 (2H, d, J = 8.4 Hz), 7.02 - 7.45 (7H, m), 7.72 - 8.03 (3H, m)

### Inventive Example 119

### 3-Methyl-4-amino-N-{2-[4-(4-fluorobenzoyl)piperidino]ethyl}-N-(4-methoxyphenyl)benzamide

Using 3-methyl-4-nitro-N-{2-[4-(4-fluorobenzoyl)piperidino]ethyl}-N-(4-methoxyphenyl)benzamide (1.02 g, 1.97 mmol), the procedure of Inventive Example 104 was repeated to obtain 955.0 mg (99.1%) of the title compound in a yellow amorphous form.
IR (KBr): 3370, 2941, 1678, 1626, 1598, 1510, 1244, 1034, 975, 838, 605 cm⁻¹
NMR (CDCl₃) δ: 1.67 - 2.31 (6H, m), 2.02 (3H, s), 2.61 (2H, t, J = 6.8 Hz), 2.53 - 3.34 (3H, m), 3.75 (3H, s), 3.98 (2H, t, J = 6.8 Hz), 6.35 (1H, d, J = 8.1 Hz), 6.82 - 7.26 (8H, m), 7.95 (2H, dd, J = 8.6 Hz, 5.5 Hz)

### Inventive Example 120

### 3-Methoxy-4-amino-N-{2-[4-(4-fluorobenzoyl)piperidino]ethyl}-N-(4-methoxyphenyl)benzamide

Using 3-methoxy-4-nitro-N-{2-[4-(4-fluorobenzoyl)piperidino]ethyl}-N-(4-methoxyphenyl)benzamide (1.57 g, 2.93 mmol), the procedure of Inventive Example 104 was repeated to obtain 1.24 g (83.8%) of the title compound in a light yellow amorphous form.
IR (KBr): 3342, 2930, 1675, 1620, 1510, 1234, 1028, 830, 601 cm⁻¹
NMR (CDCl₃) δ: 1.70 - 1.86 (4H, m), 2.04 - 2.31 (2H, m), 2.63 (2H, t, J = 6.9 Hz), 2.95 - 3.33 (3H, m), 3.75 (3H, s), 3.99 (2H, t, J = 6.9 Hz), 6.41 (1H, d, J = 7.9 Hz), 6.70 - 7.26 (8H, m), 7.95 (2H, dd, J = 8.8 Hz, 5.5 Hz)

### Inventive Example 121

### 4-Amino-N-{2-[4-(4-fluorobenzoyl)piperidino]ethyl}-N-(4-fluorophenyl)benzamide

Using 4-nitro-N-{2-[4-(4-fluorobenzoyl)piperidino]ethyl}-N-(4-fluorophenyl)benzamide (707.9 mg, 1.43 mmol), the procedure of Inventive Example 104 was repeated to obtain 255.7 mg (38.6%) of the title compound in a colorless amorphous form.
IR (KBr): 3364, 1628, 1598, 1506, 1378, 1310, 1276, 1212, 1170, 1156, 1138, 836 cm⁻¹
NMR (CDCl₃) δ: 1.78 - 2.46 (6H, m), 2.62 (2H, t, J = 6.8 Hz), 2.74 - 3.56 (5H, m), 4.00 (2H, t, J = 6.8 Hz), 6.91 - 8.22 (8H, m), 7.92 (1H, d, J = 8.4 Hz), 7.98 (1H, d, J = 8.6 Hz)

### Inventive Example 122

### 4-Amino-N-{2-[4-(4-fluorobenzoyl)piperidino]ethyl}-N-(4-methylphenyl)benzamide

Using 4-nitro-N-{2-[4-(4-fluorobenzoyl)piperidino]ethyl}-N-(4-methylphenyl)benzamide (744.5 mg, 1.52 mmol), the procedure of Inventive Example 104 was repeated to obtain 647.4 mg (92.7%) of the title compound in the form of colorless powder.
Melting point: 164 - 165°C
IR (KBr): 1680, 1620, 1598, 1506, 1376, 1298, 1274, 1170, 838 cm⁻¹
NMR (CDCl₃) δ: 1.71 - 2.16 (6H, m), 2.28 (3H, s), 2.62 (2H, dd, J = 7.3 Hz, 6.6 Hz), 2.95 - 3.17 (3H, m), 3.66 - 3.71 (2H, brs), 4.00 (2H, dd, J = 7.3 Hz, 6.6 Hz), 6.40 (2H, d, J = 8.6 Hz), 6.99 - 7.22 (8H, m), 7.92 (1H, d, J = 8.6 Hz), 7.98 (1H, d, J = 8.6 Hz)

### Inventive Example 123

### 3-Amino-N-{2-[4-(4-fluorobenzoyl)piperidino]ethyl}-N-(phenyl)benzamide

Using 3-nitro-N-{2-[4-(4-fluorobenzoyl)piperidino]ethyl}-N-(phenyl)benzamide (715.5 mg, 1.50 mmol), the procedure of Inventive Example 104 was repeated to obtain 156.8 mg (23.5%) of the title compound in a colorless amorphous form.
Melting point: 228 - 229°C
IR (KBr): 1668, 1640, 1592, 1492, 1388, 1372, 1320, 1302, 1264, 1222, 1204, 1170, 746, 698, 606 cm⁻¹
NMR (CDCl₃) δ: 2.36 - 2.69 (6H, m), 2.62 (2H, t, J = 6.6 Hz), 2.94 - 3.31 (3H, m), 3.43 - 3.69 (2H, brs), 4.05 (2H, t, J = 6.6 Hz), 6.49 - 7.23 (11H, m), 7.99 (1H, d, J = 8.6 Hz)

### Inventive Example 124

### 3-Amino-N-{2-[4-(4-fluorobenzoyl)piperidino]ethyl}-N-(4-methylphenyl)benzamide

Using 3-nitro-N-{2-[4-(4-fluorobenzoyl)piperidino]ethyl}-N-(4-methylphenyl)benzamide (735.0 mg, 1.50 mmol), the procedure of Inventive Example 104 was repeated to obtain 355.6 mg (51.6%) of the title compound in the form of colorless powder.
Melting point: 173 - 175°C
IR (KBr): 1674, 1630, 1598, 1508, 1384, 1314, 1298, 1216, 740 cm⁻¹
NMR (CDCl₃) δ: 1.59 - 2.15 (6H, m), 2.26 (3H, s), 2.59 (2H, t, J = 6.8 Hz), 2.93 - 3.27 (3H, m), 3.32 - 3.70 (2H, m), 4.00 (2H, t, J = 6.8 Hz), 6.49 - 6.57 (2H, m), 6.70 (1H, s), 6.80 - 7.22 (7H, m), 7.91 (1H, d, J = 8.6 Hz), 7.97 (1H, d, J = 8.6 Hz)

### Inventive Example 125

### 3-Amino-N-{2-[4-(4-fluorobenzoyl)piperidino]ethyl}-N-(4-fluorophenyl)benzamide

Using 3-nitro-N-{2-[4-(4-fluorobenzoyl)piperidino]ethyl}-N-(4-fluorophenyl)benzamide (491.2 mg, 1.00 mmol), the procedure of Inventive Example 104 was repeated to obtain 272.6 mg (58.8%) of the title compound in the form of colorless powder.
Melting point: 159 - 162°C
IR (KBr): 1640, 1594, 1588, 1506, 1386, 1374, 1274, 1220, 1200, 978, 844, 746, 614 cm⁻¹
NMR (CDCl₃) δ: 1.66 - 2.21 (6H, m), 2.58 (2H, t, J = 6.6 Hz), 2.92 - 3.03 (3H, m), 3.59 (2H, brs), 3.99 (2H, t, J = 6.6 Hz), 6.47 - 6.68 (3H, m), 6.82 - 7.22 (7H, m), 7.93 (1H, d, J = 8.6 Hz), 7.99 (1H, d, J = 8.6 Hz)

### Inventive Example 126

### 3-Amino-N-{2-[4-(4-fluorobenzoyl)piperidino]ethyl}-N-(3-methylthiophenyl)benzamide

Using 3-nitro-N-{2-[4-(4-fluorobenzoyl)piperidino]ethyl}-N-(3-methylthiophenyl)benzamide (785.0 mg, 1.50 mmol), the procedure of Inventive Example 104 was repeated to obtain 439.1 mg (59.5%) of the title compound in the form of colorless powder.
Melting point: 172 - 174°C
IR (KBr): 1672, 1626, 1592, 1458, 1394, 1318, 1304, 1210, 1168, 972, 862, 786, 754, 694 cm⁻¹
NMR (CDCl₃) δ: 2.33 (3H, s), 1.73 - 2.40 (6H, m), 2.60 (2H, dd, J = 6.6 Hz, 6.4 Hz), 2.94 - 3.41 (3H, m), 3.42 - 3.71 (2H, m), 4.03 (2H, dd, J = 6.6 Hz, 6.4 Hz), 6.50 - 7.25 (10H, m), 7.93 (1H, d, J = 8.6 Hz), 7.99 (1H, d, J = 8.6 Hz)

### Inventive Example 127

### 4-Amino-N-{2-[4-(4-fluorobenzoyl)piperidino]ethyl}-N-(4-chlorophenyl)benzamide

Using 4-nitro-N-{2-[4-(4-fluorobenzoyl)piperidino]ethyl}-N-(4-chlorophenyl)benzamide (760.6 mg, 1.49 mmol), the procedure of Inventive Example 104 was repeated to obtain 517.6 mg (72.4%) of the title compound in a colorless amorphous form.
IR (KBr): 1680, 1628, 1600, 1490, 1410, 1374, 1310, 1288, 1224, 1204, 1172, 1156, 1138, 1090, 974, 834, 762, 598 cm⁻¹
NMR (CDCl₃) δ: 1.85 - 2.19 (6H, m), 2.60 (2H, dd, J = 6.8, 6.6 Hz), 2.92 - 3.17 (3H, m), 3.77 (2H, brs), 3.99 (2H, dd, J = 6.8 Hz, 6.6 Hz), 6.42 (2H, d, J = 7.4 Hz), 6.98 - 7.88 (8H, m), 7.95 (2H, dd, J = 8.6 Hz, 5.5 Hz)

### Inventive Example 128

### 4-Amino-N-{2-[4-(4-fluorobenzoyl)piperidino]ethyl}-N-(3,4-dimethylphenyl)benzamide

Using 4-nitro-N-{2-[4-(4-fluorobenzoyl)piperidino]ethyl}-N-(3,4-dimethylphenyl)benzamide (751.8 mg, 1.49 mmol), the procedure of Inventive Example 104 was repeated to obtain 567.2 mg (80.4%) of the title compound in the form of light brown powder.
Melting point: 188 - 190°C
IR (KBr): 1680, 1620, 1598, 1562, 1502, 1380, 1298, 1276, 1264, 1184, 1176, 1160, 1140, 1130, 854, 838 cm⁻¹
NMR (CDCl₃) δ: 2.17 (6H, s), 1.68 - 2.36 (6H, m), 2.66 (2H, dd, J = 7.3 Hz, 6.8 Hz), 2.97 - 3.20 (3H, m), 3.72 (2H, brs), 4.01 (2H, dd, J = 7,3 Hz, 6.8 Hz), 6.40 (2H, d, J = 8.6 Hz), 6.80 - 7.26 (7H, m), 7.95 (2H, dd, J = 8.6 Hz, 5.5 Hz)

### Inventive Example 129

### 4-Amino-N-{2-[4-(4-fluorobenzoyl)piperidino]ethyl}-N-(3,5-dimethylphenyl)benzamide

Using 4-nitro-N-{2-[4-(4-fluorobenzoyl)piperidino]ethyl}-N-(3,5-dimethylphenyl)benzamide (663.2 mg, 1.32 mmol), the procedure of Inventive Example 104 was repeated to obtain 601.7 mg (96.3%) of the title compound in a light brown amorphous form.
IR (KBr): 1680, 1626, 1596, 1376, 1314, 1264, 1232, 1180, 1156, 838, 760 cm⁻¹
NMR (CDCl₃) δ: 2.20 (6H, s), 1.57 - 2.31 (6H, m), 2.62 (2H, dd, J = 7.0 Hz, 6.8 Hz), 2.95 - 3.01 (3H, m), 3.71 (2H, brs), 3.98 (2H, dd, J = 7.0 Hz, 6.8 Hz), 6.40 (2H, d, J = 8.6 Hz), 6.71 - 6.76 (3H, m), 7.03 - 7.26 (6H, m), 7.95 (2H, dd, J = 8.6 Hz, 5.5 Hz)

### Inventive Example 130

### 4-Amino-N-{3-[4-(4-fluorobenzoyl)piperidino]propyl}-N-(3-methoxyphenyl)benzamide

Using 4-nitro-N-{3-[4-(4-fluorobenzoyl)piperidino]propyl}-N-(3-methoxyphenyl)benzamide (872.0 mg, 1.68 mmol), the procedure of Inventive Example 104 was repeated to obtain 808.8 mg (98.5%) of the title compound in a colorless amorphous form.
IR (KBr): 2948, 1680, 1628, 1600, 1488, 1452, 1380, 1310, 1230, 1198, 1180, 1158 cm⁻¹
NMR (CDCl₃) δ: 1.57 - 2.20 (7H, m), 2.42 (2H, d, J = 7.5 Hz), 2.80 - 3.26 (4H, m), 3.69 (3H, s), 3.60 - 3.76 (2H, m), 3.92 (2H, d, J = 7.5 Hz), 6.40 (2H, d, J = 8.6 Hz), 6.50 - 6.75 (3H, m), 7.11 (2H, dd, J = 8.6 Hz, 5.5 Hz), 7.15 (2H, d, J = 8.6 Hz), 7.95 (2H, dd, J = 8.6 Hz, 5.5 Hz)

### Inventive Example 131

### 4-Amino-N-{2-[4-(4-fluorobenzoyl)piperidino]ethyl}-N-(3,4-methylenedioxyphenyl)benzamide

Using 4-nitro-N-{2-[4-(4-fluorobenzoyl)piperidino]ethyl}-N-(3,4-methylenedioxyphenyl)benzamide (300.0 mg, 0.578 mmol), the procedure of Inventive Example 104 was repeated to obtain 245.0 mg (86.7%) of the title compound in a light yellow amorphous form.
IR (KBr): 1680, 1602, 1503, 1485, 1446, 1389, 1338, 1299, 1233, 1209, 1173, 1153, 1131, 1035, 837, 753 cm⁻¹
NMR (CDCl₃) δ: 1.70 - 2.00 (4H, m), 2.00 - 2.36 (2H, m), 2.62 (2H, t, J = 6.8 Hz), 2.86 - 3.40 (3H, m), 3.95 (2H, t, J = 6.8 Hz), 6.88 - 7.30 (4H, m), 7.80 - 8.10 (2H, m)

### Inventive Example 132

### 4-Amino-N-{2-[4-(4-fluorobenzoyl)piperidino]ethyl}-N-(1-naphthyl)benzamide

Using 4-nitro-N-{2-[4-(4-fluorobenzoyl)piperidino]ethyl}-N-(1-naphthyl)benzamide (360.6 mg, 0.687 mmol), the procedure of Inventive Example 104 was repeated to obtain 352.9 mg (quantitative) of the title compound in a light yellow amorphous form.
IR (KBr): 3352, 1680, 1626, 1599, 1506, 1437, 1401, 1374, 1338, 1299, 1227, 1179, 1155, 1140, 1104, 972, 837, 774 cm⁻¹
NMR (CDCl₃) δ: 1.70 - 1.90 (4H, m), 1.97 - 2.34 (2H, m), 2.68 (2H, t, J = 6.5 Hz), 2.80 - 3.30 (3H, m), 3.70 (1H, quint., J = 6.5 Hz), 4.52 (1H, quint., J = 6.5 Hz), 6.25 (2H, d, J = 8.5 Hz), 6.90 - 8.20 (13H, m)

### Inventive Example 133

### 4-(Acetylamino)methyl-N-{2-[4-(4-fluorobenzoyl)piperidino]ethyl}-N-(2-methoxyethyl)benzamide

In an atmosphere of argon, 4-aminomethyl-N-{2-[4-(4-fluorobenzoyl)piperidino]ethyl}-N-(2-methoxyethyl)benzamide (80 mg, 0.181 mmol) was dissolved in methylene chloride (3 ml) to which were subsequently added dropwise triethylamine (63 µl, 0.452 mmol) and acetic anhydride (24 µl, 0.217 mmol) while cooling in an ice bath. After 20 minutes of stirring at the same temperature, the reaction solution was diluted with water (10 ml) and extracted with ethyl acetate. The resulting organic layer was washed with saturated brine and dried on anhydrous sodium carbonate. Thereafter, the solvent was removed by evaporation, and the resulting residue was purified by a silica gel column chromatography (ethyl acetate:methanol = 9:1) to obtain 68.5 mg (78.3%) of the title compound in the form of colorless solid.
Melting point: 100 - 103°C
IR (KBr): 3276, 2944, 2820, 1664, 1628, 1596, 1464, 1430, 1290, 1222, 1170, 1114 cm⁻¹
NMR (CDCl₃) δ: 1.50 - 1.93 (4H, m), 1.95 - 2.35 (2H, m), 2.03 (3H, s), 2.39 - 3.40 (5H, m), 3.32 (3H, s), 3.40 - 3.90 (6H, m), 4.42 (2H, d, J = 5.7 Hz), 6.24 (1H, br-s), 7.12 (2H, dd, J = 9 Hz, 9 Hz), 7.16 - 7.50 (4H, m), 7.94 (2H, dd, J = 9 Hz, 6 Hz)

### Inventive Example 134

### 4-(Acetylamino)methyl-N-{2-[4-(4-fluorobenzoyl)piperidino]ethyl}-N-(2-methoxyphenyl)benzamide

In an atmosphere of argon, 4-aminomethyl-N-{2-[4-(4-fluorobenzoyl)piperidino]ethyl}-N-(2-methoxyphenol)benzamide (60 mg, 0.123 mmol) was dissolved in methylene chloride (2 ml) to which were subsequently added dropwise pyridine (23 µl, 0.284 mmol) and acetic anhydride (13 µl, 0.138 mmol) while cooling in an ice bath, followed by the addition of a catalytically effective amount of 4-dimethylaminopyridine. After 20 minutes of stirring at the same temperature and additional 12 hours of stirring at room temperature, the reaction solution was diluted with water (10 ml) and extracted with ethyl acetate. The resulting organic layer was washed with saturated brine and dried on anhydrous sodium carbonate. Thereafter, the solvent was removed by evaporation, and the resulting residue was purified by a PTLC (chloroform:methanol = 20:1) to obtain 58 mg (89.0%) of the title compound in a colorless amorphous form.
IR (KBr): 3304, 2944, 1674, 1641, 1596, 1503, 1413, 1392, 1380, 1278 cm⁻¹
NMR (CDCl₃) δ: 1.43 - 2.33 (6H, m), 1.98 (3H, s), 2.62 (2H, t, J = 7.5 Hz), 2.82 - 3.30 (3H, m), 3.52 - 3.92 (1H, m), 3.67 (3H, s), 4.00 - 4.43 (1H, m), 4.31 (2H, d, J = 6 Hz), 5.60 (1H, br-s), 6.73 (1H, d, J = 8 Hz), 6.90 (1H, d, J = 9 Hz), 6.98 - 7.40 (8H, m), 7.93 (2H, dd, J = 9 Hz, 6 Hz)

### Inventive Example 135

### 4-Acetylamino-N-{2-[4-(4-fluorobenzoyl)piperidino]ethyl}-N-(2-methoxyphenyl)benzamide

Using 4-amino-N-{2-[4-(4-fluorobenzoyl)piperidino]ethyl}-N-(2-methoxyphenyl)benzamide (2.65 g, 5.58 mmol) and acetic anhydride (0.79 ml, 8.37 mmol), the procedure of Inventive Example 134 was repeated to obtain 2.44 g (84.3%) of the title compound in a colorless amorphous form.
IR (KBr): 3310, 2944, 1680, 1632, 1596, 1530, 1407, 1374, 1331, 1270, 1236, 750 cm⁻¹
NMR (CDCl₃) δ: 1.69 - 1.90 (4H, m), 2.06 (3H, s), 2.00 - 2.35 (2H, m), 2.63 (2H, t, J = 7.0 Hz), 2.80 - 3.25 (3H, m), 3.65 (3H, s), 3.70 - 3.90 (1H, m), 3.95 - 4.35 (1H, m), 6.77 (2H, t, J = 9.0 Hz), 6.96 - 7.30 (8H, m), 7.50 (1H, brs), 7.95 (2H, dd, J = 9.0 Hz, 6.0 Hz)

### Inventive Example 136

### 4-Acetylamino-N-{2-[4-(4-fluorobenzoyl)piperidino]ethyl}-N-(3-methoxyphenyl)benzamide

Using 4-amino-N-{2-[4-(4-fluorobenzoyl)piperidino]ethyl}-N-(3-methoxyphenyl)benzamide (1.00 g, 2.10 mmol) and acetic anhydride (0.24 ml, 2.54 mmol), the procedure of Inventive Example 134 was repeated to obtain 851.0 mg (78.3%) of the title compound in the form of colorless powder.
Melting point: 145.5 - 151.5°C
IR (KBr): 1680, 1632, 1600, 1530, 1488, 1400, 1314, 1282, 1158 cm⁻¹
NMR (CDCl₃) δ: 1.60 - 2.43 (5H, m), 2.10 (3H, s), 2.68 (2H, t, J = 7.0 Hz), 2.83 - 3.40 (4H, m), 3.69 (3H, s), 4.05 (2H, t, J = 7.0 Hz), 6.52 - 6.73 (3H, m), 6.78 (2H, s), 6.52 - 7.50 (5H, m), 7.13 (2H, dd, J = 8.8 Hz, 5.5 Hz), 7.96 (2H, dd, J = 8.8 Hz, 5.5 Hz)

### Inventive Example 137

### 4-Acetylamino-N-{2-[4-(4-fluorobenzoyl)piperidino]ethyl}-N-(4-methoxyphenyl)benzamide

Using 4-amino-N-{2-[4-(4-fluorobenzoyl)piperidino]ethyl}-N-(4-methoxyphenyl)benzamide (556.9 mg, 1.17 mmol) and acetic anhydride (0.13 ml, 1.41 mmol), the procedure of Inventive Example 134 was repeated to obtain 476.8 mg (78.7%) of the title compound in the form of colorless powder.
Melting point: 171 - 173°C
IR (KBr): 3316, 1671, 1632, 1599, 1527, 1512, 1437, 1404, 1377, 1314, 1296, 1245, 1203, 1173, 1153, 1113, 1029, 972, 852, 837, 762 cm⁻¹
NMR (CDCl₃-CD₃OD) δ: 1.67 - 2.00 (4H, m), 2.00 - 2.40 (2H, m), 2.12 (3H, s), 2.63 (2H, t, J = 6.9 Hz), 2.85 - 3.40 (3H, m), 3.75 (3H, s), 4.01 (2H, t, J = 6.9 Hz), 6.72 (2H, d, J = 9.0 Hz), 6.87 - 7.40 (8H, m), 6.78 (2H, s), 7.80 - 8.06 (2H, m)

### Inventive Example 138

### 4-Acetylamino-N-{2-[4-(4-fluorobenzoyl)piperidino]ethyl}-N-(2,5-dimethoxyphenyl)benzamide

Using 4-amino-N-{2-[4-(4-fluorobenzoyl)piperidino]ethyl}-N-(2,5-dimethoxyphenyl)benzamide (170.0 mg, 0.34 mmol) and acetic anhydride (0.16 ml, 1.70 mmol), the procedure of Inventive Example 134 was repeated to obtain 132.0 mg (71.5%) of the title compound in a colorless amorphous form.
IR (KBr): 3352, 2973, 1678, 1600, 1508, 1314, 1262, 1222 cm⁻¹
NMR (CDCl₃) δ: 1.76 - 2.38 (6H, m), 2.11 (3H, s), 2.62 (2H, brt, J = 7.3 Hz), 2.85 - 3.40 (3H, m), 3.68 (3H, s), 3.78 (3H, s), 4.01 - 4.08 (2H, m), 6.58 - 6.78 (2H, m), 7.03 - 7.62 (7H, m), 7.87 - 8.03 (2H, m)

### Inventive Example 139

### 3-Acetylamino-N-{2-[4-(4-fluorobenzoyl)piperidino]ethyl}-N-(3-methoxyphenyl)benzamide

Using 3-amino-N-{2-[4-(4-fluorobenzoyl)piperidino]ethyl}-N-(3-methoxyphenyl)benzamide (216.0 mg, 0.45 mmol) and acetic anhydride (0.051 ml, 0.54 mmol), the procedure of Inventive Example 134 was repeated to obtain 197.5 mg (84.9%) of the title compound in a colorless amorphous form.
IR (KBr): 3300, 2948, 1680, 1640, 1598, 1434, 1376, 1282, 1046 cm⁻¹
NMR (CDCl₃) δ: 1.60 - 2.33 (5H, m), 2.08 (3H, s), 2.60 (2H, t, J = 6.6 Hz), 2.83 - 3.30 (4H, m), 3.66 (3H, s), 4.05 (2H, t, J = 6.6 Hz), 6.53 - 6.75 (2H, m), 6.75 - 7.28 (6H, m), 7.40 (1H, brs), 7.46 - 7.70 (1H, m), 7.93 (2H, dd, J = 8.8 Hz, 5.5 Hz)

### Inventive Example 140

### 4-Acetylamino-N-{2-[4-(4-fluorobenzoyl)piperidino]ethyl}-N-(2,4-dimethoxyphenyl)benzamide

Using 4-amino-N-{2-[4-(4-fluorobenzoyl)piperidino]ethyl}-N-(2,4-dimethoxyphenyl)benzamide (203.4 mg, 0.40 mmol) and acetic anhydride (0.046 ml, 0.49 mmol), the procedure of Inventive Example 134 was repeated to obtain 161.2 mg (73.6%) of the title compound in a colorless amorphous form.
IR (KBr): 1680, 1630, 1598, 1530, 1510, 1408, 1312, 1282, 1260, 1208, 1158, 850 cm⁻¹
NMR (CDCl₃) δ: 1.68 - 4.27 (14H, m), 3.67 (3H, s), 3.73 (3H, s), 6.20 - 6.38 (2H, m), 6.93 - 7.20 (7H, m), 7.87 - 7.95 (2H, m)

### Inventive Example 141

### 4-Acetylamino-N-{2-[4-(4-fluorobenzoyl)piperidino]ethyl}-N-(3-methylphenyl)benzamide

Using 4-amino-N-{2-[4-(4-fluorobenzoyl)piperidino]ethyl}-N-(3-methylphenyl)benzamide (285.0 mg, 0.62 mmol) and acetic anhydride (0.071 ml, 0.74 mmol), the procedure of Inventive Example 134 was repeated to obtain 311.8 mg (99.9%) of the title compound in a colorless amorphous.
IR (KBr): 3310, 2940, 2790, 1680, 1629, 1602, 1527, 1446, 1374, 1227, 1155, 849 cm⁻¹
NMR (CDCl₃-CD₃OD) δ: 1.42 - 2.35 (6H, m), 2.09 (3H, s), 2.24 (3H, s), 2.62 (2H, t, J = 7.0 Hz), 2.78 - 3.38 (3H, m), 4.02 (2H, t, J = 7.0 Hz), 6.60 - 7.46 (11H, m), 7.95 (2H, dd, J = 9.0 Hz, 6.0 Hz)

### Inventive Example 142

### 4-Acetylamino-N-{2-[4-(4-fluorobenzoyl)piperidino]ethyl}-N-(phenyl)benzamide

Using 4-amino-N-{2-[4-(4-fluorobenzoyl)piperidino]ethyl}-N-(phenyl)benzamide (503.8 mg, 1.13 mmol) and acetic anhydride (0.128 ml, 1.36 mmol), the procedure of Inventive Example 134 was repeated to obtain 309.9 mg (71.0%) of the title compound in a light brown amorphous form.
IR (KBr): 1680, 1632, 1599, 1530, 1407, 1374, 1314, 1260 cm⁻¹
NMR (CDCl₃) δ: 1.77 - 1.99 (5H, m), 2.11 (3H, s), 2.26 - 2.31 (2H, m), 2.63 (2H, t, J = 6.8 Hz), 2.93 - 3.17 (3H, m), 4.04 (2H, t, J = 6.8 Hz), 6.99 - 7.40 (11H, m), 7.92 (2H, d, J = 7.9 Hz), 7.98 (1H, d, J = 7.9 Hz)

### Inventive Example 143

### 4-Acetylamino-N-{2-[4-(4-fluorobenzoyl)piperidino]ethyl}-N-(3-thiomethylphenyl)benzamide

Using 4-amino-N-{2-[4-(4-fluorobenzoyl)piperidino]ethyl}-N-(3-thiomethylphenyl)benzamide (220.6 mg, 0.45 mmol) and acetic anhydride (0.051 ml, 0.54 mmol), the procedure of Inventive Example 134 was repeated to obtain 231.2 mg (91.8%) of the title compound in a colorless amorphous form.
IR (KBr): 1680, 1630, 1598, 1530, 1476, 1438, 1406, 1372, 1312, 1260, 1226, 1204, 1156, 850, 760 cm⁻¹
NMR (CDCl₃) δ: 1.77 - 1.96 (4H, m), 2.04 - 2.19 (2H, m), 2.12 (3H, s), 2.45 (4H, s), 2.61 (2H, t, J = 6.6 Hz), 2.94 - 3.18 (3H, m), 4.03 (2H, t, J = 6.6 Hz), 6.80 - 7.38 (10H, m), 7.92 (2H, d, J = 8.6 Hz), 7.98 (1H, d, J = 8.6 Hz)

### Inventive Example 144

### 4-Acetylamino-N-{2-[4-(4-fluorobenzoyl)piperidino]ethyl}-N-(2-trifluoromethylphenyl)benzamide

Using 4-amino-N-{2-[4-(4-fluorobenzoyl)piperidino]ethyl}-N-(2-trifluoromethylphenyl)benzamide (243.0 mg, 0.474 mmol) and acetic anhydride (0.13 ml, 1.40 mmol), the procedure of Inventive Example 134 was repeated to obtain 265.0 mg (quantitative) of the title compound in a colorless amorphous form.
IR (KBr): 3348, 2942, 1680, 1636, 1600, 1316, 1262, 1172, 842, 760, 608 cm⁻¹
NMR (CDCl₃) δ: 1.66 - 1.83 (4H, m), 2.04 - 2.19 (2H, m), 2.09 (3H, s), 2.52 - 3.52 (6H, m), 4.45 (1H, brs), 7.03 - 7.87 (10H, m), 7.92 (2H, dd, J = 8.8 Hz, 5.5 Hz)

### Inventive Example 145

### 4-Acetylamino-N-{2-[4-(4-fluorobenzoyl)piperidino]ethyl}-N-(3-trifluoromethylphenyl)benzamide

Using 4-amino-N-{2-[4-(4-fluorobenzoyl)piperidino]ethyl}-N-(3-trifluoromethylphenyl)benzamide (256.0 mg, 0.474 mmol) and acetic anhydride (0.13 ml, 1.40 mmol), the procedure of Inventive Example 134 was repeated to obtain 267.0 mg (96.2%) of the title compound in a colorless amorphous form.
IR (KBr): 3335, 2949, 1680, 1598, 1408, 1330, 1262, 1128, 843, 697 cm⁻¹
NMR (CDCl₃) δ: 1.69 - 1.87 (4H, m), 2.04 -2.32 (2H, m), 2.12 (3H, s), 2.61 (2H, t, J = 6.2 Hz), 2.89 - 3.20 (3H, m), 4.05 (2H, t, J = 6.2 Hz), 7.03 - 7.44 (10H, m), 7.95 (2H, dd, J = 8.8 Hz, 5.5 Hz)

### Inventive Example 146

### 4-Acetylamino-N-{2-[4-(4-fluorobenzoyl)piperidino]ethyl}-N-(3-pyridyl)benzamide

Using 4-amino-N-{2-[4-(4-fluorobenzoyl)piperidino]ethyl}-N-(3-pyridyl)benzamide (150.0 mg, 0.335 mmol) and acetic anhydride (0.038 ml, 0.40 mmol), the procedure of Inventive Example 134 was repeated to obtain 151.0 mg (95.6%) of the title compound in a colorless amorphous form.
IR (KBr): 3290, 2920, 1680, 1641, 1599, 1530, 1428, 1407, 1374, 1227, 1158, 849 cm⁻¹
NMR (CDCl₃) δ: 1.50 - 2.35 (6H, m), 2.12 (3H, s), 2.64 (2H, t, 7.0 Hz), 2.79 - 3.32 (3H, m), 4.50 (2H, t, J = 7.0 Hz), 6.92 - 7.62 (9H, m), 7.94 (2H, dd, J = 9.0 Hz, 6.0 Hz)

### Inventive Example 147

### 2-Acetylamino-N-{2-[4-(4-fluorobenzoyl)piperidino]ethyl}-N-(3-methoxyphenyl)benzamide

Using 2-amino-N-{2-[4-(4-fluorobenzoyl)piperidino]ethyl}-N-(3-methoxyphenyl)benzamide (238.0 mg, 0.50 mmol) and acetic anhydride (0.057 ml, 0.60 mmol), the procedure of Inventive Example 134 was repeated to obtain 170.4 mg (65.9%) of the title compound in a colorless amorphous form.
IR (KBr): 3459, 2940, 1680, 1635, 1595, 1505, 1440, 1370, 1290, 1160, 1035, 960 cm⁻¹
NMR (CDCl₃) δ: 1.43 - 2.35 (5H, m), 2.20 (3H, s), 2.60 (2H, t, J = 6.0 Hz), 2.80 - 3.30 (4H, m), 3.63 (3H, s), 4.05 (2H, t, J = 6.0 Hz), 6.45 - 7.00 (5H, m), 7.08 (2H, t, J = 8.8 Hz), 7.10 (2H, dd, each J = 9.0 Hz), 7.93 (2H, dd, J = 9.0 Hz, 6.0 Hz), 8.20 (1H, d, J = 9.0 Hz), 9.15 - 9.33 (1H, m)

### Inventive Example 148

### 4-Acetylamino-N-{2-[4-(4-fluorobenzoyl)piperidino]ethyl}-N-(3-acetylaminophenyl)benzamide

Using 2-amino-N-{2-[4-(4-fluorobenzoyl)piperidino]ethyl}-N-(3-acetylaminophenyl)benzamide (230.0 mg, 0.50 mmol) and acetic anhydride (0.113 ml, 0.60 mmol), the procedure of Inventive Example 134 was repeated to obtain 248.0 mg (91.2%) of the title compound in a light yellow amorphous form.
IR (KBr): 3290, 2930, 1677, 1602, 1533, 1488, 1443, 1374, 1230, 849 cm⁻¹
NMR (CDCl₃) δ: 1.50 - 2.36 (6H, m), 2.06 (6H, s), 2.64 (2H, t, J = 7.0 Hz), 2.80 - 3.34 (3H, m), 4.01 (2H, t, J = 7.0 Hz), 6.65 - 7.63 (10H, m), 7.74 - 8.31 (4H, m)

### Inventive Example 149

### 4-Acetylamino-N-{2-[4-(4-fluorobenzoyl)piperidino]ethyl}-N-(2-methoxycarbonylphenyl)benzamide

Using 4-amino-N-{2-[4-(4-fluorobenzoyl)piperidino]ethyl}-N-(2-methoxycarbonylphenyl)benzamide (291.0 mg, 0.58 mmol) and acetic anhydride (0.11 ml, 1.16 mmol), the procedure of Inventive Example 134 was repeated to obtain 303.0 mg (96.0%) of the title compound in a colorless amorphous form.
IR (KBr): 3351, 2948, 1724, 1678, 1598, 1312, 1260, 972, 848, 758, 602 cm⁻¹
NMR (CDCl₃) δ: 1.71 - 2.23 (6H, m), 2.09 (6H, s), 2.66 - 3.22 (5H, m), 3.60 - 3.75 (1H, m), 3.82 (3H, s), 4.16 - 4.45 (1H, m), 7.02 - 7.72 (10H, m), 7.93 (2H, dd, J = 8.8, 5.5 Hz)

### Inventive Example 150

### 3-Methyl-4-acetylamino-N-{2-[4-(4-fluorobenzoyl)piperidino]ethyl}-N-(3-methoxyphenyl)benzamide

Using 4-amino-3-methyl-N-{2-[4-(4-fluorobenzoyl)piperidino]ethyl}-N-(3-methoxyphenyl)benzamide (489.0 mg, 1.00 mmol) and acetic anhydride (0.19 ml, 2.00 mmol), the procedure of Inventive Example 134 was repeated to obtain 474.0 mg (89.3%) of the title compound in a colorless amorphous form.
IR (KBr): 3298, 2949, 1678, 1512, 1246, 972, 835, 601 cm⁻¹
NMR (CDCl₃) δ: 1.71 - 2.32 (6H, m), 2.13 (6H, s), 2.60 (2H, t, J = 6.8 Hz), 3.32 (2H, t, J = 6.8 Hz), 2.95 - 3.32 (3H, m), 3.75 (3H, s), 3.99 (2H, t, J = 6.8 Hz), 6.74 (2H, d, J = 8.8 Hz), 6.96 - 7.25 (6H, m), 7.74 (1H, brs), 7.95 (2H, dd, J = 8.6 Hz, 5.5 Hz)

### Inventive Example 151

### 3-Methoxy-4-acetylamino-N-{2-[4-(4-fluorobenzoyl)piperidino]ethyl}-N-(3-methoxyphenyl)benzamide

Using 4-amino-3-methoxy-N-{2-[4-(4-fluorobenzoyl)piperidino]ethyl}-N-(3-methoxyphenyl)benzamide (253.0 mg, 0.50 mmol) and acetic anhydride (0.095 ml, 1.00 mmol), the procedure of Inventive Example 134 was repeated to obtain 213.0 mg (77.9%) of the title compound in a yellow amorphous form.
IR (KBr): 3435, 2945, 1680, 1636, 1596, 1510, 1246, 1031, 829, 600 cm⁻¹
NMR (CDCl₃) δ: 1.71 - 1.86 (4H, m), 2.04 - 2.33 (2H, m), 2.15 (3H, s), 2.62 (2H, t, J = 6.8 Hz), 2.95 - 3.33 (3H, m), 3.75 (6H, s), 4.01 (2H, t, J = 6.8 Hz), 6.74 (2H, d, J = 8.6 Hz), 6.87 - 7.26 (5H, m), 7.74 (1H, brs), 7.88 - 8.17 (3H, m)

### Inventive Example 152

### 4-Acetylamino-N-{2-[4-(4-fluorobenzoyl)piperidino]ethyl}-N-(4-fluorophenyl)benzamide

Using 4-amino-N-{2-[4-(4-fluorobenzoyl)piperidino]ethyl}-N-(4-fluorophenyl)benzamide (227.3 mg, 0.49 mmol) and acetic anhydride (0.083 ml, 0.88 mmol), the procedure of Inventive Example 134 was repeated to obtain 202.1 mg (81.6%) of the title compound in a light brown amorphous form.
IR (KBr): 1680, 1628, 1598, 1532, 1506, 1406, 1372, 1312, 1262, 1214, 850 cm⁻¹
NMR (CDCl₃) δ: 1.85 - 2.32 (6H, m), 2.11 (3H, s), 2.60 (2H, t, J = 6.6 Hz), 2.82 - 3.19 (4H, m), 4.01 (2H, t, J = 6.6 Hz), 6.90 - 7.62 (9H, m), 7.88 - 8.04 (3H, m)

### Inventive Example 153

### 4-Acetylamino-N-{2-[4-(4-fluorobenzoyl)piperidino]ethyl}-N-(4-methylphenyl)benzamide

Using 4-amino-N-{2-[4-(4-fluorobenzoyl)piperidino]ethyl}-N-(4-methylphenyl)benzamide (452.7 mg, 0.99 mmol) and acetic anhydride (0.122 ml, 1.19 mmol), the procedure of Inventive Example 134 was repeated to obtain 473.6 mg (95.4%) of the title compound in a light brown amorphous form.
IR (KBr): 1680, 1628, 1598, 1532, 1512, 1444, 1406, 1374, 1314, 1278, 1260, 1226, 1206, 1156, 1140, 974, 850, 602 cm⁻¹
NMR (CDCl₃) δ: 1.68 - 2.04 (7H, m), 2.11 (3H, s), 2.27 (3H, s), 2.61 (2H, dd, J = 7.0 Hz, 6.4 Hz), 2.94 - 3.10 (3H, m), 4.01 (2H, dd, J = 7.0, 6.4 Hz), 6.98 - 7.26 (10H, m), 7.92 (1H, d, J = 8.2 Hz), 7.98 (1H, d, J = 8.2 Hz)

### Inventive Example 154

### 4-Acetylamino-N-{2-[4-(4-fluorobenzoyl)piperidino]ethyl}-N-(phenyl)benzamide

Using 3-amino-N-{2-[4-(4-fluorobenzoyl)piperidino]ethyl}-N-(phenyl)benzamide (126.4 mg, 0.28 mmol) and acetic anhydride (0.03 ml, 0.32 mmol), the procedure of Inventive Example 134 was repeated to obtain 101.1 mg (73.1%) of the title compound in a colorless powder form.
Melting point: 198 - 201°C
IR (KBr): 1676, 1666, 1648, 1594, 1428, 1382, 1304, 1292, 1266, 1228, 1208, 746 cm⁻¹
NMR (CDCl₃) δ: 1.69 - 2.19 (7H, m), 2.13 (3H, s), 2.60 (2H, t, J = 6.6 Hz), 2.94 - 3.09 (3H, m), 4.05 (2H, t, J = 6.6 Hz), 6.92 - 7.53 (11H, m), 7.92 (1H, d, J = 8.6 Hz), 7.99 (1H, d, J = 8.6 Hz)

### Inventive Example 155

### 3-Acetylamino-N-{2-[4-(4-fluorobenzoyl)piperidino]ethyl}-N-(4-methylphenyl)benzamide

Using 3-amino-N-{2-[4-(4-fluorobenzoyl)piperidino]ethyl}-N-(4-methylphenyl)benzamide (230.1 mg, 0.50 mmol) and acetic anhydride (0.057 ml, 0.60 mmol), the procedure of Inventive Example 134 was repeated to obtain 245.2 mg (97.8%) of the title compound in a light yellow amorphous form.
IR (KBr): 1680, 1630, 1512, 750 cm⁻¹
NMR (CDCl₃) δ: 1.71 - 2.66 (7H, m), 2.13 (3H, s), 2.25 (3H, s), 2.58 (2H, dd, J = 6.8 Hz, 6.6 Hz), 2.94 - 3.19 (3H, m), 4.01 (2H, dd, J = 6.8 Hz, 6.6 Hz), 6.82 - 7.69 (10H, m), 7.93 (1H, d, J = 8.8 Hz), 7.99 (1H, d, J = 8.8 Hz)

### Inventive Example 156

### 3-Acetylamino-N-{2-[4-(4-fluorobenzoyl)piperidino]ethyl}-N-(4-fluorophenyl)benzamide

Using 3-amino-N-{2-[4-(4-fluorobenzoyl)piperidino]ethyl}-N-(fluorophenyl)benzamide (223.2 mg, 0.48 mmol) and acetic anhydride (0.055 ml, 0.58 mmol), the procedure of Inventive Example 134 was repeated to obtain 238.6 mg (98.3%) of the title compound in a light yellow amorphous form. IR (KBr): 1680, 1642, 1598, 1552, 1508, 1428, 1374, 1310,
1214, 748 cm⁻¹
NMR (CDCl₃) δ: 1.67 - 2.31 (7H, m), 2.14 (3H, s), 2.58 (2H, t, J = 6.4 Hz), 2.93 - 3.25 (3H, m), 4.01 (2H, t, J = 6.4 Hz), 6.80 - 7.56 (10H, m), 7.93 (1H, d, J = 8.6 Hz), 7.99 (1H, d, J = 8.6 Hz)

### Inventive Example 157

### 3-Acetylamino-N-{2-[4-(4-fluorobenzoyl)piperidino]ethyl}-N-(3-methylthiophenyl)benzamide

Using 3-amino-N-{2-[4-(4-fluorobenzoyl)piperidino]ethyl}-N-(3-methylthiophenyl)benzamide (196.7 mg, 0.40 mmol) and acetic anhydride (0.046 ml, 0.48 mmol), the procedure of Inventive Example 134 was repeated to obtain 172.2 mg (80.7%) of the title compound in a light brown amorphous form.
IR (KBr): 1678, 1636, 1586, 1552, 1434, 1372, 1310, 1224, 748 cm⁻¹
NMR (CDCl₃) δ: 1.73 - 2.40 (7H, m), 2.13 (3H, s), 2.33 (3H, s), 2.59 (2H, dd, J = 6.6 Hz, 6.4 Hz), 2.94 - 3.21 (3H, m), 4.03 (2H, dd, J = 6.6 Hz, 6.4 Hz), 6.85 - 7.63 (10H, m), 7.93 (1H, d, J = 8.6 Hz), 7.99 (1H, d, J = 8.6 Hz)

### Inventive Example 158

### 4-Acetylamino-N-{2-[4-(4-fluorobenzoyl)piperidino]ethyl}-N-(4-chlorophenyl)benzamide

Using 4-amino-N-{2-[4-(4-fluorobenzoyl)piperidino]ethyl}-N-(3-chlorophenyl)benzamide (328.6 mg, 0.68 mmol) and acetic anhydride (0.078 ml, 0.83 mmol), the procedure of Inventive Example 134 was repeated to obtain 345.4 mg (97.3%) of the title compound in a light yellow amorphous form.
IR (KBr): 1680, 1630, 1598, 1530, 1492, 1408, 1372, 1312, 1260, 1224, 850, 758 cm⁻¹
NMR (CDCl₃) δ: 1.69 - 2.19 (7H, m), 2.13 (3H, s), 2.59 (2H, t, J = 6.6 Hz), 2.92 - 3.09 (3H, m), 4.00 (2H, t, J = 6.6 Hz), 6.97 - 7.39 (10H, m), 7.95 (2H, dd, J = 8.6 Hz, 5.5 Hz)

### Inventive Example 159

### 4-Acetylamino-N-{2-[4-(4-fluorobenzoyl)piperidino]ethyl}-N-(3,4-dimethylphenyl)benzamide

Using 4-amino-N-{2-[4-(4-fluorobenzoyl)piperidino]ethyl}-N-(3,4-dimethylphenyl)benzamide (333.1 mg, 0.70 mmol) and acetic anhydride (0.080 ml, 0.85 mmol), the procedure of Inventive Example 134 was repeated to obtain 359.9 mg (99.7%) of the title compound in a light brown amorphous form.
IR (KBr): 2944, 1680, 1628, 1598, 1530, 1504, 1446, 1408, 1372, 1312, 1260, 1228, 1206, 1178, 1156, 1140, 850, 760 cm⁻¹
NMR (CDCl₃) δ: 1.68 - 1.86 (4H, m), 2.16 (3H, s), 2.16 (6H, s), 2.05 - 2.31 (3H, m), 2.61 (2H, t, J = 6.8 Hz), 2.94 - 3.18 (3H, m), 4.00 (2H, t, J = 6.8 Hz), 6.79 - 7.27 (9H, m), 7.95 (2H, dd, J = 8.6 Hz, 5.5 Hz)

### Inventive Example 160

### 4-Acetylamino-N-{2-[4-(4-fluorobenzoyl)piperidino]ethyl}-N-(3,5-dimethylphenyl)benzamide

Using 4-amino-N-{2-[4-(4-fluorobenzoyl)piperidino]ethyl}-N-(3,5-dimethylphenyl)benzamide (382.8 mg, 0.81 mmol) and acetic anhydride (0.092 ml, 0.99 mmol), the procedure of Inventive Example 134 was repeated to obtain 360.9 mg (86.4%) of the title compound in a light brown amorphous form.
IR (KBr): 3252, 2948, 1686, 1616, 1598, 1518, 1506, 1474, 1436, 1408, 1396, 1374, 1334, 1312, 1296, 1236, 1210, 1138, 1128, 974 cm⁻¹
NMR (CDCl₃) δ: 1.67 - 1.86 (4H, m), 2.12 (3H, s), 2.19 (6H, s), 2.04 - 2.32 (3H, m), 2.61 (2H, t, J = 6.8 Hz), 2.94 - 3.11 (3H, m), 4.00 (2H, t, J = 6.8 Hz), 6.70 - 6.76 (3H, m), 7.03 - 7.33 (6H, m), 7.95 (2H, dd, J = 8.6 Hz, 5.5 Hz)

### Inventive Example 161

### 4-Acetylamino-N-{2-[4-(4-fluorobenzoyl)piperidino]ethyl}-N-(3,4-methylenedioxyphenyl)benzamide

Using 4-amino-N-{2-[4-(4-fluorobenzoyl)piperidino]ethyl}-N-(3,4-methylenedioxyphenyl)benzamide (245.0 mg, 0.501 mmol) and acetic anhydride (0.057 ml, 0.60 mmol), the procedure of Inventive Example 134 was repeated to obtain 210.3 mg (79.0%) of the title compound in a colorless amorphous form.
IR (KBr): 1671, 1638, 1623, 1599, 1533, 1506, 1485, 1433, 1407, 1377, 1314, 1299, 1260, 1236, 1215, 1173, 1155, 1134, 1035 cm⁻¹
NMR (CDCl₃) δ: 1.62 - 1.97 (4H, m), 2.12 (3H, s), 2.00 - 2.40 (2H, m), 2.64 (2H, t, J = 6.9 Hz), 2.90 - 3.47 (3H, m), 3.99 (2H, t, J = 6.9 Hz), 5.95 (2H, s), 6.40 - 6.70 (3H, m), 6.93 - 7.43 (6H, m), 7.80 - 8.03 (2H, m)

### Inventive Example 162

### 4-Acetylamino-N-{2-[4-(4-fluorobenzoyl)piperidino]ethyl}-N-(1-naphthyl)benzamide

Using 4-amino-N-{2-[4-(4-fluorobenzoyl)piperidino]ethyl}-N-(1-naphthyl)benzamide (352.5 mg, 0.712 mmol) and acetic anhydride (0.081 ml, 0.86 mmol), the procedure of Inventive Example 134 was repeated to obtain 334.2 mg (87.4%) of the title compound in a light yellow amorphous form.
IR (KBr): 2944, 1680, 1623, 1596, 1530, 1443, 1407, 1374, 1338, 1311, 1260, 1227, 1206, 1182, 1155, 1140, 1107, 975, 849, 774 cm⁻¹
NMR (CDCl₃) δ: 1.63 - 1.88 (4H, m), 2.05 (3H, s), 1.93 - 2.31 (2H, m), 2.64 (2H, t, J = 6.8 Hz), 2.78 - 3.33 (3H, m), 3.45 - 3.87 (1H, m), 4.35 - 4.72 (1H, m), 6.90 - 8.10 (15H, m)

### Inventive Example 163

### 4-Acetylamino-N-{3-[4-(4-fluorobenzoyl)piperidino]propyl}-N-(3-methoxyphenyl)benzamide

Using 4-amino-N-{3-[4-(4-fluorobenzoyl)piperidino]propyl}-N-(3-methoxyphenyl)benzamide (300.0 mg, 0.61 mmol) and acetic anhydride (0.069 ml, 0.73 mmol), the procedure of Inventive Example 134 was repeated to obtain 312.6 mg (96.5%) of the title compound in a light yellow amorphous form.
IR (KBr): 1680, 1628, 1600, 1530, 1490, 1408, 1374, 1314, 1282, 1262, 1234, 1156, 910, 760 cm⁻¹
NMR (CDCl₃) δ: 1.60 - 2.25 (7H, m), 2.10 (3H, s), 2.44 (2H, t, J = 6.8 Hz), 2.75 - 3.30 (4H, m), 3.69 (3H, s), 3.92 (2H, t, J = 6.8 Hz), 6.46 - 6.75 (3H, m), 7.12 (2H, dd, J = 8.4 Hz), 7.15 - 7.40 (4H, m), 7.40 - 7.56 (1H, m), 7.95 (2H, d, J = 8.4 Hz, 4.3 Hz)

### Inventive Example 164

### 4-Isobutylamino-N-{2-[4-(4-fluorobenzoyl)piperidino]ethyl}-N-(2-methoxyphenyl)benzamide

Using 4-amino-N-{2-[4-(4-fluorobenzoyl)piperidino]ethyl}-N-(2-methoxyphenyl)benzamide (200.0 mg, 0.42 mmol) and isobutylyl chloride (0.053 ml, 0.51 mmol), the procedure of Inventive Example 134 was repeated to obtain 222.7 mg (97.0%) of the title compound in a colorless amorphous form.
IR (KBr): 1680, 1630, 1598, 1528, 1502, 1408, 1306, 1278, 1242, 848, 752 cm⁻¹
NMR (CDCl₃) δ: 1.20 (6H, d, J = 6.8 Hz), 1.58 - 2.78 (9H, m), 2.80 - 3.30 (3H, m), 3.55 - 3.90 (1H, m), 3.69 (3H, s), 3.90 - 4.30 (1H, m), 6.60 - 7.40 (10H, m), 7.78 - 8.07 (2H, m)

### Inventive Example 165

### 4-Butylamino-N-{2-[4-(4-fluorobenzoyl)piperidino]ethyl}-N-(2-methoxyphenyl)benzamide

Using 4-amino-N-{2-[4-(4-fluorobenzoyl)piperidino]ethyl}-N-(2-methoxyphenyl)benzamide (200.0 mg, 0.42 mmol) and butylyl chloride (0.053 ml, 0.51 mmol), the procedure of Inventive Example 134 was repeated to obtain 196.3 mg (85.5%) of the title compound in a colorless amorphous form.
IR (KBr): 2956, 1680, 1632, 1599, 1527, 1503, 1443, 1410, 1308, 1278, 1239, 1179, 1158, 1140, 1116, 1023, 975, 849 cm⁻¹
NMR (CDCl₃) δ: 0.93 (3H, t, J = 7.0 Hz), 1.50 - 2.00 (6H, m), 2.00 - 2.40 (4H, m), 2.47 - 2.75 (2H, m), 2.80 - 3.30 (3H, m), 3.60 - 3.93 (1H, m), 3.68 (3H, s), 3.97 - 4.30 (1H, m), 6.63 - 7.30 (10H, m), 7.80 - 8.03 (2H, m)

### Inventive Example 166

### 4-Butylamino-N-{2-[4-(4-fluorobenzoyl)piperidino]ethyl}-N-(3-methoxyphenyl)benzamide

Using 4-amino-N-{2-[4-(4-fluorobenzoyl)piperidino]ethyl}-N-(3-methoxyphenyl)benzamide (238.0 mg, 0.50 mmol) and butylyl chloride (0.10 ml, 0.10 mmol), the procedure of Inventive Example 134 was repeated to obtain 145.0 mg (53.2%) of the title compound in a colorless amorphous form.
IR (KBr): 2980, 1680, 1600, 1200, 970, 848, 699 cm⁻¹
NMR (CDCl₃) δ: 0.87 (3H, t, J = 7.2 Hz), 1.47 - 2.16 (8H, m), 2.41 (2H, t, J = 7.2 Hz), 2.63 (2H, t, J = 6.7 Hz), 2.95 - 3.26 (3H, m), 3.69 (3H, s), 4.06 (2H, t, J = 6.7 Hz), 6.64 - 6.72 (3H, m), 6.87 - 7.44 (7H, m), 7.91 (2H, dd, J = 8.8 Hz, 5.4 Hz)

### Inventive Example 167

### 4-Propionylamino-N-{2-[4-(4-fluorobenzoyl)piperidino]ethyl}-N-(3-methoxyphenyl)benzamide

Using 4-amino-N-{2-[4-(4-fluorobenzoyl)piperidino]ethyl}-N-(3-methoxyphenyl)benzamide (238.0 mg, 0.50 mmol) and propionyl chloride (0.077 ml, 0.60 mmol), the procedure of Inventive Example 134 was repeated to obtain 240.0 mg (90.4%) of the title compound in a yellow amorphous form.
IR (KBr): 3320, 2940, 1680, 1600, 1306, 971, 850, 699 cm⁻¹
NMR (CDCl₃) δ: 1.20 (3H, t, J = 7.5 Hz), 1.78 - 2.34 (8H, m), 2.38 (2H, t, J = 7.5 Hz), 2.63 (2H, t, J = 6.8 Hz), 2.95 - 3.26 (3H, m), 3.69 (3H, s), 4.03 (2H, t, J = 6.8 Hz), 6.63 - 6.71 (3H, m), 7.02 - 7.29 (7H, m), 7.95 (2H, dd, J = 8.8 Hz, 5.4 Hz)

### Inventive Example 168

### 4-Valerylamino-N-{2-[4-(4-fluorobenzoyl)piperidino]ethyl}-N-(3-methoxyphenyl)benzamide

Using 4-amino-N-{2-[4-(4-fluorobenzoyl)piperidino]ethyl}-N-(3-methoxyphenyl)benzamide (238.0 mg, 0.50 mmol) and valeryl chloride (0.071 ml, 0.60 mmol), the procedure of Inventive Example 134 was repeated to obtain 232.0 mg (83.0%) of the title compound in a yellow amorphous form.
IR (KBr): 3305, 2596, 1680, 1600, 1308, 972, 850, 799 cm⁻¹
NMR (CDCl₃) δ: 0.92 (3H, t, J = 6.4 Hz), 1.32 - 2.49 (10H, m), 2.63 (2H, t, J = 6.8 Hz), 2.96 - 3.28 (3H, m), 3.70 (3H, s), 4.04 (2H, t, J = 6.8 Hz), 6.63 - 6.71 (3H, m), 7.02 - 7.29 (7H, m), 7.96 (2H, dd, J = 8.8 Hz, 5.4 Hz)

### Inventive Example 169

### 4-Pivaloylamino-N-{2-[4-(4-fluorobenzoyl)piperidino]ethyl}-N-(2-methoxyphenyl)benzamide

Using 4-amino-N-{2-[4-(4-fluorobenzoyl)piperidino]ethyl}-N-(2-methoxyphenyl)benzamide (200.0 mg, 0.42 mmol) and pivaloyl chloride (0.062 ml, 0.51 mmol), the procedure of Inventive Example 134 was repeated to obtain 214.6 mg (91.2%) of the title compound in a colorless amorphous form.
IR (KBr): 2956, 1680, 1630, 1599, 1503, 1461, 1443, 1404, 1314, 1278, 1242, 1176, 1158, 1140, 1116, 1044, 1026, 975, 915, 852 cm⁻¹
NMR (CDCl₃) δ: 1.26 (9H, s), 1.50 - 1.93 (4H, m), 2.00 - 2.37 (2H, m), 2.65 (2H, t, J = 7.0 Hz), 2.80 - 3.38 (3H, m), 3.53 - 3.93 (1H, m), 3.69 (3H, s), 3.93 - 4.30 (1H, m), 6.67 - 6.92 (2H, m), 6.97 - 7.33 (6H, m), 7.80 - 8.07 (2H, m)

### Inventive Example 170

### 4-(Trifluoroacetylamino-N-{2-[4-(4-fluorobenzoyl)piperidino]ethyl}-N-(2-methoxyphenyl)benzamide

Using 4-aminomethyl-N-{2-[4-(4-fluorobenzoyl)piperidino]ethyl}-N-(2-methoxyphenyl)benzamide (60 mg, 0.123 mmol) and trifluoroacetic anhydride (19 µl, 0.135 mmol), the procedure of Inventive Example 134 was repeated to obtain 48 mg (67.0%) of the title compound in a colorless amorphous form.
IR (KBr): 3406, 3064, 2944, 1719, 1680, 1641, 1599, 1503, 1221, 1179, 1158 cm⁻¹
NMR (CDCl₃) δ: 1.46 - 1.98 (4H, m), 1.98 - 2.35 (2H, m), 2.62 (2H, t, J = 7.5 Hz), 2.79 - 3.36 (3H, m), 3.50 - 3.95 (1H, m), 3.69 (3H, s), 4.00 - 4.54 (1H, m), 4.40 (2H, d, J = 6 Hz), 6.61 - 6.83 (2H, m), 6.90 (1H, d, J = 7.5 Hz), 6.96 - 7.40 (8H, m), 7.93 (2H, dd, J = 9 Hz, 6 Hz)

### Inventive Example 171

### 4-Trifluoroacetylamino-N-{2-[4-(4-fluorobenzoyl)piperidino]ethyl}-N-(2-methoxyphenyl)benzamide

Using 4-amino-N-{2-[4-(4-fluorobenzoyl)piperidino]ethyl}-N-(2-methoxyphenyl)benzamide (200.0 mg, 0.42 mmol) and trifluoroacetic anhydride (0.18 ml, 1.26 mmol), the procedure of Inventive Example 134 was repeated to obtain 253.3 mg (quantitative) of the title compound in a colorless amorphous form.
IR (KBr): 2948, 1720, 1678, 1628, 1544, 1500, 1440, 1284, 1200, 1158, 1026, 976 cm⁻¹
NMR (CDCl₃) δ: 1.53 - 2.30 (5H, m), 2.57 (2H, t, J = 6.0 Hz), 3.46 - 3 85 (2H, m), 3.65 (3H, s), 3.90 - 4.30 (2H, m), 6.50 - 6.89 (1H, m), 6.76 (2H, t, J = 9.0 Hz), 6.90 - 7.40 (7H, m), 7.92 (2H, dd, J = 9.0 Hz, 6.0 Hz), 8.43 - 8.85 (1H, m)

### Inventive Example 172

### N-{2-[4-(4-fluorobenzoyl)piperidino]ethyl}-4-(methanesulfonylamino)methyl-N-(2-methoxyphenyl)benzamide

Using 4-aminomethyl-N-{2-[4-(4-fluorobenzoyl)piperidino]ethyl}-N-(2-methoxyphenyl)benzamide (60 mg, 0.123 mmol) and methanesulfonyl chloride (11 µl, 0.142 mmol), the procedure of Inventive Example 134 was repeated to obtain 40 mg (57.5%) of the title compound in a colorless amorphous form.
IR (KBr): 2926, 1677, 1638, 1596, 1503, 1410, 1323, 1278, 1146 cm⁻¹
NMR (CDCl₃) δ: 1.50 - 2.00 (4H, m), 2.00 - 2.37 (2H, m), 2.46 - 2.78 (2H, m), 2.70 (3H, s), 2.81 - 3.29 (3H, m), 3.40 - 3.88 (1H, m), 3.70 (3H, s), 4.00 - 4.41 (1H, m), 4.18 (2H, d, J = 6 Hz), 4.69 (1H, br-s), 6.71 (1H, d, J = 8 Hz), 6.80 (1H, d, J = 8 Hz), 6.90 - 7.35 (8H, m), 7.93 (2H, dd, J = 9 Hz, 6 Hz)

### Inventive Example 173

### N-{2-[4-(4-fluorobenzoyl)piperidino]ethyl}-4-methanesulfonylamino-N-(2-methoxyphenyl)benzamide

Using 4-amino-N-{2-[4-(4-fluorobenzoyl)piperidino]ethyl}-N-(2-methoxyphenyl)benzamide (50.0 mg, 0.11 mmol) and methanesulfonyl chloride (0.025 ml, 0.316 mmol), the procedure of Inventive Example 134 was repeated to obtain 29.5 mg (50.7%) of the title compound in a colorless amorphous form.
IR (KBr): 3310, 2944, 1680, 1629, 1599, 1503, 1464, 1395, 1335, 1278, 1236, 1155, 1116, 972 cm⁻¹
NMR (CDCl₃) δ: 1.50 - 1.93 (4H, m), 2.00 - 2.40 (2H, m), 2.64 (2H, t, J = 7.0 Hz), 2.90 (3H, s), 2.78 - 3.20 (3H, m), 3.50 - 3.90 (1H, m), 3.70 (3H, s), 3.95 - 4.37 (1H, m), 6.77 (2H, t, J = 9.0 Hz), 6.93 - 7.40 (8H, m), 7.94 (2H, dd, J = 9.0 Hz, 6.0 Hz)

### Inventive Example 174

### N-{2-[4-(4-fluorobenzoyl)piperidino]ethyl}-4-methanesulfomylamino-N-(3-methoxyphenyl)benzamide

Using 4-amino-N-{2-[4-(4-fluorobenzoyl)piperidino]ethyl}-N-(3-methoxyphenyl)benzamide (475.0mg, 1.00 mmol) and methanesulfonyl chloride (0.12 ml, 1.50 mmol), the procedure of Inventive Example 134 was repeated to obtain 527.8 mg (95.4%) of the title compound in a colorless amorphous form.
IR (KBr): 1678, 1640, 1598, 1508, 1490, 1454, 1390, 1332, 1284, 1218, 1154, 970 cm⁻¹
NMR (CDCl₃) δ: 1.50 - 2.73 (7H, m), 2.93 (3H, s), 2.73 - 3.55 (4H, m), 3.69 (3H, s), 4.20 - 4.50 (2H, m), 6.66 (2H, brd, J = 7.5 Hz), 6.85 - 7.40 (5H, m), 7.94 (2H, dd, J = 9.0 Hz, 6.0 Hz)

### Inventive Example 175

### 4-(Ethoxycarbonylamino)-N-{2-[4-(4-fluorobenzoyl)piperidino]ethyl}-N-(2-methoxyphenyl)benzamide

Using 4-aminomethyl-N-{2-[4-(4-fluorobenzoyl)piperidino]ethyl}-N-(2-methoxyphenyl)benzamide (60 mg, 0.123 mmol) and ethyl chloroformate (13 µl, 0.136 mmol), the procedure of Inventive Example 134 was repeated to obtain 50 mg (72.7%) of the title compound in a colorless amorphous form.
IR (KBr): 3340, 2938, 2806, 1716, 1680, 1641, 1599, 1503, 1413, 1239, 1137 cm⁻¹
NMR (CDCl₃) δ: 1.22 (3H, t, J = 7.5 Hz), 1.69 - 1.90 (4H, m), 2.10 - 2.34 (2H, m), 2.60 (2H, t, J = 7 Hz), 2.80 - 3.27 (3H, m), 3.65 - 3.85 (1H, m), 3.67 (3H, s), 4.00 - 4.30 (1H, m), 4.11 (2H, q, J = 7.5 Hz), 4.24 (2H, d, J = 6 Hz), 4.88 (1H, br-s), 6.73 (1H, d, J = 8 Hz), 6.80 - 7.30 (9H, m), 7.93 (2H, dd, J = 9 Hz, 6 Hz)

### Inventive Example 176

### 4-Ethoxycarbonylamino-N-{2-[4-(4-fluorobenzoyl)piperidino]ethyl}-N-(2-methoxyphenyl)benzamide

Using 4-amino-N-{2-[4-(4-fluorobenzoyl)piperidino]ethyl}-N-(2-methoxyphenyl)benzamide (185.0 mg, 0.39 mmol) and ethyl chloroformate (0.11 ml, 1.17 mmol), the procedure of Inventive Example 134 was repeated to obtain 80.0 mg (37.5%) of the title compound in a colorless oily form.
IR (neat): 3288, 2948, 1720, 1680, 1630, 1528, 1440, 1316, 1120, 1026, 976 cm⁻¹
NMR (CDCl₃) δ: 1.36 (3H, t, J = 7.2 Hz), 1.46 - 2.35 (5H, m), 2.63 (2H, t, J = 6.0 Hz), 2.76 - 3.30 (4H, m), 3.66 (3H, s), 4.10 (2H, t, J = 6.0 Hz), 4.15 (2H, q, J = 7.2 Hz), 6.53 - 6.73 (1H, m), 6.85 (2H, t, J = 8.4 Hz), 6.90 - 7.33 (8H, m), 7.95 (2H, dd, J = 9.0 Hz, 6.0 Hz)

### Inventive Example 177

### 4-[(S)-2-(tert-Butoxycarbonylamino)propionyl]aminomethyl-N-{2-[4-(4-fluorobenzoyl)piperidino]ethyl}-N-(2-methoxyethyl)benzamide

4-Aminomethyl-N-{2-[4-(4-fluorobenzoyl)piperidino]ethyl}-N-(2-methoxyethyl)benzamide (120 mg, 0.245 mmol) was dissolved in methylene chloride (5 ml) to which were subsequently added Boc-L-alanine (46.4 mg, 0.245 mmol) and EDC hydrochloride (52 mg, 0.270 mmol) while cooling in an ice bath. After 4 hours of stirring at the same temperature, the reaction solution was mixed with water (10 ml), adjusted to pH 8 with saturated sodium bicarbonate aqueous solution and then extracted with methylene chloride. Thereafter, the resulting organic layer was dried on anhydrous sodium carbonate, the solvent was removed by evaporation, and the resulting residue was purified by a silica gel column chromatography (ethyl acetate) to obtain 109 mg (68.9%) of the title compound in a colorless amorphous form.
IR (KBr): 3328, 2938, 1713, 1677, 1641, 1599, 1503, 1392, 1242, 1161 cm⁻¹
NMR (CDCl₃) δ: 1.33 (3H, d, J = 7 Hz), 1.40 (9H, s), 1.66 - 1.90 (4H, m), 2.00 - 2.32 (2H, m), 2.62 (2H, t, J = 8 Hz), 2.80 - 3.26 (3H, m), 3.60 - 3.80 (1H, m), 3.69 (3H, s), 4.00 - 4.25 (2H, s), 4.33 (2H, d, J = 6 Hz), 4.88 (1H, br-s), 6.40 (1H, br-s), 6.73 (1H, d, J = 8 Hz), 6.89 (1H, d, J = 8 Hz), 6.96 - 7.30 (8H, m), 7.94 (2H, dd, J = 9 Hz, 6 Hz)

### Inventive Example 178

### N-{2-[4-(4-Fluorobenzoyl)piperidino]ethyl}-N-(2-methoxyethyl)-4-(methylamino)methylbenzamide

4-(N-tert-Butoxycarbonyl-N-methylamino)methyl-N-{2-[4-(4-fluorobenzoyl)piperidino]ethyl}-N-(2-methoxyphenyl)benzamide (144 mg, 0.239 mmol) was dissolved in methylene chloride (4 ml) to which was subsequently added dropwise trifluoroacetic acid (0.4 ml) while cooling in an ice bath. After 2 hours of stirring at the same temperature, the solvent was removed by evaporation, and the resulting residue was diluted with methylene chloride (10 ml), washed with saturated sodium bicarbonate aqueous solution and saturated brine, and then dried on anhydrous sodium carbonate. Thereafter, the solvent was removed by evaporation to obtain 119 mg (98.9%) of the title compound in a colorless amorphous form.
IR (KBr): 2944, 2794, 1680, 1641, 1596, 1503, 1278, 1236, 750 cm⁻¹
NMR (CDCl₃) δ: 1.62 - 1,97 (5H, m), 2.00 - 2.30 (2H, m), 2.36 (3H, s), 2.63 (2H, t, J = 7.5 Hz), 2.80 - 3.26 (3H, m), 3.64 (2H, s), 3.68 (3H, s), 3.60 - 3.85 (1H, m), 4.00 - 4.26 (1H, m), 6.73 (1H, d, J = 8 Hz), 6.82 (1H, d, J = 8 Hz), 7.00 - 7.31 (8H, m), 7.94 (2H, dd, J = 9 Hz, 6 Hz)

### Inventive Example 179

### 4-[(S)-2-(tert-Aminopropionyl]aminomethyl-N-{2-[4-(4-fluorobenzoyl)piperidino]ethyl}-N-(2-methoxyethyl)benzamide

4-[(S)-2-tert-Butoxycarbonylamino)propionyl]aminomethyl-N-{2-[4-(4-fluorobenzoyl)piperidino]ethyl}-N-(2-methoxyethyl)benzamide (100 mg, 0.156 mmol) was treated in the same manner as described in Inventive Example 178 to obtain 84.7 mg (100%) of the title compound in a colorless amorphous form.
IR (KBr): 3370, 2926, 1677, 1641, 1599, 1503, 1413, 1278, 1239, 1158, 1122 cm⁻¹
NMR (CDCl₃) δ: 1.10 - 1.45 (3H, m), 1.70 - 2.58 (8H, m), 2.76 (2H, t, J = 7 Hz), 2.92 - 3.35 (4H, m), 3.68 (3H, s), 3.65 - 3.92 (1H, m), 4.05 - 4.40 (3H, m), 6.51 - 6.70 (1H, m), 6.73 (1H, d, J = 8 Hz), 6.82 - 7.30 (9H, m), 7.93 (2H, dd, J = 9 Hz, 6 Hz)

### Inventive Example 180

### 4-Amidino-N-{2-[4-(4-fluorobenzoyl)piperidino]ethyl}-N-(2-methoxyphenyl)benzamide

In an atmosphere of dry air, 4-cyano-N-{2-[4-(4-fluorobenzoyl)piperidino]ethyl}-N-(2-methoxyphenyl)benzamide (100 mg, 0.206 mmol) was dissolved in ethanol (2 ml) to which was subsequently introduced hydrogen chloride gas at -10°C for 15 minutes. After 5.5 hours of stirring at the same temperature and additional stirring at room temperature for 1 hour, ethanol was removed by evaporation, and 10% ammonia-methanol solution (1 ml) was added to the resulting residue at room temperature. After 16 hours of stirring at the same temperature, the solvent was removed by evaporation, and the resulting residue was purified by a silica gel column chromatography (ether:methanol = 10:1). Thereafter, a methylene chloride-methanol (10:1) mixture solution (5 ml) was added to the thus purified product, insoluble materials were removed by filtration and then the solvent was removed by evaporation, thereby obtain 92 mg (88.9%) of the title compound in a colorless amorphous form.
IR (KBr): 3250, 3064, 1678, 1642, 1598, 1502, 1440, 1410, 1280, 1224, 1158, 854 cm⁻¹
NMR (CDCl₃-CD₃OD) δ: 1.80 - 2.40 (4H, m), 2.40 - 4.10 (8H, m), 3.74 (3H, s), 4.13 - 4.59 (1H, m), 6.60 - 6.93 (2H, m), 6.98 - 7.37 (2H, m), 7.16 (2H, dd, J = 9 Hz, 9 Hz), 7.43 (2H, d, J = 8 Hz), 7.58 (2H, d, J = 8 Hz), 7.99 (2H, dd, J = 9 Hz, 6 Hz) MS (FAB, m/z): 120, 157, 220, 234, 296, 357, 503 (M⁺ + H)

### Inventive Example 181

### 4-Amidino-N-{2-[4-(4-fluorobenzoyl)piperidino]ethyl}-N-(4-methoxyphenyl)benzamide

Using 4-cyano-N-{2-[4-(4-fluorobenzoyl)piperidino]ethyl}-N-(4-methoxyphenyl)benzamide (728.0 mg, 1.50 mmol), the procedure of Inventive Example 180 was repeated to obtain 635.0 mg (84.3%) of the title compound in a colorless powder form.
IR (KBr): 3270, 3075, 1678, 1626, 1598, 1510, 1246, 971, 853, 730, 600 cm⁻¹
NMR (CDCl₃) δ: 1.82 - 1.91 (4H, m), 2.12 - 2.36 (2H, m), 2.62 (2H, t, J = 6.4 Hz), 2.97 - 3.41 (6H, m), 3.72 (3H, s), 4.03 (2H, t, J = 6.4 Hz), 6.74 (2H, d, J = 8.8 Hz), 7.08 (2H, d, J = 8.8 Hz), 7.05 - 7.32 (2H, m), 7.40 (2H, d, J = 8.4 Hz), 7.57 (2H, d, J = 8.4 Hz), 7.96 (2H, dd, J = 8.8 Hz, 5.5 Hz)

### Inventive Example 182

### 4-(N-acetylamidino)-N-{2-[4-(4-fluorobenzoyl)piperidino]ethyl}-N-(2-methoxyphenyl)benzamide

Using 4-amidino-N-{2-[4-(4-fluorobenzoyl)piperidino]ethyl}-N-(2-methoxyphenyl)benzamide (100 mg, 0.20 mmol), the procedure of Inventive Example 134 was repeated to obtain 81 mg (74.4%) of the title compound in a light yellow amorphous form.
IR (KBr): 3264, 2944, 1730, 1680, 1642, 1596, 1502, 1408, 1298, 1262 cm⁻¹
NMR (CDCl₃) δ: 1.56 - 1.98 (4H, m), 1.98 - 2.45 (2H, m), 2.16 (3H, s), 2.46 - 2.80 (2H, m), 2.80 - 3.36 (3H, m), 3.54 - 3.93 (1H, m), 3.70 (3H, s), 4.00 - 4.40 (1H, m), 6.62 - 6.97 (3H, m), 7.00 - 7.75 (9H, m), 8.01 (2H, dd, J = 9 Hz, 6 Hz)

### Inventive Example 183

### N-{2-[4-(4-fluorobenzoyl)piperidino]ethyl}-4-(N-methanesulfonylamidino)-N-(2-methoxyphenyl)benzamide

Using 4-amidino-N-{2-[4-(4-fluorobenzoyl)piperidino]ethyl}-N-(2-methoxyphenyl)benzamide (120 mg, 0.239 mmol), the procedure of Inventive Example 134 was repeated to obtain 81 mg (73.6%) of the title compound in a colorless amorphous form.
IR (KBr): 3406, 2944, 1680, 1641, 1596, 1533, 1503, 1410, 1278, 1155, 1113, 1020, 972, 852, 750, 603, 528 cm⁻¹
NMR (CDCl₃) δ: 1.46 - 2.00 (4H, m), 2.02 - 2.38 (2H, m), 2.46 - 2.76 (2H, m), 2.80 - 3.37 (3H, m), 3.04 (3H, s), 3.50 - 3.90 (1H, m), 3.70 (3H, s), 4.00 - 4.43 (1H, m), 6.50 - 6.91 (3H, m), 6.96 - 7.38 (7H, m), 7.55 (2H, d, J = 8 Hz), 7.95 (2H, dd, J = 9 Hz, 6 Hz)

### Inventive Example 184

### 4-(N-Ethoxycarbonylamidino)-N-{2-[4-(4-fluorobenzoyl)piperidino]ethyl}-N-(2-methoxyphenyl)benzamide

Using 4-amidino-N-{2-[4-(4-fluorobenzoyl)piperidino]ethyl}-N-(2-methoxyphenyl)benzamide (120 mg, 0.239 mmol), the procedure of Inventive Example 134 was repeated to obtain 108 mg (78.7%) of the title compound in a colorless amorphous form.
IR (KBr): 3376, 2944, 1680, 1620, 1596, 1503, 1299, 1263, 1134 cm⁻¹
NMR (CDCl₃) δ: 1.32 (3H, t, J = 7 Hz), 1.50 - 1.97 (4H, m), 2.02 - 2.38 (2H, m), 2.47 - 2.66 (2H, m), 2.80 - 3.38 (3H, m), 3.50 - 3.90 (1H, m), 3.67 (3H, s), 4.00 - 4.40 (1H, m), 4.18 (2H, q, J = 7 Hz), 6.50 - 6.90 (3H, m), 6.93 - 7.42 (7H, m), 7.63 (2H, d, J = 8 Hz), 7.94 (2H, dd, J = 9 Hz, 6 Hz)

### Inventive Example 185

### 3-[4-(4-Fluorobenzoyl)piperidino]-N-(4-methoxybenzenesulfonyl)propionamidine

In an atmosphere of argon, 3-[4-(4-fluorobenzoyl)piperidino]propionamidine (82 mg, 0.296 mmol) was suspended in THF (3 ml), and tert-butoxy potassium (38 mg, 0.328 mmol) dissolved in THF (3 ml) was added dropwise to the suspension at room temperature. After 45 minutes of stirring at the same temperature, 4-methoxybenzenesulfonyl chloride (61.6 mg, 0.296 mmol) was added and the stirring was continued for 24 hours. After removing THF by evaporation, the resulting residue was mixed with water (10 ml), extracted with ethyl acetate, washed with saturated brine and then dried on anhydrous sodium carbonate, subsequently removing the solvent by evaporation. Thereafter, the resulting residue was subjected to a silica gel column chromatography (ethyl acetate-methylene chloride:methanol = 20:1) and then mixed with ethyl acetate (5 ml) to remove insoluble materials by filtration, thereby obtaining 51 mg (38.5%) of the title compound in a light yellow oily form.
IR (neat): 3412, 2948, 1680, 1634, 1596, 1554, 1500, 1308, 1260, 1156, 1138, 1086, 732, 596, 566 cm⁻¹
NMR (CDCl₃) δ: 1.50 - 2.35 (6H, m), 2.35 - 2.82 (4H, m), 2.83 - 3.45 (3H, m), 3.83 (3H, s), 6.93 (2H, d, J = 9 Hz), 7.14 (2H, dd, J = 9 Hz, 9 Hz), 7.63 - 8.15 (5H, m), 9.12 (1H, br-s)

### Inventive Example 186

### 3-[4-(4-Fluorophenyl)piperazinyl]-N-(4-methoxybenzenesulfonyl)propionamidine

Using 3-[4-(4-fluorophenyl)piperazinyl]propionamidine (100 mg, 0.40 mmol), the procedure of Inventive Example 185 was repeated to obtain 83 mg (49.3%) of the title compound in a colorless powder form.
Melting point: 153.5 - 155°C
IR (KBr): 3356, 1592, 1570, 1512, 1284, 1252, 1140, 1122, 1088, 766 cm⁻¹
NMR (CDCl₃) δ: 2.26 - 2.90 (8H, m), 2.97 - 3.34 (4H, m), 3.84 (3H, dd, s), 6.71 - 7.20 (6H, m), 7.75 (1H, br-s), 7.88 (2H, d, J = 9 Hz), 9.02 (1H, br-s)

### Inventive Example 187

### 3-[4-(4-Fluorobenzoyl)piperidino]-N-(3-methoxybenzyl)propionamidine

In an atmosphere of argon, crude ethyl 3-[4-(4-fluorobenzoyl)piperidino]propionimidate dihydrochloride (202 mg, 0.534 mmol) was dissolved in methanol (0.5 ml) to which was subsequently added 3-methoxybenzylamine (0.1 ml, 0.763 mmol) at room temperature. After 14 hours of stirring at the same temperature, the reaction solution was mixed with saturated sodium bicarbonate aqueous solution (1 ml) and methylene chloride (10 ml), stirred and then dried on anhydrous sodium carbonate. After removing the solvent by evaporation, the resulting residue was purified by a silica gel column chromatography (chloroform:methanol = 9:1 - chloroform:10% ammonia/methanol solution = 9:1) to obtain 115 mg (54.2%) of the title compound in a light yellow oily form.
IR (neat): 2948, 1680, 1598, 1490, 1454, 1262, 1234, 1202, 1156, 736, 606 cm⁻¹
NMR (CDCl₃) δ: 1.63 - 2.29 (6H, m), 2.30 - 2.75 (4H, m), 2.80 - 3.40 (3H, m), 3.89 (3H, s), 4.30 (2H, s), 4.95 (2H, br-s), 6.60 - 7.35 (6H, m), 7.93 (2H, dd, J = 9 Hz, 6 Hz)

### Inventive Example 188

### 3-[4-(4-Fluorophenyl)piperazinyl]-N-(3-methoxybenzyl)propionamidine

Using crude ethyl 3-[4-(4-fluorophenyl)piperazinyl]propionimidate trihydrochloride (76 mg, 0.20 mmol), the procedure of Inventive Example 187 was repeated to obtain 36 mg (48.6%) of the title compound in a colorless oily form.
IR (neat): 2828, 1610, 1510, 1490, 1454, 1262, 1234, 1150, 1048, 824, 778 cm⁻¹
NMR (CDCl₃) δ: 2.30 - 2.80 (8H, m), 2.82 - 3.22 (4H, m), 3.74 (3H, s), 4.32 (2H, s), 5.74 (2H, br-s), 6.60 - 7.38 (8H, m)

### Inventive Example 189

### 4-(Ethylthioureido)-N-{2-[4-(4-fluorobenzoyl)piperidino]ethyl}-N-(2-methoxyphenyl)benzamide

4-Amino-N-{2-[4-(4-fluorobenzoyl)piperidino]ethyl)-N-(2-methoxyphenyl)benzamide (80.0 mg, 0.168 mmol) was dissolved in ethanol (2.0 ml), and the solution was mixed with ethylthio isocyanate (0.035 ml, 0.404 mmol) at room temperature and then heated under reflux for 14 hours. After cooling, the solvent was distilled off under a reduced pressure. Thereafter, the resulting residue was purified by a silica gel column chromatography (chloroform:methanol = 20:1) to obtain 95.5 mg (quantitative) of the title compound in a colorless oily form.
IR (neat): 2944, 1680, 1629, 1599, 1536, 1503, 1443, 1410, 1314, 1278, 1260, 1239, 1158, 1140, 1116, 975, 912, 854, 729, 645 cm⁻¹
NMR (CDCl₃) δ: 1.14 (3H, t, J = 7.3 Hz), 1.50 - 1.88 (4H, m), 1.96 - 2.30 (2H, m), 2.40 - 2.70 (2H, m), 2.73 - 3.27 (3H, m), 3.40 - 3.90 (3H, m), 3.72 (3H, s), 3.90 - 4.40 (1H, m), 6.60 - 7.38 (10H, m), 7.70 - 8.03 (2H, m)

### Inventive Example 190

### 4-(tert-Butylureido)-N-{2-[4-(4-fluorobenzoyl)piperidino]ethyl}-N-(2-methoxyphenyl)benzamide

Using 4-amino-N-{2-[4-(4-fluorobenzoyl)piperidino]ethyl}-N-(2-methoxyphenyl)benzamide (77.7 mg, 0.164 mmol) and tert-butyl isocyanate (0.022 ml, 0.196 mmol), the procedure of Inventive Example 189 was repeated to obtain 72.9 mg (77.6%) of the title compound in a colorless oily form.
IR (neat): 3370, 2956, 1680, 1617, 1599, 1536, 1503, 1452, 1410, 1392, 1314, 1278, 1251, 1206, 1179, 1158, 909, 729, 645, 603 cm⁻¹
NMR (CDCl₃) δ: 1.33 (9H, s), 1.58 - 1.94 (4H, m), 1.98 - 2.32 (2H, m), 2.49 - 2.75 (2H, m), 2.80 - 3.30 (3H, m), 3.50 - 3.92 (1H, m), 3.68 (3H, s), 3.92 - 4.30 (1H, m), 6.60 - 7.30 (10H, m), 7.80 - 8.07 (2H, m)

### Inventive Example 191

### 4-(Ureido)-N-{2-[4-(4-fluorobenzoyl)piperidino]ethyl}-N-(3-methoxyphenyl)benzamide

4-Amino-N-{2-[4-(4-fluorobenzoyl)piperidino]ethyl}-N-(3-methoxyphenyl)benzamide (237.2 mg, 0.50 mmol) was dissolved in acetic acid-water (1:2, 1.8 ml) to which was subsequently added dropwise an aqueous solution (0.5 ml) of potassium cyanate (81.1 mg, 0.90 mmol) . After 3 hours of stirring at room temperature, the reaction solution was mixed with saturated sodium bicarbonate aqueous solution , alkanized with 10% sodium hydroxide aqueous solution and then extracted with ethyl acetate. The resulting organic layer was washed with water and saturated brine, and dried on anhydrous sodium sulfate. Thereafter, the solvent was removed by evaporation, and the resulting residue was purified by a silica gel column chromatography (chloroform:methanol = 10:1) and recrystallized from ethyl acetate-ether to obtain 125.6 mg (48.4%) of the title compound in a colorless powder form.
Melting point: 123 - 128°C
IR (KBr): 3476, 3352, 1680, 1598, 1526, 1490, 1438, 1410, 1396, 1310, 1264, 1238, 1214, 1182, 1158, 1140, 852, 840 cm⁻¹
NMR (CDCl₃) δ: 1.77 - 2.22 (6H, m), 2.60 (2H, t, J = 6.4 Hz), 2.92 - 3.18 (3H, m), 3.69 (3H, s), 4.02 (2H, t, J = 6.4 Hz), 4.80 (2H, brs), 6.64 - 6.71 (3H, m), 6.98 - 7.25 (8H, m), 7.95 (2H, dd, J = 8.6 Hz, 5.5 Hz)

### Inventive Example 192

### 4-(N-Methylacetylamino)-N-{2-[4-(4-fluorobenzoyl)piperidino]ethyl}-N-(4-methoxyphenyl)benzamide

4-Acetylamino-N-{2-[4-(4-fluorobenzoyl)piperidino]ethyl}-N-(4-methoxyphenyl)benzamide (155.0 mg, 0.30 mmol) was dissolved in THF (3.0 ml) to which were subsequently added 60% sodium hydride (28.0 mg, 0.70 mmol) and methyl iodide (0.060 ml, 1.00 mmol) at room temperature, followed by 2 hours of stirring. The reaction solution was mixed with saturated ammonium chloride aqueous solution and extracted with ethyl acetate. The resulting organic layer was washed with water and saturated brine, and dried on anhydrous sodium sulfate. Thereafter, the solvent was removed by evaporation, and the resulting residue was recrystallized from methanol-ether to obtain 128.0 mg (80.4%) of the title compound in a colorless powder form.
IR (KBr): 2931, 1680, 1666, 1636 1598, 1510, 1242, 1029, 960, 601 cm⁻¹
NMR (CDCl₃) δ: 1.78 (3H, s), 1.78 - 1.95 (4H, m), 2.04 - 2.31 (2H, m), 2.61 (2H, t, J = 6.6 Hz), 2.95 - 3.30 (3H, m), 3.18 (3H, s), 3.74 (3H, s), 4.02 (2H, t, J = 6.6 Hz), 6.74 (2H, d, J = 8.8 Hz), 6.93 - 7.36 (8H, m), 7.96 (2H, dd, J = 8.8 Hz, 5.3 Hz)

### Inventive Example 193

### 4-(N-Methylacetylamino)-N-{2-[4-(4-fluorobenzoyl)piperidino]ethyl}-N-(3-methoxyphenyl)benzamide

Using 4-Acetylamino-N-{2-[4-(4-fluorobenzoyl)piperidino]ethyl}-N-(3-methoxyphenyl)benzamide (310.0 mg, 0.60 mmol), the procedure of Inventive Example 192 was repeated to obtain 89.2 mg (28.0%) of the title compound in a colorless amorphous form.
IR (neat): 2940, 1654, 1600, 1376, 1138, 970, 749, 699 cm⁻¹
NMR (CDCl₃) δ: 1.71 - 1.87 (4H, m), 1.78 (3H, s), 2.06 - 2.43 (2H, m), 2.63 (2H, t, J = 6.6 Hz), 2.96 - 3.25 (3H, m), 3.69 (3H, s), 4.06 (2H, t, J = 6.6 Hz), 6.64 - 6.72 (4H, m), 6.63 - 6.78 (4H, m), 6.94 - 7.40 (6H, m), 7.97 (2H, dd, J = 8.7 Hz, 5.6 Hz)

### Inventive Example 194

### 4-(Pyrol-1-yl)-N-{2-[4-(4-fluorobenzoyl)piperidino]ethyl}-N-(4-methoxyphenyl)benzamide

4-Amino-N-{2-[4-(4-fluorobenzoyl)piperidino]ethyl}-N-(4-methoxyphenyl)benzamide (237.0 mg, 0.50 mmol) was dissolved in acetic acid (5.0 ml), and the solution was mixed with 2,5-dimethoxytetrahydrofuran (0.065 ml, 0.50 mmol) and heated for 1 hour under reflux. After cooling, acetic acid was distilled off under a reduced pressure, the residue was dissolved in ethyl acetate, and the organic layer was washed with water and saturated brine, and dried on anhydrous sodium sulfate. Thereafter, the solvent was distilled off under a reduced pressure, and the resulting residue was purified by a silica gel column chromatography (hexane:ethyl acetate = 1:4) to obtain 217.0 mg (82,7%) of the title compound in a colorless amorphous form.
IR (KBr): 2943, 1678, 1638, 1510, 1330, 1246, 815, 724, 602 cm⁻¹
NMR (CDCl₃) δ: 1.80 - 2.33 (6H, m), 2.62 (2H, t, J = 6.8 Hz), 2.96 - 3.33 (3H, m), 3.75 (3H, s), 4.02 (2H, t, J = 6.8 Hz), 6.30 (2H, t, J = 9.0 Hz), 6.72 (2H, dd, J = 6.8, 2.2 Hz), 6.99 - 7.40 (10H, m), 7.96 (2H, dd, J = 8.8 Hz, 5.6 Hz)

### Inventive Example 195

### 4-(Pyrol-1-yl)-N-{2-[4-(4-fluorobenzoyl)piperidino]ethyl}-N-(3-methoxyphenyl)benzamide

Using 4-amino-N-{2-[4-(4-fluorobenzoyl)piperidino]ethyl}-N-(3-methoxyphenyl)benzamide (238.1 mg, 0.50 mmol) and 2,5-dimethoxytetrahydrofuran (0.066 ml, 0.50 mmol), the procedure of Inventive Example 195 was repeated to obtain 205.0 mg (78.0%) of the title compound in a light brown amorphous form.
IR (KBr): 1674, 1638, 1600, 1488, 1400, 1392, 1326, 1310, 1280, 1266, 1228, 1140, 842, 728 cm⁻¹
NMR (CDCl₃) δ: 1.69 - 2.25 (6H, m), 2.64 (2H, t, J = 6.6 Hz), 2.97 - 3.19 (3H, m), 3.70 (3H, s), 4.06 (2H, t, J = 6.6 Hz), 6.31 (2H, s), 6.66 (2H, s), 6.73 (1H, s), 7.03 - 7.43 (9H, m), 7.96 (2H, dd, J = 8.4 Hz, 5.5 Hz)

### Inventive Example 196

### 4-N-Dimethylamino-N-{2-[4-(4-fluorobenzoyl)piperidino]ethyl}-N-(4-methoxyphenyl)benzamide

4-Amino-N-{2-[4-(4-fluorobenzoyl)piperidino]ethyl}-N-(4-methoxyphenyl)benzamide (143.0 mg, 0.30 mmol) was dissolved in methanol (5.0 ml) to which were subsequently added 37% formaldehyde aqueous solution (1.0 ml) and sodium cyanoborohydride (38.0 mg, 0.60 mmol). The resulting mixture was stirred for 24 hours while adjusting the pH to 5 to 6 with trifluoroacetic acid. The reaction solution was mixed with ethyl acetate, and the organic layer was washed with water and saturated brine, and dried on anhydrous sodium sulfate. Thereafter, the solvent was distilled off under a reduced pressure, and the resulting residue was purified by a silica gel column chromatography (ethyl acetate) to obtain 87.0 mg (61.1%) of the title compound in a colorless amorphous form.
IR (KBr): 2944, 1678, 1600, 1510, 1444, 1246, 974, 836, 602 cm⁻¹
NMR (CDCl₃) δ: 1.66 - 2.38 (6H, m), 2.66 (2H, t, J = 6.8 Hz), 2.91 (6H, s), 2.97 - 3.36 (3H, m), 3.77 (3H, s), 4.00 (2H, t, J = 6.8 Hz), 6.42 (2H, d, J = 9.0 Hz), 6.76 (2H, d, J = 9.0 Hz), 7.00 - 7.22 (6H, m), 7.96 (2H, dd, J = 8.8 Hz, 5.5 Hz)

### Inventive Example 197

### 4-N-Dimethylamino-N-{2-[4-(4-fluorobenzoyl)piperidino]ethyl}-N-(3-methoxyphenyl)benzamide

Using 4-amino-N-{2-[4-(4-fluorobenzoyl)piperidino]ethyl}-N-(3-methoxyphenyl)benzamide (238.9 mg, 0.50 mmol), the procedure of Inventive Example 196 was repeated to obtain 92.3 mg (36.7%) of the title compound in a light yellow amorphous form.
IR (KBr): 1680, 1634, 1600, 1524, 1486, 1448, 1364, 1310, 1280, 1230, 1198, 1158, 1136, 1040, 974, 824, 760, 700 cm⁻¹
NMR (CDCl₃) δ: 1.78 - 2.17 (6H, m), 2.65 (2H, t, J = 6.8 Hz), 2.89 (6H, s), 2.89 - 3.08 (3H, m), 3.70 (3H, s), 4.02 (2H, t, J = 6.8 Hz), 6.40 (2H, d, J = 8.8 Hz), 6.71 (3H, brs), 7.11 - 7.30 (5H, m), 7.96 (2H, dd, J = 8.6 Hz, 5.7 Hz)

### Inventive Example 198

### 4-Acetylamino-N-{2-[4-(4-fluorobenzoyl)piperidino]ethyl}-N-(3-hydroxyphenyl)benzamide

4-Acetylamino-N-{2-[4-(4-fluorobenzoyl)piperidino]ethyl}-N-(3-methoxyphenyl)benzamide (50.0 mg, 0.10 mmol) was dissolved in methylene chloride (3.0 ml) to which was subsequently added a methylene chloride solution (1 ml) of boron tribromide (0.018 ml, 0.19 mmol) at 0°C. After 12 hours of stirring at 0°C to room temperature, the reaction solution was mixed with saturated sodium bicarbonate aqueous solution and extracted with ethyl acetate. The resulting organic layer was washed with water and saturated brine, and dried on anhydrous sodium sulfate. Thereafter, the solvent was distilled off under a reduced pressure, and the resulting residue was purified by a silica gel column chromatography (ethyl acetate-chloroform:methanol = 10:1) to obtain 37.3 mg (76.7%) of the title compound in a light yellow amorphous form.
IR (KBr): 3310, 3124, 2944, 1680, 1602, 1533, 1488, 1446, 1410, 1377, 1314, 1263, 1236, 1209, 1158, 909, 852, 729, 699, 648 cm⁻¹
NMR (CDCl₃) δ: 1.60 - 2.00 (4H, m), 2.00 - 2.40 (2H, m), 2.07 (3H, s), 2.63 (2H, t, J = 6.0 Hz), 2.83 - 3.40 (3H, m), 4.01 (2H, t, J = 6.0 Hz), 6.40 - 6.70 (3H, m), 6.85 - 7.40 (7H, m), 7.80 - 8.03 (2H, m)

### Inventive Example 199

### 4-Acetylamino-N-{2-[4-(4-fluorobenzoyl)piperidino]ethyl}-N-(4-hydroxyphenyl)benzamide

Using 4-acetylamino-N-{2-[4-(4-fluorobenzoyl)piperidino]ethyl}-N-(4-methoxyphenyl)benzamide (280.7 mg, 0.54 mmol), the procedure of Inventive Example 198 was repeated to obtain 215.0 mg (78.7%) of the title compound in a colorless powder form.
Melting point: 133 - 137°C
IR (KBr): 3328, 3010, 1689, 1617, 1593, 1515, 1479, 1452, 1413, 1371, 1314, 1278, 1248, 1236, 1206, 1185, 1161, 1101, 969, 852, 840, 828, 759, 669, 600 cm⁻¹
NMR (CDCl₃) δ: 1.56 - 1.93 (4H, m), 1.97 - 2.40 (2H, m), 2.10 (3H, s), 2.63 (2H, t, J = 7.0 Hz), 2.75 - 3.40 (3H, m), 3.99 (2H, t, J = 7.0 Hz), 6.65 (2H, d, J = 8.8 Hz), 6.90 (2H, d, J = 8.8 Hz), 6.97 - 7.40 (4H, m), 7.80 - 8.03 (2H, m)

### Inventive Example 200

### 4-(4-Fluorobenzoyl)-1-[3-(2-methoxyphenyl)-4-(3-methoxyphenyl)butyl]piperidine hydrochloride

4-(4-Fluorobenzoyl)-1-[3-(2-methoxyphenyl)-4-(3-methoxyphenyl)butyl]piperidine (54 mg, 0.114 mmol) was dissolved in methylene chloride (2 ml) to which was subsequently added saturated hydrogen chloride/ether solution (1 ml). After 5 minutes of stirring, the solvent was removed by evaporation, and the resulting residue was solidified in ether (5 ml) and collected by filtration to obtain 58.4 mg (100%) of the title compound in a colorless amorphous powder form.
IR (KBr): 3432, 2936, 2520, 1680, 1598, 1492, 1458, 1438, 1240, 1156 cm⁻¹
NMR (CDCl₃) δ: 1.70 - 3.10 (12H, m), 3.10 - 3.85 (4H, m), 3.70 (6H, s), 6.50 - 7.30 (1H, m), 7.90 (2H, dd, J = 9 Hz, 6 Hz), 12.0 (1H, br-s)

### Inventive Example 201

### 4-(4-Fluorobenzoyl)-1-[4-(2-methoxyphenyl)-5-(3-methoxyphenyl)pentyl]piperidine hydrochloride

Using 4-(4-fluorobenzoyl)-1-[4-(2-methoxyphenyl)-5-(3-methoxyphenyl)pentyl]piperidine (35.6 mg, 0.0727 mmol), the procedure of Inventive Example 200 was repeated to obtain 38.3 mg (100%) of the title compound in a colorless amorphous powder form.
IR (KBr): 3430, 2940, 2500, 1680, 1596, 1490, 1454, 1238, 1156 cm⁻¹
NMR (CDCl₃) δ: 1.10 - 4.00 (18H, m), 3.71 (6H, s), 6.38 - 7.35 (10H, m), 7.90 (2H, dd, J = 9 Hz, 6 Hz), 11.9 (1H, br-s)

### Inventive Example 202

### 4-(4-Fluorobenzoyl)-1-[3-(2-methoxyphenyl)-2-(2-methoxyphenyl)propyl]piperidine hydrochloride

Using 4-(4-fluorobenzoyl)-1-[3-(2-methoxyphenyl)-2-(3-methoxyphenyl)propyl]piperidine (97 mg, 0.210 mmol), the procedure of Inventive Example 200 was repeated to obtain 92 mg (88.0%) of the title compound in a colorless amorphous powder form.
IR (KBr): 3432, 2940, 2500, 1680, 1598, 1492, 1456, 1438, 1242, 1156, 1028, 950, 854, 754 cm⁻¹
NMR (CDCl₃) δ: 1.70 - 2.10 (2H, m), 2.16 - 4.10 (12H, m), 3.70, 3.84 (each 3H, s), 6.45 - 7.40 (10H, m), 7.87 (2H, dd, J = 9 Hz, 6 Hz), 12.1 (1H, br-s)

### Inventive Example 203

### 4-[4-(4-Fluorobenzoyl)piperidino]-2-(2-methoxyphenyl)-2-(3-methoxyphenyl)methylbutyronitrile hydrochloride

Using 4-[4-(4-fluorobenzoyl)piperidino]-2-(2-methoxyphenyl)-2-(3-methoxyphenyl)methylbutyronitrile (66.5 mg, 0.133 mmol), the procedure of Inventive Example 200 was repeated to obtain 71.4 mg (100%) of the title compound in a colorless amorphous powder form.
IR (KBr): 3420, 2940, 2460, 2370, 1680, 1598, 1492, 1458, 1438, 1252, 1224, 1158 cm⁻¹
NMR (CDCl₃) δ: 1.80 - 4.15 (15H, m), 3.65, 4.02 (each 3H, s), 6.42 - 7.55 (10H, m), 7.93 (2H, dd, J = 9 Hz, 6 Hz), 12.5 (1H, br-s)

### Inventive Example 204

### N-{2-[4-(4-Fluorobenzoyl)piperidino]ethyl}-4-(N-hydroxyimino)-N-(2-methoxyphenyl)benzenesulfonamide hydrochloride

Using N-{2-[4-(4-fluorobenzoyl)piperidino]ethyl}-4-(N-hydroxyimino)-N-(2-methoxyphenyl)benzenesulfonamide (65 mg, 0.12 mmol), the procedure of Inventive Example 200 was repeated to obtain 69 mg (99.8%) of the title compound in a colorless amorphous powder form.
IR (KBr): 3200, 2950, 1678, 1598, 1496, 1348, 1282, 1220, 1158, 974, 760, 728, 602 cm⁻¹
NMR (CDCl₃-CD₃OD) δ: 1.85 - 2.40 (4H, m), 3.00 - 3.90 (7H, m), 3.36 (3H, s), 3.96 - 4.35 (2H, m), 6.80 (1H, d, J = 8.5 Hz), 6.90 - 7.45 (5H, m), 7.60 (4H, s), 7.96 (2H, dd, J = 9 Hz, 6 Hz), 8.11 (1H, s)

### Inventive Example 205

### 4-Methanesulfonylamino-N-{2-[4-(4-fluorobenzoyl)piperidino]ethyl}-N-(3-methoxyphenyl)benzamide hydrochloride

Using 4-methanesulfonylamino-N-{2-[4-(4-fluorobenzoyl)piperidino]ethyl}-N-(3-methoxyphenyl)benzamide (164.0 mg, 0.30 mmol), the procedure of Inventive Example 200 was repeated to obtain 157.1 mg (88.9%) of the title compound in a colorless powder form.
Melting point: 125 - 127°C
IR (KBr): 1680, 1640, 1598, 1490, 1456, 1440, 1392, 1332, 1284, 1218, 1152, 970 cm⁻¹
NMR (CDCl₃-CD₃OD) δ: 1.90 - 2.70 (7H, m), 2.93 (3H, s), 3.00 - 3.90 (4H, m), 3.71 (3H, s), 4.23 - 4.53 (2H, m), 6.53 - 6.80 (3H, m), 6.90 - 7.40 (7H, m), 7.96 (2H, dd, J = 9.0, 6.0 Hz)

### Inventive Example 206

### 4-(Pyrol-1-yl)-N-{2-[4-(4-fluorobenzoyl)piperidino]ethyl}-N-(3-methoxyphenyl)benzamide hydrochloride

Using 4-(pyrol-1-yl)-N-{2-[4-(4-fluorobenzoyl)piperidino]ethyl}-N-(3-methoxyphenyl)benzamide (170.2 mg, 0.32 mmol), the procedure of Inventive Example 200 was repeated to obtain 170.4 mg (94.7%) of the title compound in a brown powder form.
Melting point: 131 - 134°C
IR (KBr): 3416, 1678, 1634, 1598, 1520, 1488, 1476, 1438, 1412, 1390, 1330, 1282, 1234, 1214, 1158, 1068, 844, 698 cm⁻¹
NMR (CDCl₃-CD₃OD) δ: 2.54 - 2.71 (3H, m), 3.73 (3H, s), 3.17 - 3.81 (9H, m), 4.45 (4H, t, J = 6.6 Hz), 6.73 (1H, s), 6.68 - 6.77 (3H, m), 7.09 - 7.48 (10H, m), 7.97 (2H, dd, J = 8.6 Hz, 5.3 Hz)

### Inventive Example 207

### 4-Ureido-N-{2-[4-(4-fluorobenzoyl)piperidino]ethyl}-N-(3-methoxyphenyl)benzamide hydrochloride

Using 4-ureido-N-{2-[4-(4-fluorobenzoyl)piperidino]ethyl}-N-(3-methoxyphenyl)benzamide (107.2 mg, 0.21 mmol), the procedure of Inventive Example 200 was repeated to obtain 119.5 mg (quantitative) of the title compound in a light brown powder form. Melting point: 146 - 149°C

IR (KBr): 3348, 1680, 1598, 1532, 1488, 1438, 1412, 1318, 1256, 1216, 1180, 1156, 948, 840, 760, 698 cm⁻¹
NMR (CDCl₃-CD₃OD) δ: 2.04 - 2.41 (6H, m), 3.82 (3H, s), 3.11 - 3.88 (5H, m), 4.31 - 4.37 (2H, m), 6.64 - 6.72 (4H, m), 7.08 - 7.30 (9H, m), 7.95 (2H, dd, J = 8.4 Hz, 5.3 Hz)

### Inventive Example 208

### 4-Acetylamino-N-{2-[4-(4-fluorobenzoyl)piperidino]ethyl}-N-(3-methoxyphenyl)benzamide hydrochloride

Using 4-acetylamino-N-{2-[4-(4-fluorobenzoyl)piperidino]ethyl}-N-(3-methoxyphenyl)benzamide (360.0 mg, 0.70 mmol), the procedure of Inventive Example 200 was repeated to obtain 342.0 mg (88.5%) of the title compound in a colorless powder form.
Melting point: 138 - 145°C
IR (KBr): 3445, 1676, 1640, 1600, 1316, 1218, 851, 762 cm⁻¹
NMR (CDCl₃-CD₃OD) δ: 2.15 (3H, s), 2.04 - 3.79 (11H, m), 3.70 (3H, s), 4.22 (2H, t, J = 6.8 Hz), 6.65 - 6.74 (3H, m), 7.03 - 7.49 (7H, m), 7.94 (2H, dd, J = 8.8 Hz, 5.5 Hz)

### Inventive Example 209

### 4-Amino-N-{2-[4-(4-fluorobenzoyl)piperidino]ethyl}-N-(3-methoxyphenyl)benzamide hydrochloride

Using 4-amino-N-{2-[4-(4-fluorobenzoyl)piperidino]ethyl}-N-(3-methoxyphenyl)benzamide (142.0 mg, 0.30 mmol), the procedure of Inventive Example 200 was repeated to obtain 100.0 mg (60.8%) of the title compound in a colorless powder form.
Melting point: 145 - 155°C
IR (KBr): 3440, 1678, 1600, 1490, 1210, 1158, 844 cm⁻¹
NMR (CDCl₃-CD₃OD) δ: 2.08 - 3.82 (11H, m), 3.71 (3H, s), 4.31 - 4.39 (2H, m), 6.70 - 6.82 (3H, m), 7.08 - 7.44 (7H, m), 7.95 - 8.02 (2H, m)

### Inventive Example 210

### 4-Dimethylamino-N-{2-[4-(4-fluorobenzoyl)piperidino]ethyl}-N-(3-methoxyphenyl)benzamide hydrochloride

Using 4-dimethylamino-N-{2-[4-(4-fluorobenzoyl)piperidino]ethyl}-N-(3-methoxyphenyl)benzamide (83.5 mg, 0.17 mmol), the procedure of Inventive Example 200 was repeated to obtain 87.5 mg (95.3%) of the title compound in a orange powder form.
Melting point: 119 - 121°C
IR (KBr): 3428, 2632, 2552, 2448, 1678, 1642, 1598, 1506, 1490, 1452, 1410, 1320, 1284, 1216, 1180, 1158, 1130, 952, 852, 700 cm⁻¹
NMR (CDCl₃-CD₃OD) δ: 2.51 - 2.68 (2H, m), 3.11 (6H, s), 3.19 - 3.42 (6H, m), 3.74 (3H, s), 3.49 - 3.93 (3H, m), 4.39 (2H, t, J = 6.6 Hz), 6.73 - 6.87 (3H, m), 7.14 (2H, d, J = 8.2 Hz), 7.27 - 7.32 (2H, m), 7.54 - 7.56 (3H, m), 7.99 (2H, dd, J = 8.6 Hz, 5.5 Hz)

### Inventive Example 211

### 4-[4-(4-Fluorophenyl)piperazinyl]-2-(2-methoxyphenyl)-2-(3-methoxyphenyl)methylbutyronitrile oxalate

4-[4-(4-Fluorophenyl)piperazinyl]-2-(2-methoxyphenyl)-2-(3-methoxyphenyl)methylbutyronitrile (51 mg, 0.108 mmol) was dissolved in methanol (5 ml) to which was subsequently added oxalic acid (9.8 mg, 0.108 mmol). After 5 minutes of stirring at room temperature, methanol was removed by evaporation, and the resulting residue was solidified in ether (5 ml) and collected by filtration to obtain 51.9 mg (85.3%) of the title compound in a colorless amorphous powder form.
IR (KBr): 3000, 2930, 2820, 1732, 1600, 1510, 1492, 1458, 1250, 702 cm⁻¹
NMR (CDCl₃-CD₃OD) δ: 2.70 - 3.80 (14H, m), 3.65, 3.94 (each 3H, s), 6.37 - 7.50 (12H, m)

### Inventive Example 212

### N-{2-[4-(4-Fluorobenzoyl)piperidino]ethyl}-3-methoxy-N-(2-methoxyphenyl)benzamide oxalate

Using N-{2-[4-(4-fluorobenzoyl)piperidino]ethyl}-3-methoxy-N-(2-methoxyphenyl)benzamide (107 mg, 0.218 mmol), the procedure of Inventive Example 211 was repeated to obtain 100 mg (79.2%) of the title compound in a colorless powder form.
Melting point: 170.5 - 174°C
IR (KBr): 3450, 1680, 1644, 1598, 1504, 1452, 1388, 1226 cm⁻¹
NMR (CDCl₃) δ: 1.86 - 2.50 (4H, m), 3.10 - 3.83 (7H, m), 3.62, 3.71 (each 3H, s), 3.85 - 4.40 (2H, m), 6.60 - 7.35 (10H, m), 7.67 - 8.15 (2H, m)

### Inventive Example 213

### N-{2-[4-(4-Fluorobenzoyl)piperidino]ethyl}-4-methoxy-N-(2-methoxyphenyl)benzenesulfonamide oxalate

Using N-{2-[4-(4-fluorobenzoyl)piperidino]ethyl}-4-methoxy-N-(2-methoxyphenyl)benzenesulfonamide (56.7 mg, 0.108 mmol), the procedure of Inventive Example 211 was repeated to obtain 59.8 mg (89.8%) of the title compound in a colorless amorphous powder form.
IR (KBr): 3440, 1680, 1596, 1498, 1344, 1260, 1222, 1156, 588, 562 cm⁻¹
NMR (CDCl₃-CD₃OD) δ: 1.90 - 2.50 (4H, m), 3.10 - 3.77 (2H, m), 3.40 (3H, s), 3.77 - 4.10 (2H, m), 3.86 (3H, s), 6.68 - 7.38 (8H, m), 7.53 (2H, d, J = 9 Hz), 7.95 (2H, dd, J = 9 Hz, 6 Hz)

### Inventive Example 214

### 4-Fluoro-N-{2-[4-(4-fluorobenzoyl)piperidino]ethyl}-N-(2-methoxyphenyl)benzenesulfonamide oxalate

Using 4-fluoro-N-{2-[4-(4-fluorobenzoyl)piperidino]ethyl}-N-(2-methoxyphenyl)benzenesulfonamide (81 mg, 0.157 mmol), the procedure of Inventive Example 211 was repeated to obtain 82.3 mg (86.7%) of the title compound in a beige powder form.
Melting point: 155 - 165°C
IR (KBr): 3450, 1682, 1598, 1496, 1460, 1344, 1292, 1224, 1168, 1156, 1118, 836, 586, 556 cm⁻¹
NMR (CDCl₃-CD₃OD) δ: 1.90 - 2.37 (4H, m), 2.80 - 3.70 (7H, m), 3.35 (3H, s), 3.75 - 4.20 (2H, m), 6.66 - 7.45 (8H, m), 7.60, 7.93 (each 2H, dd, J = 9 Hz, 6 Hz)

### Inventive Example 215

### N-Benzoyl-N-{2-[4-(4-fluorobenzoyl)piperidino]ethyl}-4-methoxybenzenesulfonamide oxalate

Using N-benzoyl-N-{2-[4-(4-fluorobenzoyl)piperidino]ethyl}-4-methoxybenzenesulfonamide (23.7 mg, 0.0452 mmol), the procedure of Inventive Example 211 was repeated to obtain 26.2 mg (94.3%) of the title compound in a colorless amorphous powder form.
IR (KBr): 3450, 1682, 1596, 1498, 1364, 1264, 1232, 1162, 700, 588, 554 cm⁻¹
NMR (DMSO_{d6}-CDCl₃) δ: 1.50 - 2.00 (4H, m), 2.30 - 2.70 (2H, m), 2.73 - 3.55 (5H, m), 3.70 - 4.30 (2H, m), 3.90 (3H, s), 6.87 - 7.60 (9H, m), 7.65 - 8.20 (4H, m)

### Inventive Example 216

### N-Acetyl-N-{2-[4-(4-fluorobenzoyl)piperidino]ethyl}-4-methoxybenzenesulfonamide oxalate

Using N-acetyl-N-{2-[4-(4-fluorobenzoyl)piperidino]ethyl}-4-methoxybenzenesulfonamide (27.6 mg, 0.0597 mmol), the procedure of Inventive Example 211 was repeated to obtain 30.9 mg (98.4%) of the title compound in a colorless powder form.
Melting point: 169 - 171°C IR (KBr): 3450, 1682, 1598, 1500, 1358, 1310, 1264, 1160,
1088, 558 cm⁻¹
NMR (DMSO_{d6}-CDCl₃) δ: 1.70 - 2.20 (4H, m), 2.35 (3H, s), 2.67 - 3.73 (7H, m), 3.75 - 4.24 (2H, m), 3.90 (3H, s), 6.85 - 7.33 (4H, m), 7.56 - 8.13 (4H, m)

### Inventive Example 217

### N-Benzyl-N-{2-[4-(4-fluorobenzoyl)piperidino]ethyl}-4-methoxybenzenesulfonamide oxalate

Using N-benzyl-N-{2-[4-(4-fluorobenzoyl)piperidino]ethyl)-4-methoxybenzenesulfonamide (22.8 mg, 0.0447 mmol), the procedure of Inventive Example 211 was repeated to obtain 25.9 mg (96.5%) of the title compound in a colorless amorphous powder form.
IR (KBr): 1682, 1598, 1498, 1342, 1304, 1260, 1212, 1156, 700, 560 cm⁻¹
NMR (DMSO_{d6}-CDCl₃) δ: 1.70 - 2.30 (4H, m), 2.45 - 3.65 (7H, m), 3.90 (3H, s), 4.25 (2H, s), 6.83 - 7.52 (9H, m), 7.59 - 8.08 (4H, m)

### Inventive Example 218

### N-{2-[4-(4-Fluorobenzoyl)piperidino]ethyl}-4-methoxy-N-(2-propenyl)benzenesulfonamide oxalate

Using N-{2-[4-(4-fluorobenzoyl)piperidino]ethyl}-4-methoxy-N-(2-propenyl)benzenesulfonamide (63 mg, 0.137 mmol), the procedure of Inventive Example 211 was repeated to obtain 68 mg (90.1%) of the title compound in a colorless powder form.
Melting point: 182 - 185.5°C
IR (KBr): 1684, 1598, 1498, 1412, 1344, 1304, 1260, 1214, 1154, 1094, 952, 836, 806, 700, 668, 584, 560 cm⁻¹
NMR (CDCl₃-DMSO_{d6}) δ: 1.60 - 2.20 (4H, m), 2.69 - 3.22 (4H, m), 3.22 - 3.64 (5H, m), 3.80 (2H, d, J = 6 Hz), 3.88 (3H, s), 5.16 (1H, d, J = 10.5 Hz), 5.22 (1H, d, J = 15 Hz), 5.40 - 5.90 (1H, m), 6.90 - 7.36 (4H, m), 7.75 (2H, d, J = 9 Hz), 8.02 (2H, dd, J = 9 Hz, 6 Hz)

### Inventive Example 219

### N-Ethoxycarbonylmethyl-N-{2-[4-(4-fluorobenzoyl)piperidino]ethyl}-4-methoxybenzenesulfonamide oxalate

Using N-ethoxycarbonyl-N-{2-[4-(4-fluorobenzoyl)piperidino]ethyl}-4-methoxybenzenesulfonamide (61 mg, 0.120 mmol), the procedure of Inventive Example 211 was repeated to obtain 65 mg (90.8%) of the title compound in a creamy amorphous powder form.
IR (KBr): 1748, 1680, 1598, 1500, 1342, 1304, 1262, 1214, 1184, 1156, 560 cm⁻¹
NMR (CDCl₃) δ: 1.21 (3H, t, J = 7.5 Hz), 1.80 - 2.43 (4H, m), 3.00 - 3.75 (9H, m), 3.86 (3H, s), 4.00 (2H, s), 4.10 (2H, q, J = 7.5 Hz), 6.79 - 7.32 (4H, m), 7.56 - 8.30 (6H, m)

### Inventive Example 220

### N-Benzoyl-N-{3-[4-(4-fluorophenyl)piperazinyl]propyl}-4-methoxybenzenesulfonamide oxalate

Using N-benzoyl-N-{3-[4-(4-fluorophenyl)piperazinyl]propyl}-4-methoxybenzenesulfonamide (42.2 mg, 0.0825 mmol), the procedure of Inventive Example 211 was repeated to obtain 45.6 mg (91.9%) of the title compound in a colorless powder form.
Melting point: 176 - 180°C
IR (KBr): 1682, 1596, 1512, 1360, 1266, 1162, 1018, 700, 578 cm⁻¹
NMR (CDCl₃-CD₃OD) δ: 1.97 - 2.43 (2H, m), 2.90 - 3.50 (2H, m), 3.33 (8H, s), 3.73 - 4.05 (2H, m), 3.89 (3H, s), 6.70 - 7.10 (6H, m), 7.40 (5H, s), 7.68 (2H, d, J = 9 Hz)

### Inventive Example 221

### N-{2-[4-(4-Fluorobenzoyl)piperidino]ethyl}-4-methoxymethyl-N-(2-methoxyphenyl)benzamide oxalate

Using N-{2-[4-(4-fluorobenzoyl)piperidino]ethyl}-4-methoxymethyl-N-(2-methoxyphenyl)benzamide (128 mg, 0.254 mmol), the procedure of Inventive Example 211 was repeated to obtain 128 mg (84.7%) of the title compound in a colorless powder form.
Melting point: 151 - 155°C
IR (KBr): 1680, 1640, 1598, 1502, 1412, 1388, 1306, 1224, 1158, 752 cm⁻¹
NMR (CDCl₃) δ: 1.90 - 2.56 (4H, m), 3.06 - 3.83 (7H, m), 3.30 (3H, s), 3.70 (3H, s), 3.97 - 4.45 (2H, m), 4.35 (2H, s), 4.85 (2H, br-s), 6.77 (1H, d, J = 8.5 Hz), 6.86 (1H, d, J = 6.5 Hz), 6.91 - 7.40 (8H, m), 7.95 (2H, dd, J = 9 Hz, 6 Hz)

### Inventive Example 222

### N-{2-[4-(4-Fluorobenzoyl)piperidino]ethyl}-3-methoxy-N-(2-pyridyl)benzamide oxalate

Using N-{2-[4-(4-fluorobenzoyl)piperidino]ethyl}-3-methoxy-N-(2-pyridyl)benzamide (19 mg, 0.0412 mmol), the procedure of Inventive Example 211 was repeated to obtain 22.2 mg (97.7%) of the title compound in a beige amorphous powder form.
IR (KBr): 3450, 1682, 1634, 1596, 1546, 1504, 1454, 1226 cm⁻¹
NMR (CDCl₃-CD₃OD) δ: 1.75 - 2.32 (4H, m), 2.95 - 4.00 (7H, m), 3.83 (3H, s), 4.60 - 5.00 (2H, m), 6.56 - 7.45 (6H, m), 7.53 - 8.30 (6H, m)

### Inventive Example 223

### N-{2-[4-(4-Fluorobenzoyl)piperidino]ethyl}-N-(2-methoxyphenyl)-3-nitrobenzenesulfonamide oxalate

Using N-{2-[4-(4-fluorobenzoyl)piperidino]ethyl}-N-(2-methoxyphenyl)-3-nitrobenzenesulfonamide (70 mg, 0.129 mmol), the procedure of Inventive Example 211 was repeated to obtain 73.6 mg (90.3%) of the title compound in a colorless amorphous powder form.
IR (KBr): 3450, 1680, 1598, 1532, 1496, 1354, 1220, 1172 cm⁻¹
NMR (CDCl₃-CD₃OD) δ: 1.85 - 2.60 (4H, m), 2.80 - 3.75 (7H, m), 3.35 (3H, s), 3.82 - 4.35 (2H, m), 6.67 - 7.50 (6H, m), 7.54 - 8.13 (4H, m), 8.27 - 8.60 (2H, m)

### Inventive Example 224

### N-{2-[4-(4-Fluorobenzoyl)piperidino]ethyl}-4-methoxy-N-(2-thiazolyl)benzenesulfonamide oxalate

Using N-{2-[4-(4-fluorobenzoyl)piperidino]ethyl}-4-methoxy-N-(2-thiazolyl)benzenesulfonamide (52 mg, 0.103 mmol), the procedure of Inventive Example 211 was repeated to obtain 50 mg (81.8%) of the title compound in a colorless powder form.
Melting point: 152 - 158°C
IR (KBr): 1682, 1598, 1506, 1444, 1310, 1290, 1256, 1144, 1088, 932, 704, 574, 562 cm⁻¹
NMR (CDCl₃-DMSO_{d6}) δ: 1.40 - 2.05 (4H, m), 2.27 - 2.75 (2H, m), 2.77 - 3.60 (5H, m), 3.80 (3H, s), 3.99 - 4.40 (2H, m), 6.72 (1H, d, J = 3 Hz), 6.96 (2H, d, J = 9 Hz), 7.07 - 7.40 (3H, m), 7.60 - 8.30 (4H, m)

### Inventive Example 225

### N-{2-[4-(4-Fluorobenzoyl)piperidino]ethyl}-4-methoxy-N-(3-methoxyphenyl)benzenesulfonamide oxalate

Using N-{2-[4-(4-fluorobenzoyl)piperidino]ethyl}-4-methoxy-N-(3-methoxyphenyl)benzenesulfonamide (87 mg, 0.165 mmol), the procedure of Inventive Example 211 was repeated to obtain 98.3 mg (96.6%) of the title compound in a colorless powder form.
Melting point: 163.5 - 167°C
IR (KBr): 1676, 1598, 1496, 1346, 1308, 1262, 1214, 1158, 1094, 694 cm⁻¹
NMR (CDCl₃-CD₃OD) δ: 1.80 - 2.35 (4H, m), 2.95 - 3.50 (5H, m), 3.50 - 4.17 (4H, m), 3.74, 3.88 (each 3H, s), 6.52 - 6.73 (2H, m), 6.83 - 7.40 (6H, m), 7.52 (2H, d, J = 9 Hz), 8.04 (2H, dd, J = 9 Hz, 6 Hz)

### Inventive Example 226

### N-(2-Cyanophenyl)-N-{2-[4-(4-fluorobenzoyl)piperidino]ethyl}-4-methoxybenzenesulfonamide oxalate

Using N-(2-cyanophenyl)-N-{2-[4-(4-fluorobenzoyl)-piperidino]ethyl}-4-methoxybenzenesulfonamide (107 mg, 0.205 mmol), the procedure of Inventive Example 211 was repeated to obtain 96 mg (76.6%) of the title compound in a colorless powder form.
Melting point: 167 - 173°C
IR (KBr): 1678, 1638, 1596, 1496, 1356, 1262, 1218, 1158, 704, 578, 552 cm⁻¹
NMR (CD₃OD-CDCl₃) δ: 1.85 - 2.30 (4H, m), 2.95 - 3.75 (8H, m), 3.76 - 4.14 (1H, m), 3.90 (3H, s), 6.90 - 7.30 (5H, m), 7.37 - 7.96 (5H, m), 8.00 (2H, dd, J = 9 Hz, 6 Hz)

### Inventive Example 227

### N-{2-[4-(4-Fluorobenzoyl)piperidino]ethyl}-N-(2-trifluoromethylphenyl)-4-methoxybenzenesulfonamide oxalate

Using N-{2-[4-(4-fluorobenzoyl)piperidino]ethyl}-N-(2-trifluoromethylphenyl)-4-methoxybenzenesulfonamide (46.7 mg, 0.0827 mmol), the procedure of Inventive Example 211 was repeated to obtain 49 mg (90.5%) of the title compound in a colorless powder form.
Melting point: 174 - 180°C
IR (KBr): 1680, 1596, 1498, 1356, 1316, 1264, 1224, 1160, 1036, 722, 576, 558 cm⁻¹
NMR (CD₃OD-CDCl₃) δ: 1.91 - 2.30 (4H, m), 2.90 - 3.80 (8H, m), 3.80 - 4.32 (1H, m), 3.90 (3H, s), 6.79 - 7.30 (5H, m), 7.40 - 8.07 (7H, m)

### Inventive Example 228

### N-Cyclohexyl-N-{2-[4-(4-fluorobenzoyl)piperidino]ethyl}-4-methoxybenzenesulfonamide oxalate

Using N-cyclohexyl-N-{2-[4-(4-fluorobenzoyl)piperidino]ethyl}-4-methoxybenzenesulfonamide (300 mg, 0.597 mmol), the procedure of Inventive Example 211 was repeated to obtain 322 mg (91.0%) of the title compound in a colorless powder form.
Melting point: 217 - 222°C
IR (KBr): 2925, 1716, 1686, 1598, 1500, 1322, 1260, 1214, 1148, 1128, 1102, 1026, 1010, 980, 954, 856, 700, 666, 556 cm⁻¹
NMR (CDCl₃-DMSO_{d6}) δ: 0.94 - 2.30 (14H, m), 2.80 - 4.10 (10H, m), 3.87 (3H, s), 6.85 - 7.47 (4H, m), 7.60 - 8.30 (4H, m)

### Inventive Example 229

### N-{2-[4-(4-Fluorobenzoyl)piperidino]ethyl}-N-(2-methoxyphenyl)-3-pyridinesulfonamide oxalate

Using N-{2-[4-(4-fluorobenzoyl)piperidino]ethyl}-N-(2-methoxyphenyl)-3-pyridinesulfonamide (55 mg, 0.1105 mmol), the procedure of Inventive Example 211 was repeated to obtain 53.6 mg (82.5%) of the title compound in a creamy powder form.
Melting point: 178 - 182°C
IR (KBr): 1684, 1598, 1498, 1466, 1416, 1338, 1224, 1168, 1118, 956, 782, 698, 594 cm⁻¹
NMR (CDCl₃-CD₃OD) δ: 1.90 - 2.40 (4H, m), 3.10 - 3.75 (7H, m), 3.30 (3H, s), 3.83 - 4.20 (2H, m), 6.65 - 7.56 (7H, m), 7.78 - 8.10 (2H, m), 8.60 - 8.86 (2H, m)

### Inventive Example 230

### 3-{[N-[2-[4-(4-Fluorobenzoyl)piperidino]ethyl]-2-methoxyanilino]sulfonyl}pyridine N-oxide oxalate

Using 3-{[N-[2-[4-(4-fluorobenzoyl)piperidino]ethyl]-2-methoxyanilino]sulfonyl}pyridine N-oxide (101 mg, 0.197 mmol), the procedure of Inventive Example 211 was repeated to obtain 114 mg (95.9%) of the title compound in a light orange amorphous powder form.
IR (KBr): 3450, 2940, 2820, 1664, 1628, 1596, 1464, 1428, 1290, 1280, 1212, 1170, 1112, 1048, 1014, 978 cm⁻¹
NMR (CDCl₃) δ: 1.48 - 2.30 (7H, m), 2.36 - 3.39 (5H, m), 3.32 (3H, s), 3.39 - 3.90 (6H, m), 4.71 (2H, s), 7.12 (2H, dd, J = 9 Hz, 9 Hz), 7.38 (4H, s), 7.94 (2H, dd, J = 9 Hz, 6 Hz)

### Inventive Example 231

### N-{2-[4-(4-Fluorobenzoyl)piperidino]ethyl}-N-(2-methoxyphenyl)-p-toluenesulfonamide oxalate

Using N-{2-[4-(4-fluorobenzoyl)piperidino]ethyl}-N-(2-methoxyphenyl)-p-toluenesulfonamide (41 mg, 0.0803 mmol), the procedure of Inventive Example 211 was repeated to obtain 40.5 mg (84.0%) of the title compound in a colorless powder form.
Melting point: 160 - 163°C
IR (KBr): 1682, 1598, 1496, 1336, 1224, 1158, 1118, 954, 658, 558 cm⁻¹
NMR (CDCl₃) δ: 2.00 - 2.55 (4H, m), 2.40 (3H, s), 3.00 - 3.73 (7H, m), 3.32 (3H, s), 3.80 - 4.20 (2H, m), 6.76 (1H, d, J = 8.5 Hz), 6.88 - 7.56 (7H, m), 7.47 (2H, d, J = 8 Hz), 7.93 (2H, dd, J = 9 Hz, 6 Hz)

### Inventive Example 232

### N-{2-[4-(4-Fluorobenzoyl)piperidino]ethyl}-N-(2-methoxyphenyl)-4-morpholinomethylbenzamide oxalate

Using N-{2-[4-(4-fluorobenzoyl)piperidino]ethyl}-N-(2-methoxyphenyl)-4-morpholinobenzamide (48.3 mg, 0.0863 mmol), the procedure of Inventive Example 211 was repeated to obtain 52 mg (92.7%) of the title compound in a beige amorphous powder form.
IR (KBr): 1678, 1638, 1598, 1502, 1454, 1390, 1280, 1224, 1158, 1116, 864, 756 cm⁻¹
NMR (CDCl₃-CD₃OD) δ: 1.86 - 2.35 (4H, m), 2.74 - 4.35 (17H, m), 3.52 (2H, s), 3.70 (3H, s), 6.53 - 7.40 (10H, m), 7.95 (2H, dd, J = 9 Hz, 6 Hz)

### Inventive Example 233

### N-{2-[4-(4-Fluorobenzoyl)piperidino]ethyl}-N-(2-methoxyphenyl)-4-dimethylaminomethylbenzamide oxalate

Using N-{2-[4-(4-fluorobenzoyl)piperidino]ethyl}-N-(2-methoxyphenyl)-4-dimethylaminomethylbenzamide (105 mg, 0.203 mmol), the procedure of Inventive Example 211 was repeated to obtain 117 mg (94.8%) of the title compound in a light beige amorphous powder form.
IR (KBr): 1680, 1640, 1598, 1500, 1412, 1316, 1280, 1222 cm⁻¹
NMR (CDCl₃-DMSO_{d6}) δ: 1.60 - 2.16 (4H, m), 2.20 - 3.11 (6H, m), 2.55 (6H, s), 3.15 - 4.60 (5H, m), 3.72 (3H, s), 6.55 - 6.94 (2H, m), 7.00 - 7.46 (8H, m), 8.00 (2H, dd, J = 9 Hz, 6 Hz)

### Inventive Example 234

### N-{3-[4-(4-Fluorophenyl)piperazinyl]propyl}-4-methoxy-N-(2-methoxyphenyl)benzenesulfonamide oxalate

Using N-{3-[4-(4-fluorophenyl)piperazinyl]propyl}-4-methoxy-N-(2-methoxyphenyl)benzenesulfonamide (130 mg, 0.253 mmol), the procedure of Inventive Example 211 was repeated to obtain 142 mg (93.0%) of the title compound in a colorless powder form.
Melting point: 157 - 160°C
IR (KBr): 1596, 1510, 1496, 1462, 1342, 1256, 1162, 704, 588, 562 cm⁻¹
NMR (CDCl₃-CD₃OD) δ: 1.65 - 2.10 (2H, m), 3.13 - 3.53 (2H, m), 3.33 (8H, s), 3.56 (3H, s), 3.67 (2H, t, J = 7 Hz), 3.87 (3H, s), 6.65 - 7.43 (10H, m), 7.55 (2H, d, J = 9 Hz)

### Inventive Example 235

### 4-Fluoro-N-{3-[4-(4-fluorophenyl)piperazinyl]propyl}-N-(2-methoxyphenyl)benzenesulfonamide oxalate

Using 4-fluoro-N-{3-[4-(4-fluorophenyl)piperazinyl]propyl}-N-(2-methoxyphenyl)benzenesulfonamide (103 mg, 0.205 mmol), the procedure of Inventive Example 211 was repeated to obtain 114 mg (93.8%) of the title compound in a colorless amorphous powder form.
IR (KBr): 3450, 1628, 1592, 1510, 1494, 1460, 1342, 1230, 1166, 836, 556 cm⁻¹
NMR (CDCl₃-CD₃OD) δ: 1.60 - 2.17 (2H, m), 3.04 - 3.83 (4H, m), 3.33 (8H, s), 3.39 (3H, s), 6.65 - 7.42 (10H, m), 7.62 (2H, dd, J = 9 Hz, 6 Hz)

### Inventive Example 236

### N-{3-[4-(4-Fluorophenyl)piperazinyl]propyl}-N-(2-methoxyphenyl)-3-nitrobenzenesulfonamide oxalate

Using N-{3-[4-(4-fluorophenyl)piperazinyl]propyl}-N-(2-methoxyphenyl)-3-nitrobenzenesulfonamide (195 mg, 0.369 mmol), the procedure of Inventive Example 211 was repeated to obtain 229 mg (100%) of the title compound in a yellow prism crystal form.
Melting point: 162.5 - 164°C
IR (KBr): 1628, 1532, 1512, 1498, 1460, 1352, 1258, 1228, 1168, 1120 cm⁻¹
NMR (CDCl₃) δ: 1.74 - 2.15 (2H, m), 3.00 - 3.50 (10H, m), 3.34 (3H, s), 3.55 - 3.85 (2H, m), 6.67 - 7.11 (6H, m), 7.12 - 7.30 (2H, m), 7.69 (1H, d, J = 7.5 Hz), 7.98 (1H, d, J = 7.5 Hz), 8.25 - 8.52 (2H, m), 9.44 (2H, br-s)

### Inventive Example 237

### N-{2-[4-(4-Fluorobenzoyl)piperidino]ethyl}-4-hydroxymethyl-N-(2-methoxyphenyl)benzenesulfonamide oxalate

Using N-{2-[4-(4-fluorobenzoyl)piperidino]ethyl}-4-hydroxymethyl-N-(2-methoxyphenyl)benzenesulfonamide (87 mg, 0.165 mmol), the procedure of Inventive Example 211 was repeated to obtain 90.5 mg (88.9%) of the title compound in a beige amorphous powder form.
IR (KBr): 3350, 2920, 1680, 1638, 1596, 1496, 1344, 1220, 1150 cm⁻¹
NMR (CDCl₃-CD₃OD) δ: 1.85 - 2.40 (4H, m), 2.90 - 3.70 (7H, m), 3.37 (3H, s), 3.76 - 4.14 (2H, m), 4.71 (2H, s), 6.80 (1H, d, J = 8.5 Hz), 6.99 (1H, d, J = 8.5 Hz), 7.17 (2H, dd, J = 9 Hz, 9 Hz), 7.29 (2H, d, J = 6 Hz), 7.42 (2H, d, J = 8.5 Hz), 7.60 (2H, d, J = 8.5 Hz), 7.95 (2H, dd, J = 9 Hz, 6 Hz)

### Inventive Example 238

### N-{2-[4-(4-Fluorobenzoyl)piperidino]ethyl}-4-hydroxymethyl-N-(2-methoxyphenyl)benzamide oxalate

Using N-{2-[4-(4-fluorobenzoyl)piperidino]ethyl}-4-hydroxymethyl-N-(2-methoxyphenyl)benzamide (128 mg, 0.261 mmol), the procedure of Inventive Example 211 was repeated to obtain 145 mg (95.7%) of the title compound in a light beige amorphous powder form.
IR (KBr): 3400, 1680, 1640, 1598, 1502, 1440, 1412, 1280, 1222, 1158, 1020, 954 cm⁻¹
NMR (CDCl₃-CD₃OD) δ: 1.95 - 2.35 (4H, m), 3.08 - 3.83 (7H, m), 3.70 (3H, s), 3.95 - 4.33 (2H, m), 4.56 (2H, s), 6.77 (1H, d, J = 8 Hz), 6.85 (1H, d, J = 7 Hz), 6.93 - 7.40 (8H, m), 7.95 (2H, dd, J = 9 Hz, 6 Hz)

### Inventive Example 239

### N-{2-[4-(4-Fluorobenzoyl)piperidino]ethyl}-3-hydroxymethyl-N-(2-methoxyphenyl)benzamide oxalate

Using N-{2-[4-(4-fluorobenzoyl)piperidino]ethyl}-3-hydroxymethyl-N-(2-methoxyphenyl)benzamide (148 mg, 0.302 mmol), the procedure of Inventive Example 211 was repeated to obtain 164 mg (93.5%) of the title compound in a light beige amorphous powder form.
IR (KBr): 3390, 2948, 1680, 1638, 1598, 1502, 1440, 1392, 1280, 1220, 1158, 1022 cm⁻¹
NMR (CDCl₃) δ: 1.90 - 2.40 (4H, m), 3.00 - 3.85 (7H, m), 3.70 (3H, s), 3.95 - 4.30 (2H, m), 4.50 (2H, s), 6.26 (3H, br-s), 6.75 (1H, d, J = 8 Hz), 6.83 (1H, d, J = 6.5 Hz), 6.90 - 7.56 (8H, m), 7.93 (2H, dd, J = 9 Hz, 6 Hz)

### Inventive Example 240

### N-{2-[4-(4-Fluorobenzoyl)piperidino]ethyl}-4-hydroxymethyl-N-(3-methoxybenzyl)benzamide oxalate

Using N-{2-[4-(4-fluorobenzoyl)piperidino]ethyl}-4-hydroxymethyl-N-(3-methoxybenzyl)benzamide (100 mg, 0.198 mmol), the procedure of Inventive Example 211 was repeated to obtain 110 mg (93.4%) of the title compound in a beige amorphous powder form.
IR (KBr): 3400, 1680, 1598, 1490, 1420, 1216, 1156, 852 cm⁻¹
NMR (CDCl₃-CD₃OD) δ: 1.85 - 2.35 (4H, m), 2.95 - 4.00 (9H, m), 3.80 (3H, s), 4.62 (2H, d, J = 6 Hz), 4.77 (2H, s), 6.60 - 6.96 (2H, m), 7.10 - 7.70 (6H, m), 7.16 (2H, dd, J = 9 Hz, 9 Hz), 7.94 (2H, dd, J = 9 Hz, 6 Hz)

### Inventive Example 241

### N-{2-[4-(4-Fluorobenzoyl)piperidino]ethyl}-4-hydroxymethyl-N-(2-methoxyethyl)benzamide oxalate

Using N-{2-[4-(4-fluorobenzoyl)piperidino]ethyl}-4-hydroxymethyl-N-(2-methoxyethyl)benzamide (90 mg, 0.203 mmol), the procedure of Inventive Example 211 was repeated to obtain 105 mg (97.1%) of the title compound in a light beige amorphous powder form.
IR (KBr): 3392, 1678, 1626, 1598, 1412, 1278, 1216, 1158, 1112, 1078, 1014, 952, 854, 718 cm⁻¹
NMR (CDCl₃-CD₃OD) δ: 1.85 - 2.43 (4H, m), 2.90 - 4.30 (14H, m), 3.30 (3H, s), 4.67 (2H, s), 7.17 (2H, dd, J = 9 Hz, 9 Hz), 7.20 - 7.60 (4H, m), 7.95 (2H, dd, J = 9 Hz, 6 Hz)

### Inventive Example 242

### N-{2-[4-(4-Fluorobenzoyl)piperidino]ethyl}-N-(2-methoxyphenyl)-4-dimethylaminomethylbenzenesulfonamide oxalate

Using N-{2-[4-(4-fluorobenzoyl)piperidino]ethyl}-N-(2-methoxyphenyl)-4-dimethylaminomethylbenzenesulfonamide (33.5 mg, 0.0605 mmol), the procedure of Inventive Example 211 was repeated to obtain 35 mg (89.9%) of the title compound in a colorless amorphous powder form.
IR (KBr): 1680, 1599, 1497, 1341, 1220, 1158, 759, 591 cm⁻¹
NMR (CDCl₃-CD₃OD) δ: 1.63 - 2.35 (4H, m), 2.20 (6H, s), 2.40 - 3.59 (8H, m), 3.43 (3H, s), 3.65 - 4.15 (3H, m), 6.70 - 7.43 (7H, m), 7.45 - 7.80 (3H, m), 7.95 (2H, dd, J = 9 Hz, 6 Hz)

### Inventive Example 243

### N-(2-Methoxyphenyl)-N-{2-[4-(4-dimethylaminobenzoyl)piperidino]ethyl}-4-dimethylaminomethylbenzenesulfonamide oxalate

Using N-(2-methoxyphenyl)-N-{2-[4-(4-dimethylaminobenzoyl)piperidino]ethyl}-4-dimethylaminomethylbenzenesulfonamide (47.5 mg, 0.0812 mmol), the procedure of Inventive Example 211 was repeated to obtain 51 mg (92.9%) of the title compound in a creamy amorphous powder form.
IR (KBr): 1596, 1494, 1342, 1158, 944, 754, 588 cm⁻¹
NMR (CDCl₃-CD₃OD) δ: 1.70 - 2.15 (4H, m), 2.27 (6H, m), 2.52 (4H, s), 2.70 - 3.57 (4H, m), 3.05 (6H, s), 3.42 (3H, s), 3.70 - 4.10 (3H, m), 6.65 (2H, d, J = 9 Hz), 6.80 (1H, d, J = 8 Hz), 6.96 (1H, d, J = 8 Hz), 7.10 - 7.36 (2H, m), 7.36 - 8.00 (4H, m), 7.81 (2H, d, J = 9 Hz)

### Inventive Example 244

### 4-Carbamoyl-N-{2-[4-(4-fluorobenzoyl)piperidino]ethyl}-N-(2-methoxyphenyl)benzenesulfonamide oxalate

Using 4-carbamoyl-N-{2-[4-(4-fluorobenzoyl)piperidino]ethyl}-N-(2-methoxyphenyl)benzenesulfonamide (32 mg, 0.0593 mmol), the procedure of Inventive Example 211 was repeated to obtain 33 mg (88.4%) of the title compound in a beige amorphous powder form.
IR (KBr): 3460, 1678, 1598, 1496, 1408, 1348, 1282, 1220, 1166, 1118, 708, 600 cm⁻¹
NMR (CDCl₃-CD₃OD) δ: 1.90 - 2.40 (4H, m), 2.90 - 3.75 (10H, m), 3.80 - 4.20 (2H, m), 6.84 (2H, d, J = 9 Hz), 6.99 - 7.48 (4H, m), 7.55 - 7.77 (2H, m), 7.79 - 8.20 (4H, m)

### Inventive Example 245

### N-{2-[4-(4-Fluorobenzoyl)piperidino]ethyl}-N-(2-methoxyphenyl)-4-succinimidomethylbenzamide oxalate

Using N-{2-[4-(4-fluorobenzoyl)piperidino]ethyl}-N-(2-methoxyphenyl)-4-succinimidomethylbenzamide (42 mg, 0.0735 mmol), the procedure of Inventive Example 211 was repeated to obtain 33 mg (67.9%) of the title compound in a beige amorphous powder form.
IR (KBr): 3472, 2944, 1704, 1644, 1599, 1503, 1401, 1227, 1164 cm⁻¹
NMR (CDCl₃-CD₃OD) δ: 2.00 - 2.46 (6H, m), 2.66 (4H, s), 3.20 - 3.60 (5H, m), 3.68 (3H, s), 4.00 - 4.23 (2H, m), 4.52 (2H, s), 6.76 (1H, d, J = 8 Hz), 6.88 (1H, d, J = 7 Hz), 7.00 - 7.31 (8H, m), 7.92 (2H, dd, J = 9 Hz, 6 Hz)

### Inventive Example 246

### 4-Aminomethyl-N-{2-[4-(4-fluorobenzoyl)piperidino]ethyl}-N-(2-methoxyphenyl)benzenesulfonamide oxalate

Using 4-aminomethyl-N-{2-[4-(4-fluorobenzoyl)piperidino]ethyl}-N-(2-methoxyphenyl)benzenesulfonamide (47 mg, 0.0894 mmol), the procedure of Inventive Example 211 was repeated to obtain 52 mg (94.5%) of the title compound in a colorless amorphous powder form. IR (KBr): 2940, 1678, 1598, 1496, 1342, 1282, 1218, 1158, 760, 586 cm⁻¹ NMR (CDCl₃) δ: 1.40 - 2.25 (4H, m), 2.25 - 2.89 (4H, m), 2.90 - 3.55 (2H, m), 3.29 (3H, s), 3.56 - 5.40 (9H, m), 6.53 - 6.92 (2H, m), 6.92 - 7.34 (4H, m), 7.36 - 7.71 (4H, m), 7.73 - 8.10 (2H, m)

### Inventive Example 247

### 4-Aminomethyl-N-{2-[4-(4-fluorobenzoyl)piperidino]ethyl}-N-(2-methoxyethyl)benzamide oxalate

Using 4-aminomethyl-N-{2-[4-(4-fluorobenzoyl)piperidino]ethyl}-N-(2-methoxyethyl)benzamide (51 mg, 0.1155 mmol), the procedure of Inventive Example 211 was repeated to obtain 57.6 mg (93.8%) of the title compound in a light beige amorphous powder form.
IR (KBr): 3430, 2948, 1678, 1626, 1598, 1412, 1296, 1216, 1158, 1112, 1012, 952, 852, 762 cm⁻¹
NMR (CDCl₃-CD₃OD) δ: 1.70 - 2.30 (4H, m), 2.30 - 4.40 (13H, m), 3.35 (3H, s), 4.13 (2H, br-s), 6.93 - 7.70 (6H, m), 7.73 - 8.10 (2H, m)

### Inventive Example 248

### 4-Aminomethyl-N-{2-[4-(4-fluorobenzoyl)piperidino]ethyl}-N-(2-methoxyphenyl)benzamide oxalate

Using 4-aminomethyl-N-{2-[4-(4-fluorobenzoyl)piperidino]ethyl}-N-(2-methoxyphenyl)benzamide (32 mg, 0.0654 mmol), the procedure of Inventive Example 211 was repeated to obtain 33 mg (87.1%) of the title compound in a beige amorphous powder form.
IR (KBr): 3420, 2948, 1598, 1502, 1412, 1310, 1224, 1158, 758 cm⁻¹
NMR (CDCl₃) δ: 1.50 - 2.13 (4H, m), 2.13 - 4.50 (11H, m), 3.56 (3H, s), 6.40 - 6.82 (2H, m), 6.82 - 7.70 (8H, m), 7.72 - 8.20 (2H, m)

### Inventive Example 249

### 4-(Acetylamino)methyl-N-{2-[4-(4-fluorobenzoyl)piperidino]ethyl}-N-(2-methoxyethyl)benzamide oxalate

Using 4-(acetylamino)methyl-N-{2-[4-(4-fluorobenzoyl)piperidino]ethyl}-N-(2-methoxyethyl)benzamide (68 mg, 0.141 mmol), the procedure of Inventive Example 211 was repeated to obtain 71 mg (84.7%) of the title compound in a pink amorphous powder form.
IR (KBr): 3320, 2940, 1678, 1626, 1600, 1508, 1412, 1280, 1216, 1158, 1114, 1014, 952, 852, 706 cm⁻¹
NMR (CDCl₃-CD₃OD) δ: 1.80 - 2.36 (4H, m), 2.03 (3H, s), 2.80 - 4.10 (16H, m), 3.31 (3H, s), 4.42 (2H, s), 7.15 (2H, dd, J = 9 Hz, 9 Hz), 7.20 - 7.52 (4H, m), 7.95 (2H, dd, J = 9 Hz, 6 Hz)

### Inventive Example 250

### 4-(Acetylamino)methyl-N-{2-[4-(4-fluorobenzoyl)piperidino]ethyl}-N-(2-methoxyphenyl)benzamide oxalate

Using 4-(acetylamino)methyl-N-{2-[4-(4-fluorobenzoyl)piperidino]ethyl}-N-(2-methoxyphenyl)benzamide (58 mg, 0.109 mmol), the procedure of Inventive Example 211 was repeated to obtain 40 mg (59.0%) of the title compound in a colorless powder form.
Melting point: 159 - 161°C
IR (KBr): 3400, 1728, 1680, 1626, 1599, 1563, 1503, 1413, 1278, 1224 cm⁻¹
NMR (CDCl₃-CD₃OD) δ: 1.83 - 2.46 (4H, m), 1.99 (3H, s), 2.52 - 2.75 (1H, m), 3.15 - 4.80 (6H, m), 3.71 (3H, s), 3.90 - 4.40 (2H, m), 4.30 (2H, s), 6.62 - 7.38 (10H, m), 7.83 - 8.05 (2H, m)

### Inventive Example 251

### 4-(Trifluoroacetylamino)methyl-N-{2-[4-(4-fluorobenzoyl)piperidino]ethyl}-N-(2-methoxyphenyl)benzamide oxalate

Using 4-(trifluoroacetylamino)methyl-N-{2-[4-(4-fluorobenzoyl)piperidino]ethyl}-N-(2-methoxyphenyl)benzamide (48 mg, 0.0820 mmol), the procedure of Inventive Example 211 was repeated to obtain 37 mg (66.8%) of the title compound in a colorless powder form.
Melting point: 107 - 109°C
IR (KBr): 3406, 3064, 2944, 1719, 1680, 1641, 1599, 1503, 1221, 1179, 1158 cm⁻¹
NMR (CDCl₃) δ: 1.90 - 2.40 (4H, m), 3.10 - 3.80 (7H, m), 3.70 (3H, s), 3.95 - 4.28 (2H, m), 4.42 (2H, d, J = 6 Hz), 6.45 - 7.60 (13H, m), 7.91 (2H, dd, J = 9 Hz, 6 Hz)

### Inventive Example 252

### N-{2-[4-(4-Fluorobenzoyl)piperidino]ethyl}-4-(methanesulfonylamino)methyl-N-(2-methoxyphenyl)benzamide oxalate

Using N-{2-[4-(4-fluorobenzoyl)piperidino]ethyl}-4-(methanesulfonylamino)methyl-N-(2-methoxyphenyl)benzamide (40 mg, 0.0705 mmol), the procedure of Inventive Example 211 was repeated to obtain 40 mg (86.3%) of the title compound in a colorless powder form.
Melting point: 89 - 92°C
IR (KBr): 2926, 1722, 1677, 1641, 1599, 1503, 1440, 1410, 1320, 1278, 1221 cm⁻¹
NMR (CDCl₃-CD₃OD) δ: 1.80 - 2.55 (5H, m), 2.75 (3H, s), 3.10 - 3.85 (6H, m), 3.72 (3H, s), 3.97 - 4.40 (2H, m), 4.18 (2H, s), 6.76 (1H, d, J = 8 Hz), 6.92 (1H, d, J = 8 Hz), 7.00 - 7.42 (8H, m), (2H, dd, J = 9 Hz, 6 Hz)

### Inventive Example 253

### 4-(Ethoxycarbonylamino)methyl-N-{2-[4-(4-fluorobenzoyl)piperidino]ethyl}-N-(2-methoxyphenyl)benzamide oxalate

Using 4-(ethoxycarbonylamino)methyl-N-{2-[4-(4-fluorobenzoyl)piperidino]ethyl}-N-(2-methoxyphenyl)benzamide (50 mg, 0.0890 mmol), the procedure of Inventive Example 211 was repeated to obtain 47 mg (81.0%) of the title compound in a colorless powder form.
Melting point: 118 - 120°C
IR (KBr): 2940, 1710, 1682, 1640, 1598, 1502, 1414, 1278, 1226 cm⁻¹
NMR (CDCl₃) δ: 1.22 (3H, t, J = 8 Hz), 1.70 - 2.50 (8H, m), 3.20 - 3.75 (4H, m), 3.70 (3H, s), 4.11 (2H, q, J = 8 Hz), 4.00 - 4.30 (1H, m), 4.25 (2H, d, J = 6 Hz), 6.70 - 7.31 (10H, m), 7.91 (2H, dd, J = 9 Hz, 6 Hz)

### Inventive Example 254

### N-{2-[4-(4-Fluorobenzoyl)piperidino]ethyl}-N-(2-methoxyphenyl)-4-(methylamino)methylbenzamide oxalate

Using N-{2-[4-(4-fluorobenzoyl)piperidino]ethyl}-N-(2-methoxyphenyl)-4-(methylamino)methylbenzamide (119 mg, 0.236 mmol), the procedure of Inventive Example 211 was repeated to obtain 108 mg (77.0%) of the title compound in a colorless powder form.
Melting point: 174 - 176°C
IR (KBr): 3454, 3010, 2944, 2806, 1674, 1644, 1596, 1503, 1299, 1278 cm⁻¹
NMR (CDCl₃-CD₃OD) δ: 1.70 - 1.95 (4H, m), 2.16 - 2.80 (4H, m), 2.40 (3H, s), 2.85 - 3.42 (3H, m), 3.70 (3H, s), 3.83 (2H, s), 3.80 - 4.38 (2H, m), 6.65 - 6.89 (2H, m), 6.94 - 7.36 (8H, m), 7.93 (2H, dd, J = 9 Hz, 6 Hz)

### Inventive Example 255

### 4-Amidino-N-{2-[4-(4-fluorobenzoyl)piperidino]ethyl}-N-(2-methoxyphenyl)benzamide oxalate

Using 4-amidino-N-{2-[4-(4-fluorobenzoyl)piperidino]ethyl}-N-(2-methoxyphenyl)benzamide (89 mg, 0.177 mmol), the procedure of Inventive Example 211 was repeated to obtain 91 mg (86.8%) of the title compound in a colorless amorphous powder form.
IR (KBr): 3360, 3064, 1678, 1642, 1598, 1502, 1440, 1410, 1280, 1224, 1158, 720 cm⁻¹
NMR (CDCl₃-DMSO_{d6}) δ: 1.90 - 2.70 (4H, m), 3.00 - 5.00 (9H, m), 3.76 (3H, s), 6.58 - 7.00 (2H, m), 7.00 - 7.84 (9H, m), 7.85 - 8.25 (2H, m), 9.11 (2H, br-s), 9.53 (2H, br-s) MS (FAB, m/z): 120, 157, 220, 234, 296, 357, 503 (M⁺ + H)

### Inventive Example 256

### 4-(N-Acetylamidino)-N-{2-[4-(4-fluorobenzoyl)piperidino]ethyl}-N-(2-methoxyphenyl)benzamide oxalate

Using 4-(N-acetylamidino)-N-{2-[4-(4-fluorobenzoyl)piperidino]ethyl}-N-(2-methoxyphenyl)benzamide (80 mg, 0.147 mmol), the procedure of Inventive Example 211 was repeated to obtain 84 mg (90.0%) of the title compound in a light yellow amorphous powder form.
IR (KBr): 3262, 2938, 1644, 1599, 1503, 1443, 1410, 1278, 1224, 1158, 1119, 1086, 1044, 1020, 954, 852, 756, 720 cm⁻¹
NMR (CDCl₃) δ: 2.08 - 2.38 (4H, m), 2.54 (3H, s), 3.05 - 3.60 (7H, m), 3.70 (3H, s), 4.00 - 4.33 (2H, m), 6.55 - 6.93 (2H, m), 6.94 - 7.29 (5H, m), 7.37 (2H, d, J = 7.5 Hz), 7.64 (2H, d, J = 7.5 Hz), 7.93 (2H, dd, J = 9 Hz, 6 Hz), 8.73 (1H, br-s)

### Inventive Example 257

### N-{2-[4-(4-Fluorobenzoyl)piperidino]ethyl}-4-(N-methanesulfonylamidino)-N-(2-methoxyphenyl)benzamide oxalate

Using N-{2-[4-(4-fluorobenzoyl)piperidino]ethyl}-4-(N-methanesulfonylamidino)-N-(2-methoxyphenyl)benzamide (101 mg, 0.174 mmol), the procedure of Inventive Example 211 was repeated to obtain 100 mg (85.7%) of the title compound in a light yellow amorphous powder form.
IR (KBr): 3406, 1641, 1596, 1533, 1503, 1410, 1278, 1224, 1155, 1116, 957, 852, 756, 528 cm⁻¹
NMR (CDCl₃-CD₃OD) δ: 1.80 - 2.80 (4H, m), 3.03 (3H, s), 3.14 - 3.90 (7H, m), 3.69 (3H, s), 3.95 - 4.38 (2H, m), 6.74 (1H, d, J = 8 Hz), 6.84 (1H, d, J = 7.5 Hz), 6.95 - 7.45 (6H, m), 7.62 (2H, d, J = 8.5 Hz), 7.94 (2H, dd, J = 9 Hz, 6 Hz)

### Inventive Example 258

### 4-(N-Ethoxycarbonylamidino)-N-{2-[4-(4-fluorobenzoyl)piperidino]ethyl}-N-(2-methoxyphenyl)benzamide oxalate

Using 4-(N-ethoxycarbonylamidino)-N-{2-[4-(4-fluorobenzoyl)piperidino]ethyl}-N-(2-methoxyphenyl)benzamide (107 mg, 0.186 mmol), the procedure of Inventive Example 211 was repeated to obtain 113 mg (91.4%) of the title compound in a light yellow amorphous powder form.
IR (KBr): 3388, 1623, 1599, 1503, 1461, 1407, 1368, 1299, 1263, 1224, 1158, 1140, 1119, 1020, 954, 855, 756, 720 cm⁻¹
NMR (CDCl₃-CD₃OD) δ: 1.31 (3H, t, J = 7 Hz), 1.85 - 2.34 (4H, m), 2.95 - 3.90 (7H, m), 3.70 (3H, s), 3.93 - 4.33 (2H, m), 4.17 (2H, q, J = 7 Hz), 6.75 (1H, d, J = 8 Hz), 6.84 (1H, d, J = 7.5 Hz), 6.96 - 7.45 (6H, m), 7.66 (2H, d, J = 8 Hz), 7.96 (2H, dd, J = 9 Hz, 6 Hz)

### Inventive Example 259

### 4-Nitro-N-{2-[4-(4-fluorobenzoyl)piperidino]ethyl}-N-(2-methoxyphenyl)benzamide oxalate

Using 4-nitro-N-{2-[4-(4-fluorobenzoyl)piperidino]ethyl}-N-(2-methoxyphenyl)benzamide (120 mg, 0.24 mmol), the procedure of Inventive Example 211 was repeated to obtain 106 mg (75.0%) of the title compound in a light yellow powder form.
Melting point: 201 - 203°C
IR (KBr): 2944, 2608, 1686, 1656, 1599, 1524, 1503, 1410, 1347, 1278, 1227, 1155 cm⁻¹
NMR (CDCl₃-CD₃OD) δ: 1.90 - 2.24 (4H, m), 3.10 - 3.65 (7H, m), 3.73 (3H, s), 4.04 (2H, m), 6.70 - 6.96 (2H, m), 7.00 - 7.33 (4H, m), 7.45 (2H, d, J = 9.0 Hz), 7.84 - 8.06 (4H, m)

### Inventive Example 260

### 4-{[N-[2-[4-(4-Fluorobenzoyl)piperidino]ethyl]-2-methoxyanilino]carbonyl}pyridine oxalate

Using 4-{[N-[2-[4-(4-fluorobenzoyl)piperidino]ethyl}-2-methoxyanilino]carbonyl}pyridine (200 mg, 0.43 mmol), the procedure of Inventive Example 211 was repeated to obtain 221 mg (92.5%) of the title compound in a colorless powder form.
Melting point: 98 - 100°C
IR (KBr): 1678, 1654, 1620, 1598, 1502, 1434, 1412, 1396, 1224 cm⁻¹
NMR (CDCl₃-CD₃OD) δ: 1.85 - 2.54 (6H, m), 2.86 - 3.64 (6H, m), 3.70 (3H, s), 3.90 - 4.22 (1H, m), 6.66 - 6.94 (2H, m), 7.01 - 7.22 (6H, m), 7.81 - 8.04 (2H, m), 8.37 (2H, d, J = 6.0 Hz)

### Inventive Example 261

### 4-{[N-[2-[4-(4-Fluorobenzoyl)piperidino]ethyl]-2-methoxyanilino]carbonyl}pyridine N-oxide oxalate

Using 4-{[N-[2-[4-(4-fluorobenzoyl)piperidino]ethyl]-2-methoxyanilino]carbonyl}pyridine N-oxide (269 mg, 0.56 mmol), the procedure of Inventive Example 211 was repeated to obtain 259 mg (81.2%) of the title compound in a light yellow powder form.
Melting point: 121 - 124°C
IR (KBr): 2920, 2848, 1680, 1647, 1614, 1596, 1503, 1272, 1224, 1158, 843 cm⁻¹
NMR (CDCl₃-CD₃OD) δ: 2.00 - 2.31 (4H, m), 3.13 - 3.69 (8H, m), 3.72 (3H, s), 4.00 - 4.23 (1H, m), 6.74 - 7.31 (8H, m), 7.82 - 8.03 (4H, m)

### Inventive Example 262

### 3-[4-(4-Fluorobenzoyl)piperidino]-N-(4-methoxybenzenesulfonyl)propionamidine oxalate

Using 3-[4-(4-fluorobenzoyl)piperidino]-N-(4-methoxybenzenesulfonyl)propionamidine (51 mg, 0.114 mmol), the procedure of Inventive Example 211 was repeated to obtain 50 mg (81.6%) of the title compound in a colorless powder form.
Melting point: 110 - 114°C
IR (KBr): 1680, 1638, 1598, 1500, 1412, 1260, 1226, 1140, 1086, 806, 606, 564 cm⁻¹
NMR (CDCl₃-CD₃OD) δ: 1.80 - 2.25 (4H, m), 2.60 - 3.75 (11H, m), 3.85 (3H, s), 7.00 (2H, d, J = 9 Hz), 7.19 (2H, dd, J = 9 Hz, 9 Hz), 7.84 (2H, d, J = 9 Hz), 8.00 (2H, dd, J = 9 Hz, 6 Hz)

### Inventive Example 263

### 3-[4-(4-Fluorophenyl)piperazinyl]-N-(4-methoxybenzenesulfonyl)propionamidine oxalate

Using 3-[4-(4-fluorophenyl)piperazinyl]-N-(4-methoxybenzenesulfonyl)propionamidine (82 mg, 0.195 mmol), the procedure of Inventive Example 211 was repeated to obtain 91 mg (91.4%) of the title compound in a colorless flocculent crystal form.
Melting point: 143 - 147°C
IR (KBr): 3368, 3080, 2845, 1620, 1512, 1454, 1310, 1286, 1260, 1140, 1114, 1086, 1028, 826, 806, 768, 568 cm⁻¹
NMR (CD₃OD-DMSO_{d6}) δ: 2.82 - 3.40 (12H, m), 3.83 (3H, s), 6.80 - 7.16 (1H, d, J = 9 Hz)

### Inventive Example 264

### 3-[4-(4-Fluorobenzoyl)piperidino]-N-(3-methoxybenzyl)propionamidine oxalate

Using 3-[4-(4-fluorobenzoyl)piperidino]-N-(3-methoxybenzyl)propionamidine (115 mg, 0.289 mmol), the procedure of Inventive Example 211 was repeated to obtain 129 mg (91.6%) of the title compound in a light yellow amorphous powder form.
IR (KBr): 2948, 1680, 1598, 1492, 1454, 1412, 1298, 1268, 1228, 1158, 1042, 764 cm⁻¹
NMR (CDCl₃) δ: 1.40 - 2.00 (4H, m), 2.20 - 3.50 (8H, m), 3.55 - 4.75 (3H, m), 3.73 (3H, s), 4.46 (2H, br-s), 6.60 - 7.37 (6H, m), 7.93 (2H, dd, J = 9 Hz, 6 Hz)

### Inventive Example 265

### 3-[4-(4-Fluorophenyl)piperazinyl]-N-(3-methoxybenzyl)propionamidine oxalate

Using 3-[4-(4-fluorophenyl)piperazinyl]-N-(3-methoxybenzyl)propionamidine (36 mg, 0.0972 mmol), the procedure of Inventive Example 211 was repeated to obtain 40.3 mg (90.0%) of the title compound in a creamy amorphous powder form.
IR (KBr): 1604, 1510, 1456, 1298, 1266, 1232, 1162, 1040, 826, 780, 694 cm⁻¹
NMR (CDCl₃-CD₃OD) δ: 2.45 - 3.00 (10H, m), 3.50 - 4.06 (2H, m), 3.70 (3H, s), 4.41 (2H, s), 6.66 - 7.43 (8H, m)

### Inventive Example 266

### 4-[(S)-2-Aminopropionyl]aminomethyl-N-{2-[4-(4-fluorobenzoyl)piperidino]ethyl}-N-(2-methoxyphenyl)benzamide dioxalate

4-[(S)-2-Aminopropionyl]aminomethyl-N-{2-[4-(4-fluorobenzoyl)piperidino]ethyl}-N-(2-methoxyphenyl)benzamide (84.7 mg, 0.155 mmol) was dissolved in methanol (5 ml) and mixed with oxalic acid (28 mg, 0.311 mmol). After 5 minutes of stirring at room temperature, methanol was removed by evaporation, and the resulting amorphous residue was washed with acetone (5 ml) and collected by filtration to obtain 84 mg (85.1%) of the title compound in a colorless amorphous powder form.
IR (KBr): 3076, 1718, 1682, 1636, 1598, 1502, 1440, 1412, 1278, 1224 cm⁻¹
NMR (CDCl₃-CD₃OD) δ: 1.25 - 1.55 (3H, m), 2.03 - 2.30 (4H, m), 3.25 - 3.90 (9H, m), 3.74 (3H, s), 4.00 - 4.42 (4H, m), 6.78 - 7.41 (10H, m), 7.92 - 8.15 (2H, m) MS (FAB, m/z): 220, 354, 561 M⁺ + 1)

### Inventive Example 267

### 4-Acetylamino-N-{2-[4-(4-fluorobenzoyl)piperidino]ethyl}-N-(2-methoxyphenyl)benzamide oxalate

Using 4-acetylamino-N-{2-[4-(4-fluorobenzoyl)piperidino]ethyl}-N-(2-methoxyphenyl)benzamide (135.0 mg, 0.26 mmol), the procedure of Inventive Example 211 was repeated to obtain 108.0 mg (68.2%) of the title compound in a colorless powder form.
Melting point: 190 - 192°C
IR (KBr): 3364, 2926, 2854, 1731, 1680, 1644, 1598, 1530, 1407, 1377, 1311, 1278, 1227, 1182, 1020 cm⁻¹
NMR (CDCl₃-CD₃OD) δ: 2.07 (3H, s), 2,10 - 2.30 (4H, m), 3.10 - 3.64 (8H, m), 3.70 (3H, s), 4.00 - 4.26 (2H, m), 6.70 - 6.93 (2H, m), 6.96 - 7.45 (8H, m), 7.98 (2H, dd, J = 9.0 Hz, 6.0 Hz)

### Inventive Example 268

### 4-Methanesulfonylamino-N-{2-[4-(4-fluorobenzoyl)piperidino]ethyl}-N-(2-methoxyphenyl)benzamide oxalate

Using 4-methanesulfonylamino-N-{2-[4-(4-fluorobenzoyl)piperidino]ethyl}-N-(2-methoxyphenyl)benzamide (29.5 mg, 0.05 mmol), the procedure of Inventive Example 211 was repeated to obtain 20.7 mg (60.3%) of the title compound in a colorless powder form.
Melting point: 75 - 76°C
IR (KBr): 3364, 2930, 1680, 1641, 1599, 1503, 1443, 1395, 1332, 1302, 1281, 1152 cm⁻¹
NMR (CDCl₃-CD₃OD) δ: 1.90 - 2.33 (4H, m), 2.90 (3H, s), 3.10 - 3.80 (8H, m), 3.72 (3H, s), 3.90 - 4.30 (2H, m), 6.60 - 7.40 (10H, m), 7.96 (2H, dd, J = 9.0 Hz, 6.0 Hz)

### Inventive Example 269

### 4-Acetylamino-N-{2-[4-(4-fluorobenzoyl)piperidino]ethyl}-N-(3-methoxyphenyl)benzamide fumarate

4-Acetylamino-N-{2-[4-(4-fluorobenzoyl)piperidino]ethyl}-N-(3-methoxyphenyl)benzamide (282.4 mg, 0.55 mmol) was dissolved in methanol (1.5 ml) and mixed with a methanol solution (3.0 ml) of fumaric acid (63.8 mg, 0.55 mmol) at 0°C. Thereafter, the thus precipitated crystals were collected by filtration and washed with ether to obtain 278.3 mg (80.0%) of the title compound in a colorless powder form.
Melting point: 215 - 222°C (decomposition)
IR (KBr): 3450, 1680, 1644, 1600, 1528, 1408, 1316, 1218, 1160, 852 cm⁻¹
NMR (CDCl₃-CD₃OD) δ: 1.60 - 2.43 (5H, m), 2.10 (3H, s), 2.68 (2H, t, J = 7.0 Hz), 2.83 - 3.40 (4H, m), 3.69 (3H, s), 4.05 (2H, t, J = 7.0 Hz), 6.52 - 6.73 (3H, m), 6.78 (2H, s), 6.52 - 7.50 (5H, m), 7.13 (2H, dd, J = 8.8 Hz), 7.98 (2H, dd, J = 8.8 Hz, 5.5 Hz)

### Inventive Example 270

### 4-Ethoxycarbonylamino-N-{2-[4-(4-fluorobenzoyl)piperidino]ethyl}-N-(2-methoxyphenyl)benzamide fumarate

Using 4-ethoxycarbonylamino-N-{2-[4-(4-fluorobenzoyl)piperidino]ethyl}-N-(2-methoxyphenyl)benzamide (80.0 mg, 0.15 mmol), the procedure of Inventive Example 269 was repeated to obtain 74.6 mg (77.0%) of the title compound in a light brown powder form.
Melting point: 142 - 146°C
IR (KBr): 2950, 1724, 1578, 1638, 1598, 1500, 1410, 1314, 1226, 1190 cm⁻¹
NMR (CDCl₃-CD₃OD) δ: 1.27 (3H, t, J = 7.3 Hz), 1.80 - 2.20 (5H, m), 2.40 - 3.43 (4H, m), 3.68 (3H, s), 3.93 - 4.30 (2H, m), 4.17 (2H, q, J = 7.3 Hz), 6.73 (2H, s), 6.85 (2H, t, J = 7.5), 6.90 - 7.33 (9H, m), 7.94 (2H, dd, J = 8.8 Hz, 5.5 Hz)

### Inventive Example 271

### 4-Trifluoroacetylamino-N-{2-[4-(4-fluorobenzoyl)piperidino]ethyl}-N-(2-methoxyphenyl)benzamide fumarate

Using 4-trifluoroacetylamino-N-{2-[4-(4-fluorobenzoyl)piperidino]ethyl}-N-(2-methoxyphenyl)benzamide (253.3 mg, 0.42 mmol), the procedure of Inventive Example 269 was repeated to obtain 167.7 mg (61.0%) of the title compound in a colorless powder form.
Melting point: 213 - 215°C (decomposition)
IR (KBr): 2950, 1720, 1680, 1640, 1598, 1500, 1410, 1385, 1240, 1200, 1150, 950 cm⁻¹
NMR (CDCl₃-CD₃OD) δ: 1.70 - 2.06 (5H, m), 2.20 - 3.46 (6H, m), 3.70 (3H, s), 3.76 - 4.26 (2H, m), 6.73 (2H, s), 6.81 (2H, t, J = 6.8 Hz), 6.93 - 7.51 (9H, m), 7.95 (2H, dd, J = 8.8 Hz, 5.5 Hz)

### Inventive Example 272

### 4-(3-Ethylthioureido)-N-{2-[4-(4-fluorobenzoyl)piperidino]ethyl}-N-(2-methoxyphenyl)benzamide fumarate

Using 4-(ethylthioureido)-N-{2-[4-(4-fluorobenzoyl)piperidino]ethyl}-N-(2-methoxyphenyl)benzamide (95.5 mg, 0.17 mmol), the procedure of Inventive Example 269 was repeated to obtain 87.9 mg (76.3%) of the title compound in a colorless powder form.
Melting point: 154 - 156°C
IR (KBr): 1678, 1640, 15982, 1544, 1502, 1420, 1382, 1314, 1278, 1226 cm⁻¹
NMR (CDCl₃-CD₃OD) δ: 1.16 (3H, t, J = 7.1 Hz), 1.70 - 2.12 (4H, m), 2.20 - 3.46 (11H, m), 3.72 (3H, s), 6.78 (2H, s), 6.60 - 7.40 (10H, m), 7.80 - 8.10 (2H, m)

### Inventive Example 273

### 4-tert-Butylureido)-N-{2-[4-(4-fluorobenzoyl)piperidino]ethyl}-N-(2-methoxyphenyl)benzamide fumarate

Using 4-(tert-butylureido)-N-{2-[4-(4-fluorobenzoyl)piperidino]ethyl}-N-(2-methoxyphenyl)benzamide (72.9 mg, 0.13 mmol), the procedure of Inventive Example 269 was repeated to obtain 84.2 mg (96.1%) of the title compound in a light yellow powder form.
Melting point: 110 - 114°C
IR (KBr): 2968, 1683, 1638, 1599, 1536, 1503, 1452, 1410, 1392, 1312, 1278, 1251, 1209, 1179, 1158, 1119, 975, 954, 846, 753 cm⁻¹
NMR (CDCl₃-CD₃OD) δ: 1.32 (9H, s), 1.77 - 2.20 (4H, m), 2.20 - 3.55 (8H, m), 3.69 (3H, s), 3.83 - 4.20 (1H, m), 6.66 - 7.32 (10H, m), 6.78 (2H, s), 7.80 - 8.03 (2H, m)

### Inventive Example 274

### 4-Amino-N-{2-[4-(4-fluorobenzoyl)piperidino]ethyl}-N-(2-methoxyphenyl)benzamide fumarate

Using 4-amino-N-{2-[4-(4-fluorobenzoyl)piperidino]ethyl}-N-(2-methoxyphenyl)benzamide (150.0 mg, 0.32 mmol), the procedure of Inventive Example 269 was repeated to obtain 151.6 mg (81.2%) of the title compound in a light yellow powder form.
Melting point: 152 - 158°C
IR (KBr): 1678, 1632, 1600, 1500, 1388, 1224, 1182, 1160, 756 cm⁻¹
NMR (CDCl₃-CD₃OD) δ: 1.76 - 2.03 (4H, m), 2.30 - 2.60 (2H, m), 2.70 - 3.00 (2H, m), 3.00 - 3.47 (3H, m), 3.69 (3H, s), 3.80 - 4.22 (2H, m), 6.37 (2H, d, J = 8.0 Hz), 6.70 - 6.90 (4H, m), 6.98 - 7.30 (6H, m), 7.80 - 8.07 (2H, m)

### Inventive Example 275

### 4-Isobutylylamino-N-{2-[4-(4-fluorobenzoyl)piperidino]ethyl}-N-(2-methoxyphenyl)benzamide fumarate

Using 4-isobutylylamino-N-{2-[4-(4-fluorobenzoyl)piperidino]ethyl}-N-(2-methoxyphenyl)benzamide (222.7 mg, 0.41 mmol), the procedure of Inventive Example 269 was repeated to obtain 268.0 mg (99.2%) of the title compound in a light pink powder form.
Melting point: 154 - 157°C
IR (KBr): 2968, 1683, 1641, 1599, 1530, 1503, 1455, 1410, 1386, 1305, 1278, 1251, 1224, 1179, 1155, 1116, 1020, 975, 957, 849, 753 cm⁻¹
NMR (CDCl₃-CD₃OD) δ: 1.18 (6H, d, J = 5.3 Hz), 1.75 - 2.00 (4H, m), 2.20 - 3.50 (8H, m), 3.69 (3H, s), 3.80 - 4.20 (2H, m), 6.67 - 6.93 (4H, m), 7.00 - 7.40 (8H, m), 7.83 - 8.03 (2H, m)

### Inventive Example 276

### 4-Butylamino-N-{2-[4-(4-fluorobenzoyl)piperidino]ethyl}-N-(2-methoxyphenyl)benzamide fumarate

Using 4-butylamino-N-{2-[4-(4-fluorobenzoyl)piperidino]ethyl}-N-(2-methoxyphenyl)benzamide (196.3 mg, 0.36 mmol), the procedure of Inventive Example 269 was repeated to obtain 227.8 mg (95.7%) of the title compound in a light orange powder form.
Melting point: 155 - 160°C
IR (KBr): 1683, 1638, 1599, 1530, 1503, 1455, 1407, 1386, 1308, 1251, 1224, 1203, 1179, 1155, 957, 852, 750 cm⁻¹
NMR (CDCl₃-CD₃OD) δ: 0.98 (3H, t, J = 8.0 Hz), 1.47 - 2.07 (6H, m), 2.10 - 3.48 (9H, m), 3.69 (3H, s), 3.85 - 4.30 (2H, m), 6.67 - 6.93 (4H, m), 7.00 - 7.40 (8H, m), 7.80 - 8.10 (2H, m)

### Inventive Example 277

### 4-Amino-N-{2-[4-(4-fluorobenzoyl)piperidino]ethyl}-N-(4-methoxyphenyl)benzamide fumarate

Using 4-amino-N-{2-[4-(4-fluorobenzoyl)piperidino]ethyl}-N-(4-methoxyphenyl)benzamide (132.9 mg, 0.28 mmol), the procedure of Inventive Example 269 was repeated to obtain 136.3 mg (82.4%) of the title compound in a light yellow amorphous form.
IR (KBr): 1680, 1600, 1510, 1440, 1386, 1292, 1248, 1226, 1182, 1158, 838 cm⁻¹
NMR (CDCl₃-CD₃OD) δ: 1.72 - 2.10 (4H, m), 2.30 - 2.70 (2H, m), 2.70 - 3.23 (2H, m), 3.23 - 3.58 (3H, m), 3.77 (3H, s), 4.08 (2H, t, J = 7.0 Hz), 6.42 (2H, d, J = 9.0 Hz), 6.60 - 7.33 (8H, m), 6.77 (2H, s), 7.80 - 8.10 (2H, m)

### Inventive Example 278

### 4-Acetylamino-N-{2-[4-(4-fluorobenzoyl)piperidino]ethyl}-N-(4-methoxyphenyl)benzamide fumarate

Using 4-acetylamino-N-{2-[4-(4-fluorobenzoyl)piperidino]ethyl}-N-(4-methoxyphenyl)benzamide (476.8 mg, 0.92 mmol), the procedure of Inventive Example 269 was repeated to obtain 515.6 mg (88.3%) of the title compound in a colorless powder form.
Melting point: 202 - 208°C
IR (KBr): 1683, 1641, 1596, 1512, 1443, 1410, 1386, 1317, 1293, 1257, 1227, 1179, 1158 cm⁻¹
NMR (CDCl₃-CD₃OD) δ: 1.70 - 2.03 (4H, m), 2.13 (3H, s), 2.20 - 2.92 (4H, m), 2.92 - 3.30 (3H, m), 3.78 (3H, s), 4.08 (2H, t, J = 8.0 Hz), 6.60 - 7.47 (10H, m), 6.82 (2H, s), 7.80 - 8.10 (2H, m)

### Inventive Example 279

### 4-Pivaloylamino-N-{2-[4-(4-fluorobenzoyl)piperidino]ethyl}-N-(2-methoxyphenyl)benzamide fumarate

Using 4-pivaloylamino-N-{2-[4-(4-fluorobenzoyl)piperidino]ethyl}-N-(2-methoxyphenyl)benzamide (214.6 mg, 0.38 mmol), the procedure of Inventive Example 269 was repeated to obtain 263.9 mg (quantitative) of the title compound in a light yellow amorphous form.
IR (KBr): 1680, 1641, 1599, 1503, 1443, 1404, 1314, 1278, 1245, 1224, 1158, 975, 849, 753 cm⁻¹
NMR (CDCl₃-CD₃OD) δ: 1.26 (9H, s), 1.70 - 2.03 (4H, m), 2.40 - 3.40 (7H, m), 3.69 (3H, s), 3.80 - 4.20 (2H, m), 6.60 - 6.90 (2H, m), 6.75 (2H, s), 6.90 - 7.40 (8H, m), 7.80 - 8.04 (2H, m)

### Inventive Example 280

### 4-Amino-N-{2-[4-(4-fluorobenzoyl)piperidino]ethyl}-N-(2,5-dimethoxyphenyl)benzamide fumarate

Using 4-amino-N-{2-[4-(4-fluorobenzoyl)piperidino]ethyl}-N-(2,5-dimethoxyphenyl)benzamide (152.0 mg, 0.30 mmol), the procedure of Inventive Example 269 was repeated to obtain 175.0 mg (80.4%) of the title compound in a yellow powder form.
Melting point: 199.5 - 200.8°C
IR (KBr): 3470, 3380, 2951, 1683, 1641, 1617, 1514, 1223, 850, 652 cm⁻¹
NMR (CDCl₃-CD₃OD) δ: 1.95 - 2.16 (5H, m), 2.18 - 3.80 (6H, m), 3.61 (3H, s), 3.69 (3H, s), 3.80 - 4.20 (2H, m), 6.35 (2H, d, J = 7.8 Hz), 6.72 (5H, s), 7.08 - 7.26 (4H, m), 7.91 - 8.06 (2H, m)

### Inventive Example 281

### 4-Acetylamino-N-{2-[4-(4-fluorobenzoyl)piperidino]ethyl}-N-(2,5-dimethoxyphenyl)benzamide fumarate

Using 4-acetylamino-N-{2-[4-(4-fluorobenzoyl)piperidino]ethyl}-N-(2,5-dimethoxyphenyl)benzamide (109.0 mg, 0.20 mmol), the procedure of Inventive Example 269 was repeated to obtain 111.0 mg (84.3%) of the title compound in a colorless powder form.
Melting point: 207 - 208°C
IR (KBr): 3460, 1945, 1679, 1640, 1598, 1506, 1316, 1223, 855 cm⁻¹
NMR (CDCl₃-CD₃OD) δ: 1.94 - 2.15 (4H, m), 2.09 (3H, s), 2.61 - 3.53 (7H, m), 3.61 (3H, s), 3.69 (3H, s), 4.01 (2H, brs), 6.71 (2H, d, J = 4.8 Hz), 7.05 - 7.41 (7H, m), 7.89 - 8.04 (2H, m)

### Inventive Example 282

### 4-Amino-N-{2-[4-(4-fluorobenzoyl)piperidino]ethyl}-N-(3-methoxyphenyl)benzamide fumarate

Using 4-amino-N-{2-[4-(4-fluorobenzoyl)piperidino]ethyl}-N-(3-methoxyphenyl)benzamide (185.1 mg, 0.39 mmol), the procedure of Inventive Example 269 was repeated to obtain 215.8 mg (93.8%) of the title compound in a colorless powder form.
Melting point: 155 - 157°C
IR (KBr): 3444, 3360, 1680, 1628, 1600, 1516, 1440, 1384, 1314, 1214, 1180, 1042 cm⁻¹
NMR (CDCl₃-CD₃OD) δ: 1.80 - 2.23 (5H, m), 2.56 - 3.60 (8H, m), 3.70 (3H, s), 4.18 (2H, t, J = 7.5 Hz), 6.42 (2H, d, J = 8.6 Hz), 6.53 - 6.72 (2H, m), 6.75 (2H, s), 7.00 - 7.30 (6H, m), 6.75 (2H, s), 7.95 (2H, dd, J = 8.8 Hz, 5.5 Hz)

### Inventive Example 283

### 3-Amino-N-{2-[4-(4-fluorobenzoyl)piperidino]ethyl}-N-(3-methoxyphenyl)benzamide fumarate

Using 3-amino-N-{2-[4-(4-fluorobenzoyl)piperidino]ethyl}-N-(3-methoxyphenyl)benzamide (153.7 mg, 0.32 mmol), the procedure of Inventive Example 269 was repeated to obtain 176.9 mg (93.5%) of the title compound in a light yellow amorphous form.
IR (KBr): 3460, 3364, 1680, 1638, 1598, 1456, 1318, 1234, 1158, 1080, 980 cm⁻¹
NMR (CDCl₃-CD₃OD) δ: 1.80 - 2.16 (5H, m), 2.56 - 3.50 (8H, m), 3.68 (3H, s), 4.11 (2H, t, J = 5.7 Hz), 6.40 - 7.33 (10H, m), 6.75 (2H, s), 7.96 (2H, dd, J = 8.8 Hz, 5.5 Hz)

### Inventive Example 284

### 3-Acetylamino-N-{2-[4-(4-fluorobenzoyl)piperidino]ethyl}-N-(3-methoxyphenyl)benzamide fumarate

Using 3-acetylamino-N-{2-[4-(4-fluorobenzoyl)piperidino]ethyl}-N-(3-methoxyphenyl)benzamide (197.5 mg, 0.38 mmol), the procedure of Inventive Example 269 was repeated to obtain 176.6 mg (73.6%) of the title compound in a light red powder form.
Melting point: 122 - 125°C
IR (KBr): 3300, 2930, 1676, 1640, 1598, 1456, 1318, 1234, 1158, 1080, 980 cm⁻¹
NMR (CDCl₃-CD₃OD) δ: 1.73 - 2.20 (5H, m), 2.12 (3H, s), 2.33 - 2.75 (2H, m), 2.83 (2H, t, J = 7.5 Hz), 2.95 - 3.46 (2H, m), 3.68 (3H, s), 4.12 (2H, t, J = 7.5 Hz), 6.53 - 6.90 (3H, m), 6.76 (2H, s), 6.93 - 7.43 (6H, m), 7.73 (1H, d, J = 7.5 Hz), 7.96 (2H, dd, J = 9.0 Hz, 6.0 Hz)

### Inventive Example 285

### 4-Amino-N-{2-[4-(4-fluorobenzoyl)piperidino]ethyl}-N-(2,5-dimethoxyphenyl)benzamide fumarate

Using 4-amino-N-{2-[4-(4-fluorobenzoyl)piperidino]ethyl}-N-(2,5-dimethoxyphenyl)benzamide (102.3 mg, 0.20 mmol), the procedure of Inventive Example 269 was repeated to obtain 92.4 mg (74.3%) of the title compound in a colorless amorphous form.
Melting point: 105 - 106°C
IR (KBr): 1710, 1680, 1600, 1510, 1438, 1410, 1308, 1278, 1224, 1208, 1182, 1158 cm⁻¹
NMR (CDCl₃-CD₃OD) δ: 2.17 - 2.20 (4H, m), 2.25 - 3.91 (15H, m), 3.69 (3H, s), 3.77 (3H, s), 6.38 - 6.51 (3H, m), 6.81 (2H, s), 7.00 - 7.34 (5H, m), 7.99 - 8.09 (2H, m)

### Inventive Example 286

### 4-Acetylamino-N-{2-[4-(4-fluorobenzoyl)piperidino]ethyl}-N-(2,5-dimethoxyphenyl)benzamide fumarate

Using 4-acetylamino-N-{2-[4-(4-fluorobenzoyl)piperidino]ethyl}-N-(2,5-dimethoxyphenyl)benzamide (102.1 mg, 0.19 mmol), the procedure of Inventive Example 269 was repeated to obtain 80.0 mg (64.7%) of the title compound in a colorless amorphous form.
Melting point: 109 - 110°C
IR (KBr): 1681, 1636, 1597, 1529, 1510, 1439, 1406, 1312, 1258, 1223, 1208, 1181, 1158, 1027, 851 cm⁻¹
NMR (CDCl₃-CD₃OD) δ: 1.93 - 2.06 (4H, m), 2.27 (3H, s), 2.35 - 3.25 (2H, m), 3.27 - 4.84 (8H, m), 3.69 (3H, s), 3.77 (3H, s), 6.38 - 6.51 (3H, m), 6.81 (2H, s), 7.00 - 7.34 (5H, m), 7.00 - 7.34 (5H, m), 7.99 - 8.09 (2H, m)

### Inventive Example 287

### 4-Amino-N-{2-[4-(4-fluorobenzoyl)piperidino]ethyl}-N-(3-methylphenyl)benzamide fumarate

Using 4-amino-N-{2-[4-(4-fluorobenzoyl)piperidino]ethyl}-N-(3-methylphenyl)benzamide (184.5 mg, 0.40 mmol), the procedure of Inventive Example 269 was repeated to obtain 72.0 mg (31.2%) of the title compound in a light yellow amorphous form.
IR (KBr): 3330, 2935, 2450, 1680, 1629, 1599, 1440, 1380, 1302, 1230, 1155, 843 cm⁻¹
NMR (CDCl₃-CD₃OD) δ: 1.15 - 2.10 (6H, m), 2.26 (3H, s), 2.34 - 3.50 (7H, m), 4.10 (2H, t, J = 7.0 Hz), 6.40 (2H, d, J = 9.0 Hz), 6.77 (2H, s), 6.61 - 7.31 (8H, m), 7.94 (2H, dd, J = 9.0 Hz, 6.0 Hz)

### Inventive Example 288

### 4-Acetylamino-N-{2-[4-(4-fluorobenzoyl)piperidino]ethyl}-N-(3-methylphenyl)benzamide fumarate

Using 4-acetylamino-N-{2-[4-(4-fluorobenzoyl)piperidino]ethyl}-N-(3-methylphenyl)benzamide (236.0 mg, 0.47 mmol), the procedure of Inventive Example 269 was repeated to obtain 246.0 mg (84.8%) of the title compound in a colorless powder form.
Melting point: 196 - 202°C
IR (KBr): 3250, 2400, 1683, 1644, 1599, 1530, 1407, 1380, 1230, 1161, 849 cm⁻¹
NMR (CDCl₃-CD₃OD) δ: 2.11 (3H, s), 2.26 (3H, s), 1.50 - 2.60 (6H, m), 2.76 (2H, t, J = 7.0 Hz), 2.93 - 3.53 (3H, m), 4.09 (2H, t, J = 7.0 Hz), 6.80 (2H, s), 6.70 - 7.43 (11H, m), 7.95 (2H, dd, J = 9.0 Hz, 6.0 Hz)

### Inventive Example 289

### 4-Acetylamino-N-{2-[4-(4-fluorobenzoyl)piperidino]ethyl}-N-(phenyl)benzamide fumarate

Using 4-acetylamino-N-{2-[4-(4-fluorobenzoyl)piperidino]ethyl}-N-(phenyl)benzamide (314.3 mg, 0.64 mmol), the procedure of Inventive Example 269 was repeated to obtain 349.8 mg (90.5%) of the title compound in a colorless powder form.
Melting point: 210 - 214°C
IR (KBr): 1686, 1641, 1599, 1527, 1497, 1452, 1410, 1389, 1314, 1260, 1227, 1185, 1158, 852, 759 cm⁻¹
NMR (CDCl₃-CD₃OD) δ: 1.86 - 1.97 (6H, m), 2.09 (3H, s), 2.28 - 2.55 (4H, m), 2.66 - 2.94 (4H, m), 3.04 - 3.08 (2H, m), 4.11 (2H, dd, J = 7.0 Hz, 6.6 Hz), 6.78 (2H, s), 7.06 - 7.42 (9H, m), 7.93 (2H, dd, J = 9.0 Hz, 6.0 Hz)

### Inventive Example 290

### 4-Amino-N-{2-[4-(4-fluorobenzoyl)piperidino]ethyl}-N-(3-methylthiophenyl)benzamide fumarate

Using 4-amino-N-{2-[4-(4-fluorobenzoyl)piperidino]ethyl}-N-(3-methylthiophenyl)benzamide (148.2 mg, 0.30 mmol), the procedure of Inventive Example 269 was repeated to obtain 488.0 mg (80.3%) of the title compound in a colorless powder form.
Melting point: 224 - 227°C
IR (KBr): 1710, 1680, 1600, 1512, 1312, 1204, 1192, 836 cm⁻¹
NMR (CDCl₃-CD₃OD) δ: 2.17 - 2.20 (4H, m), 2.43 (3H, s), 3.29 - 4.09 (15H, m), 6.38 - 6.98 (5H, m), 7.00 - 7.33 (5H, m), 7.98 - 8.01 (2H, m)

### Inventive Example 291

### 4-Amino-N-{2-[4-(4-fluorobenzoyl)piperidino]ethyl}-N-(2-trifluoromethylphenyl)benzamide fumarate

Using 4-amino-N-{2-[4-(4-fluorobenzoyl)piperidino]ethyl}-N-(2-trifluoromethylphenyl)benzamide (102.6 mg, 0.20 mmol), the procedure of Inventive Example 269 was repeated to obtain 112.0 mg (89.0%) of the title compound in a colorless powder form.
Melting point: 175 - 176°C
IR (KBr): 3480, 3380, 1676, 1640, 1602, 1314, 1226, 1174, 1128, 646 cm⁻¹
NMR (CDCl₃-CD₃OD) δ: 1.81 - 1.91 (4H, m), 2.19 - 2.44 (2H, m), 2.66 - 4.51 (7H, m), 6.46 (4H, brd, J = 8.6 Hz), 6.79 (2H, s), 7.05 - 7.61 (8H, m), 7.88 - 8.04 (2H, m)

### Inventive Example 292

### 4-Acetylamino-N-{2-[4-(4-fluorobenzoyl)piperidino]ethyl}-N-(2-trifluoromethylphenyl)benzamide fumarate

Using 4-acetylamino-N-{2-[4-(4-fluorobenzoyl)piperidino]ethyl}-N-(2-trifluoromethylphenyl)benzamide (139.0 mg, 0.25 mmol), the procedure of Inventive Example 269 was repeated to obtain 143.0 mg (85.1%) of the title compound in a colorless powder form.
Melting point: 188 - 190°C
IR (KBr): 3380, 1680, 1600, 1532, 1318, 1262, 1166, 1076, 705, 646 cm⁻¹
NMR (CDCl₃-CD₃OD) δ: 1.88 - 1.99 (4H, m), 2.09 (3H, s), 2.36 - 2.65 (2H, m), 2.82 (2H, t, J = 6.9 Hz), 3.06 - 3.23 (3H, m), 4.15 (2H, t, J = 6.9 Hz), 6.77 (2H, s), 7.06 - 7.46 (10H, m), 7.96 (2H, dd, J = 8.8 Hz, 5.5 Hz)

### Inventive Example 293

### 4-Amino-N-{2-[4-(4-fluorobenzoyl)piperidino]ethyl}-N-(3-trifluoromethylphenyl)benzamide fumarate

Using 4-amino-N-{2-[4-(4-fluorobenzoyl)piperidino]ethyl}-N-(3-trifluoromethylphenyl)benzamide (140.0 mg, 0.27 mmol), the procedure of Inventive Example 269 was repeated to obtain 110.1 mg (64.1%) of the title compound in a light brown powder form.
Melting point: 149 - 150°C
IR (KBr): 3476, 2952, 1680, 1600, 1336, 1130, 840, 701 cm⁻¹
NMR (CDCl₃-CD₃OD) δ: 1.75 - 1.94 (4H, m), 2.20 - 2.40 (4H, m), 2.65 - 3.41 (5H, m), 4.08 (2H, t, J = 6.8 Hz), 6.43 (2H, t, J = 8.4 Hz), 6.78 (2H, s), 7.03 - 7.42 (8H, m), 7.95 (2H, dd, J = 8.6 Hz, 5.5 Hz)

### Inventive Example 294

### 4-Acetylamino-N-{2-[4-(4-fluorobenzoyl)piperidino]ethyl}-N-(3-trifluoromethylphenyl)benzamide fumarate

Using 4-acetylamino-N-{2-[4-(4-fluorobenzoyl)piperidino]ethyl}-N-(3-trifluoromethylphenyl)benzamide (139.0 mg, 0.25 mmol), the procedure of Inventive Example 269 was repeated to obtain 157.0 mg (94.0%) of the title compound in a colorless powder form.
Melting point: 205 - 206°C
IR (KBr): 3343, 1680, 1600, 1314, 1264, 1174, 1130, 850, 763, 609 cm⁻¹
NMR (CDCl₃-CD₃OD) δ: 1.82 - 1.98 (4H, m), 2.09 (3H, s), 2.33 - 2.60 (2H, m), 2.80 (2H, t, J = 6.6 Hz), 3.05 - 3.30 (2H, m), 4.13 (2H, t, J = 6.6 Hz), 6.78 (2H, s), 7.05 - 7.46 (10H, m), 7.96 (2H, dd, J = 8.8 Hz, 5.5 Hz)

### Inventive Example 295

### 4-Acetylamino-N-{2-[4-(4-fluorobenzoyl)piperidino]ethyl}-N-(3-pyridyl)benzamide fumarate

Using 4-acetylamino-N-{2-[4-(4-fluorobenzoyl)piperidino]ethyl}-N-(3-pyridyl)benzamide (151.0 mg, 0.31 mmol), the procedure of Inventive Example 269 was repeated to obtain 145.0 mg (77.6%) of the title compound in a colorless powder form.
Melting point: 200 - 208°C
IR (KBr): 3245, 1683, 1644, 1599, 1527, 1428, 1410, 1314, 1230, 1182, 849 cm⁻¹
NMR (CDCl₃-CD₃OD) δ: 2.12 (3H, s), 1.60 - 2.44 (6H, m), 2.68 (2H, t, J = 7.0 Hz), 2.97 - 3.72 (3H, m), 4.09 (2H, t, J = 7.0 Hz), 6.80 (2H, s), 6.97 - 7.60 (8H, m), 7.95 (2H, dd, J = 9.0 Hz, 6.0 Hz), 8.24 - 8.70 (3H, m)

### Inventive Example 296

### 4-Acetylamino-N-{2-[4-(4-fluorobenzoyl)piperidino]ethyl}-N-(3-methylthiophenyl)benzamide fumarate

Using 4-acetylamino-N-{2-[4-(4-fluorobenzoyl)piperidino]ethyl}-N-(3-methylthiophenyl)benzamide (167.2 mg, 0.31 mmol), the procedure of Inventive Example 269 was repeated to obtain 177.7 mg (88.2%) of the title compound in a colorless powder form.
Melting point: 128 - 133°C
IR (KBr): 1680, 1640, 1598, 1532, 1476, 1440, 1408, 1372, 1314, 1260, 1226, 1180, 1158, 972, 954, 852, 760 cm⁻¹
NMR (CDCl₃-CD₃OD) δ: 2.11 (3H, s), 1.89 - 2.52 (10H, m), 2.50 (3H, s), 2.74 - 2.77 (2H, m), 3.04 - 3.45 (4H, m), 4.09 (2H, dd, J = 6.6 Hz, 6.4 Hz), 6.77 - 7.40 (10H, m), 7.95 (2H, dd, J = 8.6 Hz, 6.0 Hz)

### Inventive Example 297

### 2-Acetylamino-N-{2-[4-(4-fluorobenzoyl)piperidino]ethyl}-N-(3-methoxyphenyl)benzamide fumarate

Using 2-acetylamino-N-{2-[4-(4-fluorobenzoyl)piperidino]ethyl}-N-(3-methoxyphenyl)benzamide (170.4 mg, 0.33 mmol), the procedure of Inventive Example 270 was repeated to obtain 146.2 mg (70.0%) of the title compound in a colorless powder form.
Melting point: 105 - 107°C
IR (KBr): 3450, 1678, 1598, 1492, 1386, 1284, 1158 cm⁻¹
NMR (CDCl₃-CD₃OD) δ: 2.23 (3H, s), 1.63 - 2.50 (5H, m), 2.68 (2H, t, J = 6.6 Hz), 2.85 - 3.46 (2H, m), 3.67 (3H, s), 4.10 (2H, t, J = 6.6 Hz), 6.40 - 6.92 (4H, m), 6.80 (2H, s), 6.92 - 7.35 (5H, m), 7.95 (2H, dd, J = 9.0 Hz, 6.0 Hz), 8.17 (1H, d, J = 8.4 Hz), 9.30 (1H, brs)

### Inventive Example 298

### 4-Amino-N-{2-[4-(4-fluorobenzoyl)piperidino]ethyl}-N-(3-aminophenyl)benzamide fumarate

Using 4-amino-N-{2-[4-(4-fluorobenzoyl)piperidino]ethyl}-N-(3-aminophenyl)benzamide (123.0 mg, 0.27 mmol), the procedure of Inventive Example 269 was repeated to obtain 107.8 mg (70.1%) of the title compound in a light yellow amorphous form.
IR (KBr): 3350, 1680, 1599, 1494, 1440, 1218, 1158, 840 cm⁻¹
NMR (CDCl₃-CD₃OD) δ: 1.80 - 2.30 (4H, m), 2.70 - 3.77 (11H, m), 4.17 (2H, t, J = 7.0 Hz), 6.18 - 6.57 (5H, m), 6.73 (2H, s), 6.80 - 7.32 (5H, m), 7.95 (2H, dd, J = 9.0 Hz, 6.0 Hz)

### Inventive Example 299

### 4-Acetylamino-N-{2-[4-(4-fluorobenzoyl)piperidino]ethyl}-N-(3-acetylaminophenyl)benzamide fumarate

Using 4-acetylamino-N-{2-[4-(4-fluorobenzoyl)piperidino]ethyl}-N-(3-acetylaminophenyl)benzamide (231.0 mg, 0.424 mmol), the procedure of Inventive Example 269 was repeated to obtain 201.0 mg (75.6%) of the title compound in a colorless powder form.
Melting point: 214.5 - 222.5°C
IR (KBr): 3260, 3060, 1689, 1326, 1596, 1530, 1446, 1389, 1248, 1155, 855 cm⁻¹
NMR (CDCl₃-DMSO-d₆) δ: 1.52 - 3.29 (11H, m), 2.10 (6H, s), 2.51 - 3.36 (6H, m), 4.13 (2H, t, J = 7.0 Hz), 6.80 (2H, s), 6.92 - 7.55 (10H, m), 7.97 (2H, dd, J = 9.0 Hz, 6.0 Hz), 8.49 - 8.78 (2H, m)

### Inventive Example 300

### 4-Acetylamino-N-{2-[4-(4-fluorobenzoyl)piperidino]ethyl}-N-(2-methoxycarbonylphenyl)benzamide fumarate

Using 4-acetylamino-N-{2-[4-(4-fluorobenzoyl)piperidino]ethyl}-N-(2-methoxycarbonylphenyl)benzamide (164.0 mg, 0.30 mmol), the procedure of Inventive Example 269 was repeated to obtain 150.4 mg (75.6%) of the title compound in a colorless powder form.
Melting point: 176 - 186°C
IR (KBr): 3342, 1714, 1680, 1598, 1314, 1264, 843, 759, 675, 600 cm⁻¹
NMR (CDCl₃-CD₃OD) δ; 1.83 - 2.01 (4H, m), 2.08 (3H, s), 2.51 - 3.36 (6H, m), 3.80 (3H, s), 4.17 - 4.41 (1H, m), 6.73 (2H, s), 7.06 - 7.70 (10H, m), 7.96 (2H, dd, J = 8.6 Hz, 5.5 Hz)

### Inventive Example 301

### 4-Amidino-N-{2-[4-(4-fluorobenzoyl)piperidino]ethyl}-N-(4-methoxyphenyl)benzamide fumarate

Using 4-amidino-N-{2-[4-(4-fluorobenzoyl)piperidino]ethyl}-N-(4-methoxyphenyl)benzamide (150.0 mg, 0.30 mmol), the procedure of Inventive Example 269 was repeated to obtain 185.0 mg (quantitative) of the title compound in a colorless amorphous form.
Melting point: 137 - 164°C
IR (KBr): 3370, 3045, 1678, 1638, 1510, 848, 652, 603 cm⁻¹
NMR (CDCl₃-CD₃OD) δ: 2.05 - 2.27 (4H, m), 2.73 - 3.43 (7H, m), 3.72 (3H, s), 4.09 - 4.29 (2H, m), 6.74 (2H, s), 6.75 (2H, d, J = 8.8 Hz), 7.07 - 7.63 (8H, m), 7.94 (2H, dd, J = 8.6 Hz, 5.3 Hz)

### Inventive Example 302

### 4-Acetylamino-3-methyl-N-{2-[4-(4-fluorobenzoyl)piperidino]ethyl}-N-(4-methoxyphenyl)benzamide fumarate

Using 4-acetylamino-3-methyl-N-{2-[4-(4-fluorobenzoyl)piperidino]ethyl}-N-(4-methoxyphenyl)benzamide (159.0 mg, 0.30 mmol), the procedure of Inventive Example 269 was repeated to obtain 176.4 mg (90.7%) of the title compound in a colorless powder form.
Melting point: 171 - 172°C IR (KBr): 3440, 2951, 1682, 1644, 1598, 1512, 1304, 1032, 847,
637 cm⁻¹
NMR (CDCl₃-CD₃OD) δ: 1.71 - 2.04 (4H, m), 2.14 (6H, s), 2.42 - 2.66 (2H, m), 2.85 (2H, t, J = 7.5 Hz), 3.11 - 3.39 (3H, m), 3.75 (3H, s), 4.09 (2H, t, J = 7.5 Hz), 6.69 - 6.79 (4H, m), 6.95 - 7.32 (6H, m), 7.53 - 7.62 (1H, m), 7.96 (2H, dd, J = 8.8 Hz, 5.5 Hz)

### Inventive Example 303

### 4-Acetylamino-3-methoxy-N-{2-[4-(4-fluorobenzoyl)piperidino]ethyl}-N-(4-methoxyphenyl)benzamide fumarate

Using 4-acetylamino-3-methoxy-N-{2-[4-(4-fluorobenzoyl)piperidino]ethyl}-N-(4-methoxyphenyl)benzamide (137.0 mg, 0.25 mmol), the procedure of Inventive Example 269 was repeated to obtain 160.0 mg (96.4%) of the title compound in a colorless needle crystal form.
Melting point: 189 - 193°C
IR (KBr): 3440, 2945, 1680, 1638, 1598, 1510, 1301, 759, 643 cm⁻¹
NMR (CDCl₃-CD₃OD) δ: 1.89 - 2.04 (4H, m), 2.16 (3H, s), 2.35 - 3.31 (7H, m), 3.73 (3H, s), 3.76 (3H, s), 4.08 (2H, t, J = 7.3 Hz), 6.78 (2H, s), 6.70 - 7.32 (8H, m), 7.88 - 8.14 (3H, m)

### Inventive Example 304

### 4-Acetylamino-N-{2-[4-(4-fluorobenzoyl)piperidino]ethyl}-N-(4-methylphenyl)benzamide fumarate

Using 4-acetylamino-N-{2-[4-(4-fluorobenzoyl)piperidino]ethyl}-N-(4-methylphenyl)benzamide (307.9 mg, 0.61 mmol), the procedure of Inventive Example 269 was repeated to obtain 335.3 mg (89.0%) of the title compound in a colorless powder form.
Melting point: 217 - 221°C (decomposition)
IR (KBr): 1682, 1638, 1594, 1530, 1510, 1452, 1414, 1390, 1316, 1262, 1228, 1184, 1158, 1136, 1110, 974, 852, 764, 636 cm⁻¹
NMR (CDCl₃-CD₃OD) δ: 1.79 - 1.84 (4H, m), 2.08 (3H, s), 2.27 (3H, s), 2.56 - 2.70 (4H, m), 2.95 - 3.23 (3H, m), 3.94 - 4.08 (3H, m), 6.74 - 7.56 (10H, m), 7.95 (2H, dd, J = 8.8 Hz, 5.5 Hz)

### Inventive Example 305

### 4-Acetylamino-N-{2-[4-(4-fluorobenzoyl)piperidino]ethyl}-N-(4-fluorophenyl)benzamide fumarate

Using 4-acetylamino-N-{2-[4-(4-fluorobenzoyl)piperidino]ethyl}-N-(4-fluorophenyl)benzamide (158.8 mg, 0.31 mmol), the procedure of Inventive Example 269 was repeated to obtain 131.9 mg (68.4%) of the title compound in a colorless amorphous form.
Melting point: 210 - 214°C (decomposition)
IR (KBr): 1684, 1632, 1594, 1530, 1510, 1410, 1386, 1332, 1316, 1264, 1224 cm⁻¹
NMR (CDCl₃-CD₃OD) δ: 1.77 - 1.83 (4H, m), 2.08 (3H, s), 2.09 - 2.29 (2H, m), 2.59 - 2.67 (4H, m), 2.88 - 3.04 (4H, m), 3,14 - 3.78 (2H, m), 4.00 (2H, dd, J = 6.4 Hz, 6.2 Hz), 6.96 (2H, d, J = 8.6 Hz), 7.03 - 7.22 (5H, m), 7.44 (2H, d, J = 8.6 Hz), 7.58 (1H, brs), 7.89 - 8.04 (2H, m)

### Inventive Example 306

### 4-(Pyrol-1-yl)-N-{2-[4-(4-fluorobenzoyl)piperidino]ethyl}-N-(4-methoxyphenyl)benzamide fumarate

Using 4-(pyrol-1-yl)-N-{2-[4-(4-fluorobenzoyl)piperidino]ethyl}-N-(4-methoxyphenyl)benzamide (131.0 mg, 0.25 mmol), the procedure of Inventive Example 269 was repeated to obtain 144.0 mg (90.0%) of the title compound in a colorless needle crystal form.
Melting point: 167 - 169°C
IR (KBr): 3446, 2951, 1680, 1610, 1512, 1330, 836, 710 cm⁻¹
NMR (CDCl₃-CD₃OD) δ: 1.94 - 2.04 (4H, m), 2.07 - 2.88 (4H, m), 3.12 - 3.34 (3H, m), 3.75 (3H, s), 4.13 (2H, d, J = 6.3 Hz), 6.31 (2H, t, J = 2.2 Hz), 6.76 (2H, s), 6.77 (2H, d, J = 8.8 Hz), 6.96 - 7.40 (10H, m), 7.96 (2H, dd, J = 8.8 Hz, 5.3 Hz)

### Inventive Example 307

### 4-Acetylamino-N-{2-[4-(4-fluorobenzoyl)piperidino]ethyl}-N-(3-hydroxyphenyl)benzamide fumarate

Using 4-acetylamino-N-{2-[4-(4-fluorobenzoyl)piperidino]ethyl}-N-(3-hydroxyphenyl)benzamide (154.3 mg, 0.31 mmol), the procedure of Inventive Example 269 was repeated to obtain 167.6 mg (88.3%) of the title compound in a light yellow amorphous form.
IR (KBr): 2974, 1683, 1641, 1599, 1530, 1485, 1443, 1410, 1380, 1314, 1257, 1182, 1152, 972, 954, 849, 759 cm⁻¹
NMR (CDCl₃-CD₃OD) δ: 1.80 - 2.05 (4H, m), 2.10 (3H, s), 2.37 - 3.67 (7H, m), 4.13 (2H, t, J = 7.0 Hz), 6.77 (2H, s), 6.30 - 7.40 (10H, m), 7.80 - 8.05 (2H, m)

### Inventive Example 308

### 4-(N-Methyl-acetylamino)-N-{2-[4-(4-fluorobenzoyl)piperidino]ethyl}-N-(4-methoxyphenyl)benzamide fumarate

Using 4-(N-methyl-acetylamino)-N-{2-[4-(4-fluorobenzoyl)piperidino]ethyl}-N-(3-methoxyphenyl)benzamide (115.5 mg, 0.22 mmol), the procedure of Inventive Example 269 was repeated to obtain 141.0 mg (quantitative) of the title compound in a colorless needle crystal form.
Melting point: 143 - 145°C
IR (KBr): 3450, 1680, 1652, 1600, 1512, 1250, 857, 651 cm⁻¹
NMR (CDCl₃-CD₃OD) δ: 1.78 (3H, s), 1.78 - 2.03 (4H, m), 2.29 - 2.60 (2H, m), 2.81 (2H, t, J = 7.0 Hz), 3.08 - 3 40 (3H, m), 3.17 (3H, s), 3.75 (3H, s), 4.10 (2H, t, J = 7.0 Hz), 6.74 (2H, d, J = 8.8 Hz), 6.78 (2H, s), 6.96 - 7.37 (8H, m), 7.96 (2H, dd, J = 8.8 Hz, 5.5 Hz)

### Inventive Example 309

### 4-(N-Dimethylamino)-N-{2-[4-(4-fluorobenzoyl)piperidino]ethyl}-N-(4-methoxyphenyl)benzamide fumarate

Using 4-(N-dimethylamino)-N-{2-[4-(4-fluorobenzoyl)piperidino]ethyl}-N-(4-methoxyphenyl)benzamide (110.0 mg, 0.22 mmol), the procedure of Inventive Example 269 was repeated to obtain 114.0 mg (61.1%) of the title compound in a colorless needle crystal form.
Melting point: 226 - 228°C (decompose)
IR (KBr): 3440, 2940, 1600, 1510, 1370, 1032, 832, 644 cm⁻¹
NMR (CDCl₃-CD₃OD) δ: 1.95 - 2.06 (4H, m), 2.62 - 3.45 (7H, m), 2.92 (6H, s), 3.77 (3H, s), 4.11 (2H, t, J = 7.0 Hz), 6.43 (2H, d, J = 9.0 Hz), 6.77 (2H, s), 6.78 (2H, d, J = 9.0 Hz), 6.97 - 7.30 (6H, m), 7.97 (2H, dd, J = 8.8 Hz, 5.5 Hz)

### Inventive Example 310

### 3-Acetylamino-N-{2-[4-(4-fluorobenzoyl)piperidino]ethyl}-N-(phenyl)benzamide fumarate

Using 3-acetylamino-N-{2-[4-(4-fluorobenzoyl)piperidino]ethyl}-N-(phenyl)benzamide (91.1 mg, 0.19 mmol), the procedure of Inventive Example 269 was repeated to obtain 74.3 mg (65.9%) of the title compound in a light yellow powder form.
Melting point: 137 - 138°C
IR (KBr): 1676, 1648, 1596, 1560, 1494, 1430, 1382, 1306, 1290, 1264, 1228, 1158, 976, 748, 698 cm⁻¹
NMR (CDCl₃-CD₃OD) δ: 1.77 - 1.93 (4H, m), 2.13 (3H, s), 2.27 - 2.43 (2H, m), 2.67 (2H, t, J = 6.6 Hz), 2.97 - 3.84 (8H, m), 4.07 (2H, t, J = 6.6 Hz), 6.79 - 6.84 (2H, m), 7.14 - 7.34 (9H, m), 7.93 (1H, d, J = 8.6 Hz), 7.99 (1H, d, J = 8.6 Hz)

### Inventive Example 311

### 3-Acetylamino-N-{2-[4-(4-fluorobenzoyl)piperidino]ethyl}-N-(4-methylthiophenyl)benzamide fumarate

Using 4-acetylamino-N-{2-[4-(4-fluorobenzoyl)piperidino]ethyl}-N-(4-methylthiophenyl)benzamide (127.2 mg, 0.24 mmol), the procedure of Inventive Example 269 was repeated to obtain 88.1 mg (56.9%) of the title compound in a light yellow powder form.
Melting point: 95 - 97°C
IR (KBr): 1680, 1640, 1588, 1552, 1374, 1314, 1226 cm⁻¹
NMR (CDCl₃-CD₃OD) δ: 1.80 - 2.12 (4H, m), 2.13 (3H, s), 2.35 (3H, s), 2.31 - 2.54 (4H, m), 2.77 (2H, t, J = 6.6 Hz), 3.06 - 3.31 (6H, m), 4.12 (2H, t, J = 6.6 Hz), 6.81 - 7.39 (10H, m), 7.93 (1H, d, J = 8.4 Hz), 7.99 (1H, d, J = 8.4 Hz)

### Inventive Example 312

### 3-Acetylamino-N-{2-[4-(4-fluorobenzoyl)piperidino]ethyl}-N-(4-methylphenyl)benzamide fumarate

Using 4-acetylamino-N-{2-[4-(4-fluorobenzoyl)piperidino]ethyl}-N-(4-methylphenyl)benzamide (199.4 mg, 0.40 mmol), the procedure of Inventive Example 269 was repeated to obtain 230.2 mg (93.2%) of the title compound in a light brown amorphous form.
Melting point: 93 - 95°C
IR (KBr): 1680, 1598 cm⁻¹
NMR (CDCl₃-CD₃OD) δ: 1.87 - 2.43 (6H, m), 2.13 (3H, s), 2.28 (3H, s), 2.70 (2H, t, J = 6.6 Hz), 2.98 - 3.62 (4H, m), 4.07 (2H, t, J = 6.6 Hz), 6.82 - 7.28 (9H, m), 7.69 - 7.72 (1H, m), 7.93 (1H, d, J = 8.8 Hz), 7.99 (1H, d, J = 8.8 Hz)

### Inventive Example 313

### 3-Acetylamino-N-{2-[4-(4-fluorobenzoyl)piperidino]ethyl}-N-(4-fluorophenyl)benzamide fumarate

Using 3-acetylamino-N-{2-[4-(4-fluorobenzoyl)piperidino]ethyl}-N-(4-fluorophenyl)benzamide (182.5 mg, 0.36 mmol), the procedure of Inventive Example 269 was repeated to obtain 184.2 mg (82.3%) of the title compound in a light brown amorphous form.
Melting point: 93 - 95°C
IR (KBr): 1682, 1640, 1598, 1554, 1508, 1378, 1320, 1220, 1156 cm⁻¹
NMR (CDCl₃-CD₃OD) δ: 1.84 - 1.96 (4H, m), 2.12 (3H, s), 2.20 - 2.45 (3H, m), 2.61 - 3.42 (9H, m), 4.04 (2H, t, J = 6.4 Hz), 6.69 - 7.70 (10H, m), 7.93 (1H, d, J = 8.6 Hz), 7.99 (1H, d, J = 8.8 Hz)

### Inventive Example 314

### 4-Acetylamino-N-{2-[4-(4-fluorobenzoyl)piperidino]ethyl}-N-(4-hydroxyphenyl)benzamide fumarate

Using 4-acetylamino-N-{2-[4-(4-fluorobenzoyl)piperidino]ethyl}-N-(4-hydroxyphenyl)benzamide (215.0 mg, 0.43 mmol), the procedure of Inventive Example 269 was repeated to obtain 254.0 mg (96.0%) of the title compound in a light yellow powder form.
Melting point: 72 - 74°C
IR (KBr): 3044, 1680, 1598, 1512, 1448, 1408, 1378, 1316, 1264, 1228, 1182, 1158, 976, 952, 842, 760 cm⁻¹
NMR (CDCl₃-CD₃OD) δ: 1.70 - 2.13 (6H, m), 2.08 (3H, s), 2.30 - 2.73 (5H, m), 3.88 - 4.20 (2H, m), 6.53 - 6.99 (4H, m), 6.75 (2H, s), 7.00 - 7.40 (6H, m), 7.80 - 8.05 (2H, m)

### Inventive Example 315

### 4-Propionylamino-N-{2-[4-(4-fluorobenzoyl)piperidino]ethyl}-N-(3-methoxyphenyl)benzamide fumarate

Using 4-propionylamino-N-{2-[4-(4-fluorobenzoyl)piperidino]ethyl}-N-(3-methoxyphenyl)benzamide (106.0 mg, 0.20 mmol), the procedure of Inventive Example 269 was repeated to obtain 110.0 mg (85.3%) of the title compound in a colorless powder form.
Melting point: 214 - 218°C
IR (KBr): 3445, 1680, 1600, 1296, 1216, 853, 638 cm⁻¹
NMR (CDCl₃-CD₃OD) δ: 1.70 (3H, t, J = 7.4 Hz), 1.83 - 2.00 (4H, m), 2.22 - 3.86 (9H, m), 3.70 (3H, s), 3.88 - 4.20 (2H, m), 6.61 - 6.77 (5H, m), 7.05 - 7.45 (7H, m), 7.98 - 8.04 (2H, m)

### Inventive Example 316

### 4-Valerylamino-N-{2-[4-(4-fluorobenzoyl)piperidino]ethyl}-N-(3-methoxyphenyl)benzamide fumarate

Using 4-varelylamino-N-{2-[4-(4-fluorobenzoyl)piperidino]ethyl}-N-(3-methoxyphenyl)benzamide (142.0 mg, 0.25 mmol), the procedure of Inventive Example 269 was repeated to obtain 152.0 mg (85.9%) of the title compound in a colorless powder form.
Melting point: 229 - 230°C (decomposition)
IR (KBr): 3450, 2951, 1678, 1600, 1296, 1180, 857, 646 cm⁻¹
NMR (CDCl₃-CD₃OD) δ: 0.92 (3H, t, J = 6.4 Hz), 1.00 - 4.37 (22H, m), 3.71 (3H, s), 6.64 - 6.77 (5H, m), 7.06 - 7.45 (7H, m), 7.96 (2H, dd, J = 8.6 Hz, 5.5 Hz)

### Inventive Example 317

### 4-Acetylamino-N-{2-[4-(4-fluorobenzoyl)piperidino]ethyl}-N-(3,4-methylenedioxyphenyl)benzamide fumarate

Using 4-acetylamino-N-{2-[4-(4-fluorobenzoyl)piperidino]ethyl}-N-(3,4-methylenedioxyphenyl)benzamide (210.3 mg, 0.40 mmol), the procedure of Inventive Example 269 was repeated to obtain 195.9 mg (76.5%) of the title compound in a colorless powder form.
Melting point: 223 - 224°C
IR (KBr): 1710, 1680, 1644, 1596, 1530, 1506, 1485, 1452, 1410, 1389, 1317, 1257, 1218, 1182, 1161, 1041, 849 cm⁻¹
NMR (CDCl₃-CD₃OD) δ: 1.70 - 2.00 (4H, m), 2.11 (3H, s), 2.20 - 2.57 (2H, m), 2.74 (2H, t, J = 6.9 Hz), 2.87 - 3.47 (3H, m), 4.03 (2H, t, J = 6.9 Hz), 5.95 (2H, s), 6.37 - 6.70 (3H, m), 6.79 (2H, s), 6.90 - 7.50 (6H, m), 7.80 - 8.05 (2H, m)

### Inventive Example 318

### 4-Acetylamino-N-{2-[4-(4-fluorobenzoyl)piperidino]ethyl}-N-(3,4-dimethylphenyl)benzamide fumarate

Using 4-acetylamino-N-{2-[4-(4-fluorobenzoyl)piperidino]ethyl}-N-(3,4-dimethylphenyl)benzamide (218.2 mg, 0.42 mmol), the procedure of Inventive Example 269 was repeated to obtain 223.6 mg (84.3%) of the title compound in a colorless powder form.
Melting point: 210 - 212°C (decomposition)
IR (KBr): 1710, 1682, 1656, 1642, 1534, 1504, 1408, 1382, 1316, 1262, 1234, 1218, 1182, 1160, 848, 762, 636 cm⁻¹
NMR (CDCl₃-CD₃OD) δ: 2.09 (3H, s), 2.19 (6H, brs), 1.96 - 2.92 (10H, m), 3.01 (2H, t, J = 7.0 Hz), 3.20 - 3.54 (3H, m), 4.16 (2H, t, J = 6.8 Hz), 6.73 (2H, s), 6.87 - 7.47 (7H, m), 8.05 (2H, dd, J = 8.6 Hz, 5.5 Hz)

### Inventive Example 319

### 4-Acetylamino-N-{2-[4-(4-fluorobenzoyl)piperidino]ethyl}-N-(4-chlorophenyl)benzamide fumarate

Using 4-acetylamino-N-{2-[4-(4-fluorobenzoyl)piperidino]ethyl}-N-(4-chlorophenyl)benzamide (159.0 mg, 0.30 mmol), the procedure of Inventive Example 269 was repeated to obtain 140.8 mg (73.0%) of the title compound in a colorless powder form.
Melting point: 218 - 220°C (decomposition)
IR (KBr): 1682, 1636, 1596, 1530, 1492, 1414, 1384, 1318, 1262, 1228, 1186 cm⁻¹
NMR (CDCl₃-CD₃OD) δ: 1.78 - 1.84 (4H, m), 2.10 (3H, s), 2.10 - 2.31 (2H, m), 2.61 - 2.68 (3H, m), 2.93 - 3.83 (7H, m), 4.01 (2H, t, J = 6.8 Hz), 6.75 (2H, s), 7.00 - 7.23 (4H, m), 7.40 - 7.53 (4H, m), 7.96 (2H, dd, J = 8.6 Hz, 5.5 Hz)

### Inventive Example 320

### 4-Acetylamino-N-{2-[4-(4-fluorobenzoyl)piperidino]ethyl}-N-(1-naphthyl)benzamide fumarate

Using 4-acetylamino-N-{2-[4-(4-fluorobenzoyl)piperidino]ethyl}-N-(1-naphthyl)benzamide (334.2 mg, 0.62 mmol), the procedure of Inventive Example 269 was repeated to obtain 360.7 mg (88.8%) of the title compound in a light yellow powder form.
Melting point: 217 - 221°C (decomposition)
IR (KBr): 3050, 1680, 1644, 1599, 1530, 1350, 1407, 1383, 1314, 1260, 1230, 1182, 1158, 849, 771 cm⁻¹
NMR (CDCl₃-CD₃OD) δ: 1.50 - 1.97 (4H, m), 2.02 (3H, s), 2.20 - 2.60 (2H, m), 2.60 - 3.43 (5H, m), 3.55 - 3.92 (1H, m), 4.40 - 4.75 (1H, m), 6.73 (2H, s), 6.90 - 8.08 (15H, m)

### Inventive Example 321

### 4-Acetylamino-N-{3-[4-(4-fluorobenzoyl)piperidino]propyl}-N-(3-methoxyphenyl)benzamide fumarate

Using 4-acetylamino-N-{3-[4-(4-fluorobenzoyl)piperidino]propyl}-N-(3-methoxyphenyl)benzamide (312.6 mg, 0.59 mmol), the procedure of Inventive Example 269 was repeated to obtain 308.7 mg (80.9%) of the title compound in a colorless powder form.
Melting point: 190 - 193°C
IR (KBr): 1682, 1634, 1600, 1528, 1490, 1408, 1314, 1258, 1182, 1050, 844 cm⁻¹
NMR (CDCl₃-CD₃OD) δ: 1.76 - 2.25 (6H, m), 2.09 (3H, s), 2.56 - 3.00 (4H, m), 3.69 (3H, s), 3.94 (2H, dd, J = 7.0 Hz, 6.5 Hz), 6.50 - 6.80 (3H, m), 6.74 (2H, s), 6.95 - 7.50 (7H, m), 7.96 (2H, dd, J = 8.5 Hz, 5.3 Hz)

### Inventive Example 322

### 4-Amino-N-{3-[4-(4-fluorobenzoyl)piperidino]propyl}-N-(3-methoxyphenyl)benzamide fumarate

Using 4-amino-N-{3-[4-(4-fluorobenzoyl)piperidino]propyl}-N-(3-methoxyphenyl)benzamide (300.0 mg, 0.61 mmol), the procedure of Inventive Example 269 was repeated to obtain 321.3 mg (81.1%) of the title compound in a colorless powder form.
Melting point: 170 - 172°C
IR (KBr): 1680, 1598, 1562, 1488, 1452, 1394, 1306, 1282, 1230, 1180, 1158 cm⁻¹
NMR (CDCl₃-CD₃OD) δ: 1.70 - 2.23 (6H, m), 2.50 - 2.95 (4H, m), 3.00 - 3.53 (5H, m), 3.70 (3H, s), 3.92 (2H, t, J = 7.0 Hz), 6.41 (2H, d, J = 8.4 Hz), 6.50 - 6.85 (2H, m), 6.74 (2H, s), 6.95 - 7.36 (6H, m), 7.95 (2H, dd, J = 8.6 Hz, 5.5 Hz)

### Inventive Example 323

### 4-(N-Methyl-acetylamino)-N-{2-[4-(4-fluorobenzoyl)piperidino]ethyl}-N-(3-methoxyphenyl)benzamide fumarate

Using 4-(N-methyl-acetylamino)-N-{2-[4-(4-fluorobenzoyl)piperidino]ethyl}-N-(3-methoxyphenyl)benzamide (81.8 mg, 0.154 mmol), the procedure of Inventive Example 269 was repeated to obtain 69.7 mg (69.8%) of the title compound in a colorless powder form.
Melting point: 161 - 167°C
IR (KBr): 3440, 1648, 1600, 1382, 1304, 1158, 854, 700 cm⁻¹
NMR (CDCl₃-CD₃OD) δ: 1.78 (3H, s), 1.84 - 2.01 (4H, m), 2.22 - 2.64 (2H, m), 2.82 (2H, t, J = 6.7 Hz), 3.18 (3H, s), 3.09 - 3.40 (3H, m), 3.70 (3H, s), 4.14 (2H, t, J = 6.7 Hz), 6.63 - 6.78 (4H, m), 6.96 - 7.41 (6H, m), 7.95 (2H, dd, J = 8.6 Hz, 5.5 Hz)

### Inventive Example 324

### 4-Acetylamino-N-{2-[4-(4-fluorobenzoyl)piperidino]ethyl}-N-(3-methoxyphenyl)benzamide tartarate

Using 4-acetylamino-N-{2-[4-(4-fluorobenzoyl)piperidino]ethyl}-N-(3-methoxyphenyl)benzamide (155.0 mg, 0.30 mmol) and tartaric acid (35.0 mg, 0.30 mmol), the procedure of Inventive Example 269 was repeated to obtain 143.0 mg (75.3%) of the title compound in a colorless powder form.
Melting point: 152 - 175°C
IR (KBr): 3440, 3325, 1686, 1600, 1312, 1216, 851 cm⁻¹
NMR (CDCl₃-CD₃OD) δ: 2.10 (3H, s), 1.99 - 4.44 (12H, m), 3.70 (3H, s), 6.62 - 6.77 (3H, m), 7.07 - 7.45 (7H, m), 7.91 - 8.06 (2H, m)

### Inventive Example 325

### 4-Acetylamino-N-{2-[4-(4-fluorobenzoyl)piperidino]ethyl}-N-(3-methoxyphenyl)benzamide maleate

Using 4-acetylamino-N-{2-[4-(4-fluorobenzoyl)piperidino]ethyl}-N-(3-methoxyphenyl)benzamide (155.0 mg, 0.30 mmol) and maleic acid (45.0 mg, 0.30 mmol), the procedure of Inventive Example 269 was repeated to obtain 164.0 mg (82.0%) of the title compound in a colorless powder form.
Melting point: 212.0 - 213°C
IR (KBr): 3447, 1686, 1640, 1598, 1320, 1214, 856 cm⁻¹
NMR (CDCl₃-CD₃OD) δ: 2.11 (3H, s), 2.16 - 3.75 (11H, m), 3.70 (3H, s), 4.27 (2H, t, J = 7.0 Hz), 6.23 (2H, s), 6.58 - 6.77 (3H, m), 7.08 - 7.45 (7H, m), 7.98 (2H, dd, J = 8.7 Hz, 5.4 Hz)

### Inventive Example 326

### 4-Amino-N-{2-[4-(4-fluorobenzoyl)piperidino]ethyl}-N-(3-methoxyphenyl)benzamide hemifumarate

4-Aamino-N-{2-[4-(4-fluorobenzoyl)piperidino]ethyl}-N-(3-methoxyphenyl)benzamide (475.0 mg, 1.00 mmol) was dissolved in methanol (4.0 ml) and mixed with a methanol solution (3.0 ml) of fumaric acid (58.0 mg, 0.50 mmol) at 0°C. Thereafter, the thus precipitated crystals were collected by filtration and recrystallised from an ethanol-water mixture solution to obtain 481.0 mg (90.0%) of the title compound in a colorless powder form.
Melting point: 176 - 179°C
IR (KBr): 3450, 3340, 1672, 1632, 1602, 1510, 1490, 1362, 1318, 1284, 1202, 1122, 960, 840 cm⁻¹
NMR (CDCl₃-DMSO_{d6}) δ: 1.50 - 2.00 (5H, m), 2.13 - 2.49 (2H, m), 2.72 (2H, t, J = 7.5 Hz), 2.90 - 3.25 (4H, m), 3.69 (3H, s), 4.05 (2H, t, J = 7.5 Hz), 6.40 (2H, d, J = 8.5 Hz), 6.50 - 6.80 (2H, m), 6.75 (1H, s), 6.93 - 7.26 (6H, m), 7.95 (2H, dd, J = 8.8 Hz, 5.5 Hz)

While the invention has been described in detail and with reference to specific embodiments thereof, it will be apparent to one skilled in the art that various changes and modifications can be made therein without departing from the spirit and scope thereof.

## Claims

1. A substituted cyclic amine compound represented by the following general formula (1) 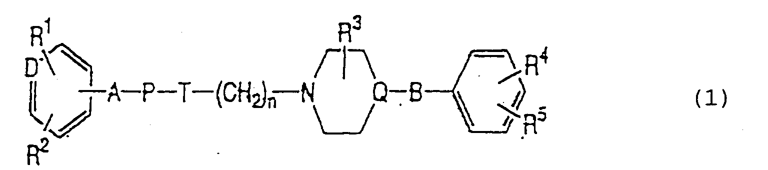 wherein R¹ and R² may be the same or different from each other and each represents a hydrogen atom, a hydroxyl group, a straight or branched-chain alkyl group, an alkenyl group, a substituted or unsubstituted aralkyl group, an alkoxy group, a hydroxyalkyl group, an acyl group, a substituted or unsubstituted amino group, a substituted or unsubstituted aminoalkyl group, a substituted or unsubstituted imino group, a substituted or unsubstituted iminoalkyl group, a substituted or unsubstituted guanidino group, a substituted or unsubstituted guanidinoalkyl group, a substituted or unsubstituted amidino group, a substituted or unsubstituted amidinoalkyl group, a substituted or unsubstituted ureido group, a substituted or unsubstituted ureidoalkyl group, a substituted or unsubstituted thioureido group, a substituted or unsubstituted thioureidoalkyl group, a carboxyl group, an alkoxycarbonyl group, a formyl group, a substituted or unsubstituted carbamoyl group, a substituted or unsubstituted carbamoylalkyl group, a formylalkyl group, an alkoxycarbonylalkyl group, a carboxyalkyl group, a mercapto group, an alkylthio group, a mercaptoalkyl group, a nitro group, a cyano group, a halogenoalkyl group, a trihalogenomethyl group or a halogen atom, R³ represents a hydrogen atom, a hydroxyl group, a straight or branched-chain alkyl group or an alkoxy group, R⁴ and R⁵ may be the same or different from each other and each represents a hydrogen atom, a hydroxyl group, a straight or branched-chain alkyl group, an alkenyl group, a halogen atom, an alkoxy group, a mercapto group, an alkylthio group, a substituted or unsubstituted amino group or a trihalogenomethyl group, D represents a methine moiety, a nitrogen atom or =N(→O)-, A represents a methylene moiety, a carbonyl group or a sulfonyl group, P is a group represented by -CR⁶(R⁷)- or -NR⁸-, each of R⁶, R⁷ and R⁸ represents a hydrogen atom, a straight or branched-chain alkyl group, a cyclic alkyl group, an alkenyl group, a substituted or unsubstituted aralkyl group, a substituted or unsubstituted phenyl group, a substituted or unsubstituted naphthyl group, a substituted or unsubstituted heterocyclic moiety, an acyl group, a hydroxyalkyl group, a substituted or unsubstituted aminoalkyl group, a cyano group, a substituted or unsubstituted carbamoylalkyl group, a carboxyalkyl group or an alkoxycarbonylalkyl group, Q represents a methine group or a substituted or unsubstituted nitrogen atom, each of T and B is a single bond or represents a group selected from the member consisting of methylene, carbonyl, -CH(OH)- and -C(=NH)-, and n is an integer of 1 to 6; or a pharmaceutically acceptable salt thereof.

2. A synthetic intermediate of the compound (1) of claim 1, represented by the following general formula (2) 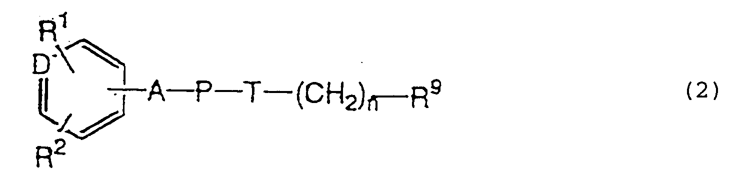 wherein R¹ and R² may be the same or different from each other and each represents a hydrogen atom, a hydroxyl group, a straight or branched-chain alkyl group, an alkenyl group, a substituted or unsubstituted aralkyl group, an alkoxy group, a hydroxyalkyl group, an acyl group, a substituted or unsubstituted amino group, a substituted or unsubstituted aminoalkyl group, a substituted or unsubstituted imino group, a substituted or unsubstituted iminoalkyl group, a substituted or unsubstituted guanidino group, a substituted or unsubstituted guanidinoalkyl group, a substituted or unsubstituted amidino group, a substituted or unsubstituted amidinoalkyl group, a substituted or unsubstituted ureido group, a substituted or unsubstituted ureidoalkyl group, a substituted or unsubstituted thioureido group, a substituted or unsubstituted thioureidoalkyl group, a carboxyl group, an alkoxycarbonyl group, a formyl group, a substituted or unsubstituted carbamoyl group, a substituted or unsubstituted carbamoylalkyl group, a formylalkyl group, an alkoxycarbonylalkyl group, a carboxyalkyl group, a mercapto group, an alkylthio group, a mercaptoalkyl group, a nitro group, a cyano group, a halogenoalkyl group, a trihalogenomethyl group or a halogen atom, R⁹ represents a hydroxyl group, a p-toluenesulfonyloxy group, a methanesulfonyloxy group, trifluoromethanesulfonyloxy group, a halogen atom, a tetrahydropyranyloxy group, a methoxy group, an ethoxy group, a tert-butoxy group, a benzyloxy group, an acetoxy group, a trimethylsilyloxy group, a tert-butyldimethyloxy group or a pivaloyloxy group, D represents a methine moiety, a nitrogen atom or =N(→O)-, A represents a methylene moiety, a carbonyl group or a sulfonyl group, P is a group represented by -CR⁶(R⁷)- or -NR⁸-, each of R⁶, R⁷ and R⁸ represents a hydrogen atom, a straight or branched-chain alkyl group, a cyclic alkyl group, an alkenyl group, a substituted or unsubstituted aralkyl group, a substituted or unsubstituted phenyl group, a substituted or unsubstituted naphthyl group, a substituted or unsubstituted heterocyclic moiety, an acyl group, a hydroxyalkyl group, a substituted or unsubstituted aminoalkyl group, a cyano group, a substituted or unsubstituted carbamoylalkyl group, a carboxyalkyl group or an alkoxycarbonylalkyl group, T is a single bond or represents a group selected from the member consisting of methylene, carbonyl, -CH(OH)- and -C(=NH)-, and n is an integer of 1 to 6.

3. A synthetic intermediate of the compound (1) of claim 1, represented by the following general formula (3) 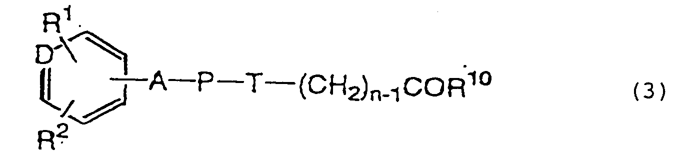 wherein R¹ and R² may be the same or different from each other and each represents a hydrogen atom, a hydroxyl group, a straight or branched-chain alkyl group, an alkenyl group, a substituted or unsubstituted aralkyl group, an alkoxy group, a hydroxyalkyl group, an acyl group, a substituted or unsubstituted amino group, a substituted or unsubstituted aminoalkyl group, a substituted or unsubstituted imino group, a substituted or unsubstituted iminoalkyl group, a substituted or unsubstituted guanidino group, a substituted or unsubstituted guanidinoalkyl group, a substituted or unsubstituted amidino group, a substituted or unsubstituted amidinoalkyl group, a substituted or unsubstituted ureido group, a substituted or unsubstituted ureidoalkyl group, a substituted or unsubstituted thioureido group, a substituted or unsubstituted thioureidoalkyl group, a carboxyl group, an alkoxycarbonyl group, a formyl group, a substituted or unsubstituted carbamoyl group, a substituted or unsubstituted carbamoylalkyl group , a formylalkyl group, an alkoxycarbonylalkyl group, a carboxyalkyl group, a mercapto group, an alkylthio group, a mercaptoalkyl group, a nitro group, a cyano group, a halogenoalkyl group, a trihalogenomethyl group or a halogen atom, R¹⁰ represents a hydrogen atom, a hydroxyl group, a methoxy group, an ethoxy group or a tert-butoxy group, D represents a methine moiety, a nitrogen atom or =N(→O)-, A represents a methylene moiety, a carbonyl group or a sulfonyl group, P is a group represented by -CR⁶(R⁷)- or -NR⁸-, each of R⁶, R⁷ and R⁸ represents a hydrogen atom, a straight or branched-chain alkyl group, a cyclic alkyl group, an alkenyl group, a substituted or unsubstituted aralkyl group, a substituted or unsubstituted phenyl group, a substituted or unsubstituted naphthyl group, a substituted or unsubstituted heterocyclic moiety, an acyl group, a hydroxyalkyl group, a substituted or unsubstituted aminoalkyl group, a cyano group, a substituted or unsubstituted carbamoylalkyl group, a carboxyalkyl group or an alkoxycarbonylalkyl group, T is a single bond or represents a group selected from the member consisting of methylene, carbonyl, -CH(OH)- and -C(=NH)-, and n is an integer of 1 to 6.

4. A process for producing a substituted cyclic amine compound represented by the following general formula (1) 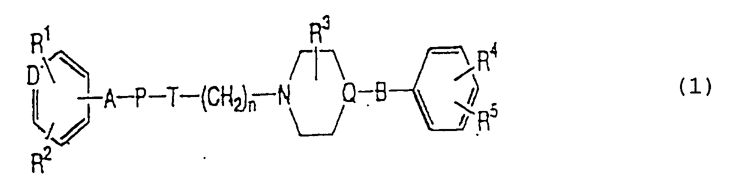 wherein R¹, R², R³, R⁴, R⁵, D, A, P, Q, T, B and n are as defined in the foregoing, or a pharmaceutically acceptable salt thereof,
which comprises reacting a compound represented by the following general formula (2a) 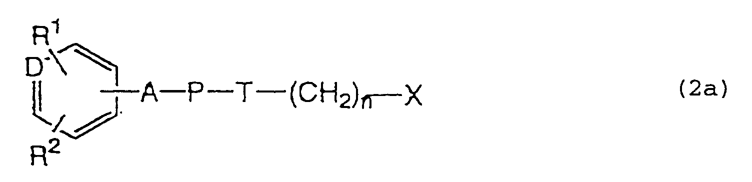 wherein R¹ and R² may be the same or different from each other and each represents a hydrogen atom, a hydroxyl group, a straight or branched-chain alkyl group, an alkenyl group, a substituted or unsubstituted aralkyl group, an alkoxy group, a hydroxyalkyl group, an acyl group, a substituted or unsubstituted amino group, a substituted or unsubstituted aminoalkyl group, a substituted or unsubstituted imino group, a substituted or unsubstituted iminoalkyl group, a substituted or unsubstituted guanidino group, a substituted or unsubstituted guanidinoalkyl group, a substituted or unsubstituted amidino group, a substituted or unsubstituted amidinoalkyl group, a substituted or unsubstituted ureido group, a substituted or unsubstituted ureidoalkyl group, a substituted or unsubstituted thioureido group, a substituted or unsubstituted thioureidoalkyl group, a carboxyl group, an alkoxycarbonyl group, a formyl group, a substituted or unsubstituted carbamoyl group, a substituted or unsubstituted carbamoylalkyl group, a formylalkyl group, an alkoxycarbonylalkyl group, a carboxyalkyl group, a mercapto group, an alkylthio group, a mercaptoalkyl group, a nitro group, a cyano group, a halogenoalkyl group, a trihalogenomethyl group or a halogen atom, D represents a methine moiety, a nitrogen atom or =N(→O)-, A represents a methylene moiety, a carbonyl group or a sulfonyl group, P is a group represented by -CR⁶(R⁷)- or -NR⁸-, each of R⁶, R⁷ and R⁸ represents a hydrogen atom, a straight or branched-chain alkyl group, a cyclic alkyl group, an alkenyl group, a substituted or unsubstituted aralkyl group, a substituted or unsubstituted phenyl group, a substituted or unsubstituted naphthyl group, a substituted or unsubstituted heterocyclic moiety, an acyl group, a hydroxyalkyl group, a substituted or unsubstituted aminoalkyl group, a cyano group, a substituted or unsubstituted carbamoylalkyl group, a carboxyalkyl group or an alkoxycarbonylalkyl group, T is a single bond or represents a group selected from the member consisting of methylene, carbonyl, -CH(OH)- and -C(=NH)-, X represents a halogen atom, a p-toluenesulfonyloxy group, a methanesulfonyloxy group or a trifluoromethanesulfonyloxy group and n is an integer of 1 to 6, with a compound represented by the following general formula (4) 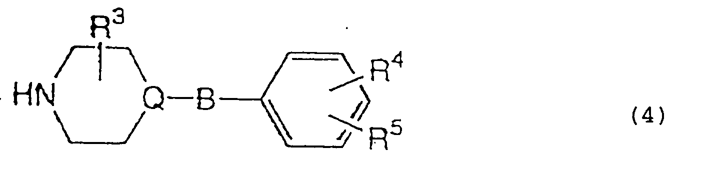 wherein R³ represents a hydrogen atom, a hydroxyl group, a straight or branched-chain alkyl group or an alkoxy group, R⁴ and R⁵ may be the same or different from each other and each represents a hydrogen atom, a hydroxyl group, a straight or branched-chain alkyl group, an alkenyl group, a halogen atom, an alkoxy group, a mercapto group, an alkylthio group, a substituted or unsubstituted amino group or a trihalogenomethyl group, Q represents a methine group or a substituted or unsubstituted nitrogen atom and B is a single bond or represents a group selected from the member consisting of methylene, carbonyl, -CH(OH)- and -C(=NH)-.

5. The process for producing a substituted cyclic amine compound (1) or a pharmaceutically acceptable salt thereof according to claim 4, wherein a compound represented by the following general formula (3a) 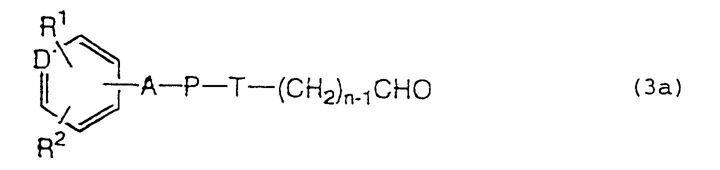 wherein R¹ and R² may be the same or different from each other and each represents a hydrogen atom, a hydroxyl group, a straight or branched-chain alkyl group, an alkenyl group, a substituted or unsubstituted aralkyl group, an alkoxy group, a hydroxyalkyl group, an acyl group, a substituted or unsubstituted amino group, a substituted or unsubstituted aminoalkyl group, a substituted or unsubstituted imino group, a substituted or unsubstituted iminoalkyl group, a substituted or unsubstituted guanidino group, a substituted or unsubstituted guanidinoalkyl group, a substituted or unsubstituted amidino group, a substituted or unsubstituted amidinoalkyl group, a substituted or unsubstituted ureido group, a substituted or unsubstituted ureidoalkyl group, a substituted or unsubstituted thioureido group, a substituted or unsubstituted thioureidoalkyl group, a carboxyl group, an alkoxycarbonyl group, a formyl group, a substituted or unsubstituted carbamoyl group, a substituted or unsubstituted carbamoylalkyl group, a formylalkyl group, an alkoxycarbonylalkyl group, a carboxyalkyl group, a mercapto group, an alkylthio group, a mercaptoalkyl group, a nitro group, a cyano group, a halogenoalkyl group, a trihalogenomethyl group or a halogen atom, D represents a methine moiety, a nitrogen atom or =N(→O)-, A represents a methylene moiety, a carbonyl group or a sulfonyl group, P is a group represented by -CR⁶(R⁷) - or -NR⁸-, each of R⁶, R⁷ and R⁸ represents a hydrogen atom, a straight or branched-chain alkyl group, a cyclic alkyl group, an alkenyl group, a substituted or unsubstituted aralkyl group, a substituted or unsubstituted phenyl group, a substituted or unsubstituted naphthyl group, a substituted or unsubstituted heterocyclic moiety, an acyl group, a hydroxyalkyl group, a substituted or unsubstituted aminoalkyl group, a cyano group, a substituted or unsubstituted carbamoylalkyl group, a carboxyalkyl group or an alkoxycarbonylalkyl group, T is a single bond or represents a group selected from the member consisting of methylene, carbonyl, -CH(OH)- and -C(=NH)- and n is an integer of 1 to 6, is reduced by allowing it to react with the compound (4) of claim 4.

6. The process for producing a substituted cyclic amine compound (1) or a pharmaceutically acceptable salt thereof according to claim 4, wherein a compound represented by the following general formula (5) 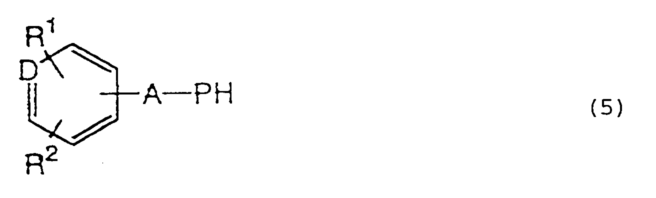 wherein R¹ and R² may be the same or different from each other and each represents a hydrogen atom, a hydroxyl group, a straight or branched-chain alkyl group, an alkenyl group, a substituted or unsubstituted aralkyl group, an alkoxy group, a hydroxyalkyl group, an acyl group, a substituted or unsubstituted amino group, a substituted or unsubstituted aminoalkyl group, a substituted or unsubstituted imino group, a substituted or unsubstituted iminoalkyl group, a substituted or unsubstituted guanidino group, a substituted or unsubstituted guanidinoalkyl group, a substituted or unsubstituted amidino group, a substituted or unsubstituted amidinoalkyl group, a substituted or unsubstituted ureido group, a substituted or unsubstituted ureidoalkyl group, a substituted or unsubstituted thioureido group, a substituted or unsubstituted thioureidoalkyl group, a carboxyl group, an alkoxycarbonyl group, a formyl group, a substituted or unsubstituted carbamoyl group, a substituted or unsubstituted carbamoylalkyl group, a formylalkyl group, an alkoxycarbonylalkyl group, a carboxyalkyl group, a mercapto group, an alkylthio group, a mercaptoalkyl group, a nitro group, a cyano group, a halogenoalkyl group, a trihalogenomethyl group or a halogen atom, D represents a methine moiety, a nitrogen atom or =N(→O)-, A represents a methylene moiety, a carbonyl group or a sulfonyl group, P is a group represented by -CR⁶(R⁷)- or -NR⁸- and each of R⁶, R⁷ and R⁸ represents a hydrogen atom, a straight or branched-chain alkyl group, a cyclic alkyl group, an alkenyl group, a substituted or unsubstituted aralkyl group, a substituted or unsubstituted phenyl group, a substituted or unsubstituted naphthyl group, a substituted or unsubstituted heterocyclic moiety, an acyl group, a hydroxyalkyl group, a substituted or unsubstituted aminoalkyl group, a cyano group, a substituted or unsubstituted carbamoylalkyl group, a carboxyalkyl group or an alkoxycarbonylalkyl group, is allowed to react with a compound represented by the following general formula (6) 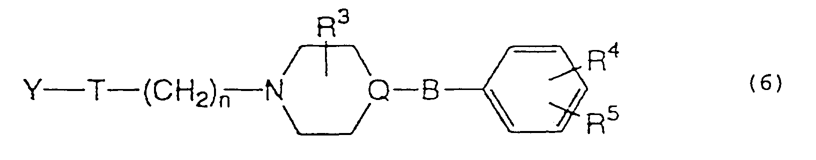 wherein R³ represents a hydrogen atom, a hydroxyl group, a straight or branched-chain alkyl group or an alkoxy group, R⁴ and R⁵ may be the same or different from each other and each represents a hydrogen atom, a hydroxyl group, a straight or branched-chain alkyl group, an alkenyl group, a halogen atom, an alkoxy group, a mercapto group, an alkylthio group, a substituted or unsubstituted amino group or a trihalogenomethyl group, Y represents a halogen atom, a p-toluenesulfonyloxy group, a methanesulfonyloxy group or a trifluoromethanesulfonyloxy group, Q represents a methine group or a substituted or unsubstituted nitrogen atom, each of T and B is a single bond or represents a group selected from the member consisting of methylene, carbonyl, -CH(OH)- and - C(=NH)- and n is an integer of 1 to 6.

7. The process for producing a substituted cyclic amine compound (1) or a pharmaceutically acceptable salt thereof according to claim 4, wherein a compound represented by the following general formula (7) 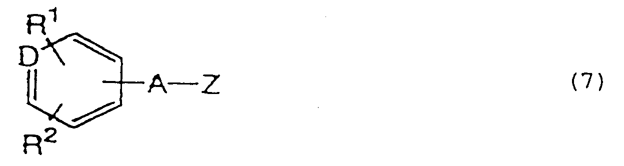 wherein R¹ and R² may be the same or different from each other and each represents a hydrogen atom, a hydroxyl group, a straight or branched-chain alkyl group, an alkenyl group, a substituted or unsubstituted aralkyl group, an alkoxy group, a hydroxyalkyl group, an acyl group, a substituted or unsubstituted amino group, a substituted or unsubstituted aminoalkyl group, a substituted or unsubstituted imino group, a substituted or unsubstituted iminoalkyl group, a substituted or unsubstituted guanidino group, a substituted or unsubstituted guanidinoalkyl group, a substituted or unsubstituted amidino group, a substituted or unsubstituted amidinoalkyl group, a substituted or unsubstituted ureido group, a substituted or unsubstituted ureidoalkyl group, a substituted or unsubstituted thioureido group, a substituted or unsubstituted thioureidoalkyl group, a carboxyl group, an alkoxycarbonyl group, a formyl group, a substituted or unsubstituted carbamoyl group, a substituted or unsubstituted carbamoylalkyl group, a formylalkyl group, an alkoxycarbonylalkyl group, a carboxyalkyl group, a mercapto group, an alkylthio group, a mercaptoalkyl group, a nitro group, a cyano group, a halogenoalkyl group, a trihalogenomethyl group or a halogen atom, D represents a methine moiety, a nitrogen atom or =N(→O)-, A represents a methylene moiety, a carbonyl group or a sulfonyl group and Z represents a halogen atom, a p-toluenesulfonyloxy group, a methanesulfonyloxy group or a trifluoromethanesulfonyloxy group, is allowed to react with a compound represented by the following general formula (8) 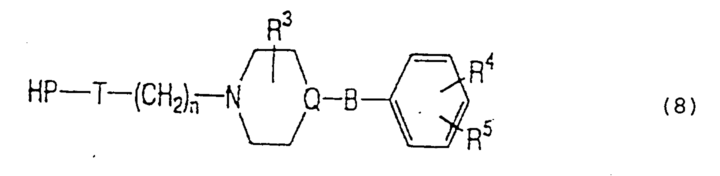 wherein R³ represents a hydrogen atom, a hydroxyl group, a straight or branched-chain alkyl group or an alkoxy group, R⁴ and R⁵ may be the same or different from each other and each represents a hydrogen atom, a hydroxyl group, a straight or branched-chain alkyl group, an alkenyl group, a halogen atom, an alkoxy group, a mercapto group, an alkylthio group, a substituted or unsubstituted amino group or a trihalogenomethyl group, P is a group represented by -CR⁶(R⁷)-or -NR⁸-, each of R⁶, R⁷ and R⁸ represents a hydrogen atom, a straight or branched-chain alkyl group, a cyclic alkyl group, an alkenyl group, a substituted or unsubstituted aralkyl group, a substituted or unsubstituted phenyl group, a substituted or unsubstituted naphthyl group, a substituted or unsubstituted heterocyclic moiety, an acyl group, a hydroxyalkyl group, a substituted or unsubstituted aminoalkyl group, a cyano group, a substituted or unsubstituted carbamoylalkyl group, a carboxyalkyl group or an alkoxycarbonylalkyl group, Q represents a methine group or a substituted or unsubstituted nitrogen atom, each of T and B is a single bond or represents a group selected from the member consisting of methylene, carbonyl, -CH(OH)- and -C(=NH)- and n is an integer of 1 to 6.

8. A process for producing a substituted cyclic amine compound represented by the following general formula (1a) 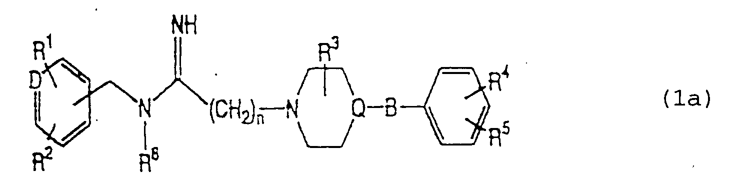 wherein R¹, R², R³, R⁴, R⁵, R⁸, D, Q, B and n are as defined above, or a pharmaceutically acceptable salt thereof, which comprises reacting a compound represented by the following general formula (5a) 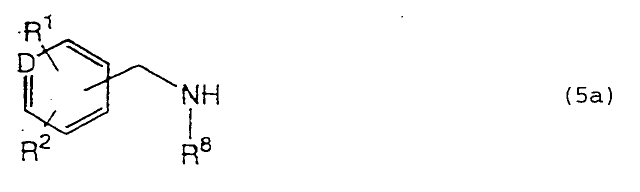 wherein R¹ and R² may be the same or different from each other and each represents a hydrogen atom, a hydroxyl group, a straight or branched-chain alkyl group, an alkenyl group, a substituted or unsubstituted aralkyl group, an alkoxy group, a hydroxyalkyl group, an acyl group, a substituted or unsubstituted amino group, a substituted or unsubstituted aminoalkyl group, a substituted or unsubstituted imino group, a substituted or unsubstituted iminoalkyl group, a substituted or unsubstituted guanidino group, a substituted or unsubstituted guanidinoalkyl group, a substituted or unsubstituted amidino group, a substituted or unsubstituted amidinoalkyl group, a substituted or unsubstituted ureido group, a substituted or unsubstituted ureidoalkyl group, a substituted or unsubstituted thioureido group, a substituted or unsubstituted thioureidoalkyl group, a carboxyl group, an alkoxycarbonyl group, a formyl group, a substituted or unsubstituted carbamoyl group, a substituted or unsubstituted carbamoylalkyl group, a formylalkyl group, an alkoxycarbonylalkyl group, a carboxyalkyl group, a mercapto group, an alkylthio group, a mercaptoalkyl group, a nitro group, a cyano group, a halogenoalkyl group, a trihalogenomethyl group or a halogen atom, R⁸ represents a hydrogen atom, a straight or branched-chain alkyl group, a cyclic alkyl group, an alkenyl group, a substituted or unsubstituted aralkyl group, a substituted phenyl group, a substituted or unsubstituted heterocyclic moiety, an acyl group, a hydroxyalkyl group, a substituted or unsubstituted aminoalkyl group, a cyano group, a substituted or unsubstituted carbamoylalkyl group, a carboxyalkyl group or an alkoxycarbonylalkyl group and D represents a methine moiety, a nitrogen atom or =N(→O)-, with a compound represented by the following general formula (9) 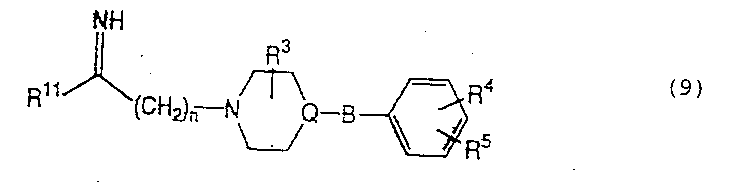 wherein R³ represents a hydrogen atom, a hydroxyl group, a straight or branched-chain alkyl group or an alkoxy group, R⁴ and R⁵ may be the same or different from each other and each represents a hydrogen atom, a hydroxyl group, a straight or branched-chain alkyl group, an alkenyl group, a halogen atom, an alkoxy group, a mercapto group, an alkylthio group, a substituted or unsubstituted amino group or a trihalogenomethyl group, R¹¹ represents an alkoxy group, an alkylthio group or a halogen atom, B is a single bond or represents a group selected from the member consisting of methylene, carbonyl, -CH(OH)- and -C(=NH)-, Q represents a methine group or a substituted or unsubstituted nitrogen atom and n is an integer of 1 to 6.

9. A pharmaceutical composition for circulatory organ use comprising a substituted cyclic amine compound of the formula (I) or a pharmaceutically acceptable salt as disclosed in claim 1, together with a pharmaceutically acceptable carrier.
